# EUROPEAN PATENT APPLICATION

(11) **EP 4 803 545 A2**
(43) Date of publication of application: **09.09.2026**
(21) Application number: 26193877.3
(22) Date of filing: 16.02.2022
(51) Int. Cl.: C07K 16/32

(54) **LINKER-PAYLOADS AND CONJUGATES THEREOF**

(30) Priority: 16.02.2021 FI 20217033; 01.04.2021 FI 20217058
(62) Document number(s) of the earlier application(s) in accordance with Art. 76 EPC: 22705851.8 / 4 294 453
(71) Applicant: Glykos Finland Oy, 00790 Helsinki (FI)
(72) Inventor: SAARINEN, Juhani, 00370 Helsinki (FI); SATOMAA, Tero, 00700 Helsinki (FI); PYNNÖNEN, Henna, 01700 Vantaa (FI); AITIO, Olli, 00330 Helsinki (FI)
(74) Representative: Papula Oy

(57) **Abstract**

Linker-payloads and their conjugates are disclosed.

## Description

### TECHNICAL FIELD

The disclosure relates to a linker, a linker-payload conjugate, a targeting unit-linker-payload conjugate, methods for preparing the same, a pharmaceutical composition and a method of treating and/or modulating the growth of and/or prophylaxis of tumor cells.

### SUMMARY

A linker-payload conjugate of Formula I, Formula IG, Formula IGX or Formula III 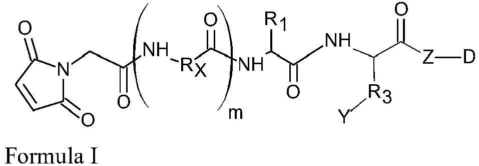 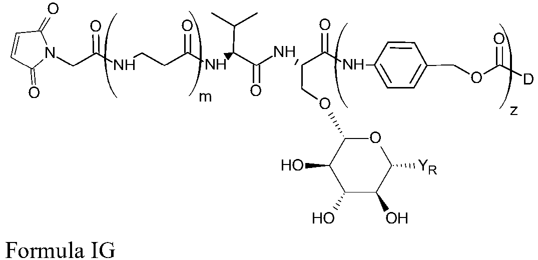 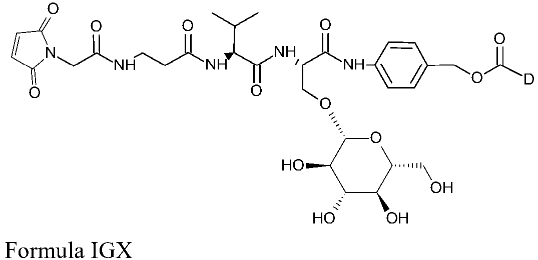 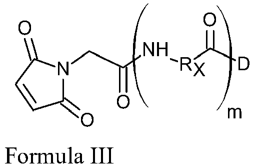 is disclosed; wherein R₁ is an amino acid side chain; R_{X} is a linear C₁-C₆ alkylene group, a branched C₁-C₆ alkylene group, -CH₂CH₂-, or -CH(R₂)-, wherein R₂ is an amino acid side chain; Y is absent or a hydrophilic group; R₃ is an amino acid side chain; Z is either absent or a self-immolative group; D is a payload molecule; and m is either 0 or 1.

A targeting unit-linker-payload conjugate of Formula II, Formula IIs, Formula IIG, Formula IIGs, Formula IIGX, Formula IIGXs, Formula IV or Formula IVs 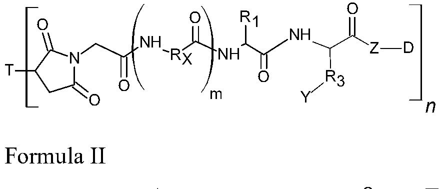 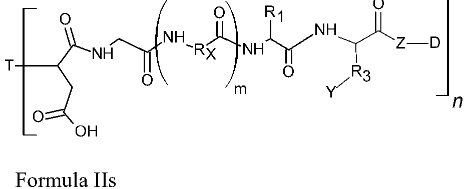 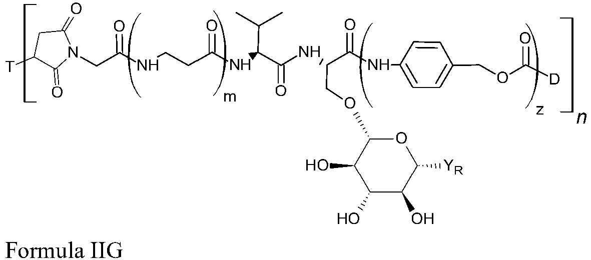 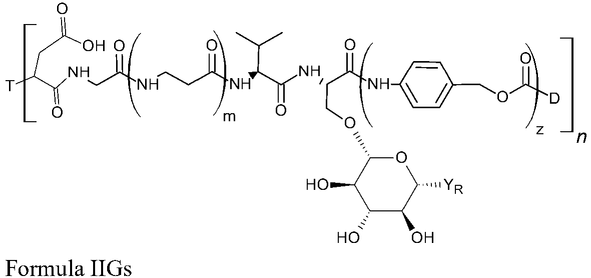 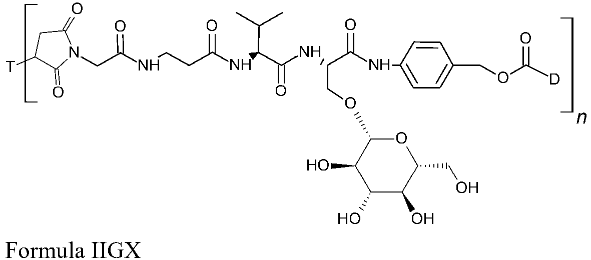 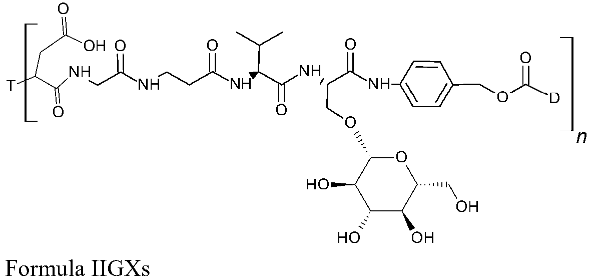 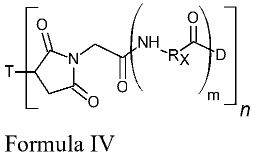 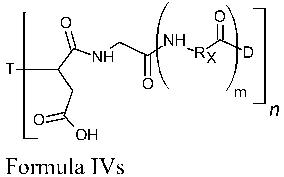 is disclosed; wherein T is a targeting unit; R₁ is an amino acid side chain; R_{X} is a linear C₁-C₆ alkylene group, a branched C₁-C₆ alkylene group, -CH₂CH₂-, or -CH(R₂)-, wherein R₂ is an amino acid side chain; Y is absent or a hydrophilic group; R₃ is an amino acid side chain; Z is either absent or a self-immolative group; D is a payload molecule; m is either 0 or 1; and n ≥ 1, or n is in the range of 1 to about 20, or 1 to about 15, or 1 to about 10, or 2 to 10, or 2 to 6, or 2 to 5, or 2 to 4; or n is 1, 2, 3, 4, 5, 6, 7, 8, 9, 10, 11, 12, 13, 14, 15, 16, 17, 18, 19, or 20.

### FIGURE LEGENDS

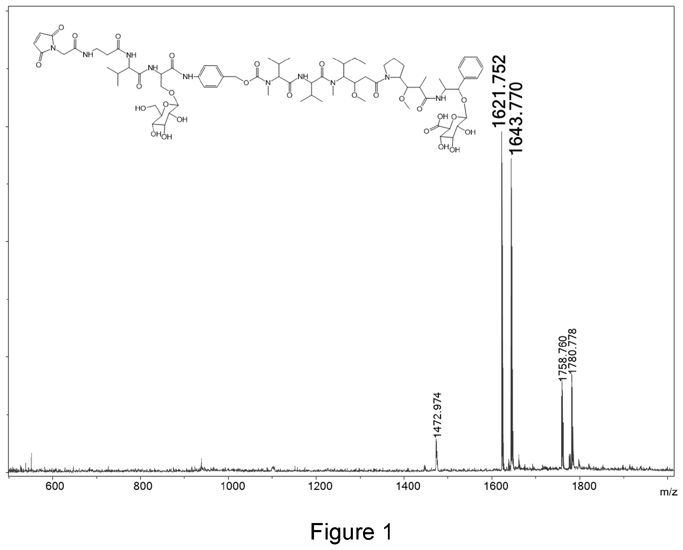

**Figure 1** shows matrix-assisted laser desorption-ionization time-of-flight (MALDI-TOF) mass spectrometric analysis of maleimidoacetyl-β-Ala-Val-Ser(Glc)-PAB-MMAU. Observed signals at m/z 1621.752 for the [M+Na]⁺ ion and at m/z 1643.770 for the [M-H+2Na]⁺ ion demonstrate successful preparation of the linker-drug conjugate.

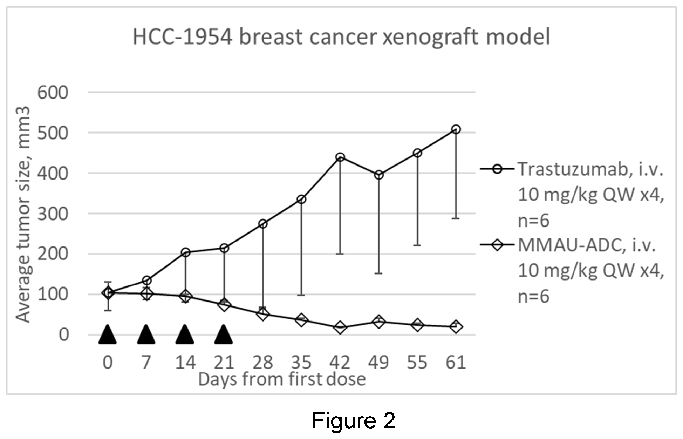

**Figure 2** shows in vivo efficacy of maleimide-stabilized MMAU-ADC in tumorxenografted mice. Four intravenous 10 mg/kg doses at seven day intervals (QWx4) of either trastuzumab or trastuzumab ADC with maleimidoacetyl-β-Ala-Val-Ser(Glc)-PAB-MMAU payloads (MMAU-ADC, DAR=8, maleimide-stabilized) were given to six mice/group (n=6) after HCC-1954 tumors had grown to average volume of 100 mm³ (day 0). MMAU-ADC effectively shrinked tumors in all mice without regrowth during the 61 days' treatment and follow-up period, while tumors grew steadily to >500 mm³ average volume in the trastuzumab-treated mice. Black triangles show the times of the i.v. dosings. Error bars show the standard deviation (SD).

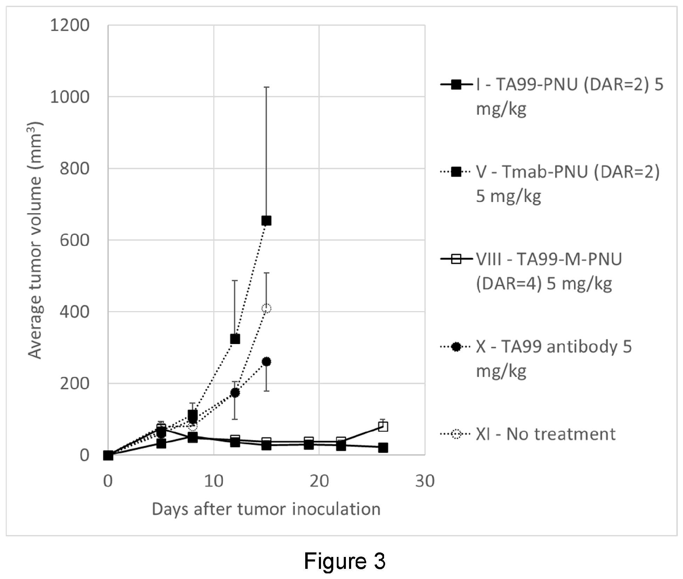

**Figure 3** shows in vivo efficacy of maleimide-stabilized PNU-ADC in syngeneic tumor-engrafted mice. A single intravenous 5 mg/kg dose of either mouse monoclonal TA99 IgG2a antibody, glycoconjugated TA99 ADC with DBCO-Val-Ser(GlcA)-EDA-PNU payloads (TA99-PNU ADC, DAR=2) or TA99 ADC with maleimidoacetyl-EDA-PNU payloads (TA99-M-PNU ADC, DAR=4) were given to six mice/group (n=6) after B16-F10 mouse melanoma tumors had established (day 2). Both ADCs effectively shrinked tumors in all mice and they survived to the end of the experiment, while tumors grew very rapidly in both antibody-treated mice and non-treated mice and most of them had to be sacrificed before the end of the experiment (day 29). Black triangles show the time of the i.v. dosing. Error bars show the standard error of the mean (SEM). Tracking of the average tumor volume ends when first mice in the group dies due to tumor growth.

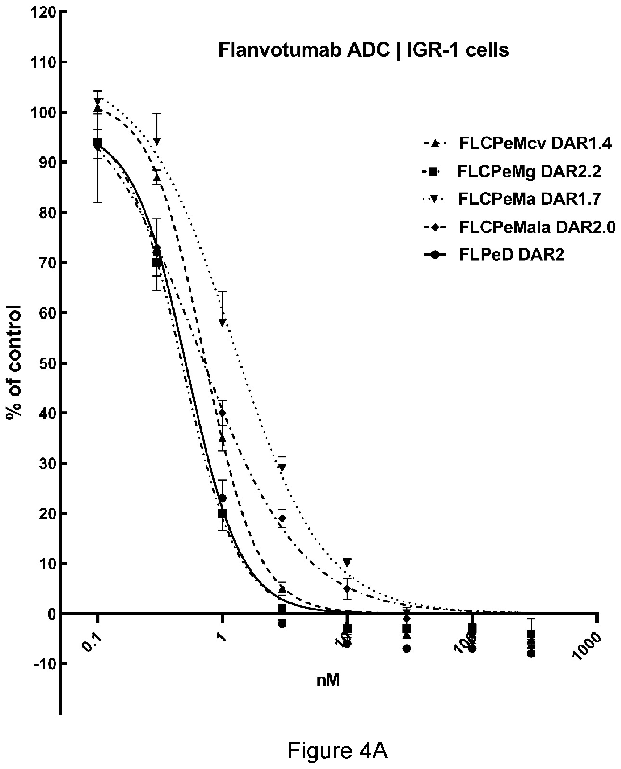

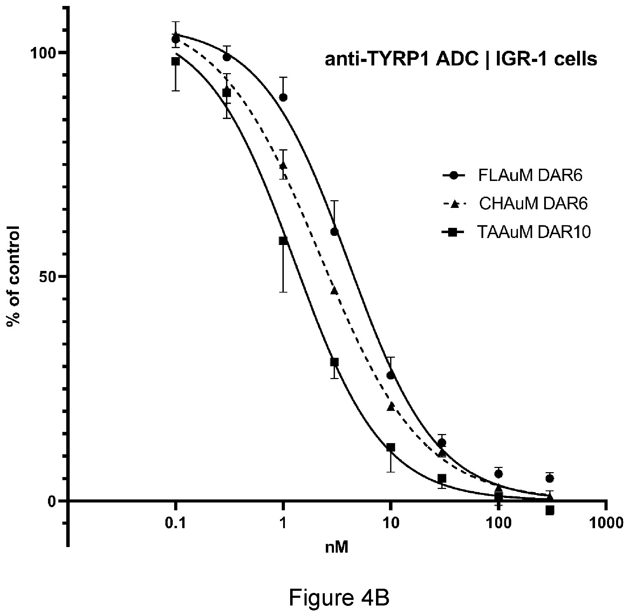

**Figure 4** shows cytotocity of flanvotumab PNU ADCs FLCPeMcv, FLCPeMg, FLCPeMa, FLCPeMala and FLPeD (Figure 4A) and anti-TYRP1 MMAU ADCs FLAuM, CHAuM and TAAuM (Figure 4B) in IGR-1 cells.

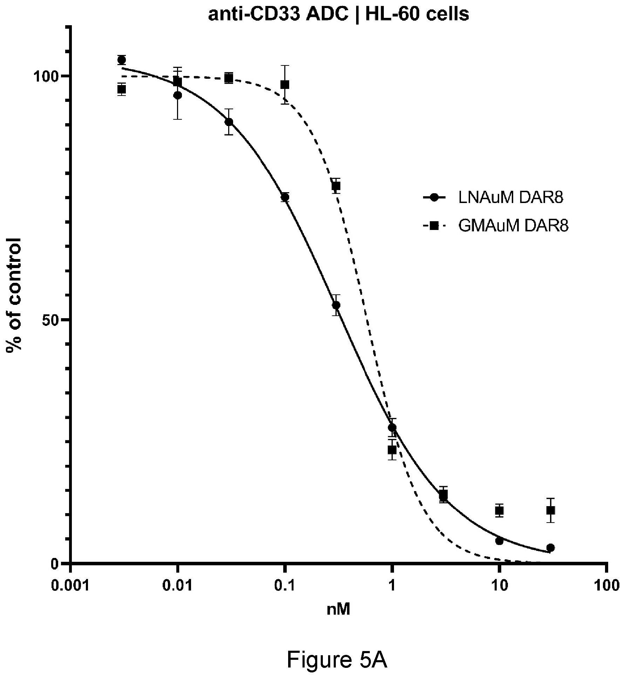

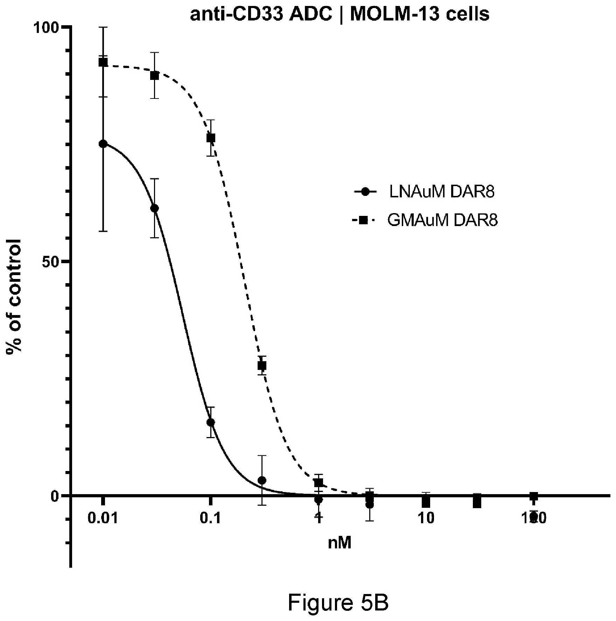

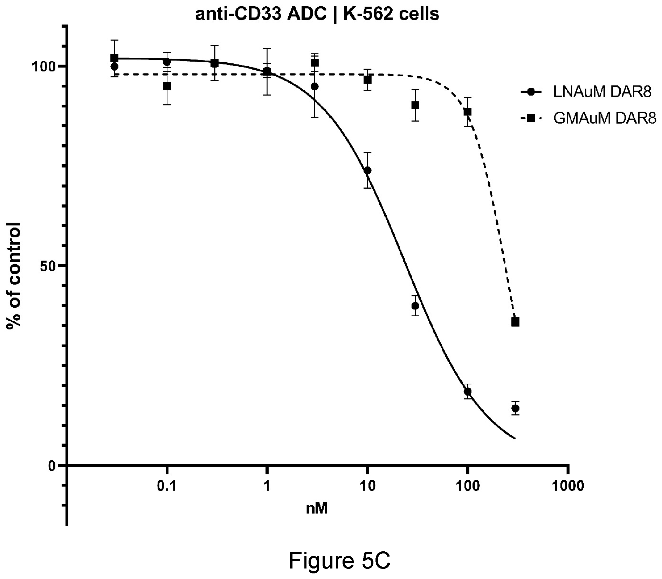

**Figure 5** shows cytotocity of lintuzumab MMAU (LNAuM) and gemtuzumab MMAU (GMAuM) ADCs in (Figure 5A) HL-60 cells, (Figure 5B) MOLM-13 and (Figure 5C) K-562 cells.

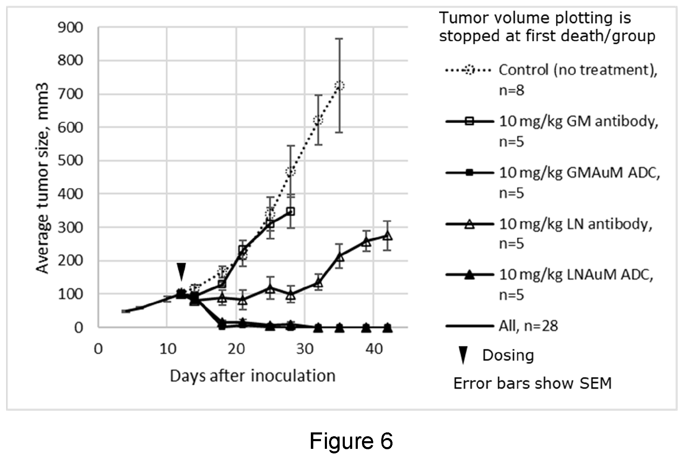

**Figure 6** shows in vivo efficacy of anti-CD33 ADCs against HL-60 leukemia cell xenografted mice. After subcutaneous tumors reached average size of 100 mm³, a single intravenous 10 mg/kg dose of unconjugated antibodies gemtuzumab (GM) or lintuzumab (LN), or MMAU ADCs GMAuM or LNAuM (both with AuM linker-payload, DAR=8) were given to five mice/group (n=5, day 12 after inoculation). The control group received no treatment (n=8). In both ADC treatment groups the tumors disappeared in all mice, showing effective anti-cancer activity. LN antibody treatment inhibited tumor growth and the mice in the group survived to the end of the study. Tumors grew rapidly in both the control group and the GM treatment group, leading to deaths of mice due to tumor growth before the end of the experiment at 30 days after the treatment. Black triangle shows the time of the i.v. dosing. Error bars show the standard error of the mean (SEM). Tracking of the average tumor volume ends when first mice in the group dies due to tumor growth.

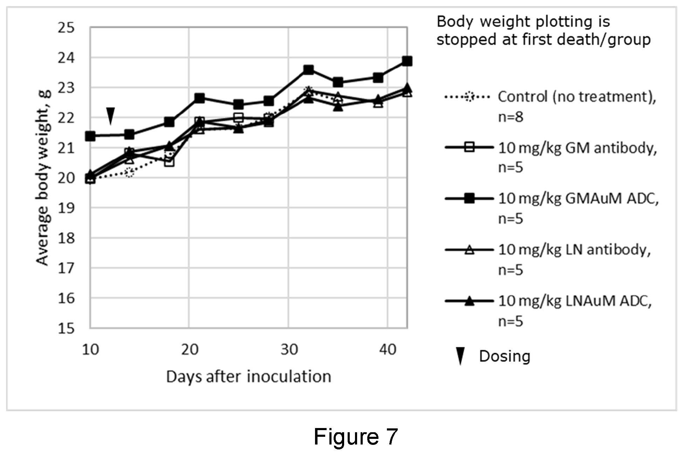

**Figure 7** shows average mouse body weight in the treatment groups of the same experiment as shown in Figure 6. There was no change in body weight increase during the study in any group. Black triangle shows the time of the i.v. dosing. Tracking of the average body weight ends when first mice in the group dies due to tumor growth.

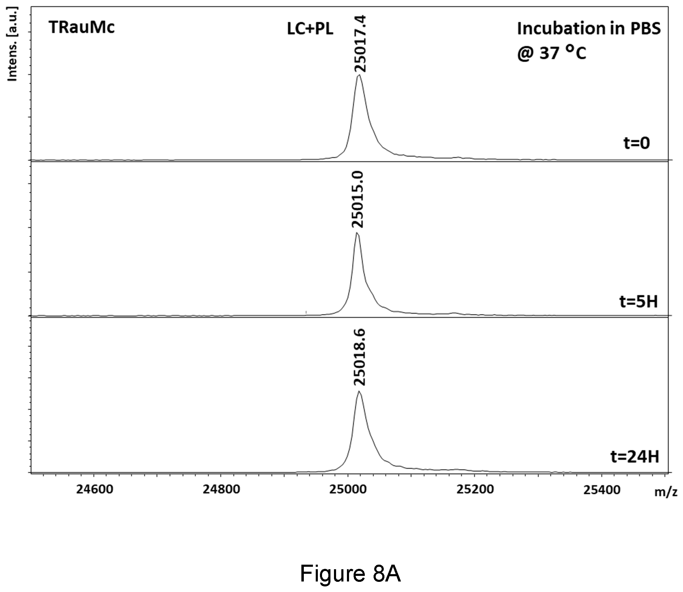

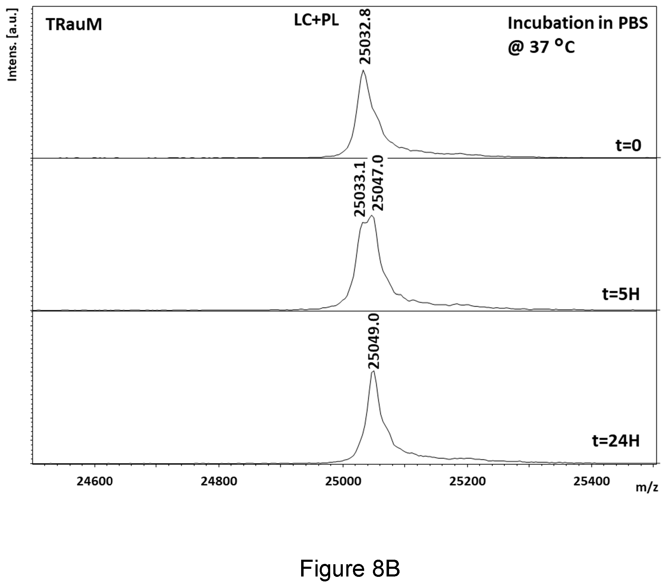

**Figure 8** shows stabilization of maleimide conjugate by hydrolysis for between TRAuMc with maleimidocaproyl linker (Figure 8A) and TRAuM with maleimidoacetyl-β-alanyl linker (Figure 8B). Both ADCs were incubated in PBS at 37°C for 24 h and the stabilization reaction was followed at time points of 0 h, 5 h and 24 h by MALDI-TOF MS of the ADC. The maleimide of TRAuM ADC was effectively stabilized whereas TRAuMc did not stabilize during the 24 h incubation.

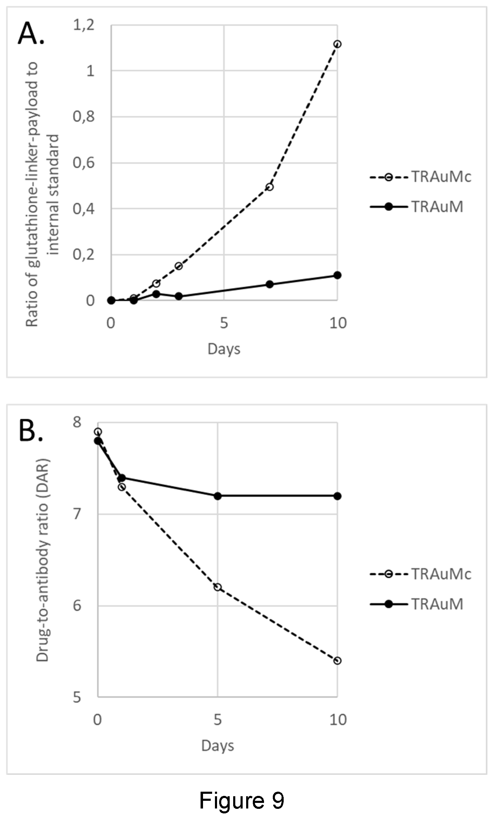

**Figure 9** shows differential de-conjugation rates of maleimide conjugate ADCs TRAuMc and TRAuM in incubation with oxidized glutathione (Figure 9A) and human serum albumin (Figure 9B). The ADCs were incubated in parallel at physiological pH at 37°C and the de-conjugation reaction was followed at various time points by MALDI-TOF MS (A), wherein the ratio of glutathione-linker-payload to internal standard is directly relative to the de-conjugation rate, and RP-HPLC (B), wherein the lowering of drug-to-antibody ratio (DAR) indicates higher de-conjugation rate.

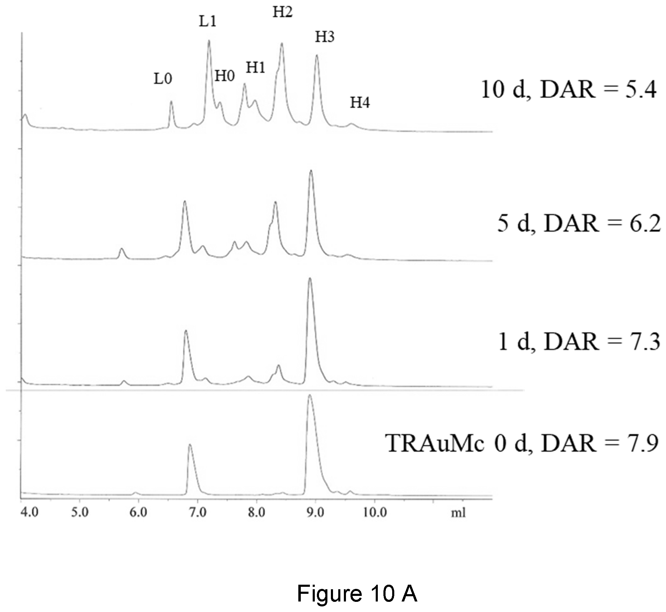

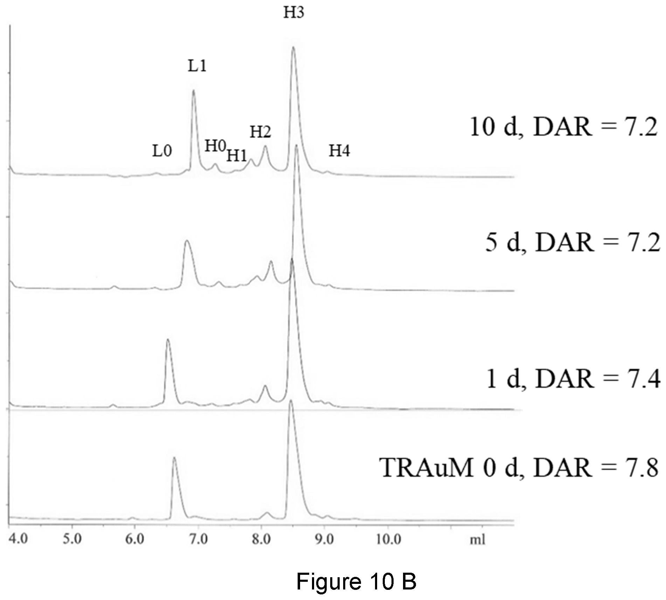

**Figure 10** shows DAR analyses by RP-HPLC of maleimide conjugate ADCs TRAuMc (Figure 10A) and TRAuM (Figure 10B) during incubation with human serum albumin at physiological pH at 37°C. De-conjugation of the linker payloads was higher for TRAuMc, resulting in lower DAR at the end of the experiment, whereas TRAuM had only small reduction of DAR. Individual components of the reduced ADC are indicated: L0, light chain without linker-payload; L1, light chain with 1 linker-payload; H0, heavy chain without linker-payload; H1, H2, H3 and H4, heavy chain with 1-4 linker-payloads, respectively. x-axis shows elution volume and y-axis shows absorbance at 280 nm.

### DETAILED DESCRIPTION

Linkers that are conjugated to a payload molecule are disclosed. In an embodiment, the linkers comprise a stabilizing group.

The presence of a stabilizing group in the linker may provide several benefits, such as higher *in vivo* stability, and therefore, both i) improved efficacy towards the target and ii) improved safety to non-target cells and tissues.

In an embodiment, the linkers comprise a non-cleavable group.

In an embodiment, the linkers comprise a cleavable group.

In an embodiment, the linkers comprise a cleavable group and a stabilizing group.

The presence of a cleavable group in the linker may provide several benefits, such as i) improved efficacy towards the target and ii) improved safety to non-target cells and tissues.

In an embodiment, the linkers comprise a cleavable hydrophilic group and a stabilizing group.

The presence of a cleavable hydrophilic group in the linker may provide several benefits, such as i) higher water solubility of the final product, ii) higher resistance towards aggregation in aqueous solutions, iii) ability to link a higher number of payload molecules per molecule of cell binder, iv) higher *in vivo* stability, and/or therefore, both v) improved efficacy towards the target and vi) improved safety to non-target cells and tissues.

In an embodiment, the presence of a cleavable hydrophilic group and a stabilizing group in the linker may prevent premature cleavage of the linker before the conjugate reaches its target, which further contributes to the benefits named above.

In an embodiment, the presence of a hydrophilic group and a stabilizing group in the linker improves the pharmacokinetics of the conjugate improving its in vivo exposure, which may further contribute to the benefits named above.

In this context, the term "linker" should be understood as referring to the moiety or portion of a molecule represented by Formulas I and III that does not comprise D, or as referring to the moiety or portion of a molecule represented by Formulas II, IIs, IV and IVs that does not comprise D and T.

The present disclosure provides a linker-payload conjugate of Formula I 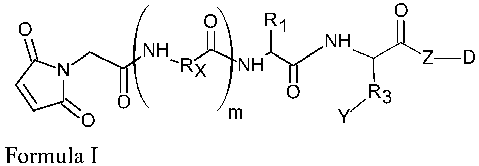 wherein R₁ is an amino acid side chain; Rx is a linear C₁-C₆ alkylene group, a branched C₁-C₆ alkylene group, -CH₂CH₂-, or CH(R₂), wherein R₂ is an amino acid side chain; Y is absent or a hydrophilic group; R₃ is an amino acid side chain; Z is either absent or a self-immolative group; D is a payload molecule; and m is either 0 or 1.

In an embodiment, m is 1 and Rx is -CH₂CH₂-.

In an embodiment, m is 0.

The present disclosure further provides a linker-payload conjugate of Formula IG 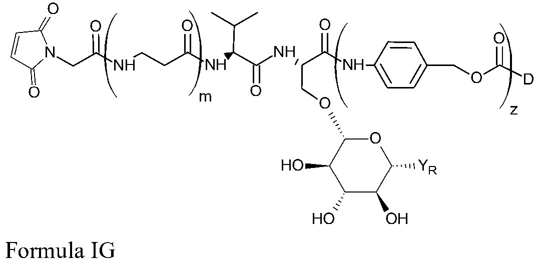 wherein Y_{R} is either CH₂OH or COOH; D is a payload molecule; m is either 0 or 1; z is either 0 or 1; both of the two α-amino acids are in L configuration; and the saccharide is in β-D-glucopyranose configuration.

In an embodiment, m is 1 and z is 1 and Y_{R} is CH₂OH.

In an embodiment, m is 1 and z is 1 and Y_{R} is COOH.

In an embodiment, m is 1 and z is 0 and Y_{R} is CH₂OH.

In an embodiment, m is 1 and z is 0 and Y_{R} is COOH.

In an embodiment, m is 0 and z is 1 and Y_{R} is CH₂OH.

In an embodiment, m is 0 and z is 1 and Y_{R} is COOH.

In an embodiment, m is 0 and z is 0 and Y_{R} is CH₂OH.

In an embodiment, m is 0 and z is 0 and Y_{R} is COOH.

In an embodiment, the linker-payload conjugate is of Formula IGX 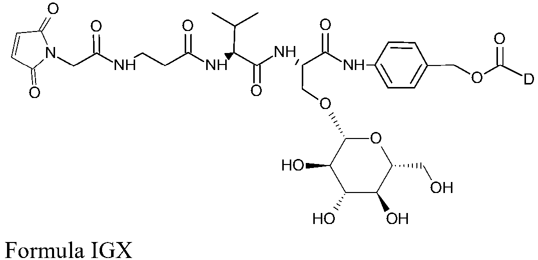 wherein D is a payload molecule.

The present disclosure further provides a targeting unit-linker-payload conjugate of Formula II or Formula IIs 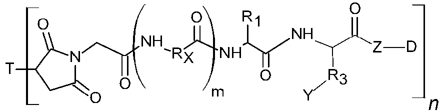 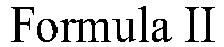 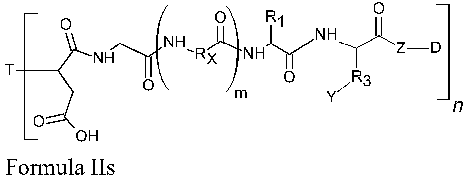 wherein T is a targeting unit; R₁ is an amino acid side chain; R_{X} is a linear C₁-C₆ alkylene group, a branched C₁-C₆ alkylene group, -CH₂CH₂-, or CH(R₂), wherein R₂ is an amino acid side chain; Y is absent or a hydrophilic group; R₃ is an amino acid side chain; Z is either absent or a self-immolative group; D is a payload molecule; m is either 0 or 1; and n 1.

The present disclosure further provides a targeting unit-linker-payload conjugate of Formula IIG or IIGs 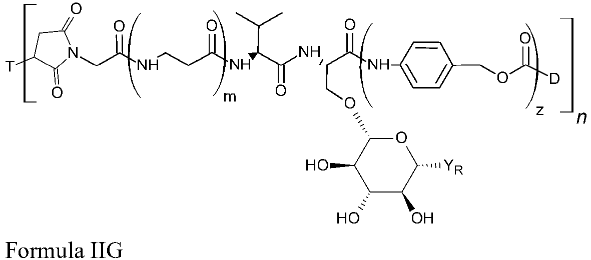 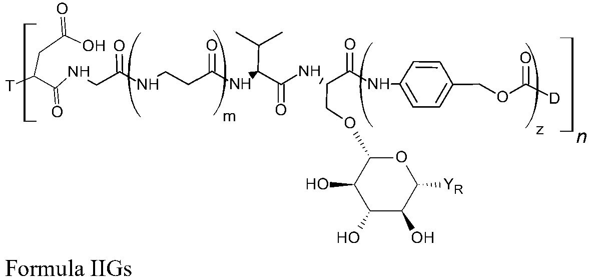 wherein T is a targeting unit; Y_{R} is either CH₂OH or COOH; D is a payload molecule; m is either 0 or 1; z is either 0 or 1; n 1; both of the two α-amino acids are in L configuration; and the saccharide is in β-D-glucopyranose configuration.

In an embodiment, m is 1 and z is 1 and Y_{R} is COOH.

In an embodiment, m is 1 and z is 0 and Y_{R} is CH₂OH.

In an embodiment, m is 1 and z is 0 and Y_{R} is COOH.

In an embodiment, m is 0 and z is 1 and Y_{R} is CH₂OH.

In an embodiment, m is 0 and z is 1 and Y_{R} is COOH.

In an embodiment, m is 0 and z is 0 and Y_{R} is CH₂OH.

In an embodiment, m is 0 and z is 0 and Y_{R} is COOH.

In an embodiment, a targeting unit-linker-payload conjugate is of Formula IIGX or
Formula IIGXs 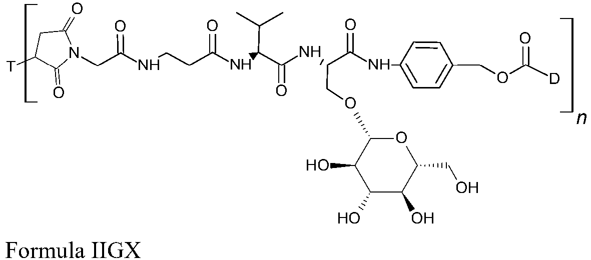 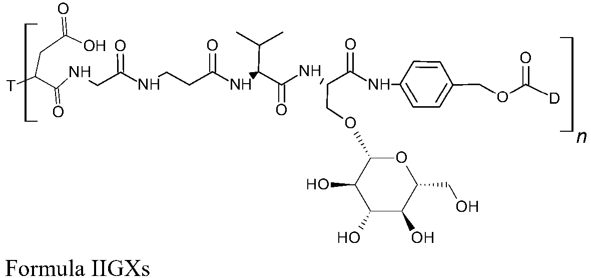 wherein T is a targeting unit; D is a payload molecule; and n 1.

The present disclosure further provides a linker-payload conjugate of Formula III 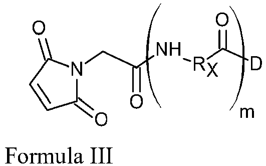 wherein R_{X} is a linear C₁-C₆ alkylene group, a branched C₁-C₆ alkylene group, - CH₂CH₂-, or CH(R₂), wherein R₂ is an amino acid side chain; D is a payload molecule; and m is either 0 or 1.

In an embodiment, m is 1 and Rx is -CH₂CH₂-.

In an embodiment, m is 0.

The present disclosure further provides a targeting unit-linker-payload conjugate of Formula IV or Formula IVs 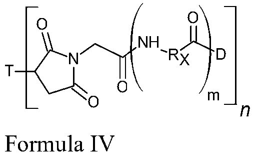 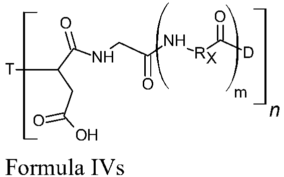 wherein T is a targeting unit; R_{X} is a linear C₁-C₆ alkylene group, a branched C₁-C₆ alkylene group, -CH₂CH₂-, or CH(R₂), wherein R₂ is an amino acid side chain; D is a payload molecule; m is either 0 or 1; and n 1.

In an embodiment, the presence of the maleimidoacetyl group bound to the rest of the molecule via an amide in the linker leads to more efficient stabilization of the maleimide according to Formula II, Formula IIG, Formula IIGX or Formula IV into a hydrolysed maleimide according to the corresponding Formula II, Formula IIG, Formula IIGX or Formula IV, respectively. In an embodiment, hydrolysed maleimide according to Formula IIs, Formula IIGs, Formula IIGXs or Formula IVs is resistant to degradation of the thioether bond to the targeting unit in the presence of free thiols (so-called reverse Michael reaction). This has the added utility as the targeting unit-linker-payload conjugate according to Formula IIs, Formula IIGs, Formula IIGXs or Formula IVs may be more stable especially in the bloodstream and in tissues, thereby increasing the *in vivo* efficacy of the conjugate.

In an embodiment, the stabilization of the maleimide to the hydrolysed maleimide occurs spontaneously, in other words the structure of the targeting unit-linker-payload conjugate according to Formula IIs, Formula IIGs, Formula IIGXs or Formula IVs is such that it promotes the hydrolysis reaction.

In an embodiment, the stabilization of the maleimide to the hydrolysed maleimide is further facilitated by an increase of pH. In an embodiment, the pH is increased to above 7, about 7.4, between 7-8, about 8, above 8, between 8-9, about 8.5, about 9, or above 9. In an embodiment, the pH is returned back after the stabilization of the maleimide.

In an embodiment, the stabilization of the maleimide to the hydrolysed maleimide is further facilitated by an increase of temperature. In an embodiment, the temperature is increased above +4°C, to about +10°C, above +10°C, between +10-20°C, about +20°C, about +22°C, between +20-30°C, about +30°C, between +30-40°C, about +37°C, about +40°C, or above +40°C or above +50°C. In an embodiment, the temperature is returned back after the stabilization of the maleimide.

In an embodiment, the stabilization of the maleimide to the hydrolysed maleimide is further facilitated by an increase of temperature and an increase of pH. In an embodiment, the stabilization of the maleimide is facilitated by an increase of temperature to between +20-40°C and an increase of pH to between 7-9. In an embodiment, the stabilization of the maleimide is facilitated by an increase of temperature to between +20-40°C and an increase of pH above 8. In an embodiment, the stabilization of the maleimide is facilitated by an increase of temperature to between +30-40°C and an increase of pH to about 8. In an embodiment, both the temperature and the pH are returned back after the stabilization of the maleimide.

In an embodiment, a targeting unit-linker-payload conjugate comprises at least 1 hydrolysed maleimide according to Formula IIs, Formula IIGs, Formula IIGXs or Formula IVs.

In an embodiment, a targeting unit-linker-payload conjugate comprises at least 2 hydrolysed maleimides according to Formula IIs, Formula IIGs, Formula IIGXs or Formula IVs.

In an embodiment, a targeting unit-linker-payload conjugate comprises at least 3 hydrolysed maleimides according to Formula IIs, Formula IIGs, Formula IIGXs or Formula IVs.

In an embodiment, a targeting unit-linker-payload conjugate comprises at least 4 hydrolysed maleimides according to Formula IIs, Formula IIGs, Formula IIGXs or Formula IVs.

In an embodiment, a targeting unit-linker-payload conjugate comprises at least 5 hydrolysed maleimides according to Formula IIs, Formula IIGs, Formula IIGXs or Formula IVs.

In an embodiment, a targeting unit-linker-payload conjugate comprises at least 6 hydrolysed maleimides according to Formula IIs, Formula IIGs, Formula IIGXs or Formula IVs.

In an embodiment, a targeting unit-linker-payload conjugate comprises at least 7 hydrolysed maleimides according to Formula IIs, Formula IIGs, Formula IIGXs or Formula IVs.

In an embodiment, a targeting unit-linker-payload conjugate comprises at least 8 hydrolysed maleimides according to Formula IIs, Formula IIGs, Formula IIGXs or Formula IVs.

In an embodiment, a targeting unit-linker-payload conjugate comprises at least 9 hydrolysed maleimides according to Formula IIs, Formula IIGs, Formula IIGXs or Formula IVs.

In an embodiment, a targeting unit-linker-payload conjugate comprises at least 10 hydrolysed maleimides according to Formula IIs, Formula IIGs, Formula IIGXs or Formula IVs.

In an embodiment, a targeting unit-linker-payload conjugate comprises 100 % of hydrolysed maleimides according to Formula IIs, Formula IIGs, Formula IIGXs or Formula IVs.

In an embodiment, a targeting unit-linker-payload conjugate comprises at least 1/n hydrolysed maleimides according to Formula IIs, Formula IIGs, Formula IIGXs or Formula IVs, wherein n is at least 1.

In an embodiment, a targeting unit-linker-payload conjugate comprises at least 2/n hydrolysed maleimides according to Formula IIs, Formula IIGs, Formula IIGXs or Formula IVs, wherein n is at least 2.

In an embodiment, a targeting unit-linker-payload conjugate comprises at least 3/n hydrolysed maleimides according to Formula IIs, Formula IIGs, Formula IIGXs or Formula IVs, wherein n is at least 3.

In an embodiment, a targeting unit-linker-payload conjugate comprises at least 4/n hydrolysed maleimides according to Formula IIs, Formula IIGs, Formula IIGXs or Formula IVs, wherein n is at least 4.

In an embodiment, a targeting unit-linker-payload conjugate comprises at least 5/n hydrolysed maleimides according to Formula IIs, Formula IIGs, Formula IIGXs or Formula IVs, wherein n is at least 5.

In an embodiment, a targeting unit-linker-payload conjugate comprises at least 6/n hydrolysed maleimides according to Formula IIs, Formula IIGs, Formula IIGXs or Formula IVs, wherein n is at least 6.

In an embodiment, a targeting unit-linker-payload conjugate comprises at least 7/n hydrolysed maleimides according to Formula IIs, Formula IIGs, Formula IIGXs or Formula IVs, wherein n is at least 7.

In an embodiment, a targeting unit-linker-payload conjugate comprises at least 8/n hydrolysed maleimides according to Formula IIs, Formula IIGs, Formula IIGXs or Formula IVs, wherein n is at least 8.

In an embodiment, a targeting unit-linker-payload conjugate comprises at least 9/n hydrolysed maleimides according to Formula IIs, Formula IIGs, Formula IIGXs or Formula IVs, wherein n is at least 9.

In an embodiment, a targeting unit-linker-payload conjugate comprises at least 10/n hydrolysed maleimides according to Formula IIs, Formula IIGs, Formula IIGXs or Formula IVs, wherein n is at least 10.

In an embodiment, n is in the range of 1 to about 20, or 1 to about 15, or 1 to about 10, or 2 to 10, or 2 to 6, or 2 to 5, or 2 to 4; or n is 1, 2, 3, 4, 5, 6, 7, 8, 9, 10, 11, 12, 13, 14, 15, 16, 17, 18, 19, or 20.

In an embodiment, n is in the range of 3 to about 20, or 3 to about 15, or 3 to about 10, or 3 to about 9, or 3 to about 8, or 3 to about 7, or 3 to about 6, or 3 to 5, or 3 to 4.

In an embodiment, n is in the range of 4 to about 20, or 4 to about 15, or 4 to about 10, or 4 to about 9, or 4 to about 8, or 4 to about 7, or 4 to about 6, or 4 to 5.

In an embodiment, n is 5.

In an embodiment, n is 6.

In an embodiment, n is 7.

In an embodiment, n is 8.

In an embodiment, n is 9.

In an embodiment, n is 10.

In an embodiment, the terms "drug-to-antibody ratio" or "DAR" mean the number of payload molecules conjugated to an antibody. In an embodiment, the term "drug-to-antibody ratio" may be shortened as "DAR". In an embodiment, the terms "drug-to-antibody ratio" and "DAR" may be used interchangeably. In an embodiment, DAR may be used to describe the number of payloads per targeting unit in a targeting unit-linker-payload conjugate.

In the Formulas of the present disclosure the variable n is generally used to show the DAR. In an embodiment, the variable n is an integer. In an embodiment, the variable n is an integer when used in the Formulas of the present disclosure.

In an embodiment, DAR means the average number of payload molecules conjugated to an antibody in a composition comprising targeting unit-linker-payload conjugates with different DAR values. In this context, DAR is equal to the ratio of the total amount of the payload in the composition to the total amount of the targeting unit in the composition. In this context, DAR is also equal to the weighed average of the targeting unit-linker-payload conjugates with different DAR values. A person skilled in the art can determine the DAR with various different analytical methods. For example, mass spectrometry (MALDI-TOF MS), RP-HPLC (PLRP-S chromatogram), and UV spectrophotometry (A280/A480 method) were used to determine DAR values in the examples of the present disclosure.

A skilled person will recognize that the linker-payload conjugate moiety linked to targeting unit as represented in Formula II is essentially the same as represented by Formula I. In the targeting unit-linker-payload conjugate, the targeting unit, T, and the payload, D, have thus reacted at the two ends of the linker. Using the linkers according to the disclosure, one or more payload molecules can be introduced to a targeting unit. Using the hydrophilic linkers according to the disclosure, a higher number of payload molecules can be introduced. Thus, using the hydrophilic linkers according to the disclosure, a higher DAR can be achieved.

In an embodiment, Y is selected from the group consisting of a saccharide, phosphate ester, sulfate ester, a phosphodiester and a phosphonate.

In an embodiment, Y is a saccharide.

The term "saccharide" should be understood as referring to single simple sugar moieties or monosaccharides or their derivatives, as well as combinations of two or more single sugar moieties or monosaccharides covalently linked to form disaccharides, oligosaccharides, and polysaccharides.

The term "monosaccharide" should be understood to include trioses, tetroses, pentoses, hexoses, heptoses, octoses or nonoses. One or several of the hydroxyl groups in the chemical structure can be replaced with other groups such as hydrogen, amino, amine, acylamido, acetylamido, halogen, mercapto, acyl, acetyl, phosphate or sulphate ester, and the like; and the saccharides can also comprise other functional groups such as carboxyl, carbonyl, hemiacetal, acetal and thio groups. A monosaccharide can selected from the group including, but not limited to, simple aldoses such as glyceraldehyde, erythrose, threose, ribose, arabinose, xylose, lyxose, allose, altrose, glucose, mannose, gulose, idose, galactose, talose and mannoheptulose; simple ketoses such as dihydroxyacetone, erythrulose, ribulose, xylulose, psicose, fructose, sorbose, tagatose and sedoheptulose; deoxysugars such as fucose, 2-deoxyglucose, 2-deoxyribose and rhamnose; sialic acids such as ketodeoxynonulosonic acid, N-acetylneuraminic acid and 9-O-acetyl-N-acetylneuraminic acid; uronic acids such as glucuronic acid, galacturonic acid and iduronic acid; amino sugars such as 2-amino-2-deoxygalactose and 2-amino-2-deoxyglucose; acylamino sugars such as 2-acetamido-2-deoxygalactose, 2-acetamido-2-deoxyglucose and N-glycolylneuraminic acid; phosphorylated and sulphated sugars such as 6-phosphomannose, 6-sulpho-N-acetylglucosamine and 3-sulphogalactose; and derivatives and modifications thereof. The monosaccharide can also be a non-reducing carbohydrate such as inositol or alditol or their derivative.

Saccharides and monosaccharides may be in D- or L-configuration; in open-chain, pyranose or furanose form; α or β anomer; and any combination thereof.

The term "oligosaccharide" should be understood as referring to saccharides composed of two or several monosaccharides linked together by glycosidic bonds having a degree of polymerization in the range of from 2 to about 20. The term "oligosaccharide" should be understood as referring to hetero- and homopolymers that can be either branched, linear or cyclical. In an embodiment, the oligosaccharide has a reducing end and a non-reducing end, whether or not the saccharide at the reducing end is in fact a reducing sugar.

The term "disaccharide" should be understood as referring to an oligosaccharide composed of two monosaccharides linked together by a glycosidic bond. Examples of disaccharides include, but are not limited to, lactose, N-acetyllactosamine, galactobiose, maltose, isomaltose and cellobiose.

The term "trisaccharide" should be understood as referring to a saccharide composed of three monosaccharides linked together by glycosidic bonds. Examples of trisaccharides include, but are not limited to, maltotriose, sialyllactose, globotriose, lacto-N-triose and gangliotriose.

In an embodiment, the saccharide is a monosaccharide, a disaccharide, a trisaccharide or an oligosaccharide.

In an embodiment, the saccharide comprises β-D-galactose, N-acetyl-β-D-galactosamine, N-acetyl-α-D-galactosamine, N-acetyl-β-D-glucosamine, β-D-glucuronic acid, α-L-iduronic acid, α-D-galactose, α-D-glucose, β-D-glucose, α-D-mannose, β-D-mannose, α-L-fucose, β-D-xylose, neuraminic acid or any analogue or modification thereof.

In an embodiment, the saccharide consists of β-D-galactose, N-acetyl-β-D-galactosamine, N-acetyl-α-D-galactosamine, N-acetyl-β-D-glucosamine, β-D-glucuronic acid, α-L-iduronic acid, α-D-galactose, α-D-glucose, β-D-glucose, α-D-mannose, β-D-mannose, α-L-fucose, β-D-xylose, neuraminic acid or any analogue or modification thereof.

In an embodiment, the saccharide consists of β-D-glucose, N-acetyl-β-D-glucosamine, β-D-glucuronic acid or α-L-fucose.

In an embodiment, the saccharide comprises β-D-glucose.

In an embodiment, the saccharide consists of β-D-glucose.

In an embodiment, the saccharide comprises β-D-glucuronic acid.

In an embodiment, the saccharide consists of β-D-glucuronic acid.

In an embodiment, the modification is sulfate, phosphate, carboxyl, amino, or O-acetyl modification of the monosaccharide.

The term "analogue" or "being analogous to" should be understood so that the analogue or the analogous monosaccharide is cleavable by the same enzyme than the monosaccharide to which it is analogous.

The term "modification" or "modification of a monosaccharide" should be understood so that the modification is a covalent modification of a monosaccharide resulting from substitution of a functional group or an atom of the monosaccharide.

In an embodiment, the modification is selected from the group of sulfate, phosphate, carboxyl, amino, and O-acetyl modification.

In an embodiment, Y is cleavable by an enzyme.

In an embodiment, Y is cleavable by an enzyme, for example, an intracellular enzyme, a lysosomal enzyme or a cytoplasmic enzyme.

In an embodiment, the cleavable hydrophilic group Y is a saccharide and cleavable by an enzyme.

In an embodiment, the saccharide is β-D-glucose, N-acetyl-β-D-glucosamine, β-D-glucuronic acid or α-L-fucose.

In an embodiment, the saccharide is β-D-glucose.

In an embodiment, the saccharide is β-D-glucuronic acid.

In an embodiment, the enzyme is an intracellular enzyme, a lysosomal enzyme or a cytoplasmic enzyme.

In an embodiment, the intracellular enzyme is a glucosidase, a hexosaminidase, an N-acetylglucosaminidase, a glucuronidase or a fucosidase.

In an embodiment, the lysosomal enzyme is a glucosidase, a hexosaminidase, an N-acetylglucosaminidase, a glucuronidase or a fucosidase.

In an embodiment, the lysosomal enzyme is β-glucosidase.

In an embodiment, the lysosomal enzyme is a glucuronidase.

In an embodiment, the cytoplasmic enzyme is a glucosidase, a hexosaminidase, an N-acetylglucosaminidase, a glucuronidase or a fucosidase.

In an embodiment, the saccharide such as β-D-glucose or β-D-glucuronic acid is cleavable by a lysosomal or an intracellular enzyme. This embodiment has the utility that lysosomal or intracellular enzymes may remove the saccharide inside a cell. A skilled person is capable of selecting a saccharide that is cleavable by a lysosomal or an intracellular enzyme based on biochemical literature; various such enzymes having different specificities are known.

In an embodiment, the lysosomal or intracellular enzyme is capable of removing the entire saccharide inside a cell.

In an embodiment, one or more of the glycosidic bonds of the saccharide are essentially stable in neutral pH and/or in serum.

In an embodiment, all glycosidic bonds of the saccharide are essentially stable in neutral pH and/or in serum.

In an embodiment, one or more of the glycosidic bonds of the saccharide are cleavable in tumor microenvironment outside a cell. This embodiment has the added utility that the saccharide may be removed more efficiently inside a tumor than in normal tissue and the molecule may be more efficiently taken up by cancer cells than by normal cells.

In an embodiment, the saccharide protects the linker from cleavage by a peptidase before the saccharide is cleaved by a glycosidase enzyme.

In an embodiment, the saccharide is β-D-glucose that protects the linker from cleavage by a peptidase before the saccharide is cleaved by β-glucosidase.

In an embodiment, the saccharide is β-D-glucuronic acid that protects the linker from cleavage by a peptidase before the saccharide is cleaved by glucuronidase.

In an embodiment, the saccharide protects the linker from cleavage by cathepsin before the saccharide is cleaved by a glycosidase enzyme.

In an embodiment, the saccharide is β-D-glucose that protects the linker from cleavage by cathepsin before the saccharide is cleaved by β-glucosidase.

In an embodiment, the saccharide is β-D-glucuronic acid that protects the linker from cleavage by a cathepsin before the saccharide is cleaved by glucuronidase.

In an embodiment, the lysosomal or intracellular enzyme is selected from the group consisting of β-galactosidase, β-hexosaminidase, α-N-acetylgalactosaminidase, β-N-acetylglucosaminidase, β-glucuronidase, α-L-iduronidase, α-galactosidase, α-glucosidase, β-glucosidase, α-mannosidase, β-mannosidase, α-fucosidase, β-xylosidase and neuraminidase.

In an embodiment, the human glycohydrolase is selected from the group consisting of β-galactosidase, β-hexosaminidase, α-N-acetylgalactosaminidase, β-N-acetylglucosaminidase, β-glucuronidase, α-L-iduronidase, α-galactosidase, α-glucosidase, β-glucosidase, α-mannosidase, β-mannosidase, α-fucosidase, β-xylosidase and neuraminidase.

In an embodiment, Y is phosphate ester.

In an embodiment, Y is sulfate ester.

In an embodiment, Y is a phosphodiester.

In an embodiment, the phosphodiester is pyrophosphate, O-P(=O)(OH)-OP(=O)(OH)₂.

In an embodiment, the phosphodiester is a substituted pyrophosphate selected from the group of O-P(=O)(OH)-O-P(=O)(OH)OR and O-P(=O)(OH)-O-P(=O)(OH)R, wherein R is selected from the group of P(=O)(OH)R, CH₃, an alkyl group and an aryl group. In an embodiment, the alkyl group is CH₂CH₂NH₂. In an embodiment, the aryl group is benzyl.

In an embodiment, Y is a phosphonate.

In an embodiment, the phosphonate is bisphosphonate, O-P(=O)(OH)-CH₂-P(=O)(OH)₂.

In an embodiment, the phosphonate is substituted bisphosphonate selected from the group of O-P(=O)(OH)-CH₂-P(=O)(OH)OR and O-P(=O)(OH)-CH₂-P(=O)(OH)R, wherein R is selected from the group of P(=O)(OH)R, CH₃, an alkyl group and an aryl group. In an embodiment, the alkyl group is CH₂CH₂NH₂. In an embodiment, the aryl group is benzyl.

Phosphodiester and bisphosphonate groups can be prepared as described in Yates and Fiedler, ACS Chem. Biol. 2016, 11, 1066-1073, and incorporated as protected modified amino acids such as protected phospho diester-modified or protected bisphosphonate-modified serine building blocks in standard peptide synthesis chemistry to produce the linker moieties according to the present disclosure.

In an embodiment, the cleavable hydrophilic group Y inhibits an endopeptidase from liberating the payload D from the conjugate until Y is first cleaved away from the conjugate.

In an embodiment, R₃ is a side chain of an amino acid.

In an embodiment, R₃ is the side chain of an α-amino acid, serine, threonine or tyrosine.

In an embodiment, R₃ is the side chain of serine.

As used herein, "amino acid side chain" refers the monovalent hydrogen or nonhydrogen substituent bonded to the α-carbon of an α-amino acid, including α-amino acid and non-α-amino acids. Exemplary amino acid side chains include, but are not limited to, the α-carbon substituent of glycine, alanine, valine, leucine, isoleucine, methionine, tryptophan, phenylalanine, proline, serine, threonine, cysteine, tyrosine, asparagine, glutamine, aspartic acid, glutamic acid, lysine, arginine, histidine, and citrulline.

In an embodiment, the amino acid side chain is selected from the α-carbon substituents of the amino acid structures shown below, wherein the amino acid side chains are highlighted by dashed boxes below.

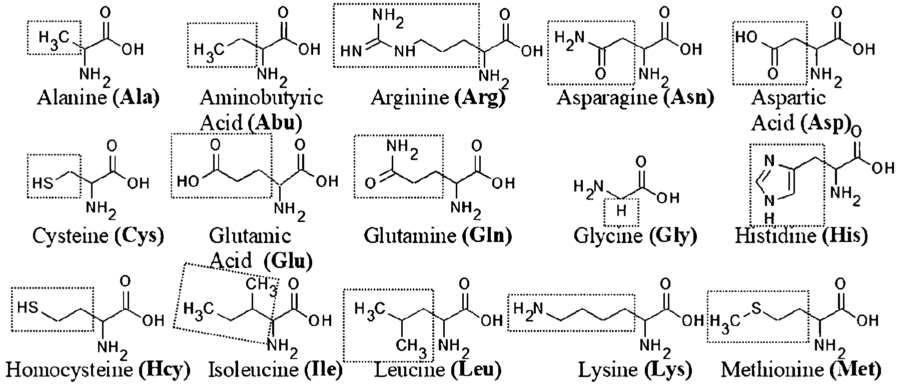

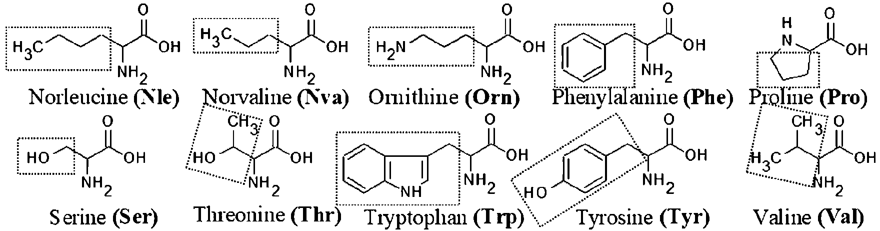

The amino acid may be in L- or D-configuration; in free amino acid or amino acid residue form; and any combination thereof.

The term "alkyl" should be understood as referring to a straight or branched chain saturated or unsaturated hydrocarbon having the indicated number of carbon atoms (e.g., "C₁-C₈ alkyl" refers to an alkyl group having from 1 to 8 carbon atoms). When the number of carbon atoms is not indicated, the alkyl group has from 1 to 8 carbon atoms. Representative "C₁-C₈ alkyl" groups include (but are not limited to) methyl (Me, CH₃), ethyl (Et, CH₂CH₃), 1-propyl (n-Pr, n-propyl, CH₂CH₂CH₃), 2-propyl (i-Pr, isopropyl, CH(CH₃)₂), 1-butyl (n-Bu, n-butyl, CH₂CH₂CH₂CH₃), 2-methyl-1-propyl (i-Bu, isobutyl, CH₂CH(CH₃)₂), 2-butyl (s-Bu, s-butyl, CH(CH₃)CH₂CH₃), 2-methyl-2-propyl (t-Bu, tert-butyl, C(CH₃)₃), 1-pentyl (n-pentyl, CH₂CH₂CH₂CH₂CH₃), 2-pentyl (CH(CH₃)CH₂CH₂CH₃), 3-pentyl (CH(CH₂CH₃)₂), 2-methyl-2-butyl (C(CH₃)₂CH₂CH₃), 3-methyl-2-butyl (CH(CH₃)CH(CH₃)₂), 3-methyl-1-butyl (CH₂CH₂CH(CH₃)₂), 2-methyl-1-butyl (CH₂CH(CH₃)CH₂CH₃), 1-hexyl (CH₂CH₂CH₂CH₂CH₂CH₃), 2-hexyl (CH(CH₃)CH₂CH₂CH₂CH₃), 3-hexyl (CH(CH₂CH₃)(CH₂CH₂CH₃)), 2-methyl-2-pentyl (C(CH₃)₂CH₂CH₂CH₃), 3-methyl-2-pentyl (CH(CH₃)CH(CH₃)CH₂CH₃), 4-methyl-2-pentyl (CH(CH₃)CH₂CH(CH₃)₂), 3-methyl-3-pentyl (C(CH₃)(CH₂CH₃)₂), 2-methyl-3-pentyl (CH(CH₂CH₃)CH(CH₃)₂), 2,3-dimethyl-2-butyl (C(CH₃)₂CH(CH₃)₂), and 3,3-dimethyl-2-butyl (CH(CH₃)C(CH₃)₃). An alkyl group can be unsubstituted or substituted with one or more groups including, but not limited to, OH, O(C₁-C₈ alkyl), aryl, COR', OCOR', CONH₂, CONHR', CONR'₂, NHCOR', SH, SO₂R', SOR', OSO₂OH, OPO(OH)₂, halogen, N₃, NH₂, NHR', NR'₂, NHCO(C₁-C₈ alkyl) or CN, wherein each R' is independently either H, C₁-C₈ alkyl or aryl. The term "alkyl" should also be understood as referring to an alkylene, a saturated, branched or straight chain or cyclic hydrocarbon radical of 1-18 carbon atoms, and having two monovalent radical centers derived by the removal of two hydrogen atoms from the same or two different carbon atoms of a parent alkane. Typical such alkylenes include (but are not limited to) methylene (CH₂) 1,2-ethyl (CH₂CH₂), 1,3-propyl (CH₂CH₂CH₂), 1,4-butyl (CH₂CH₂CH₂CH₂), and the like. The term "alkyl" should also be understood as referring to arylalkyl and heteroarylalkyl radicals as described below.

The term "arylalkyl" should be understood as referring to an acyclic alkyl radical in which one of the hydrogen atoms bonded to a carbon atom, typically a terminal or sp³ carbon atom, is replaced with an aryl radical. Typical arylalkyl groups include (but are not limited to) benzyl, 2-phenylethan-1-yl, 2-phenylethen-1-yl, naphthylmethyl, 2-naphthylethan-1-yl, 2-naphthylethen-1-yl, naphthobenzyl, 2-naphthophenylethan-1-yl, and the like. The arylalkyl group comprises 6 to 20 carbon atoms, e.g., the alkyl moiety, including alkanyl, alkenyl or alkynyl groups, of the arylalkyl group is 1 to 6 carbon atoms and the aryl moiety is 5 to 14 carbon atoms.

The term "heteroarylalkyl" should be understood as referring to an acyclic alkyl radical in which one of the hydrogen atoms bonded to a carbon atom, typically a terminal or sp³ carbon atom, is replaced with a heteroaryl radical. Typical heteroarylalkyl groups include (but are not limited to) 2-benzimidazolylmethyl, 2-furylethyl, and the like. The heteroarylalkyl group comprises 6 to 20 carbon atoms, e.g., the alkyl moiety, including alkanyl, alkenyl or alkynyl groups, of the heteroarylalkyl group is 1 to 6 carbon atoms and the heteroaryl moiety is 5 to 14 ring atoms, typically 1 to 3 heteroatoms selected from N, O, P, and S, with the remainder being carbon atoms. The heteroaryl moiety of the heteroarylalkyl group may be a monocycle having 3 to 7 ring members (2 to 6 carbon atoms) or a bicycle having 7 to 10 ring members (4 to 9 carbon atoms) and 1 to 3 heteroatoms selected from N, O, P, and S, for example: a bicyclo [4,5], [5,5], [5,6], or [6,6] system.

The term "alkynyl" should be understood as referring to a C₂-C₁₈ hydrocarbon containing normal, secondary, tertiary or cyclic carbon atoms with at least one site of unsaturation, i.e., a carbon-carbon, sp triple bond. Examples include, but are not limited to acetylenic (C≡CH) and propargyl (CH₂C≡CH). The term "alkynyl" should also be understood as referring to an alkynylene, an unsaturated, branched or straight chain or cyclic hydrocarbon radical of 2-18 carbon atoms, and having two monovalent radical centers derived by the removal of two hydrogen atoms from carbon atoms of a parent alkyne. Typical alkynylene radicals include (but are not limited to) acetylene (C=C), propargyl (CH₂C≡C), and 4-pentynyl (CH₂CH₂CH₂C≡C).

The term "alkenyl" should be understood as referring to a C₂-C₁₈ hydrocarbon containing normal, secondary, tertiary or cyclic carbon atoms with at least one site of unsaturation, i.e., a carbon-carbon, sp² double bond. Examples include, but are not limited to ethylene or vinyl (CH=CH₂), allyl (CH₂CH=CH₂), cyclopentenyl (C₅H₇), and 5-hexenyl (CH₂CH₂CH₂CH₂CH=CH₂). The term "alkenyl" should also be understood as referring to an alkenylene, an unsaturated, branched or straight chain or cyclic hydrocarbon radical of 2-18 carbon atoms, and having two monovalent radical centers derived by the removal of two hydrogen atoms from the same or two different carbon atoms of a parent alkene. Typical alkenylene radicals include, but are not limited to 1,2-ethylene (CH=CH).

In the context of this specification, the term "substituted", when used as adjective to "alkyl", "heteroalkyl", "cycloalkyl", "heterocycloalkyl", "aryl", "heteroaryl", "alkylaryl" and the like, indicates that said "alkyl", "heteroalkyl", "cycloalkyl", "heterocycloalkyl", "aryl", "alkylaryl" or "heteroaryl" group contains one or more substituents, which may include, but are not limited to, OH, =O, =S, =NRh, =N-ORh, SH, NH₂, NO₂, NO, N₃, CF₃, CN, OCN, SCN, NCO, NCS, C(O)NH₂, C(O)H, C(O)OH, halogen, Rh, SRh, S(O)Rh, S(O)ORh, S(O)₂Rh, S(O)₂ORh, OS(O)Rh, OS(O)ORh, OS(O)₂Rh, OS(O)₂ORh, OP(O)(ORh)(ORi), P(O)(ORh)(ORi), ORh, NHRi, N(Rh)Ri, +N(Rh)(Ri)Rj, Si(Rh)(Ri)(Rj), C(O)Rh, C(O)ORh, C(O)N(Ri)Rh, OC(O)Rh, OC(O)ORh, OC(O)N(Rh)Ri, N(Ri)C(O)Rh, N(Ri)C(O)ORh, N(Ri)C(O)N(Rj)Rh, and the thio derivatives of these substituents, or a protonated or deprotonated form of any of these substituents, wherein Rh, Ri, and Rj are independently selected from H and optionally substituted C₁₋₁₅ alkyl, C₁₋₁₅ heteroalkyl, C₃₋₁₅ cycloalkyl, C₃₋₁₅ heterocycloalkyl, C₄₋₁₅ aryl, or C₄₋₁₅ heteroaryl or a combination thereof, two or more of Rh, Ri, and Rj optionally being joined to form one or more carbocycles or heterocycles.

The term "alkyl" as used herein may refer to a straight chain or branched, saturated or unsaturated hydrocarbon substituent. Examples of alkyl groups include, but are not limited to, methyl, ethyl, propyl, butyl, pentyl, hexyl, octyl, decyl, isopropyl, sec-butyl, isobutyl, tert-butyl, isopentyl, 2-methylbutyl, vinyl, allyl, 1-butenyl, 2-butenyl, isobutylenyl, 1-pentenyl, and 2-pentenyl.

The term "heteroalkyl" as used herein may refer to a straight chain or branched, saturated or unsaturated hydrocarbon substituent in which at least one carbon is replaced by a heteroatom. Examples include, but are not limited to, methyloxymethyl, ethyloxymethyl, methyloxyethyl, ethyloxyethyl, methylaminomethyl, dimethylaminomethyl, methylaminoethyl, dimethylaminoethyl, methylthiomethyl, ethylthiomethyl, ethylthioethyl, and methylthioethyl.

The term "cycloalkyl" as used herein may refer to a saturated or unsaturated non-aromatic carbocycle substituent, which may consist of one ring or two or more rings fused together. Examples include, but are not limited to, cyclopropyl, cyclobutyl, cyclopentyl, cyclopentenyl, cyclopentadienyl, cyclohexyl, cyclohexenyl, 1,3-cyclohexadienyl, decalinyl, and 1,4-cyclohexadienyl.

The term "heterocycloalkyl" as used herein may refer to a saturated or unsaturated non-aromatic cyclic hydrocarbon substituent, which may consist of one ring or two or more rings fused together, wherein at least one carbon in one of the rings is replaced by a heteroatom. Examples include, but are not limited to, tetrahydrofuranyl, pyrrolidinyl, piperidinyl, 1,4-dioxanyl, decahydroquinolinyl, piperazinyl, oxazolidinyl, and morpholinyl.

The term "heterocycloalkyl" as used herein may refer to a saturated or unsaturated non-aromatic cyclic hydrocarbon substituent, which may consist of one ring or two or more rings fused together, wherein at least one carbon in one of the rings is replaced by a heteroatom. Examples include, but are not limited to, tetrahydrofuranyl, pyrrolidinyl, piperidinyl, 1,4-dioxanyl, decahydroquinolinyl, piperazinyl, oxazolidinyl, and morpholinyl.

The term "alkylaryl" as used herein may refer to an aryl attached to an alkyl, wherein the terms alkyl and aryl are as defined above. Examples include, but are not limited to, benzyl and ethylbenzene radical.

The extension "-ylene" as opposed to "-yl" in for example "alkylene" as opposed to "alkyl" indicates that said for example "alkylene" is a divalent moiety connected to one or two other moieties via two covalent single bonds or one double bond as opposed to being a monovalent group connected to one moiety via one covalent single bond in said for example "alkyl". The term "alkylene" therefore may refer to a straight chain or branched, saturated or unsaturated hydrocarbon moiety; the term "heteroalkylene" as used herein may refer to a straight chain or branched, saturated or unsaturated hydrocarbon moiety in which at least one carbon is replaced by a heteroatom; the term "arylene" as used herein may refer to a carbocyclic aromatic moiety, which may consist of one ring or two or more rings fused together; the term "heteroarylene" as used herein may refer to a carbocyclic aromatic moiety, which may consist of one ring or two or more rings fused together, wherein at least one carbon in one of the rings is replaced by a heteroatom; the term "cycloalkylene" as used herein may refer to a saturated or unsaturated non-aromatic carbocycle moiety, which may consist of one ring or two or more rings fused together; the term "heterocycloalkylene" as used herein may refer to a saturated or unsaturated non-aromatic cyclic hydrocarbon moiety, which may consist of one ring or two or more rings fused together, wherein at least one carbon in one of the rings is replaced by a heteroatom. Exemplary divalent moieties include those examples given for the monovalent groups hereinabove in which one hydrogen atom is removed.

The prefix "poly" in "polyalkylene", "polyheteroalkylene", "polyarylene", "polyheteroarylene", polycycloalkylene", "polyheterocycloalkylene", and the like, indicates that two or more of such "-ylene" moieties, e.g., alkylene moieties, are joined together to form a branched or unbranched multivalent moiety containing one or more attachment sites for adjacent moieties.

In an embodiment, the alkyl group is unsubstituted or substituted C₁-C₈ alkyl, heteroalkyl, cycloalkyl, heterocycloalkyl, arylalkyl, heteroarylalkyl, alkynyl or alkenyl.

In an embodiment, R₂ is an aryl group.

The term "aryl" as used herein may refer to a carbocyclic aromatic substituent, which may consist of one ring or two or more rings fused together. Examples of aryl groups include, but are not limited to, phenyl, naphthyl, and anthracenyl.

The term "heteroaryl" as used herein may refer to a carbocyclic aromatic substituent, which may consist of one ring or two or more rings fused together, wherein at least one carbon in one of the rings is replaced by a heteroatom. Examples of heteroaryl groups include, but are not limited to, pyridinyl, furanyl, pyrrolyl, triazolyl, pyrazolyl, imidazolyl, thiophenyl, indolyl, benzofuranyl, benzimidazolyl, indazolyl, benzotriazolyl, benzisoxazolyl, and quinolinyl.

Certain linkers of the disclosure possess chiral centers or double bonds; the enantiomeric, diastereomeric, and geometric mixtures of two or more isomers, in any composition, as well as the individual isomers are encompassed within the scope of the present disclosure.

In an embodiment, the aryl group is aryl or heteroaryl.

In an embodiment, m is 0.

In an embodiment, Y is β-D-glucose; R₃ is the side chain of an α-amino acid, serine, threonine or tyrosine; and R₁ is selected from the group of side chains of α-amino acids, valine, phenylalanine, tyrosine, leusine, isoleusine, arginine, alanine, lysine and glycine.In an embodiment, Y is β-D-glucose; R₃ is the side chain of serine; and R₁ is the side chain of valine.

In an embodiment, Y is β-D-glucuronic acid; R₃ is the side chain of an α-amino acid, serine, threonine or tyrosine; and R₁ is selected from the group of side chains of α-amino acids, valine, phenylalanine, tyrosine, leusine, isoleusine, arginine, alanine, lysine and glycine

In an embodiment, Y is β-D-glucuronic acid; R₃ is the side chain of serine; and R₁ is the side chain of valine.

In an embodiment, m is 1.

In an embodiment, R₁ is an amino acid side chain.

In an embodiment, R₁ is selected from the group of side chains of α-amino acids, valine, phenylalanine, tyrosine, leusine, isoleusine, arginine, alanine, lysine and glycine.

In an embodiment, R₁ is selected from the group of side chains of valine, phenylalanine and alanine.

In an embodiment, R₁ is the side chain of valine.

In an embodiment, R_{X} is a linear C₁-C₆ alkylene group

In an embodiment, R_{X} is a branched C₁-C₆ alkylene group

In an embodiment, R_{X} is CH(R₂), wherein R₂ is an amino acid side chain

In an embodiment, R_{X} is -CH₂CH₂-.

In an embodiment, Y is a saccharide; R₃ is a side chain of an amino acid; m = 1; and R₁ is selected from the group of side chains of α-amino acids, valine, phenylalanine, tyrosine, leusine, isoleusine, arginine, alanine, lysine and glycine.

In an embodiment, Y is a saccharide; R₃ is the side chain of serine; m = 1; R_{X} is - CH₂CH₂-; and R₁ is the side chain of valine.

In an embodiment, Y is β-D-glucose; R₃ is the side chain of serine; m = 1; R_{X} is - CH₂CH₂-; and R₁ is the side chain of valine.

In an embodiment, Y is β-D-glucuronic acid; R₃ is the side chain of serine; m = 1; Rx is -CH₂CH₂-; and R₁ is the side chain of valine. In an embodiment, Z is absent.

In an embodiment, Z comprises a self-immolative group.

In an embodiment, Z comprises a self-immolative group linking the linker of the present disclosure and the payload molecule. The term "self-immolative" refers to a functional chemical moiety that is capable of covalently linking together chemical moieties (e.g. MMAU to the linker of the present disclosure) and that will spontaneously separate from e.g. the MMAU or a cytotoxic drug if its bond to the rest of the linker is cleaved.

In an embodiment, the self-immolative group is para-aminobenzyloxycarbonyl (PABC), orto-aminobenzyloxycarbonyl, an α-amino acid and an oligopeptide. In an embodiment, the oligopeptide is di-, tri- or tetrapeptide of α-amino acids. Said group is capable of spontaneously cleaving itself from the conjugate after the linker has been cleaved by an enzyme.

In an embodiment, the self-immolative group is a para-aminobenzyloxycarbonyl (PABC) group.

In an embodiment, Y is a saccharide; R₃ is a side chain of an amino acid; m = 1; R₁ is selected from the group of side chains of α-amino acids, valine, phenylalanine, tyrosine, leusine, isoleusine, arginine, alanine, lysine and glycine; and Z is PABC.

In an embodiment, Y is a saccharide; R₃ is the side chain of serine; m = 1; R₁ is the side chain of valine; and Z is PABC.

In an embodiment, Y is β-D-glucose; R₃ is the side chain of serine; m = 1; R₁ is the side chain of valine; and Z is PABC.

In an embodiment, Y is β-D-glucose; R₃ is the side chain of serine; m = 1; R_{X} is - CH₂CH₂-; R₁ is the side chain of valine; and Z is PABC.In an embodiment, Y is β-D-glucuronic acid; R₃ is the side chain of serine; m = 1; Rx is -CH₂CH₂-; R₁ is the side chain of valine; and Z is PABC.

In an embodiment, Y is β-D-glucose; R₃ is the side chain of serine; m = 1; R_{X} is - CH₂CH₂-; R₁ is the side chain of valine; and Z is absent.In an embodiment, Y is β-D-glucuronic acid; R₃ is the side chain of serine; m = 1; Rx is -CH₂CH₂-; R₁ is the side chain of valine; and Z is absent.

In an embodiment, D is selected from the group consisting of a cytotoxic drug, an immunomodulatory agent, a labeling agent, a chelator and a radioactive agent.

In an embodiment, the immunomodulatory agent is selected from the group of corticosteroids such as cortisol, cortisone, prednisone, prednisolone, methylprednisolone, dexamethasone, betamethasone, triamcinolone, fludrocortisone and deoxycorticosterone, or an analogue thereof; and thalidomide, lenalidomide (CC-5013), CC-4047, or an analogue thereof.

In an embodiment, the labeling agent is selected from the group of fluorescent label, magnetic label and isotope label.

In an embodiment, the chelator is selected from the group of NOTA, DOTA, TRAP and analogous chelators.

In an embodiment, the radioactive agent is selected from the group of positron emitter of oxygen, nitrogen, iron, carbon, or gallium, ⁴³K, ⁵²Fe, ⁵⁷Co, ⁶⁷Cu, ⁶⁷Ga, ⁶⁸Ga, ¹²³I, ¹²⁵I, ¹³¹I, ¹³²I, or ⁹⁹Tc.

In an embodiment, D is a cytotoxic drug selected from the group consisting of a tubulin-binding agent, a tubulin-disrupting agent, an auristatin, a DNA-binding agent and a DNA-alkylating and/or crosslinking agent.

In an embodiment, D is an auristatin.

In an embodiment, the auristatin is monomethylauristatin E (MMAE) saccharide conjugate of Formula AS. 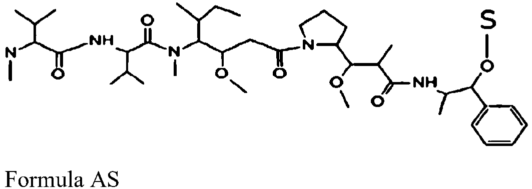 wherein S is a saccharide.

In an embodiment, the saccharide comprises or is a monosaccharide selected from the group consisting of β-D-galactose, N-acetyl-β-D-galactosamine, N-acetyl-α-D-galactosamine, N-acetyl-β-D-glucosamine, β-D-glucuronic acid, α-L-iduronic acid, α-D-galactose, α-D-glucose, β-D-glucose, α-D-mannose, β-D-mannose, α-L-fucose, β-D-xylose, neuraminic acid and any analogue or modification thereof.

In an embodiment, D is monomethylauristatin E β-D-glucuronide (MMAU).

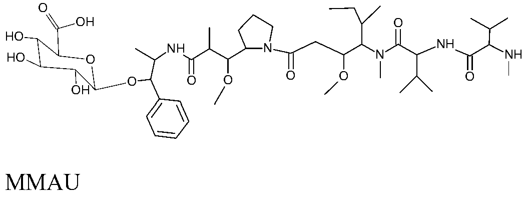

In an embodiment, D is monomethylauristatin F 3-hydroxypropanamide (MMAFP).

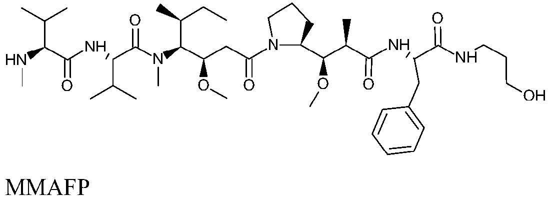

In an embodiment, D is monomethylauristatin F 3-hydroxypropanamide O-glycoside according to Formula FS. 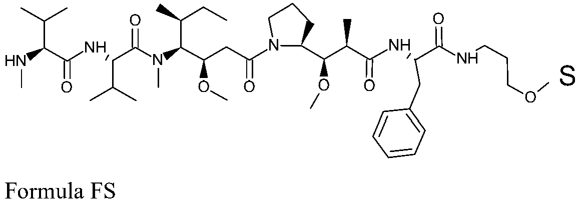 wherein S is a saccharide.

In an embodiment, the saccharide comprises or is a monosaccharide selected from the group consisting of β-D-galactose, N-acetyl-β-D-galactosamine, N-acetyl-α-D-galactosamine, N-acetyl-β-D-glucosamine, β-D-glucuronic acid, α-L-iduronic acid, α-D-galactose, α-D-glucose, β-D-glucose, α-D-mannose, β-D-mannose, α-L-fucose, β-D-xylose, neuraminic acid and any analogue or modification thereof.

In an embodiment, D is monomethylauristatin F 3-hydroxypropanamide β-D-glucuronide (MMAFU). 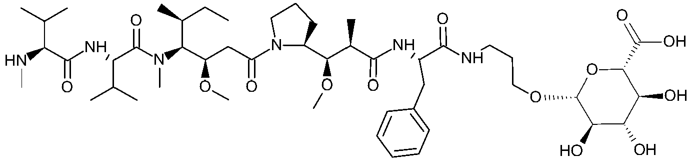 MMAFU

In an embodiment, the payload molecule D comprises an amine moiety, through which the payload molecule is bound so as to form a secondary or tertiary amine.

In the context of this specification, the term "cytotoxic drug" may refer to any cytotoxic drug or cytotoxic drug derivative. It may also refer to the cytotoxic drug moiety of the conjugate according to one or more embodiments; said cytotoxic drug moiety may be modified as described in this specification, e.g. by the addition of a linker of the present disclosure. The term "cytotoxic drug" may also refer to a cytotoxic agent.

The cytotoxic drug may be any compound that results in the death of a cell, or induces cell death, or in some manner decreases cell viability. The cytotoxic drug can be any of many small molecule drugs, including, but not limited to, dolastatins; auristatins; epothilones; daunorubicins and doxorubicins; alkylating agents, such as thiotepa and cyclophosphamide (CYTOXAN^{™}); alkyl sulfonates such as busulfan, improsulfan and piposulfan; aziridines, such as benzodopa, carboquone, meturedopa, and uredopa; ethylenimines and methylamelamines including altretamine, triethylenemelamine, trietylene-phosphoramide, triethylenethiophosphaoramide and trimethylolomelamine; acetogenins (especially bullatacin and bullatacinone); camptothecins (including the synthetic analogue topotecan); bryostatin; callystatin; CC-1065 (including its adozelesin, carzelesin and bizelesin synthetic analogues); cryptophycins (particularly cryptophycin 1 and cryptophycin 8); duocarmycin (including the synthetic analogues, KW-2189 and CBI-TMI); eleutherobin; pancratistatin; sarcodictyins; spongistatin; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics, such as the enediyne antibiotics (e.g. calicheamicins, especially calicheamicin γ1; dynemicin, including dynemicin A; esperamicin; as well as neocarzinostatin chromophore and related chromoprotein enediyne antiobiotic chromomophores), aclacinomysins, actinomycin, authramycin, azaserine, bleomycins, cactinomycin, carabicin, caminomycin, carzinophilin; chromomycins, dactinomycin, detorubicin, 6-diazo-5-oxo-L-norleucine, other doxorubicin derivatives including morpholino-doxorubicin, cyanomorpholino-doxorubicin, 2-pyrrolino-doxorubicin and deoxydoxorubicin, epirubicin, esorubicin, idarubicin, marcellomycin, nitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites, such as methotrexate and 5-fluorouracil (5-FU); folic acid analogues, such as denopterin, methotrexate, pteropterin, trimetrexate; purine analogs, such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-fluorouracil; androgens, such as calusterone, dromostanolone propionate, epitiostanol, mepitiostane, testolactone; anti-adrenals, such as aminoglutethimide, mitotane, trilostane; folic acid replenisher, such as frolinic acid; aceglatone; aldophosphamide glycoside; aminolevulinic acid; amsacrine; bestrabucil; bisantrene; edatraxate; defofamine; demecolcine; diaziquone; elfomithine; elliptinium acetate; etoglucid; gallium nitrate; hydroxyurea; lentinan; lonidamine; maytansinoids, such as maytansine and N-glucosylmaytansinoids, ansamitocins, DM-1, DM-4; mitoguazone; mitoxantrone; mopidamol; nitracrine; pentostatin; phenamet; pirarubicin; podophyllinic acid; 2-ethylhydrazide; procarbazine; PSK^{®}; razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g. paclitaxel (TAXOL^{®}, Bristol-Myers Squibb Oncology, Princeton, N.J.) and doxetaxel (TAXOTERE^{®}, Rhone-Poulenc Rorer, Antony, France); chlorambucil; gemcitabine; 6-thioguanine; mercaptopurine; methotrexate; platinum coordination complex such as cisplatin, carboplatin and vinblastine; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylomithine (DMFO); retinoic acid; capecitabine; anti-hormonal agents that act to regulate or inhibit hormone action on tumours, such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens, such as flutamide, nilutamide, bicalutamide, leuprolide, and goserelin; tubulysins; amanitins, such as α-amanitin; and pharmaceutically acceptable salts, acids; and saccharide derivatives of any of the above as disclosed in the International Patent Publication No. WO/2016/001485 the content of which is hereby incorporated in its entirety.

In an embodiment, the cytotoxic drug is a dolastatin, auristatin, doxorubicin, DM1, epirubicin, duocarmycin or any analogue or derivative thereof.

In an embodiment, the cytotoxic drug is a dolastatin, auristatin, doxorubicin, or any analogue or derivative thereof.

In an embodiment, the cytotoxic drug is dolastatin 10 or any derivative thereof.

In an embodiment, the cytotoxic drug is dolastatin 15 or any derivative thereof.

In an embodiment, the cytotoxic drug is auristatin F or any derivative thereof.

In an embodiment, the cytotoxic drug is dolastatin 10, dolastatin 15, or auristatin F.

In an embodiment, the cytotoxic drug is dolastatin 10.

In an embodiment, the cytotoxic drug is dolastatin 15.

In an embodiment, the cytotoxic drug is auristatin F.

In an embodiment, the cytotoxic drug is auristatin U (MMAU).

In an embodiment, the cytotoxic drug is auristatin FP (MMAFP).

In an embodiment, the cytotoxic drug is auristatin FU (MMAFU). Examples of suitable dolastatins include monomethyl and desmethyl dolastatins 10, 15, C, D and H, monomethyl and desmethyl isodolastatin H, and analogues and derivatives thereof. Dolastatins 10 and 15 are the most potent cytotoxic agents among the α-amino acidly occurring dolastatins. Monomethyl and desmethyl dolastatins 10 and 15 can be prepared by chemical synthesis according to standard peptide synthesis chemistry.

Examples of suitable auristatins that can be used include (but are not limited to) monomethyl and desmethyl auristatins E, F, FP, FU, EB, EFP, PY, PYE, PE, PHE, TP, 2-AQ and 6-AQ.

In an embodiment, the cytotoxic drug is daunorubicin or doxorubicin.

In an embodiment, the rubicin or the doxorubicin derivative is nemorubicin (3'-deamino-3'-[2"(S)-methoxy-4"-morpholinyl]doxorubicin; MMDX) or a modification or derivative thereof.

In an embodiment, the rubicin or the doxorubicin derivative is 3'-deamino-3",4'-anhydro-[2"(S)-methoxy-3"(R)-oxy-4"-morpholinyl]doxorubicin (PNU-159682) or a modification or derivative thereof.

In an embodiment, the rubicin or the doxorubicin derivative is PNU-EDA, PNU-EDA' or a modification or derivative thereof. 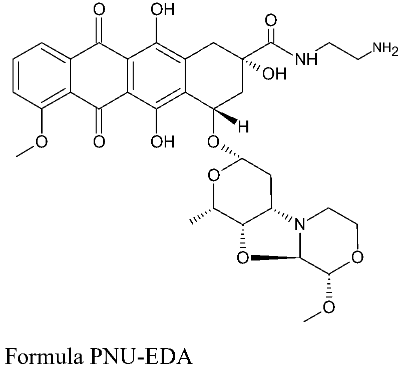 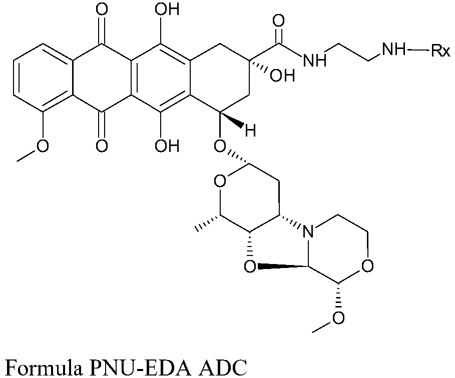 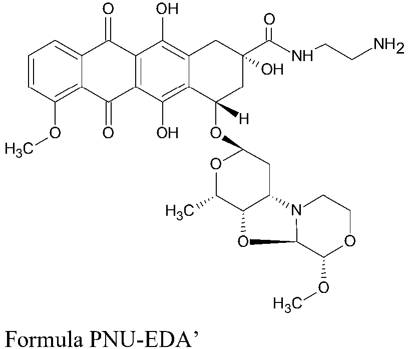 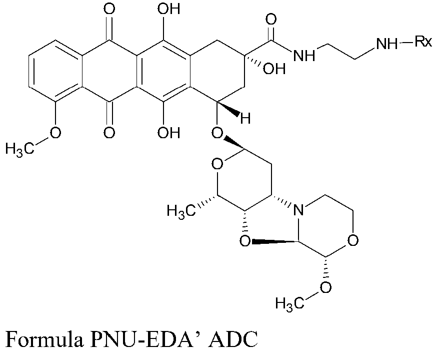 wherein Rx is L -antibody and L is the linker described in this specification, wherein the "linker" should be understood as referring to the moiety or portion of a molecule represented by Formulas I and III that does not comprise D, or as referring to the moiety or portion of a molecule represented by Formulas II, IIs, IV and IVs that does not comprise D and T.

In an embodiment, the cytotoxic drug is a maytansinoid. The maytansinoid may be an N-glucosylmaytansinoid. 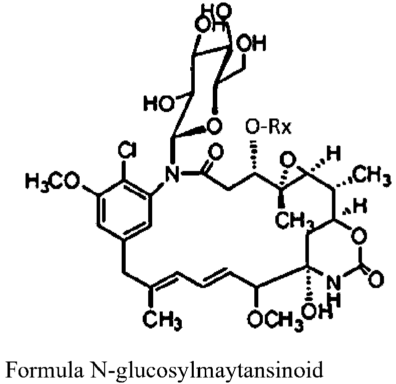 wherein Rx is L-antibody and L is the linker described in this specification, wherein the "linker" should be understood as referring to the moiety or portion of a molecule represented by Formulas I and III that does not comprise D, or as referring to the moiety or portion of a molecule represented by Formulas II, IIs, IV and IVs that does not comprise D and T.

In an embodiment, the cytotoxic drug is maytansine, an ansamitocin, DM1 or DM4 (also known as DM-4).

In an embodiment, the cytotoxic drug is DM1. DM1 is also known as DM-1 and mertansine.

In an embodiment, the cytotoxic drug is a rubicin. Suitable rubicins may be e.g. daunorubicins, doxorubicins, detorubicin, other doxorubicin derivatives including morpholino-doxorubicin, cyanomorpholino-doxorubicin, 2-pyrrolino-doxorubicin, deoxydoxorubicin, epirubicin, esorubicin, idarubicin, rodorubicin, zorubicin, and pirarubicin.

In an embodiment, the cytotoxic drug is epirubicin.

In an embodiment, the cytotoxic drug is duocarmycin. Suitable duocarmyxins may be e.g. duocarmycin A, duocarmycin B1, duocarmycin B2, duocarmycin C1, duocarmycin C2, duocarmycin D, duocarmycin SA, duocarmycin MA, and CC-1065. The term "duocarmycin" should be understood as referring also to synthetic analogs of duocarmycins, such as adozelesin, bizelesin, carzelesin, KW-2189 and CBI-TMI.

In an embodiment, the cytotoxic drug comprises a duocarmycin fragment that can alkylate DNA. In an embodiment, the cytotoxic drug comprises two or more duocarmycin fragments that can alkylate DNA. In an embodiment, the cytotoxic drug comprises two duocarmycin fragments that can alkylate DNA.

In an embodiment, the duocarmycin is a duocarmycin-saccharide conjugate of Formula DS. 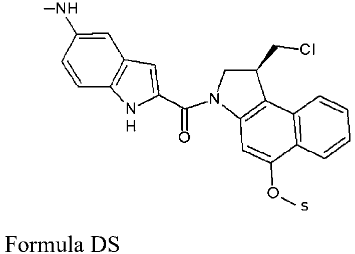 wherein S is a saccharide.

In an embodiment, the saccharide comprises or is a monosaccharide selected from the group consisting of β-D-galactose, N-acetyl-β-D-galactosamine, N-acetyl-α-D-galactosamine, N-acetyl-β-D-glucosamine, β-D-glucuronic acid, α-L-iduronic acid, α-D-galactose, α-D-glucose, β-D-glucose, α-D-mannose, β-D-mannose, α-L-fucose, β-D-xylose, neuraminic acid and any analogue or modification thereof.

Examples of suitable dolastatins include monomethyl and desmethyl dolastatins 10, 15, C, D and H, monomethyl and desmethyl isodolastatin H, and analogues and derivatives thereof.

In an embodiment, the cytotoxic drug is a tubulysin.

In an embodiment, the cytotoxic drug is an amanitin, such as an α-amanitin.

In an embodiment, the cytotoxic drug is a cryptophycin.

In an embodiment, the auristatin is monomethylauristatin E.

In an embodiment, the auristatin is MMAU.

In an embodiment, the auristatin is monomethylauristatin F, W or M.

In an embodiment, the cytotoxic drug is a pyrrolobenzodiazepine (PBD), a PBD dimer or an analogue thereof.

In an embodiment, the pyrrolobenzodiazepine (PBD), a PBD dimer or an analogue thereof is selected from the group of naturally occurring and synthetic analogues, abbeymycin, chicamycin, DC-81; mazethramycin, neothramycins A and B, porothramycin, prothracarcin;, sibanomicin (DC- 102), sibiromycin and tomamycin.

In an embodiment, the pyrrolobenzodiazepine (PBD), a PBD dimer or an analogue thereof is a non-crosslinking analogue described in Miller et al. 2016, Mol Cancer Ther; 15(8); 1-9.

D may be a cytotoxic drug selected from the group consisting of dolastatin; auristatin; epothilone; daunorubicin; doxorubicin; an alkylating agent, such as thiotepa or cyclophosphamide (CYTOXAN^{™}); alkyl sulfonate such as busulfan, improsulfan or piposulfan; aziridine, such as benzodopa, carboquone, meturedopa, or uredopa; ethylenimine and/or methylamelamine, such as altretamine, triethylenemelamine, trietylene-phosphoramide, triethylenethiophosphaoramide or trimethylolomelamine; acetogenin, such as bullatacin or bullatacinone; camptothecin, such as the synthetic analogue topotecan; bryostatin; callystatin; CC-1065 and/or its adozelesin, carzelesin or bizelesin synthetic analogue; cryptophycin, such as cryptophycin 1 or cryptophycin 8); duocarmycin (including the synthetic analogues, KW-2189 and CBI-TMI); eleutherobin; pancratistatin; sarcodictyins; spongistatin; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics, such as the enediyne antibiotics (e.g. calicheamicins, especially calicheamicin γ1; dynemicin, including dynemicin A; esperamicin; as well as neocarzinostatin chromophore and related chromoprotein enediyne antiobiotic chromomophores), aclacinomysins, actinomycin, authramycin, azaserine, bleomycins, cactinomycin, carabicin, caminomycin, carzinophilin; chromomycins, dactinomycin, detorubicin, 6-diazo-5-oxo-L-norleucine, other doxorubicin derivatives including morpholino-doxorubicin, cyanomorpholino-doxorubicin, 2-pyrrolino-doxorubicin and deoxydoxorubicin, epirubicin, esorubicin, idarubicin, marcellomycin, nitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites, such as methotrexate and 5-fluorouracil (5-FU); folic acid analogues, such as denopterin, methotrexate, pteropterin, trimetrexate; purine analogs, such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-fluorouracil; androgens, such as calusterone, dromostanolone propionate, epitiostanol, mepitiostane, testolactone; anti-adrenals, such as aminoglutethimide, mitotane, trilostane; folic acid replenisher, such as frolinic acid; aceglatone; aldophosphamide glycoside; aminolevulinic acid; amsacrine; bestrabucil; bisantrene; edatraxate; defofamine; demecolcine; diaziquone; elfomithine; elliptinium acetate; etoglucid; gallium nitrate; hydroxyurea; lentinan; lonidamine; maytansinoids, such as maytansine and N-glucosylmaytansinoids, ansamitocins, DM-1, DM-4; mitoguazone; mitoxantrone; mopidamol; nitracrine; pentostatin; phenamet; pirarubicin; podophyllinic acid; 2-ethylhydrazide; procarbazine; PSK^{®}; razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g. paclitaxel (TAXOL^{®}, Bristol-Myers Squibb Oncology, Princeton, N.J.) and doxetaxel (TAXOTERE^{®}, Rhone-Poulenc Rorer, Antony, France); chlorambucil; gemcitabine; 6-thioguanine; mercaptopurine; methotrexate; platinum coordination complex such as cisplatin, carboplatin and vinblastine; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylomithine (DMFO); retinoic acid; capecitabine; tamoxifen, raloxifene, aromatase inhibiting 4(5)-imidazoles, 4-hydroxytamoxifen, trioxifene, keoxifene, LY117018, onapristone, and toremifene (Fareston); and anti-androgens, such as flutamide, nilutamide, bicalutamide, leuprolide, and goserelin; tubulysins; amanitins, such as α-amanitin; and pharmaceutically acceptable salts, acids; dolastatin 10 or any derivative thereof; dolastatin 15 or any derivative thereof; auristatin F or any derivative thereof; monomethyl and desmethyl dolastatins 10, 15, C, D and H, monomethyl and desmethyl isodolastatin H, and analogues and derivatives thereof; monomethyl and desmethyl auristatins E, F, EB, EFP, PY, PYE, PE, PHE, TP, 2-AQ and 6-AQ; maytansinoids; N-glucosylmaytansinoid; maytansine, an ansamitocin, DM1 (also known as mertansine) or DM4 (also known as DM-4); daunorubicins, doxorubicins, detorubicin, other doxorubicin derivatives including morpholino-doxorubicin, cyanomorpholino-doxorubicin, 2-pyrrolino-doxorubicin, deoxydoxorubicin, epirubicin, esorubicin, idarubicin, rodorubicin, zorubicin, and pirarubicin; duocarmycin A, duocarmycin B1, duocarmycin B2, duocarmycin C1, duocarmycin C2, duocarmycin D, duocarmycin SA, duocarmycin MA, and CC-1065; synthetic analogs of duocarmycins, such as adozelesin, bizelesin, carzelesin, KW-2189 and CBI-TMI; duocarmycin-saccharide conjugate of Formula DS; tubulysin; α-amanitin; cryptophycin; monomethylauristatin E; an auristatin saccharide conjugate of Formula AS; MMAU; monomethylauristatin F, W or M; a pyrrolobenzodiazepine (PBD), abbeymycin, chicamycin, DC-81, mazethramycin, neothramycins A and B, porothramycin, prothracarcin, sibiromycin, tomamycin, and a PBD dimer; or an analogue of any of the above.

In an embodiment, the linker-payload conjugate is according to any one of the following Formulas CMa-CMzz.

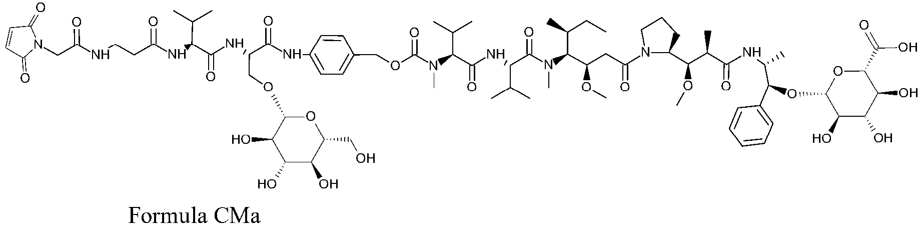

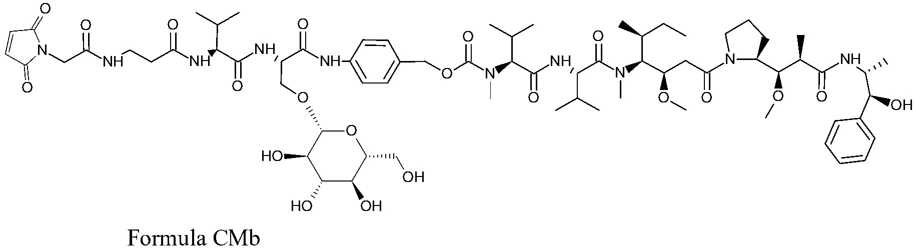

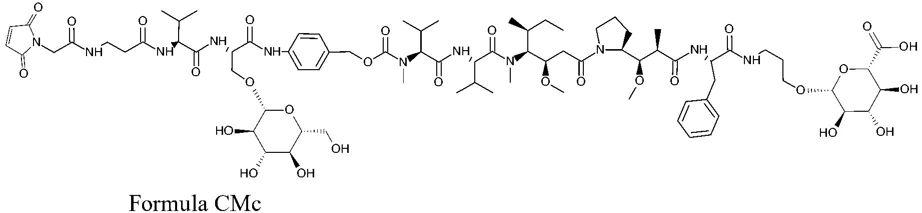

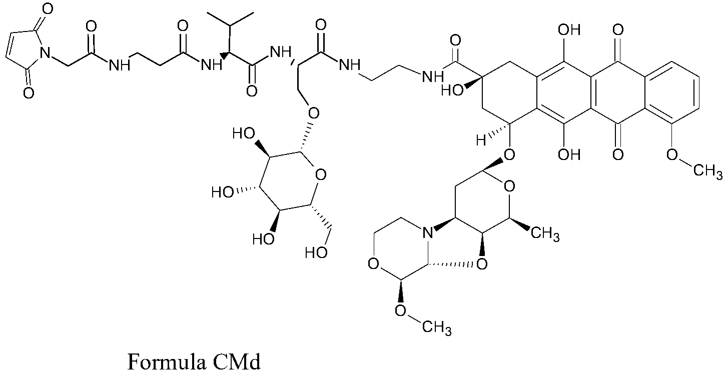

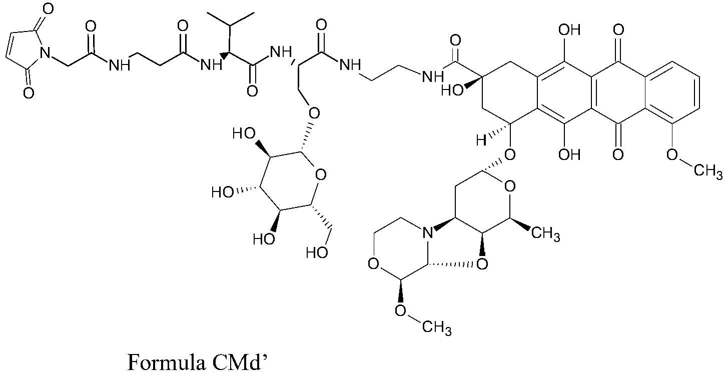

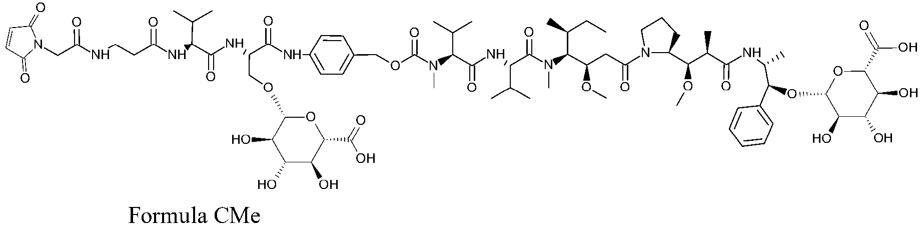

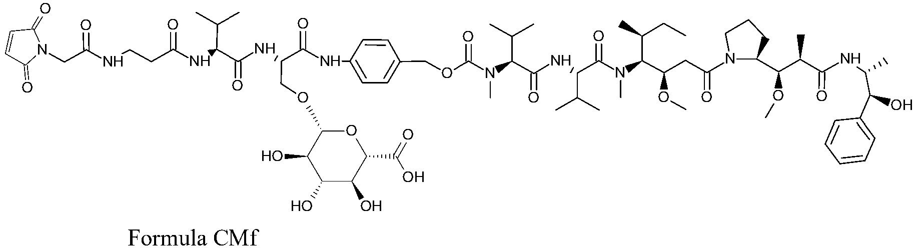

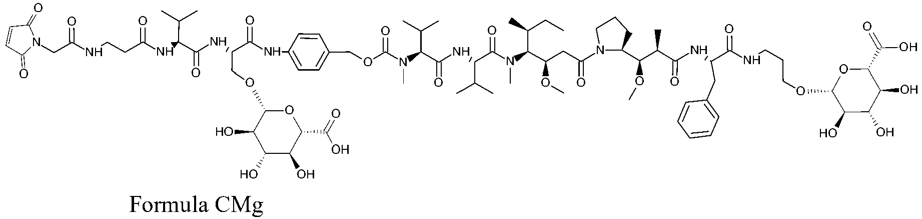

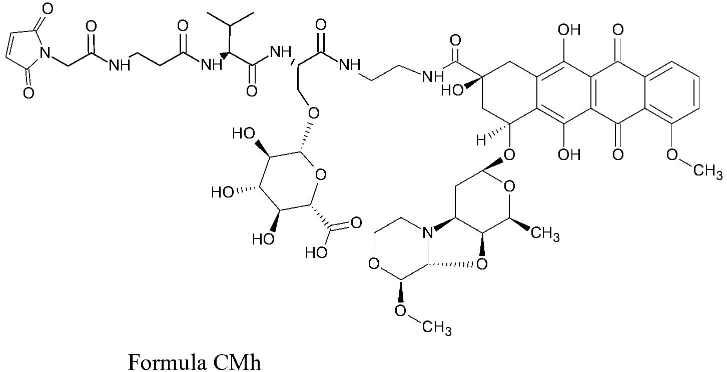

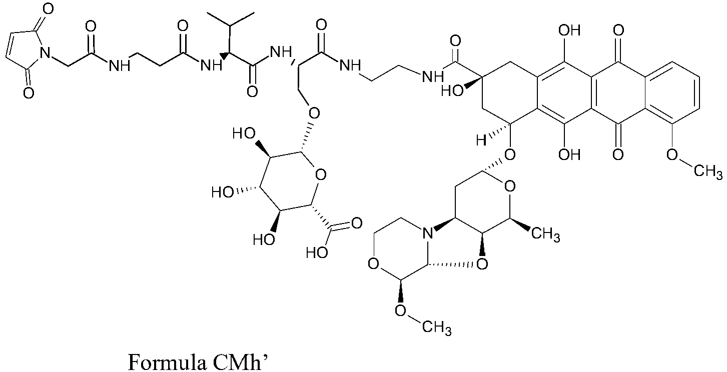

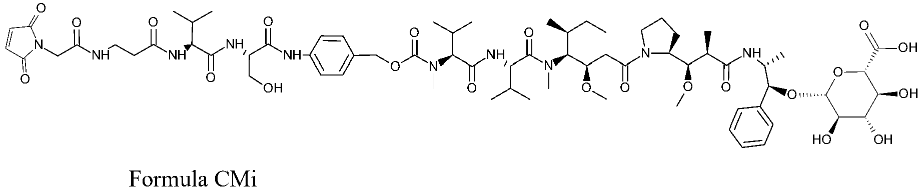

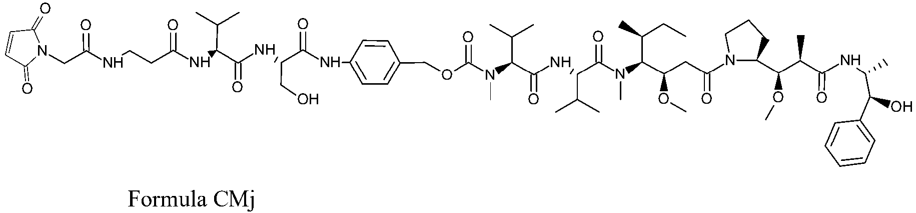

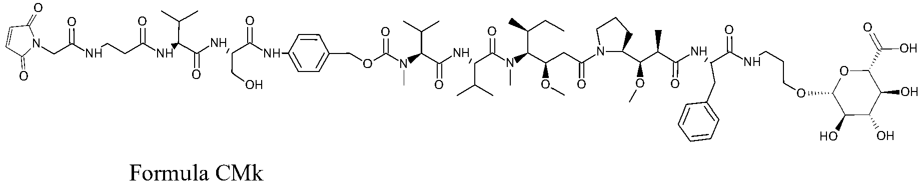

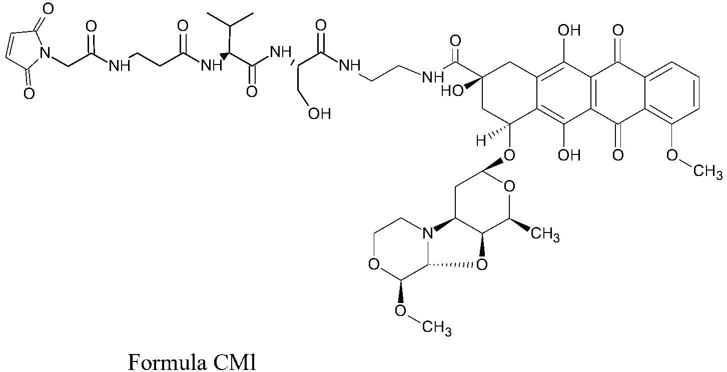

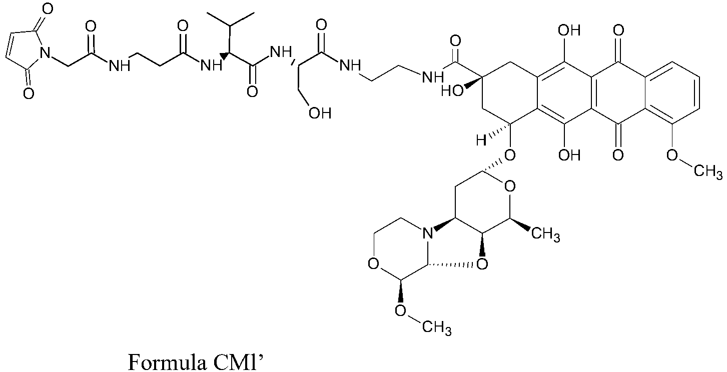

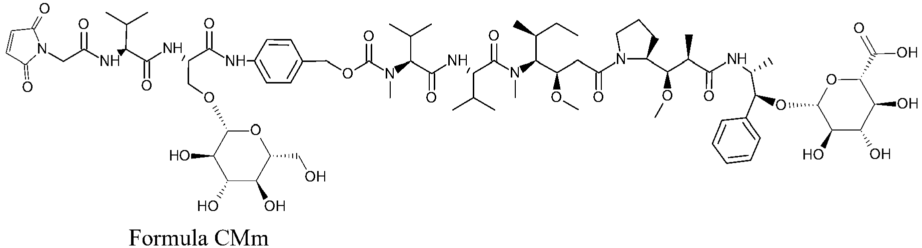

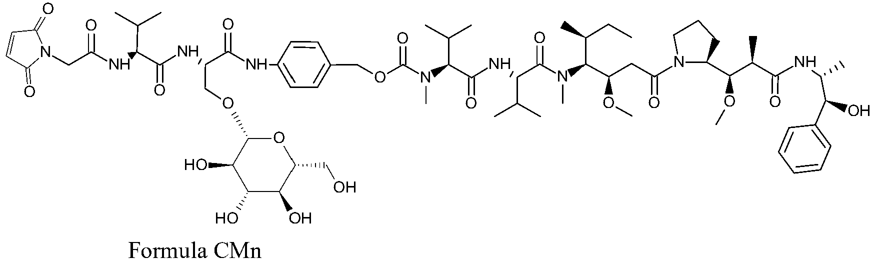

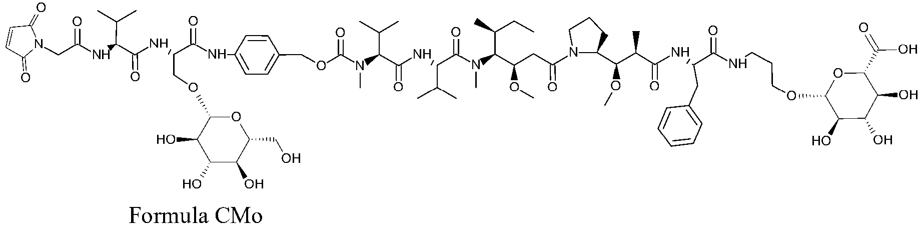

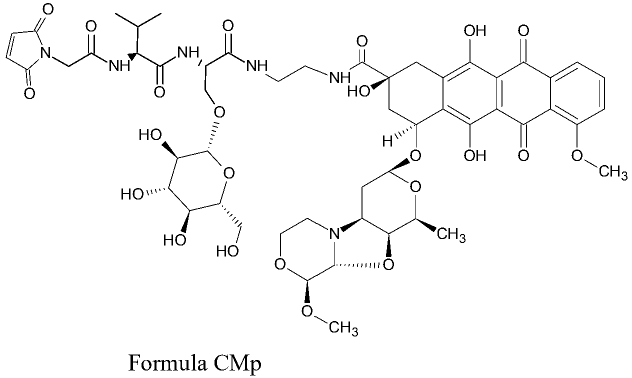

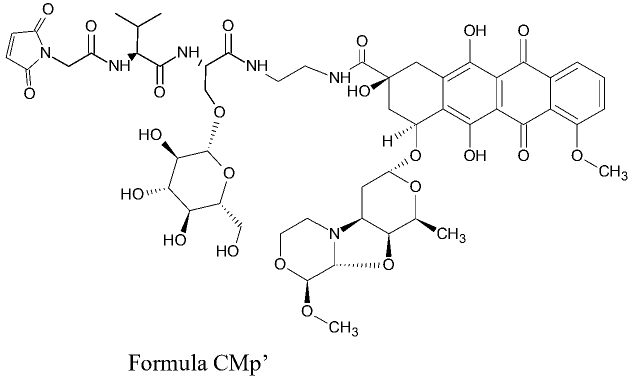

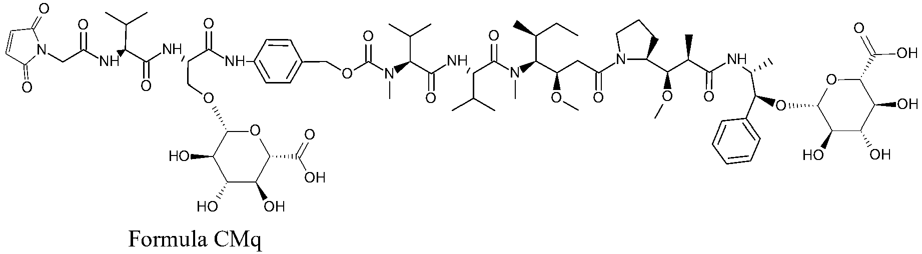

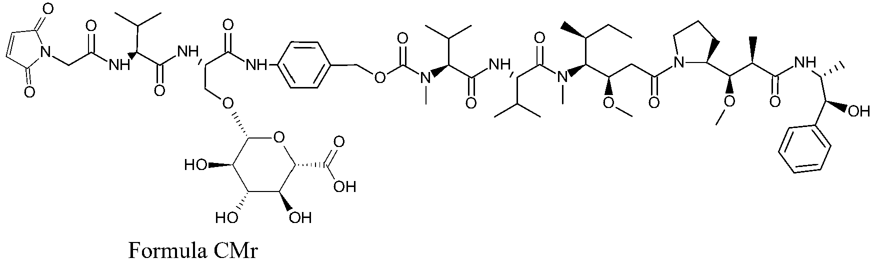

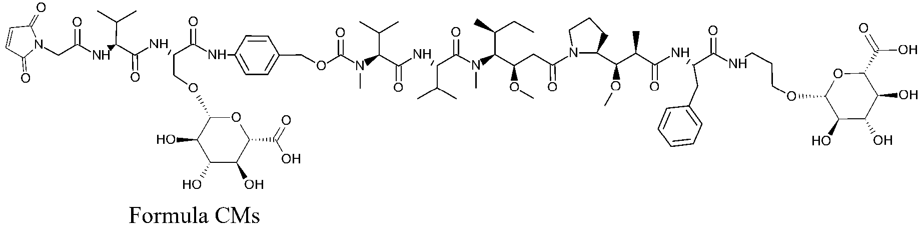

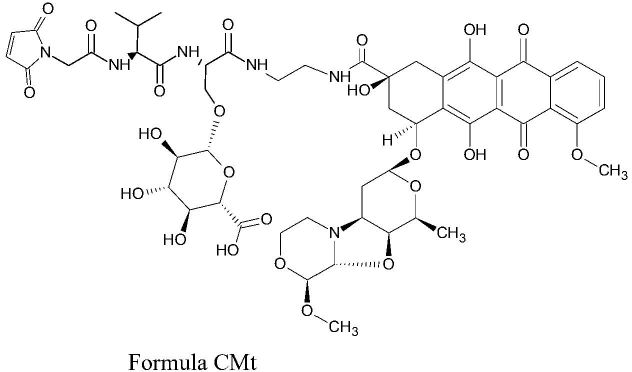

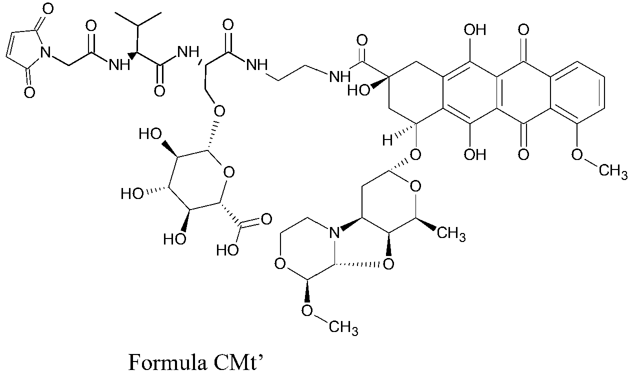

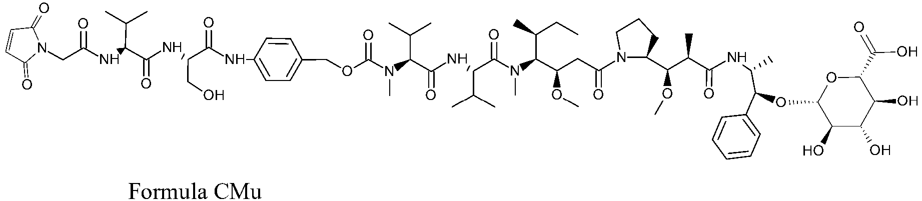

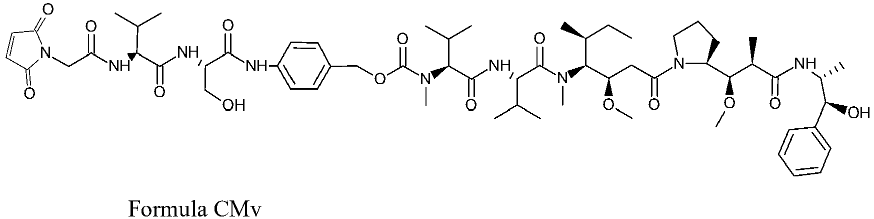

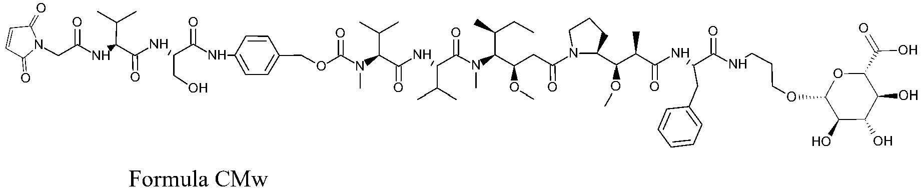

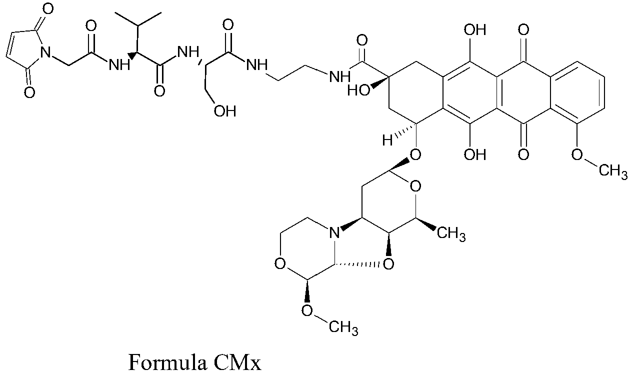

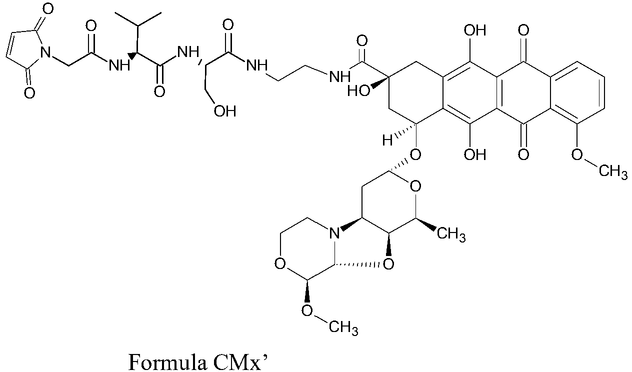

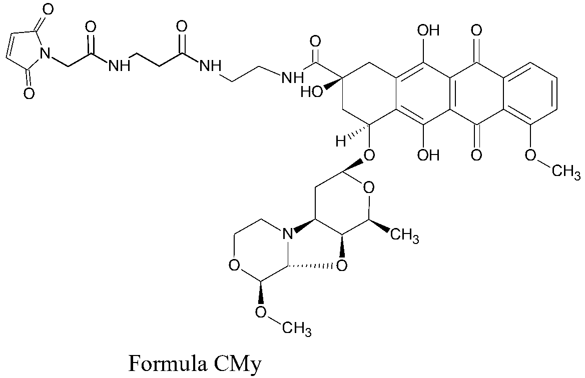

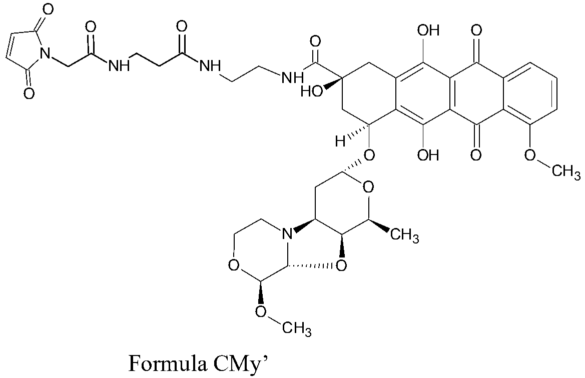

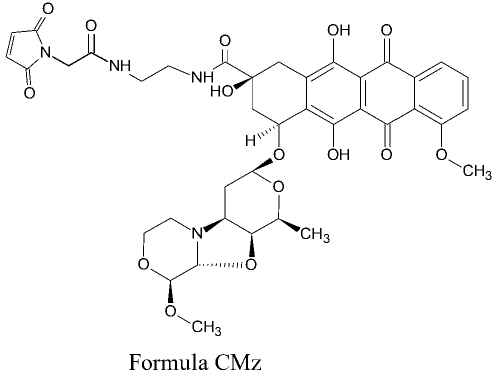

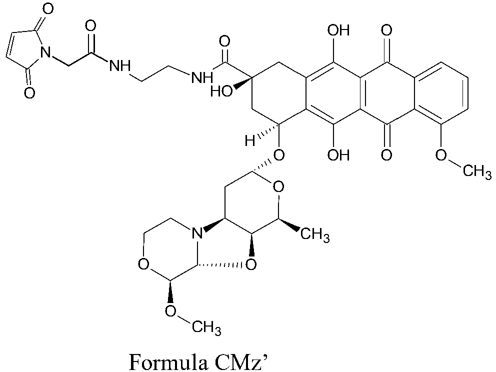

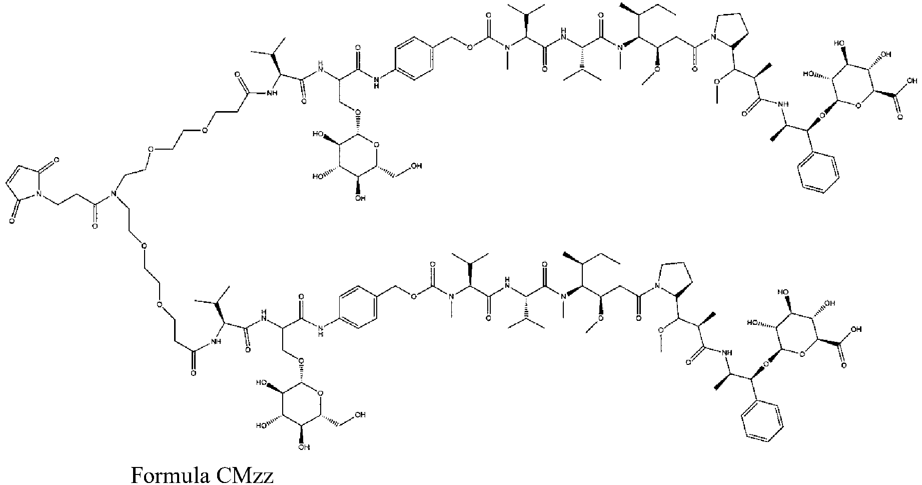

In an embodiment, the targeting unit T is a molecule that specifically binds to a target molecule. In the context of the present disclosure, the specific binding has the meaning that the targeting molecule has a reasonably higher binding affinity to its target than to unrelated molecules. An example of the specific binding is the binding of an antibody to its target epitope.

In an embodiment, the targeting unit T is a molecule that specifically binds to a target molecule on a surface of a target cell.

In an embodiment, the targeting unit T is small-molecule weight ligand, a lectin, a peptide, an aptamer, or an antibody.

In an embodiment, the targeting unit T is an antibody.

In an embodiment, the antibody is a cysteine engineered antibody.

The antibody, or a cysteine engineered antibody, may, in principle, be any antibody or its binding fragment, for instance an IgG, an scFv, a single domain antibody, an Fv, a VHH antibody, a diabody, a tandem diabody, a Fab, a Fab', a F(ab')2, a Db, a dAb-Fc, a taFv, a scDb, a dAb2, a DVD-Ig, a Bs(scFv)4-IgG, a taFv-Fc, a scFv-Fc-scFv, a Db-Fc, a scDb-Fc, a scDb-CH3, or a dAb-Fc-dAb.

In an embodiment, the antibody, or cysteine engineered antibody, is a human antibody or a humanized antibody. In this context, the term "human antibody", as it is commonly used in the art, is to be understood as meaning antibodies having variable regions in which both the framework and complementary determining regions (CDRs) are derived from sequences of human origin. In this context, the term "humanized antibody", as it is commonly used in the art, is to be understood as meaning antibodies wherein residues from a CDR of an antibody of human origin are replaced by residues from a CDR of a nonhuman species (such as mouse, rat or rabbit) having the desired specificity, affinity and capacity.

The antibodies of the disclosure may be cysteine engineered to facilitate conjugation to a payload. Based on the disclosure one skilled in the art could readily fabricate cysteine engineered antibodies as described herein. As used herein a "cysteine engineered antibody" or "cysteine engineered" may refer to an antibody, or immunoreactive fragment thereof, wherein at least one amino acid in the heavy and/or light chain is deleted, altered or substituted (preferably with another amino acid) to provide at least one free cysteine or unpaired cysteine in the heavy and/or light chain. "Engineered cysteine", as used herein, may refer to a cysteine amino acid being present in the cysteine engineered antibody (e.g., the cysteine engineered flanvotumab).

In some embodiments, the cysteine engineered antibody is a cysteine engineered antibody or antibody fragment, and the cysteine amino acid is introduced by substituting a non-cysteine amino acid in the corresponding parent antibody or antibody fragment (e.g., at N297C (Kabat numbering) of the heavy chain constant region) with the cysteine amino acid.

In some embodiments, the cysteine engineered antibody is a cysteine engineered antibody or antibody fragment, and the cysteine amino acid is replaced by substituting a cysteine amino acid in the corresponding parent antibody or antibody fragment (e.g., at C220S (Kabat numbering) of the heavy chain constant region) with the non-cysteine amino acid.

"Parent antibody", as used herein, refers to the corresponding antibody of the cysteine engineered antibody prior to the engineering process (e.g., the engineering process introducing the engineered cysteine). It is understood that the parent antibody may be wild type, mutated, or synthetic.

"Cysteine engineered", as used herein, refers to the feature of an antibody (e.g., an antibody or antibody fragment)) as including at least one engineered cysteine or free cysteine.

In accordance with the present disclosure, the term "engineered cysteine" or "cysteine engineered" means deleting or substituting an amino acid in the heavy chain or light chain of an antibody to provide at least one free cysteine or unpaired cysteine in the heavy and/or light chain.

In an embodiment, "engineered cysteine" or "cysteine engineered" means substituting a non-cysteine amino acid in the heavy chain and/or light chain of an antibody by a cysteine.

In an embodiment, "engineered cysteine" or "cysteine engineered" means substituting a cysteine amino acid in the heavy chain and/or light chain of an antibody by a non-cysteine amino acid.

In certain embodiments the unpaired cysteine residue will comprise or be an unpaired intrachain cysteine residue. In other embodiments the free cysteine residue will comprise or be an unpaired interchain cysteine residue. In still other embodiments the free cysteine may be engineered into the amino acid sequence of the antibody (e.g., in the CH3 domain). In any event the cysteine engineered antibody can be of various isotypes, for example, IgG, IgE, IgA or IgD; and within those classes the antibody can be of various subclasses, for example, IgG1, IgG2, IgG3 or IgG4. For IgG constructs the light chain of the antibody can comprise either a kappa or lambda isotype each incorporating a C214 that, in selected embodiments, may be unpaired due to a lack of a C220 residue in the IgG1 heavy chain.

Thus, as used herein, the terms "free cysteine" or "unpaired cysteine" may be used interchangeably unless otherwise dictated by context and shall mean any cysteine (or thiol containing) constituent (e.g., a cysteine residue) of an antibody, whether naturally present or specifically incorporated in a selected residue position using molecular engineering techniques, that is not part of a naturally occurring (or "native") disulfide bond under physiological conditions. In certain embodiments the free cysteine may comprise a naturally occurring cysteine whose native interchain or intrachain disulfide bridge partner has been substituted, eliminated or otherwise altered to disrupt the naturally occurring disulfide bridge under physiological conditions thereby rendering the unpaired cysteine suitable for conjugation. In other embodiments the free or unpaired cysteine will comprise a cysteine residue that is selectively placed at a predetermined site within the antibody heavy or light chain amino acid sequences. It will be appreciated that, prior to conjugation, free or unpaired cysteines may be present as a thiol (reduced cysteine), as a capped cysteine (oxidized) or as part of a non-native intra- or intermolecular disulfide bond (oxidized) with another cysteine or thiol group on the same or different molecule depending on the oxidation state of the system. Accordingly, in certain embodiments the free or unpaired cysteines (whether naturally occurring or incorporated) will be subject to selective reduction and subsequent conjugation to provide compositions or pharmaceutical compositions of the linker-payload conjugates of the present disclosure.

In certain embodiments the cysteine engineered antibody comprises at least one amino acid deletion or substitution of an intrachain or interchain cysteine residue. As used herein "interchain cysteine residue" means a cysteine residue that is involved in a native disulfide bond either between the light and heavy chain of an antibody or between the two heavy chains of an antibody while an "intrachain cysteine residue" is one naturally paired with another cysteine in the same heavy or light chain. In one embodiment the deleted or substituted interchain cysteine residue is involved in the formation of a disulfide bond between the light and heavy chain. In another embodiment the deleted or substituted cysteine residue is involved in a disulfide bond between the two heavy chains. In a typical embodiment, due to the complementary structure of an antibody, in which the light chain is paired with the VH and CH1 domains of the heavy chain and wherein the CH2 and CH3 domains of one heavy chain are paired with the CH2 and CH3 domains of the complementary heavy chain, a mutation or deletion of a single cysteine in either the light chain or in the heavy chain would result in two unpaired cysteine residues in the cysteine engineered antibody.

In some embodiments an interchain cysteine residue is deleted. In other embodiments an interchain cysteine is substituted for another amino acid (e.g., a naturally occurring amino acid). For example, the amino acid substitution can result in the replacement of an interchain cysteine with a neutral (e.g. serine, threonine or glycine) or hydrophilic (e.g. methionine, alanine, valine, leucine or isoleucine) residue. In certain embodiments an interchain cysteine is replaced with a serine.

In some embodiments the deleted or substituted cysteine residue is on the light chain (either kappa or lambda) thereby leaving a free cysteine on the heavy chain. In other embodiments the deleted or substituted cysteine residue is on the heavy chain leaving the free cysteine on the light chain constant region. Upon assembly it will be appreciated that deletion or substitution of a single cysteine in either the light or heavy chain of an intact antibody results in a cysteine engineered antibody having two unpaired cysteine residues.

With regard to the introduction or addition of a cysteine residue or residues to provide a free cysteine (as opposed to disrupting a native disulfide bond) compatible position(s) on the antibody or antibody fragment may readily be discerned by one skilled in the art. Accordingly, in selected embodiments the cysteine(s) may be introduced in the CH1 domain, the CH2 domain or the CH3 domain or any combination thereof depending on the desired DAR, the selected payload and the antibody target. In other embodiments the cysteines may be introduced into a kappa or lambda CL domain and, in certain embodiments, in the C-terminal region of the CL domain. In each case other amino acid residues proximal to the site of cysteine insertion may be altered, removed or substituted to facilitate molecular stability, conjugation efficiency or provide a protective environment for the payload once it is attached. In certain embodiments, the substituted residues occur at any accessible sites of the antibody. By substituting such surface residues with cysteine, reactive thiol groups are thereby positioned at readily accessible sites on the antibody and may be selectively reduced. In certain embodiments, the substituted residues occur at accessible sites of the antibody. By substituting those residues with cysteine, reactive thiol groups are thereby positioned at accessible sites of the antibody and may be used to selectively conjugate the antibody.

In an embodiment, cysteine engineered antibody has one or more amino acid deletions or substitutions of heavy chain selected from the group consisting of A40, P41, A84, V89, S112, S113, A114, S115, T116, G118, V152, S153, N155, A168, Q171, C220, 225, 226, 229, 247, V278, N297, 339, S371, 375, 376, S396, and E400 (according to Kabat).

In an embodiment the asparagine at position 297 (N297) on the IgG heavy chain is deleted or substituted.

In an embodiment, the heavy chain is N297C.

In an embodiment the cysteine at position 220 (C220) on the IgG heavy chain is deleted or substituted.

In an embodiment, the heavy chain is C220S.

In an embodiment the cysteine at position 226 or position 229 on the heavy chain is deleted or substituted.

In an embodiment, the heavy chain is C226S.

In an embodiment, the heavy chain is C229S.

In an embodiment, cysteine engineered antibody has one or more amino acid deletions or substitutions of light chain selected from the group consisting of V110, S114, S121, S127, 143, 147, A153, 159, 163, 165, S168, V205, and 214 (according to Kabat).

In an embodiment the cysteine at position V205C or C214 of the IgG light chain (kappa or lambda) is deleted or substituted.

In an embodiment, the light chain is V205C.

In an embodiment, the light chain is C214S.

Additional substitution positions and methods of fabricating cysteine engineered antibodies are set forth in WO 2006/034488, WO 2006/065533 and WO 2014/124316 which are incorporated herein in its entirety.

The expression "Kabat numbering" refers to the numbering system used for heavy chain variable domains or light chain variable domains of the compilation of antibodies in Kabat, E. A. et al., Sequences of Proteins of Immunological Interest, 5th Ed. Public Health Service, National Institutes of Health, Bethesda, Md. (1991). Using this numbering system, the actual linear amino acid sequence may contain fewer or additional amino acids corresponding to a shortening of, or insertion into, a framework region (FR) or complementary determining region (CDR) of the variable domain. The Kabat numbering of residues may be determined for a given antibody by alignment at regions of homology of the sequence of the antibody with a "standard" Kabat numbered sequence. Unless expressly otherwise indicated, any numbers referring to the amino acid (sequence) of a heavy chain or light chain in this specification are according to the Kabat numbering.

The expression "Eu numbering" refers to the Eu index as in Kabat, E. A. et al., Sequences of Proteins of Immunological Interest, 5th Ed. Public Health Service, National Institutes of Health, Bethesda, Md., NIH publication no. 91-3242, pp. 662, 680, 689 (1991). The "Eu index as in Kabat" refers to the residue numbering of the human IgG1 Eu antibody (Edelman, G. M. et al., Proc. Natl. Acad. Sci. USA, 63, 78-85 (1969)).

In an embodiment, the antibody is capable of binding a cell surface antigen.

In an embodiment, the cell surface antigen is a tumor antigen and/or a cancer antigen.

In an embodiment, the antibody is selected from the group consisting of bevacizumab, tositumomab, etanercept, trastuzumab, adalimumab, alemtuzumab, gemtuzumab ozogamicin, efalizumab, rituximab, infliximab, abciximab, basiliximab, palivizumab, omalizumab, daclizumab, cetuximab, panitumumab, epratuzumab, 2G12, lintuzumab, nimotuzumab, flanvotumab and ibritumomab tiuxetan.

In an embodiment, the antibody is capable of binding a target molecule selected from the group consisting of CD2, CD3, CD4, CD5, CD6, CD11, CD8, CD11a, CD19, CD20, CD22, CD25, CD26, CD30, CD33, CD34, CD37, CD38, CD40, CD44, CD46, CD52, CD56, CD79, CD105, CD138, epidermal growth factor receptor 1 (EGFR), epidermal growth factor receptor 2 (HER2/neu), HER3 or HER4 receptor, LFA-1, Mac1, p150.95, VLA-4, ICAM-1, VCAM, EpCAM, alpha4/beta7 integrin, alpha v/beta3 integrin including either alpha or beta subunits thereof (e.g. anti-CD11a, anti-CD18 or anti-CD11b antibodies), tissue factor (TF), tumor necrosis factor alpha (TNF-α), human vascular endothelial growth factor (VEGF), glycoprotein IIb/IIIa, TGF-beta, alpha interferon (alpha-IFN), IL-8, IL-2 receptor, IgE, respiratory syncytial virus (RSV), HIV-1 envelope glycoprotein gp120, cancer-associated high-mannose type N-glycans, blood group antigen Apo2, death receptor, flk2/flt3 receptor, obesity (OB) receptor, mp1 receptor, CTLA-4, transferrin receptor, Lewis y, GD3, TYRP-1 (melanoma antigen TA99/gp75), PD-L1, PD-L2, TIM-3, MUC1, CA6 MUC1 antigen, and protein C.

In an embodiment, the antibody is selected from the group consisting of an anti-EGFR antibody, an epidermal growth factor receptor 2 (HER2/neu) antibody, an anti-CD22 antibody, an anti-CD30 antibody, an anti-CD33 antibody, an anti-Lewis y antibody, anti-TYRP-1, an anti-CD20 antibody and an anti-hematologic target antibody.

In an embodiment, the antibody is capable of binding an anti-hematologic target molecule selected from the group consisting of CD19, CD20, CD22, CD25, CD30, CD33, CD37, CD38, CD52, CD56, CD70, CD74, CD79, CD98, CD117, CD105, CD123, CD138, CD157, BCMA and CD319 (SLAMF7).

In the context of this specification, the term "anti-hematologic" may be understood as referring to a target molecule that may be involved or associated with a hematological cancer.

In an embodiment, the target molecule is selected from the group consisting of CD19, CD22, CD33, CD52 and CD123.

In an embodiment, the antibody is an anti-hematologic target antibody.

In an embodiment, the anti-hematologic target antibody is selected from the group consisting of loncastuximab, blinatumomab, tafasitamab, coltuximab, denintuzumab, obexelimab, inebilizumab, MOR00208, MDX-1342, MEDI-551, SAR3419, rituximab, ofatumumab, veltuzumab, ocrelizumab, obinutuzumab, oaratuzumab, ublituximab, nofetumomab, ibritumomab, epratuzumab, inotuzumab ozogamicin, bectumomab, moxetumomab, pinatuzumab, DCDT2980S, basiliximab, daclizumab, camidanlumab, inolimomab, ADCT-301, IMTOX-25, brentuximab, iratumumab, AVE9633, lintuzumab, gemtuzumab, vadastuximab, otlertuzumab, lilotomab, naratuximab, BI836826, AGS67E, IMGN529, daratumumab, isatuximab, mezagitamab, felzartamab, MOR202, MOR03087, alemtuzumab, lorvotuzumab mertansine, vorsetuzumab mafodotin, SGN-70A, polatuzumab, indatuximab, MDX-1203, milatuzumab-doxorubicin, IGN523, LOP-628, CSL360, talacotuzumab, XmAb14045, KHK2823, BT062, belantamab mafodotin, teclistamab and elotuzumab.

In an embodiment, the target antibody is selected from the group consisting of epratuzumab, lintuzumab, coltuximab, denintuzumab, loncastuximab, alemtuzumab and talacotuzumab.

In an embodiment, the antibody is selected from the group consisting of abagovomab, actoxumab, adecatumumab, afutuzumab, altumomab, amatuximab, anifrolumab, apolizumab, atinumab, atlizumab, atorolimumab, bapineuzumab, basiliximab, bavituximab, belimumab, benralizumab, bertilimumab, besilesomab, bezlotoxumab, bimagrumab, bivatuzumab, blinatumomab, blosozumab, brentuximab, briakinumab, brodalumab, canakinumab, cantuzumab, caplacizumab, capromab, carlumab, catumaxomab, CC49, cedelizumab, cixutumumab, clazakizumab, clenoliximab, clivatuzumab, conatumumab, concizumab, crenezumab, CR6261, dacetuzumab, dalotuzumab, daratumumab, demcizumab, denosumab, detumomab, drozitumab, duligotumab, dupilumab, dusigitumab, ecromeximab, eculizumab, edobacomab, edrecolomab, eldelumab, elotuzumab, elsilimomab, enavatuzumab, enlimomab, enokizumab, enoticumab, ensituximab, epitumomab, epratuzumab, ertumaxomab, etaracizumab, etrolizumab, evolocumab, exbivirumab, fanolesomab, faralimomab, farletuzumab, fasinumab, felvizumab, fezakinumab, ficlatuzumab, figitumumab, flanvotumab, fontolizumab, foralumab, foravirumab, fresolimumab, fulranumab, futuximab, galiximab, ganitumab, gantenerumab, gavilimomab, gevokizumab, girentuximab, glembatumumab, golimumab, gomiliximab, guselkumab, ibalizumab, icrucumab, imciromab, imgatuzumab, inclacumab, indatuximab, intetumumab, inolimomab, inotuzumab, ipilimumab, iratumumab, itolizumab, ixekizumab, keliximab, labetuzumab, lambrolizumab, lampalizumab, lebrikizumab, lemalesomab, lerdelimumab, lexatumumab, libivirumab, ligelizumab, lintuzumab, lirilumab, lodelcizumab, lorvotuzumab, lucatumumab, lumiliximab, mapatumumab, margetuximab, maslimomab, mavrilimumab, matuzumab, mepolizumab, metelimumab, milatuzumab, minretumomab, mitumomab, mogamulizumab, morolimumab, motavizumab, moxetumomab, muromonab, namilumab, narnatumab, natalizumab, nebacumab, necitumumab, nerelimomab, nesvacumab, nimotuzumab, nivolumab, binutuzumab, ocaratuzumab, ocrelizumab, odulimomab, ofatumumab, olaratumab, olokizumab, onartuzumab, oregovomab, orticumab, otelixizumab, oxelumab, ozanezumab, ozoralizumab, pagibaximab, panobacumab, parsatuzumab, pascolizumab, pateclizumab, patritumab, pemtumomab, perakizumab, pertuzumab, pidilizumab, pinatuzumab, pintumomab, placulumab, polatuzumab, ponezumab, priliximab, pritoxaximab, pritumumab, quilizumab, racotumomab, radretumab, rafivirumab, ramucirumab, raxibacumab, regavirumab, reslizumab, rilotumumab, robatumumab, roledumab, romosozumab, rontalizumab, rovelizumab, ruplizumab, samalizumab, sarilumab, satumomab, secukinumab, seribantumab, setoxaximab, sevirumab, sibrotuzumab, sifalimumab, siltuximab, simtuzumab, siplizumab, sirukumab, solanezumab, solitomab, sonepcizumab, sontuzumab, stamulumab, suvizumab, tabalumab, tacatuzumab, talizumab, tanezumab, taplitumomab, tefibazumab, tenatumomab, teneliximab, teplizumab, teprotumumab, TGN1412, ticilimumab, tildrakizumab, tiga-tuzumab, tocilizumab, toralizumab, tovetumab, tralokinumab, TRBS07, tregalizumab, tremelimumab, tucotuzumab, tuvirumab, ublituximab, urelumab, urtoxazumab, ustekinumab, vantictumab, vapaliximab, vatelizumab, vedolizumab, veltuzumab, vepalimomab, vesencumab, visilizumab, volociximab, vorsetuzumab, votumumab, zalutumumab, zanolimumab, zatuximab, ziralimumab, 2G12 (anti-HIV-1 envelope glycoprotein gp120), TA99, avelumab, DS6, huDS6, and zolimomab.

In an embodiment, the antibody is selected from the group consisting of an anti-EGFR antibody, an epidermal growth factor receptor 2 (HER2/neu) antibody, an anti-CD22 antibody, an anti-CD30 antibody, an anti-CD33 antibody, an anti-CD20 antibody, an anti-TYRP-1 antibody, an anti-PD-L1 antibody, an anti-PD-L2 antibody, an anti-TIM-3 antibody, an anti-MUC1 antibody, and an anti-CA6 antibody.

In an embodiment, the antibody is an anti-EGFR antibody.

In an embodiment, an anti-EGFR antibody is cetuximab, imgatuzumab, matuzumab, nimotuzumab, necitumumab, panitumumab, or zalutumumab.

In an embodiment, the antibody is an epidermal growth factor receptor 2 (HER2/neu) antibody.

In an embodiment, an anti-HER2 antibody is margetuximab, pertuzumab, trastuzumab, ertumaxomab, or 520C9XH22.

In an embodiment, the antibody is an anti-CD19 antibody.

In an embodiment, the anti-CD19 antibody is loncastuximab, blinatumomab, tafasitamab, coltuximab, denintuzumab, obexelimab or inebilizumab.

In an embodiment, loncastuximab has the heavy chain sequence set forth in SEQ ID NO: 1.

In an embodiment, loncastuximab has the light chain sequence set forth in SEQ ID NO: 2.

In an embodiment, coltuximab has the heavy chain sequence set forth in SEQ ID NO: 3.

In an embodiment, coltuximab has the light chain sequence set forth in SEQ ID NO: 4.

In an embodiment, denintuzumab has the heavy chain sequence set forth in SEQ ID NO: 5.

In an embodiment, denintuzumab has the light chain sequence set forth in SEQ ID NO: 6.

In an embodiment, the antibody is an anti-CD52 antibody.

In an embodiment, the anti-CD52 antibody is alemtuzumab.

In an embodiment, alemtuzumab has the heavy chain sequence set forth in SEQ ID NO: 7.

In an embodiment, alemtuzumab has the light chain sequence set forth in SEQ ID NO: 8.

In an embodiment, the antibody is an anti-CD37 antibody.

In an embodiment, the anti-CD37 antibody is otlertuzumab, lilotomab, naratuximab or BI 836826.

In an embodiment, the antibody is an anti-CD38 antibody.

In an embodiment, the anti-CD38 antibody is daratumumab, isatuximab, mezagitamab or felzartamab.

In an embodiment, daratumumab has the heavy chain sequence set forth in SEQ ID NO: 9.

In an embodiment, daratumumab has the light chain sequence set forth in SEQ ID NO: 10.

In an embodiment, isatuximab has the heavy chain sequence set forth in SEQ ID NO: 11.

In an embodiment, isatuximab has the light chain sequence set forth in SEQ ID NO: 12.

In an embodiment, mezagitamab has the heavy chain sequence set forth in SEQ ID NO: 13.

In an embodiment, mezagitamab has the light chain sequence set forth in SEQ ID NO: 14.

In an embodiment, felzartamab has the heavy chain sequence set forth in SEQ ID NO: 15.

In an embodiment, felzartamab has the light chain sequence set forth in SEQ ID NO: 16.

In an embodiment, the antibody is an anti-CD56 antibody.

In an embodiment, the anti-CD56 antibody is lorvotuzumab.

In an embodiment, lorvotuzumab has the heavy chain sequence set forth in SEQ ID NO: 17.

In an embodiment, lorvotuzumab has the light chain sequence set forth in SEQ ID NO: 18.

In an embodiment, the antibody is an anti-CD79 antibody.

In an embodiment, the anti-CD79 antibody is polatuzumab.

In an embodiment, polatuzumab has the heavy chain sequence set forth in SEQ ID NO: 19.

In an embodiment, polatuzumab has the light chain sequence set forth in SEQ ID NO: 20.

In an embodiment, the antibody is an anti-CD138 antibody.

In an embodiment, the anti-CD138 antibody is indatuximab.

In an embodiment, indatuximab has the heavy chain sequence set forth in SEQ ID NO: 21.

In an embodiment, indatuximab has the light chain sequence set forth in SEQ ID NO: 22.

In an embodiment, the antibody is an anti-BCMA antibody.

In an embodiment, the anti-BCMA antibody is belantamab or teclistamab.

In an embodiment, belantamab has the heavy chain sequence set forth in SEQ ID NO: 23.

In an embodiment, belantamab has the light chain sequence set forth in SEQ ID NO: 24.

In an embodiment, the antibody is an anti-CD20 antibody.

In an embodiment, the anti-CD20 antibody is rituximab, ublituximab, obinutuzumab, ocaratuzumab, ocrelizumab, veltuzumab, ofatumumab, nofetumomab or ibritumomab.

In an embodiment, ocrelizumab has the heavy chain sequence set forth in SEQ ID NO: 25.

In an embodiment, ocrelizumab has the light chain sequence set forth in SEQ ID NO: 26.

In an embodiment, the antibody is an anti-CD22 antibody.

In an embodiment, an anti-CD22 antibody is bectumomab, moxetumomab, epratuzumab, inotuzumab, or pinatuzumab.

In an embodiment, inotuzumab has the heavy chain sequence set forth in SEQ ID NO: 27.

In an embodiment, inotuzumab has the light chain sequence set forth in SEQ ID NO: 28.

In an embodiment, epratuzumab has the heavy chain sequence set forth in SEQ ID NO: 29.

In an embodiment, epratuzumab has the light chain sequence set forth in SEQ ID NO: 30.

In an embodiment, the antibody is an anti-CD25 antibody.

In an embodiment, an anti-CD25 antibody is camidanlumab, daclizumab, inolimomab and basiliximab.

In an embodiment, camidanlumab has the heavy chain sequence set forth in SEQ ID NO: 31.

In an embodiment, camidanlumab has the light chain sequence set forth in SEQ ID NO: 32.

In an embodiment, the antibody is an anti-CD30 antibody.

In an embodiment, an anti-CD30 antibody is brentuximab vedotin (or the antibody portion of the brentuximab vedotin) or iratumumab.

In an embodiment, brentuximab has the heavy chain sequence set forth in SEQ ID NO: 33.

In an embodiment, brentuximab has the light chain sequence set forth in SEQ ID NO: 34.

In an embodiment, the antibody is an anti-CD33 antibody.

In an embodiment, an anti-CD33 antibody is gemtuzumab, SGN-CD33A or lintuzumab.

In an embodiment, lintuzumab has the heavy chain sequence set forth in SEQ ID NO: 35.

In an embodiment, lintuzumab has the light chain sequence set forth in SEQ ID NO: 36.

In an embodiment, gemtuzumab has the heavy chain sequence set forth in SEQ ID NO: 37.

In an embodiment, gemtuzumab has the light chain sequence set forth in SEQ ID NO: 38.

In an embodiment, the anti-CD33 antibody is vadastuximab.

In an embodiment, vadastuximab has the heavy chain sequence set forth in SEQ ID NO: 39.

In an embodiment, vadastuximab has the light chain sequence set forth in SEQ ID NO: 40.

In an embodiment, the anti-CD33 antibody is a cysteine engineered antibody.

In an embodiment, the cysteine engineered anti-CD33 antibody has heavy chain selected from the group consisting of N296C and C219S.

In an embodiment, the cysteine engineered anti-CD33 antibody is lintuzumab. In other words, the cysteine engineered anti-CD33 antibody is cysteine engineered lintuzumab.

In the context of this specification, the term "cysteine engineered lintuzumab" may be understood as referring to an antibody having otherwise the amino acid sequence and/or structure of lintuzumab, except that it has been cysteine engineered. The cysteine engineered lintuzumab may thus be obtainable or obtained by cysteine engineering lintuzumab. Although lintuzumab has been used as an example of the use of the term, the same may apply to any parent antibody described herein.

In an embodiment, the cysteine engineered lintuzumab has the heavy chain sequence having the substitution N296C set forth in SEQ ID NO: 41.

In an embodiment, the cysteine engineered lintuzumab has the heavy chain sequence having the substitution C219S set forth in SEQ ID NO: 42.

In an embodiment, the cysteine engineered anti-CD33 antibody is gemtuzumab.

In an embodiment, the cysteine engineered gemtuzumab has heavy chain selected from the group consisting of N293C and C130S.

In an embodiment, the cysteine engineered gemtuzumab has the heavy chain sequence having the substitution N293C set forth in SEQ ID NO: 43.

In an embodiment, the cysteine engineered gemtuzumab has the heavy chain sequence having the substitution C130S set forth in SEQ ID NO: 44.

In an embodiment, the antibody is an anti-TYRP-1 antibody.

In an embodiment, the anti-TYRP-1 antibody is TA99, chimeric TA99 or flanvotumab.

In an embodiment, the anti-TYRP-1 antibody is flanvotumab.

In an embodiment, flanvotumab has the heavy chain sequence set forth in SEQ ID NO: 45.

In an embodiment, flanvotumab has the light chain sequence set forth in SEQ ID NO: 46.

In an embodiment, chimeric TA99 has the heavy chain sequence set forth in SEQ ID NO: 47.

In an embodiment, chimeric TA99 has the light chain sequence set forth in SEQ ID NO: 48.

In an embodiment, the anti-TYRP-1 antibody is a cysteine engineered antibody.

In an embodiment, the cysteine engineered anti-TYRP-1 antibody is TA99, chimeric TA99 or flanvotumab.

In an embodiment, the cysteine engineered anti-TYRP-1 antibody is flanvotumab.

In an embodiment, the cysteine engineered flanvotumab has the heavy chain sequence having a substitution selected from the group consisting of N299C and C222S.

In an embodiment, the cysteine engineered flanvotumab has the heavy chain sequence having the substitution N299C set forth in SEQ ID NO: 49.

In an embodiment, the cysteine engineered flanvotumab has the heavy chain sequence having the substitution C222S set forth in SEQ ID NO: 50.

In an embodiment, the cysteine engineered chimeric TA99 has the heavy chain sequence having the substitution N301C set forth in SEQ ID NO: 51.

In an embodiment, the cysteine engineered chimeric TA99 has the heavy chain sequence having the substitution C224S set forth in SEQ ID NO: 52.

In an embodiment, the antibody is an anti-PD-L1 antibody.

In an embodiment, the anti-PD-L1 antibody is avelumab.

In an embodiment, the antibody is an anti-PD-L2 antibody.

In an embodiment, the antibody is an anti-TIM-3 antibody.

In an embodiment, the antibody is an anti-MUC1 antibody.

In an embodiment, the antibody is an anti-CA6 antibody.

In an embodiment, the anti-CA6 antibody is DS6 or huDS6.

In an embodiment, Y is β-D-glucuronic acid; R₃ is the side chain of an α-amino acid, serine, threonine or tyrosine; m = 1; R₁ is selected from the group of side chains of α-amino acids, valine, phenylalanine, tyrosine, leusine, isoleusine, arginine, alanine, lysine and glycine; Z is absent; R_{X} is -CH₂CH₂-; and D is a cytotoxic drug.

In an embodiment, Y is β-D-glucuronic acid; R₃ is the side chain of an α-amino acid, serine, threonine or tyrosine; m = 1; R₁ is selected from the group of side chains of α-amino acids, valine, phenylalanine, tyrosine, leusine, isoleusine, arginine, alanine, lysine and glycine; Z is PABC; R_{X} is -CH₂CH₂-; and D is a cytotoxic drug.

In an embodiment, Y is β-D-glucuronic acid; R₃ is the side chain of serine; m = 1; R₁ is the side chain of valine; R_{X} is -CH₂CH₂-; Z is PABC; and D is a cytotoxic drug.

In an embodiment, Y is β-D-glucuronic acid; R₃ is the side chain of an α-amino acid, serine, threonine or tyrosine; m = 1; R₁ is selected from the group of side chains of α-amino acids, valine, phenylalanine, tyrosine, leusine, isoleusine, arginine, alanine, lysine and glycine; Z is PABC; Rx is -CH₂CH₂-; and D is an auristatin.

In an embodiment, Y is β-D-glucuronic acid; R₃ is the side chain of an α-amino acid, serine, threonine or tyrosine; m = 1; R₁ is selected from the group of side chains of α-amino acids, valine, phenylalanine, tyrosine, leusine, isoleusine, arginine, alanine, lysine and glycine; Z is PABC; Rx is -CH₂CH₂-; and D is a rubicin or a doxorubicin derivative.

In an embodiment, Y is β-D-glucuronic acid; R₃ is the side chain of an α-amino acid, serine, threonine or tyrosine; m = 1; R₁ is selected from the group of side chains of α-amino acids, valine, phenylalanine, tyrosine, leusine, isoleusine, arginine, alanine, lysine and glycine; Z is absent; Rx is -CH₂CH₂-; and D is a rubicin or a doxorubicin derivative.

In an embodiment, Y is a saccharide; R₃ is the side chain of serine; m = 1; R₁ is the side chain of valine; Z is PABC; Rx is -CH₂CH₂-; and D is a rubicin or a doxorubicin derivative.

In an embodiment, Y is a saccharide; R₃ is the side chain of serine; m = 1; R₁ is the side chain of valine; Z is absent; Rx is -CH₂CH₂-; and D is a rubicin or a doxorubicin derivative.

In an embodiment, Y is β-D-glucuronic acid; R₃ is the side chain of serine; m = 1; R₁ is the side chain of valine; Z is PABC; Rx is -CH₂CH₂-; and D is an auristatin.

In an embodiment, Y is β-D-glucose; R₃ is the side chain of serine; m = 1; R₁ is the side chain of valine; Z is PABC; Rx is -CH₂CH₂-; and D is a rubicin or a doxorubicin derivative.

In an embodiment, Y is β-D-glucose; R₃ is the side chain of serine; m = 1; R₁ is the side chain of valine; Z is absent; Rx is -CH₂CH₂-; and D is a rubicin or a doxorubicin derivative.

In an embodiment, Y is β-D-glucuronic acid; R₃ is the side chain of serine; m = 1; R₁ is the side chain of valine; Z is PABC; Rx is -CH₂CH₂-; and D is a rubicin or a doxorubicin derivative.

In an embodiment, Y is β-D-glucuronic acid; R₃ is the side chain of serine; m = 1; R₁ is the side chain of valine; Z is absent; Rx is -CH₂CH₂-; and D is a rubicin or a doxorubicin derivative.

In an embodiment,
(i) R₃ is selected from the group consisting of a side chain of an α-amino acid, serine, threonine and tyrosine,
(ii) Z is selected from the group consisting of para-aminobenzyloxycarbonyl (PABC); orto-aminobenzyloxycarbonyl; an amino acid; and a peptide; or Z is absent;
(iii) R₁ is selected from the group consisting of the side chain of valine, sthe ide chain of phenylalanine, the side chain of tyrosine, the side chain of leusine, the side chain of isoleusine, the side chain of arginine, the side chain of alanine, the side chain of lysine and the side chain of glycine; and/or
(iv) R_{X} is selected from the group consisting of a linear C₁-C₆ alkylene group; a branched C₁-C₆ alkylene group; CH(R₂), wherein R₂ is an amino acid side chain; and -CH₂CH₂-.

In an embodiment, the targeting unit-linker-payload conjugate is according to any one of the following Formulas TMa-TMz.

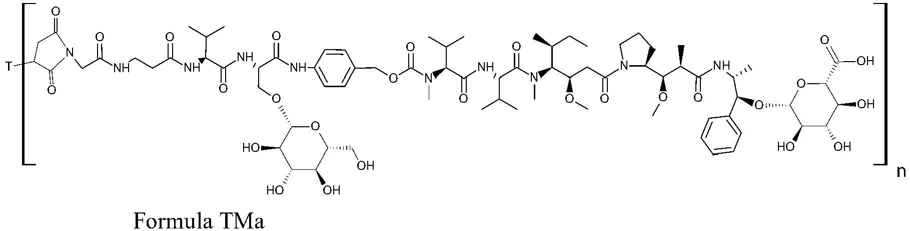

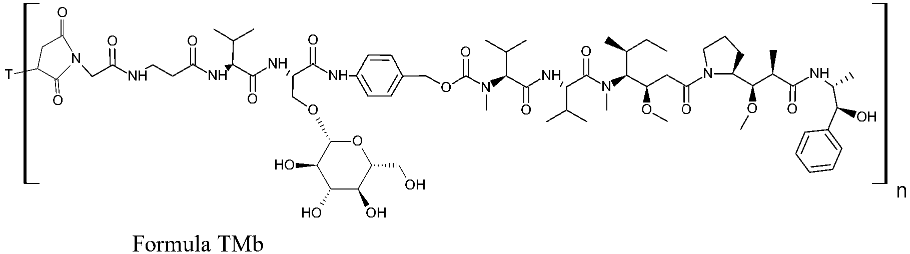

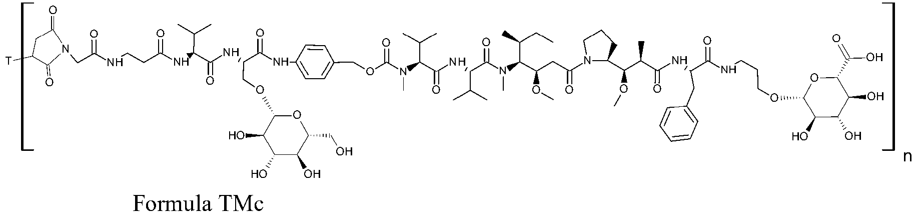

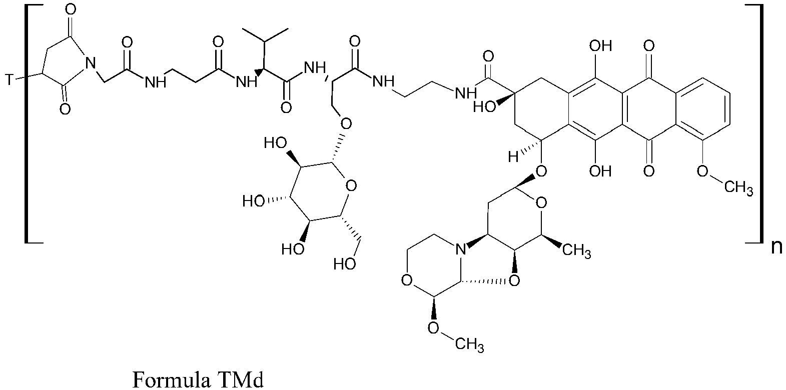

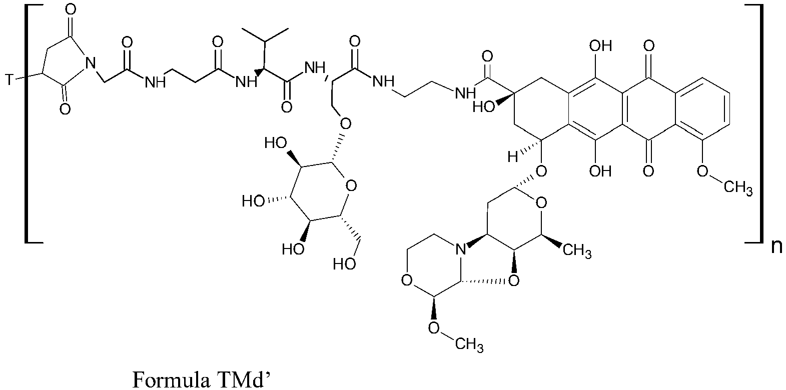

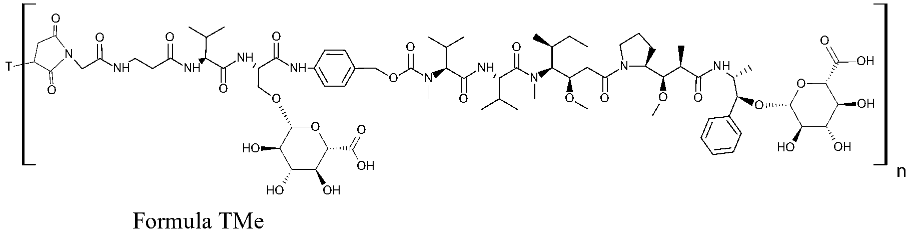

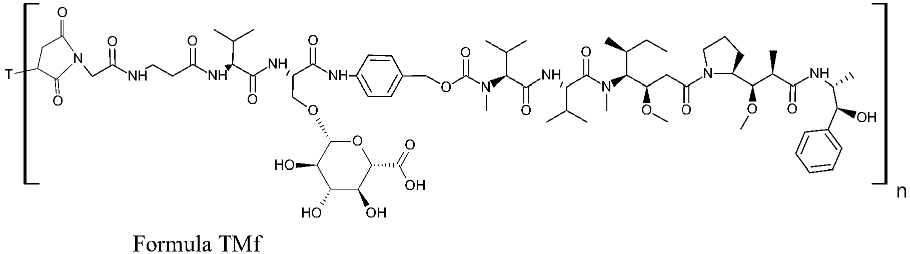

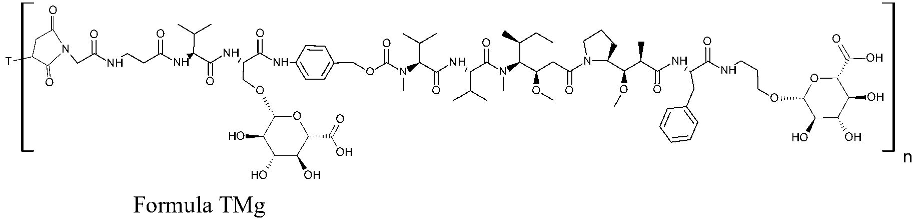

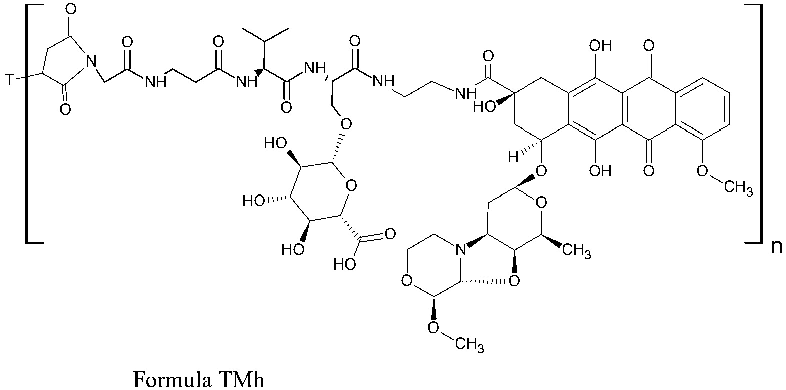

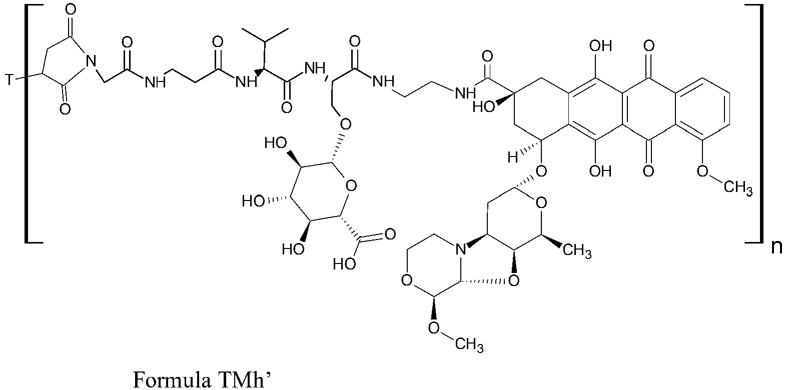

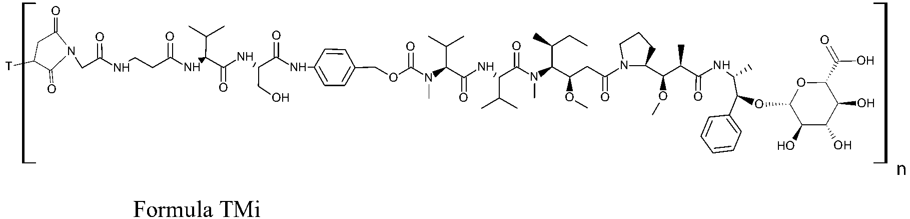

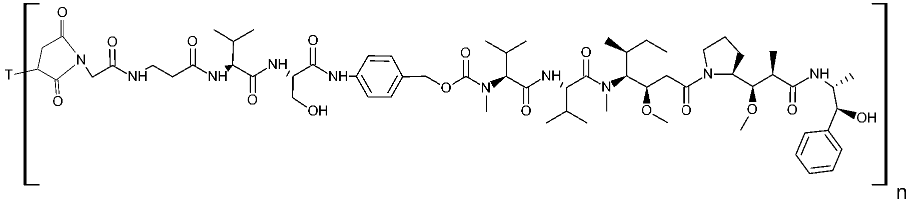

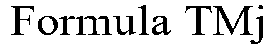

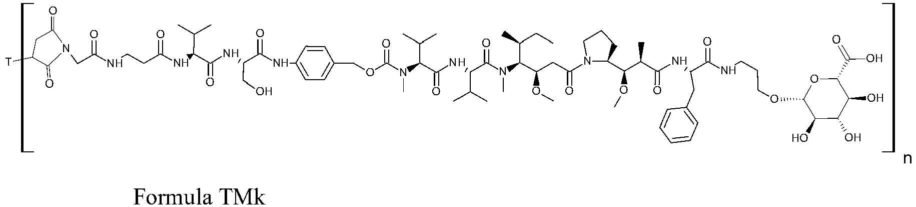

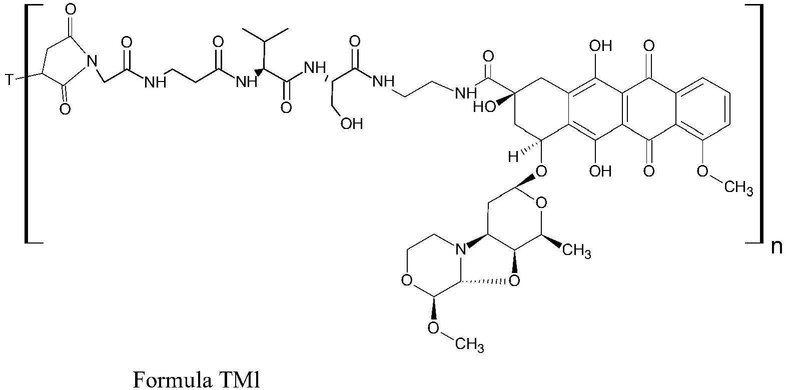

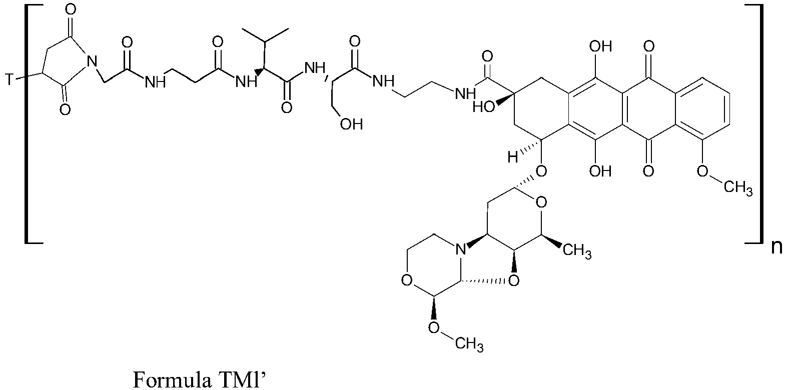

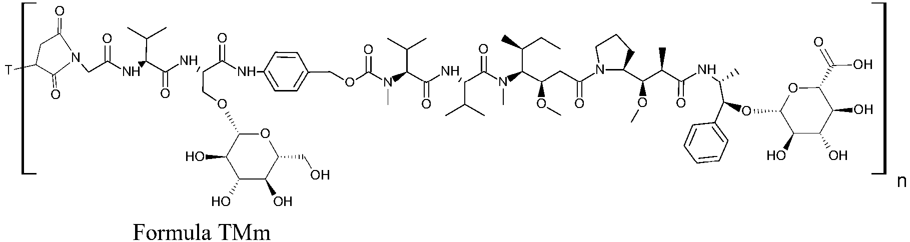

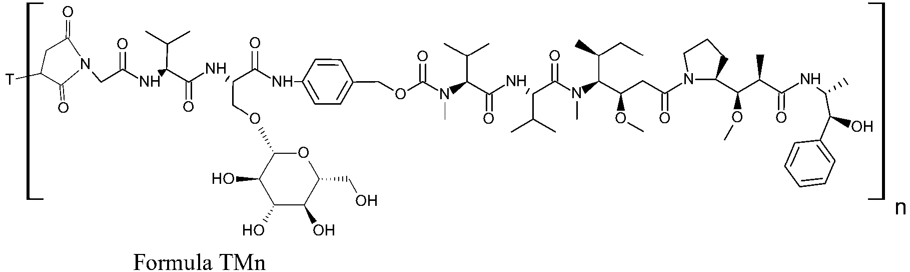

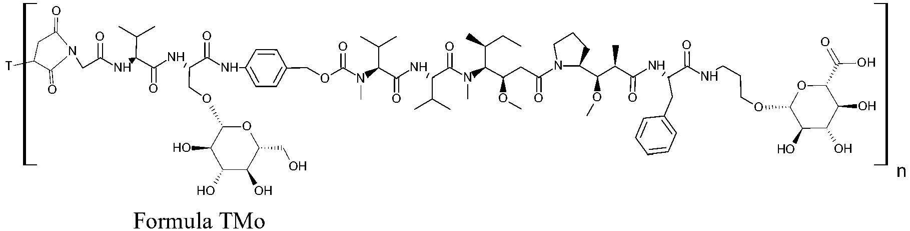

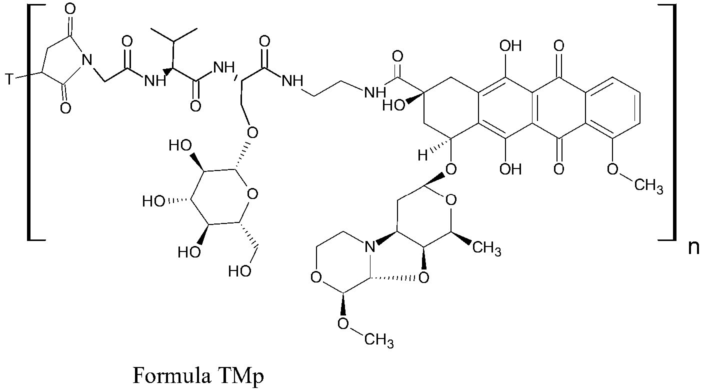

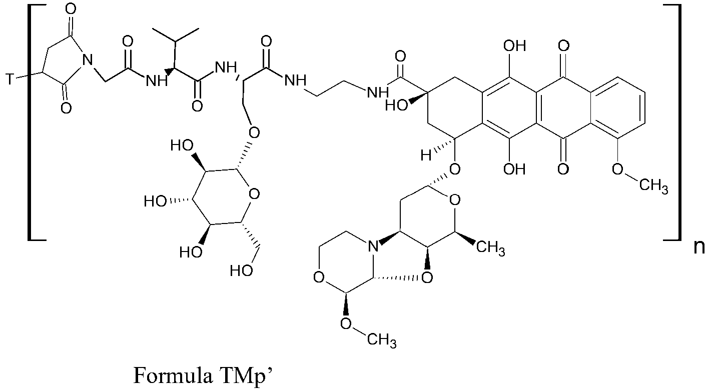

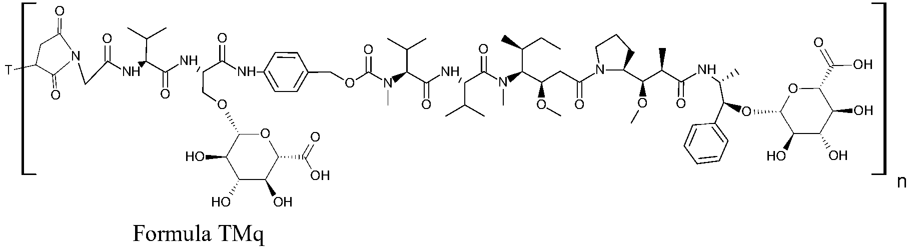

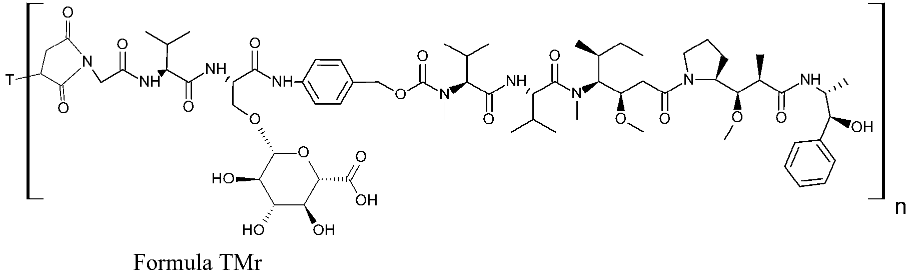

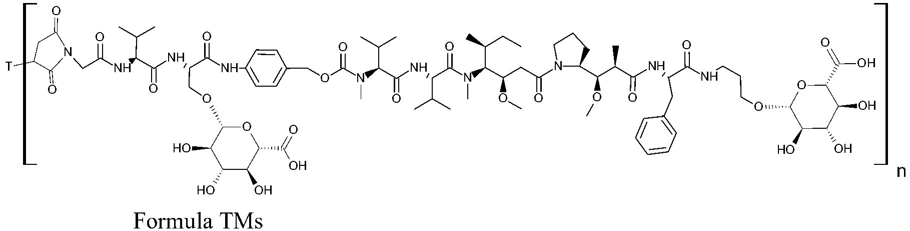

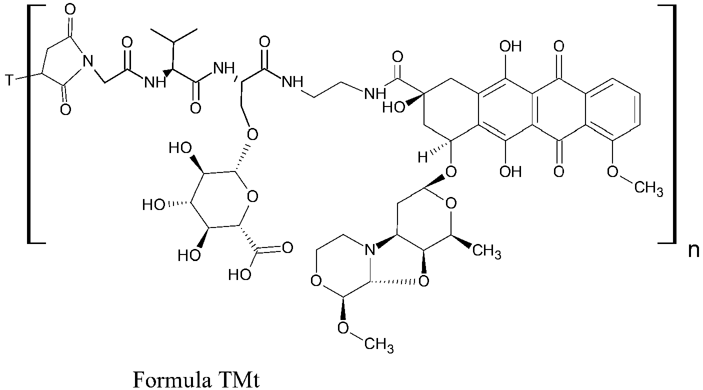

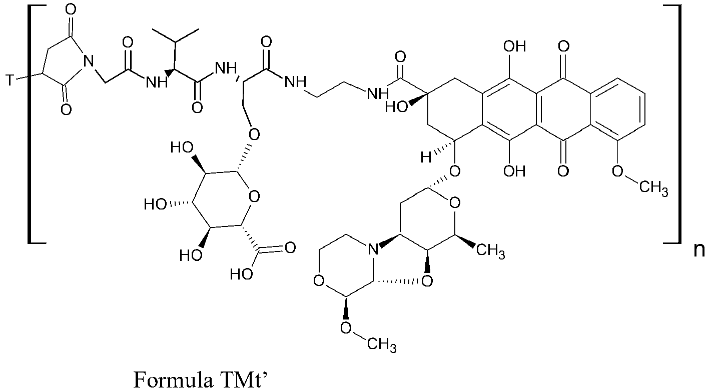

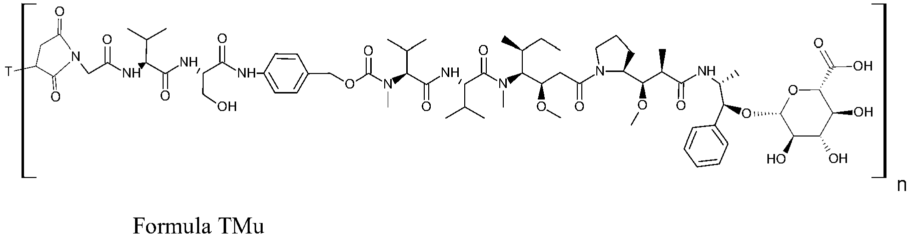

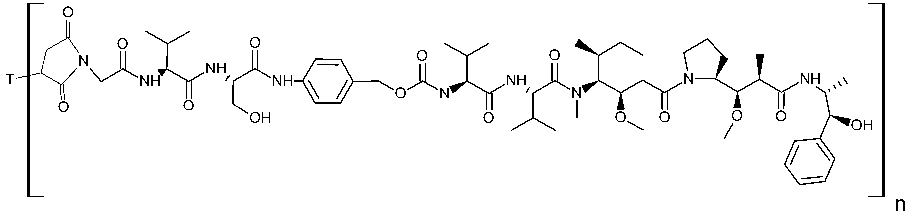

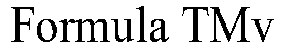

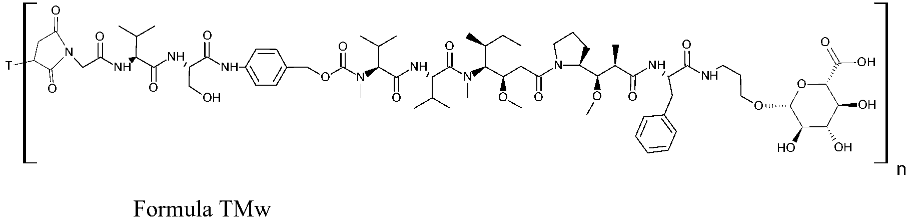

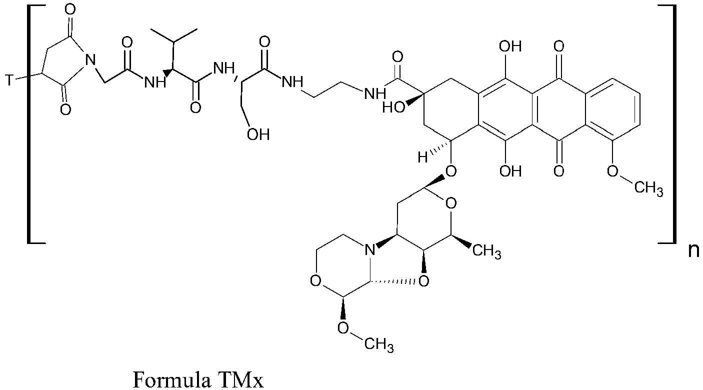

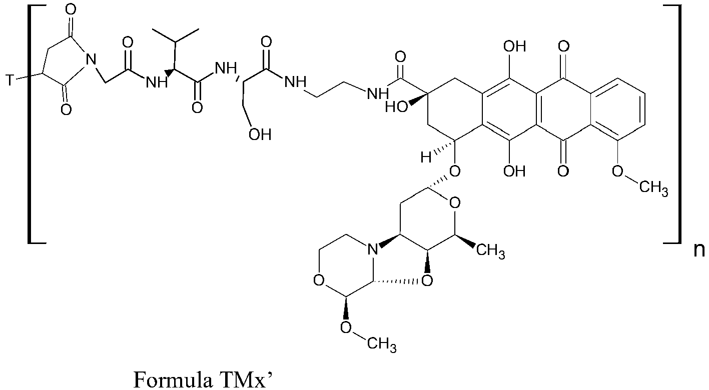

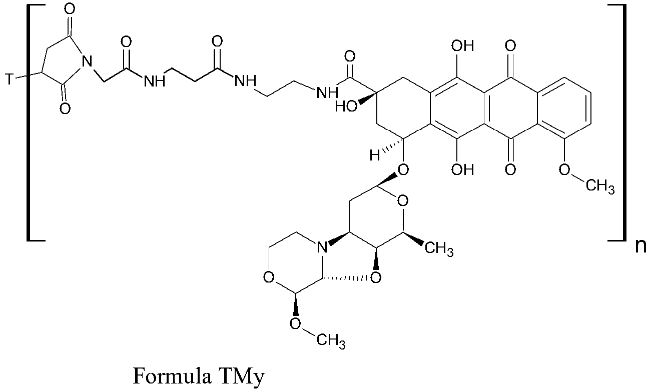

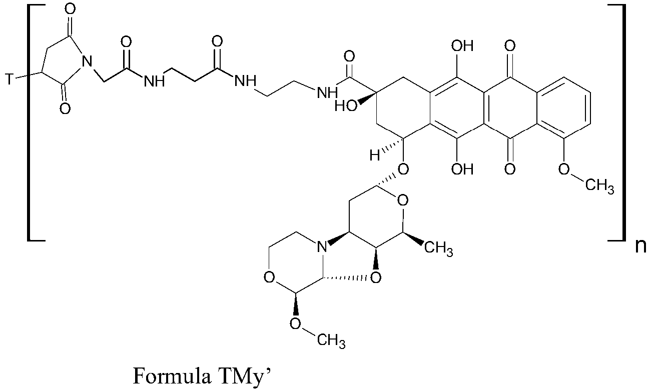

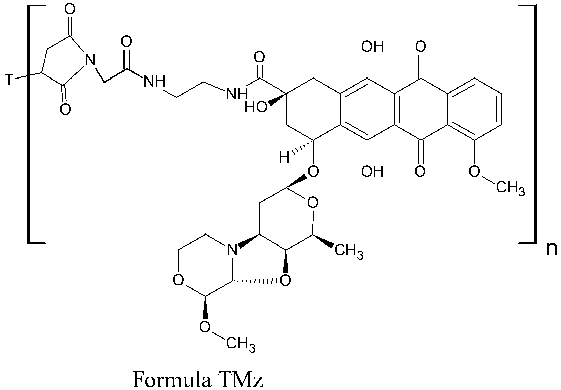

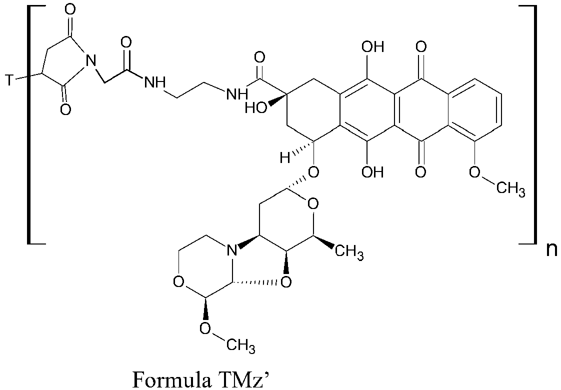

In an embodiment, the targeting unit-linker-payload conjugate is according to any one of the following Formulas TMsa-TMsz.

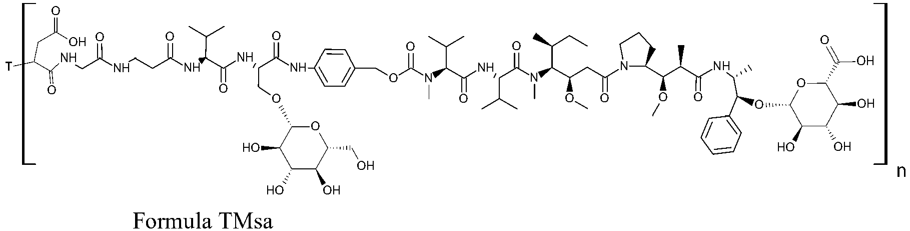

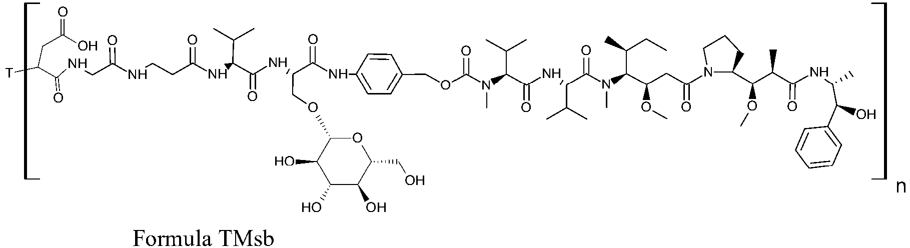

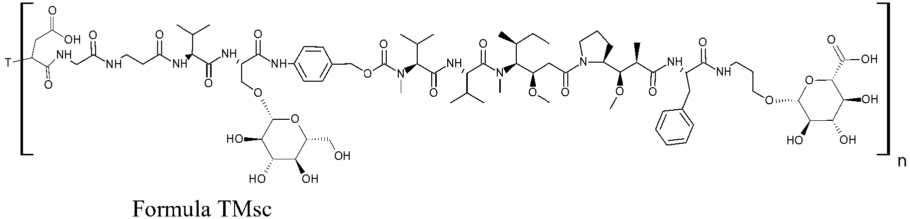

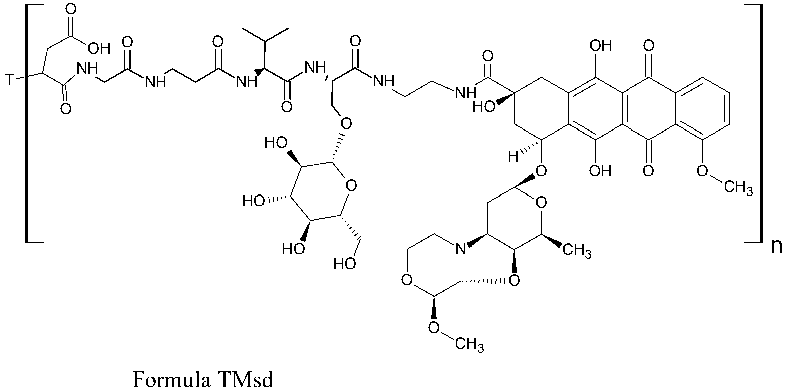

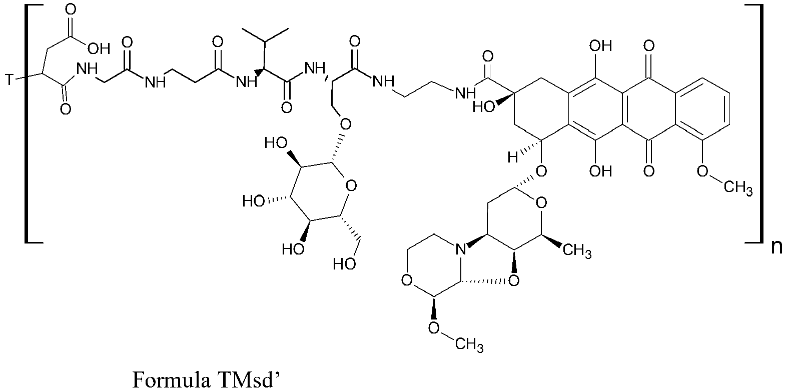

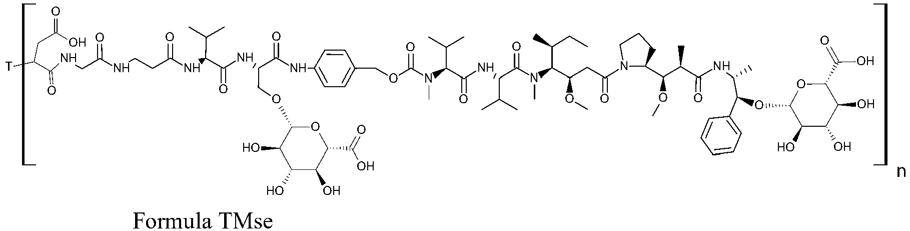

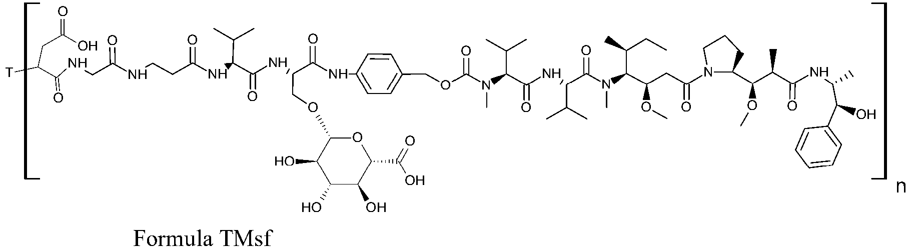

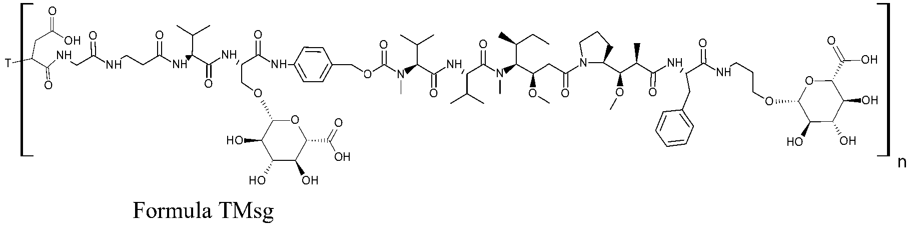

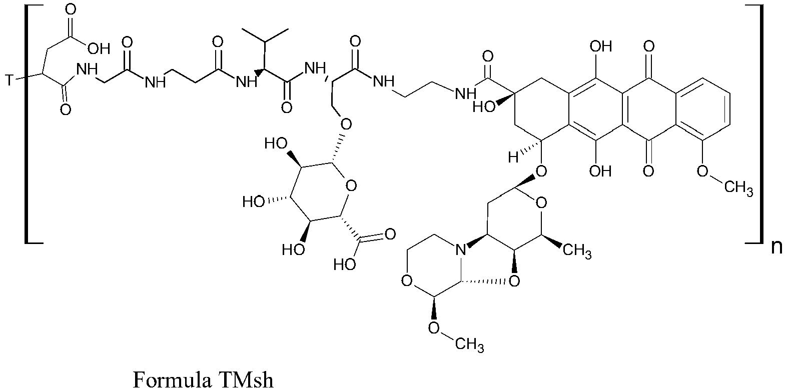

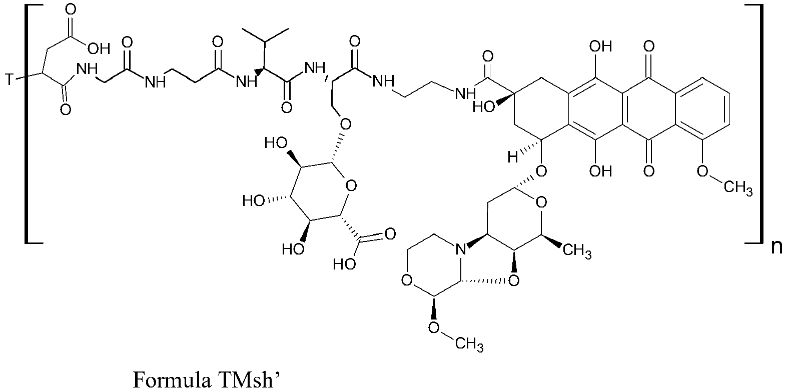

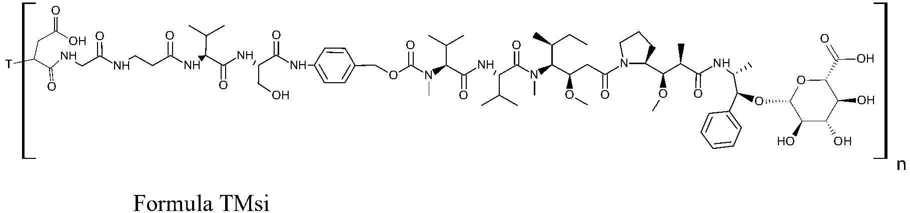

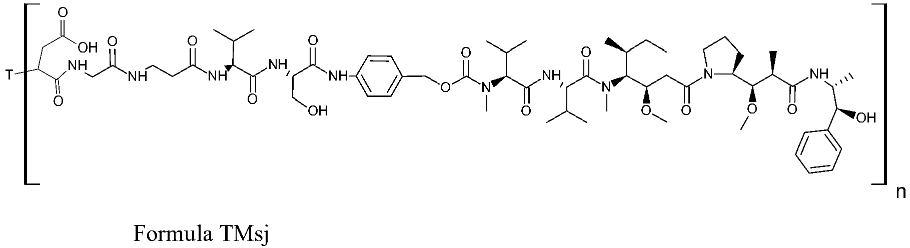

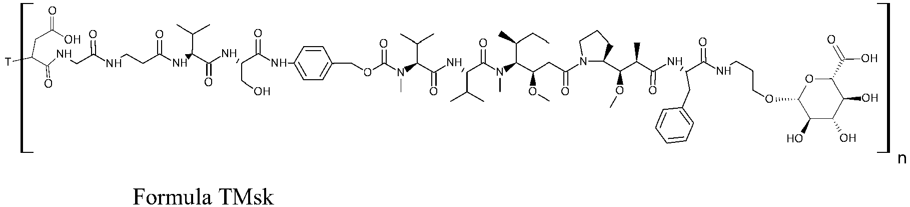

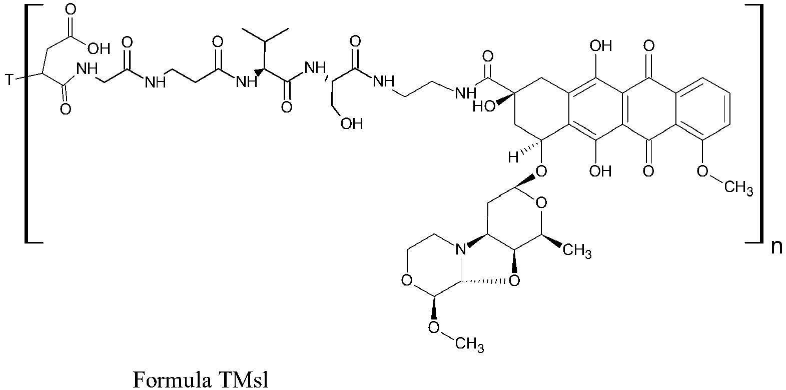

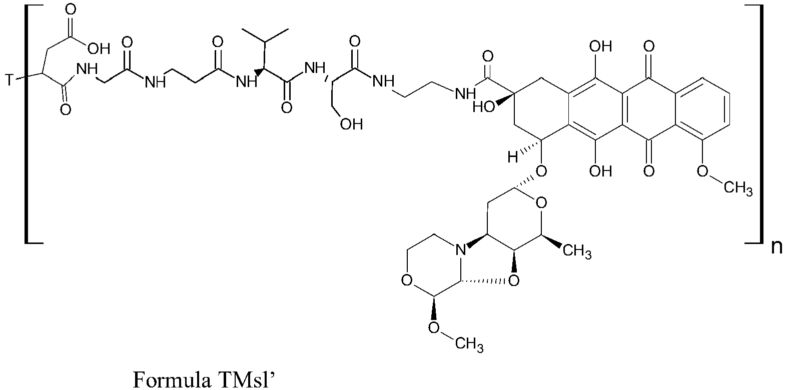

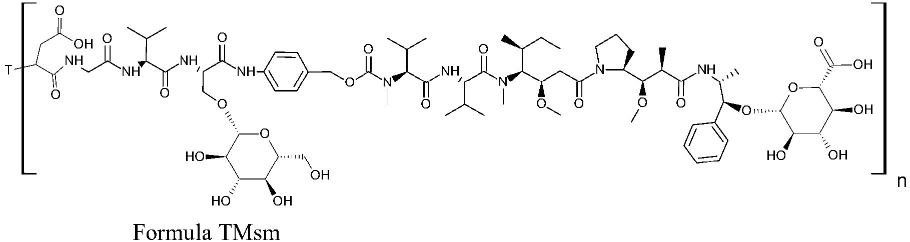

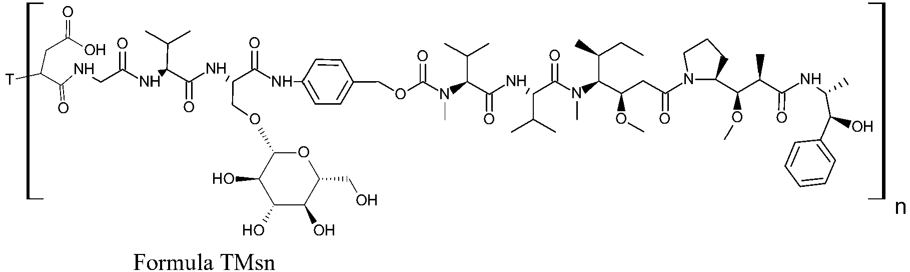

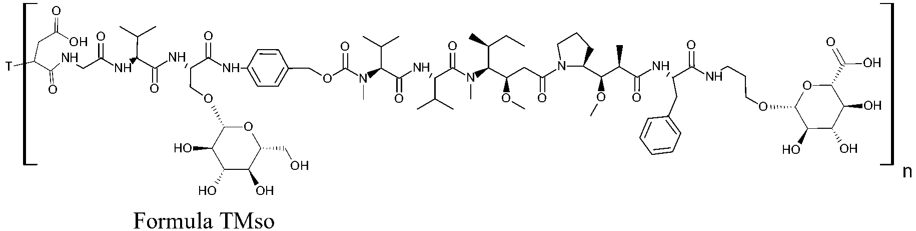

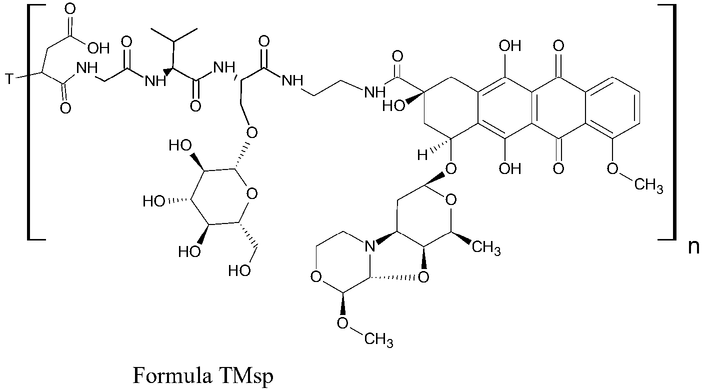

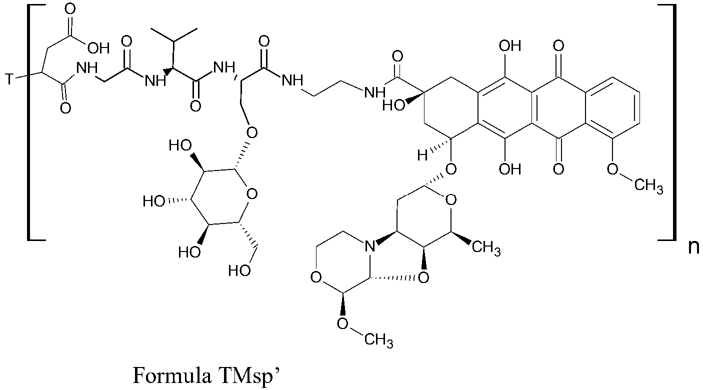

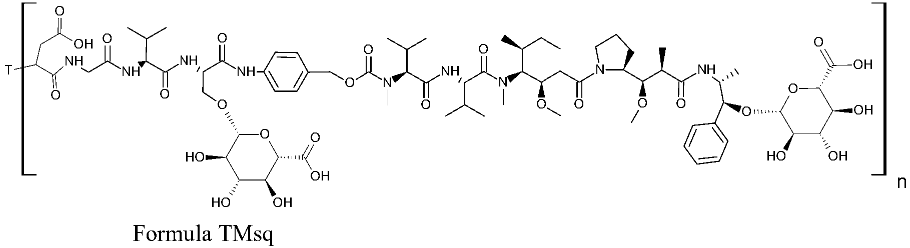

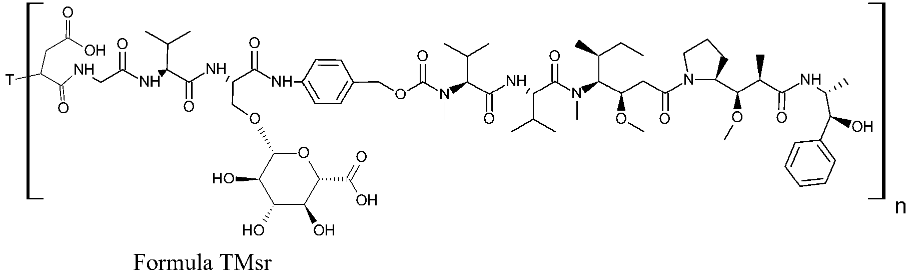

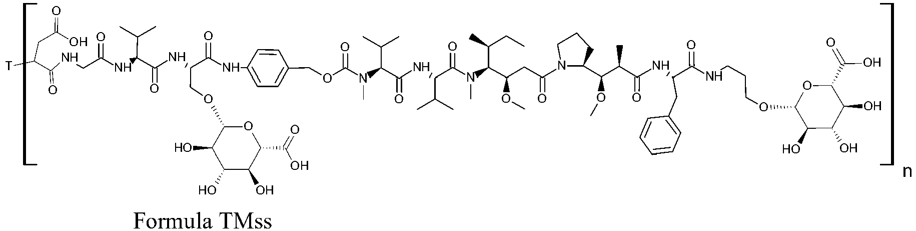

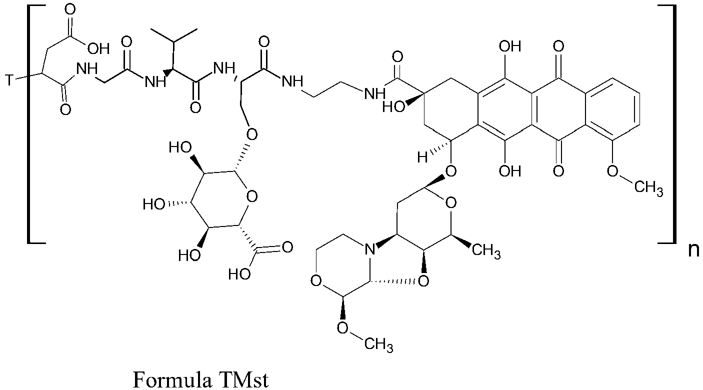

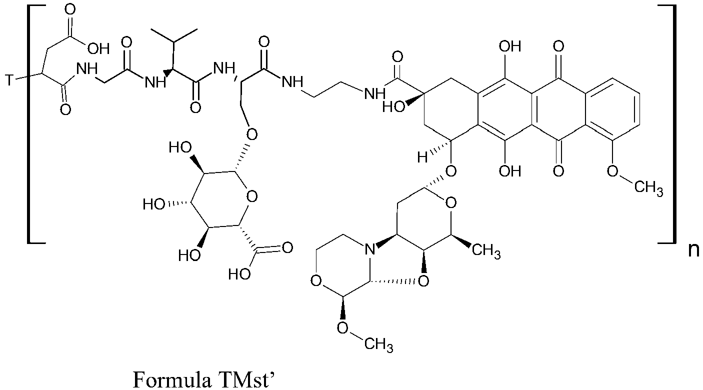

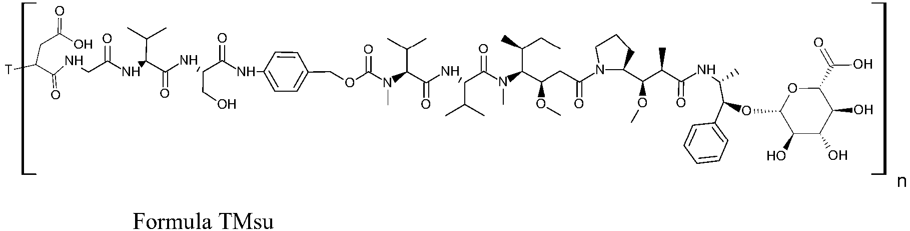

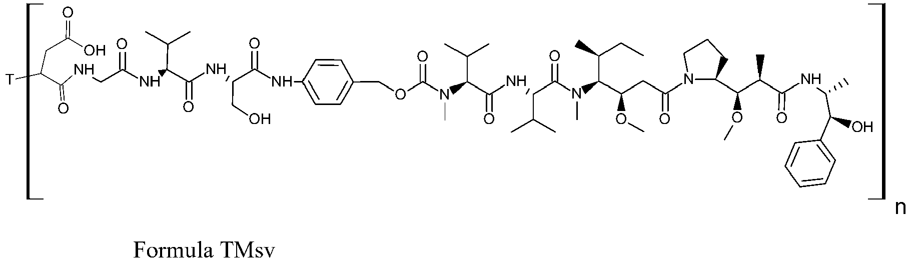

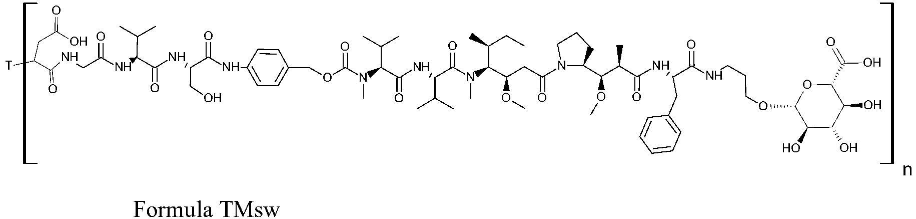

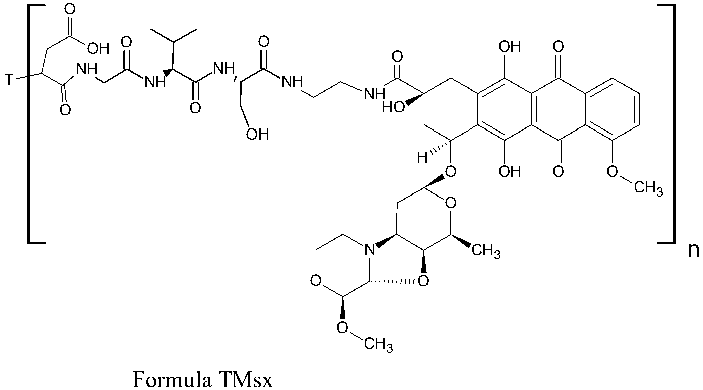

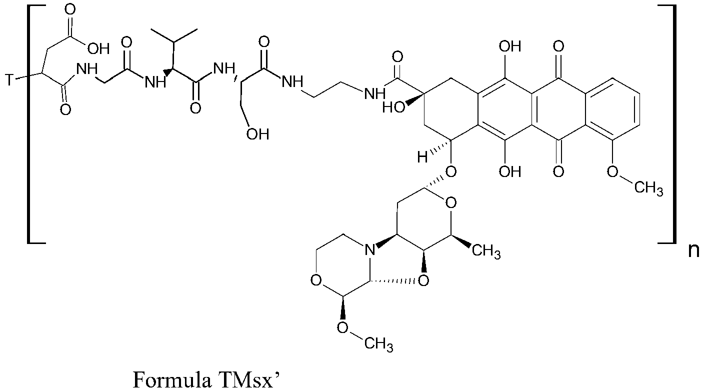

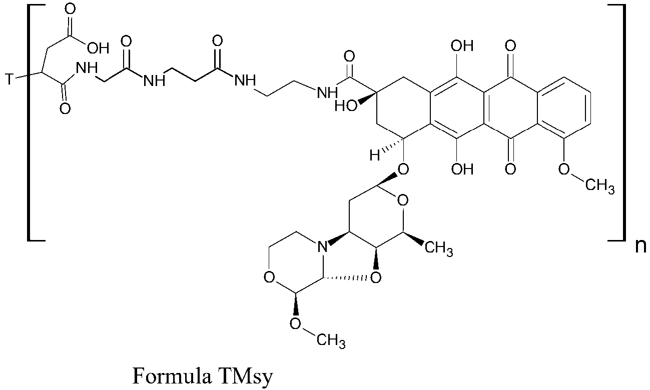

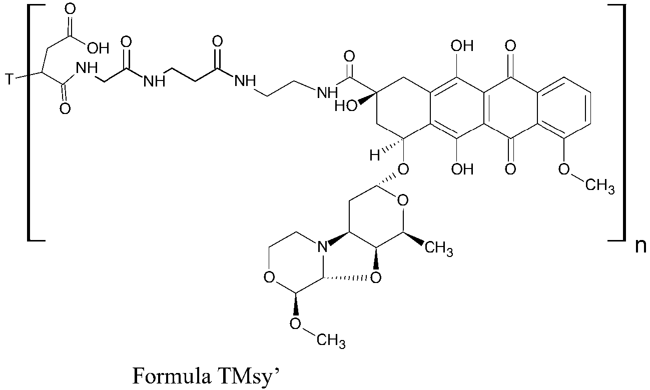

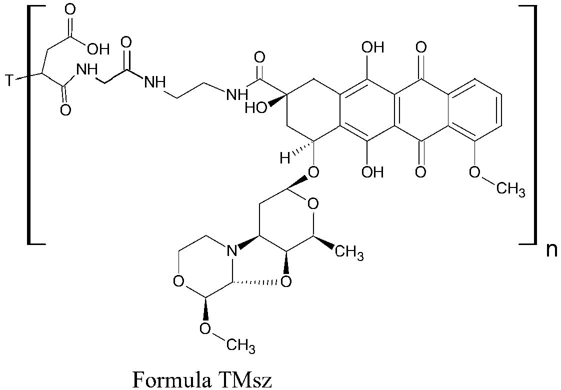

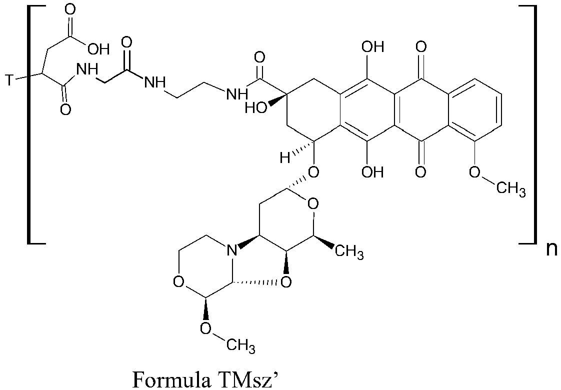

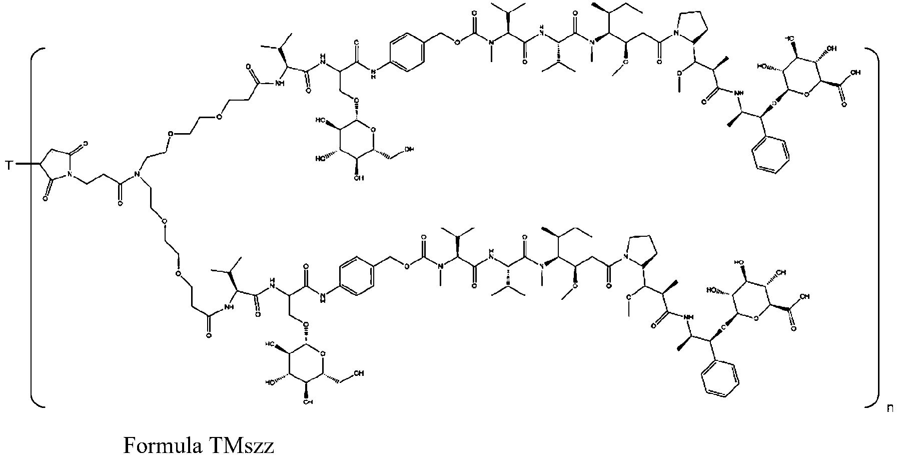

In an embodiment, the targeting unit-linker-payload conjugate is antibody-maleimidoacetyl-β-Ala-Val-Ser(Glc)-PAB-MMAU 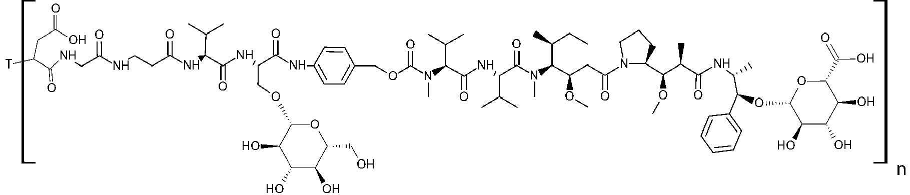 wherein T is an antibody. n may be any value or range of values of n described in this specification.

In an embodiment, the targeting unit-linker-payload conjugate is antibody-maleimidoacetyl-β-Ala-Val-Ser(Glc)-PAB-MMAU 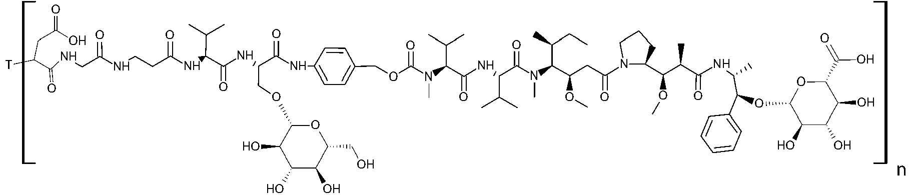 wherein T is an antibody and n is 8.
In an embodiment, the targeting unit-linker-payload conjugate is anti-HER2 antibody-maleimidoacetyl-β-Ala-Val-Ser(Glc)-PAB-MMAU 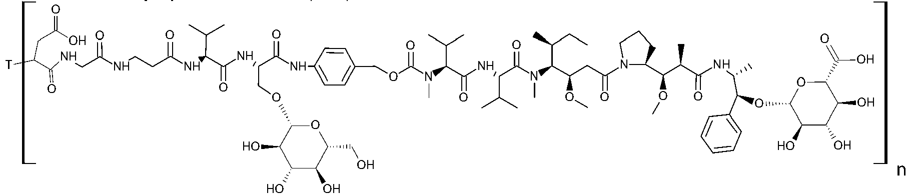 wherein T is an anti-HER2 antibody. In an embodiment, n is about 8.

In an embodiment, the targeting unit-linker-payload conjugate is trastuzumab-maleimidoacetyl-p-Ala-Val-Ser(Glc)-PAB-MMAU 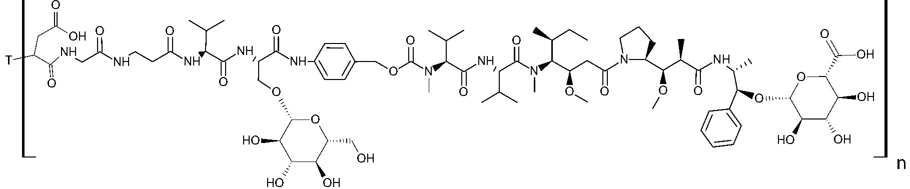 wherein T is trastuzumab. In an embodiment, n is about 8.

In an embodiment, the targeting unit-linker-payload conjugate is anti-HER2 antibody-maleimidoacetyl-β-Ala-Val-Ser(GlcA)-PAB-MMAU 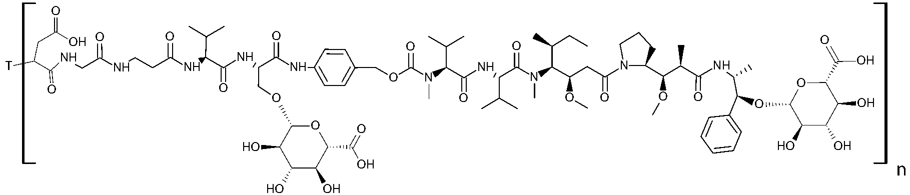 wherein T is an anti-HER2 antibody. In an embodiment, n is about 8.

In an embodiment, the targeting unit-linker-payload conjugate is trastuzumab-maleimidoacetyl-β-Ala-Val-Ser(GlcA)-PAB-MMAU 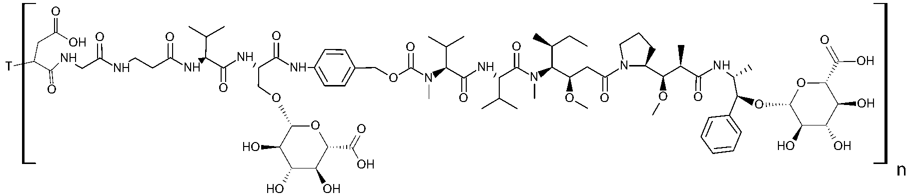 wherein T is trastuzumab. In an embodiment, n is about 8.

In an embodiment, the targeting unit-linker-payload conjugate is anti-CD33 antibody-maleimidoacetyl-p-Ala-Val-Ser(Glc)-PAB-MMAU 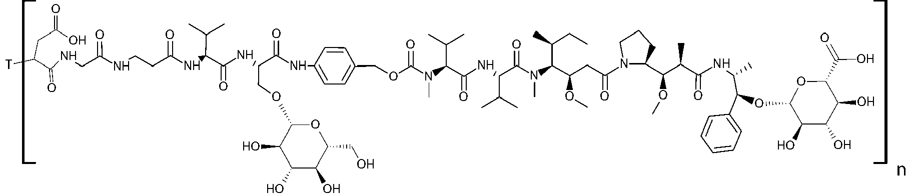 wherein T is an anti-CD33 antibody. In an embodiment, n is 6, 7 or 8.

In an embodiment, the targeting unit-linker-payload conjugate is lintuzumab-maleimidoacetyl-β-Ala-Val-Ser(Glc)-PAB-MMAU 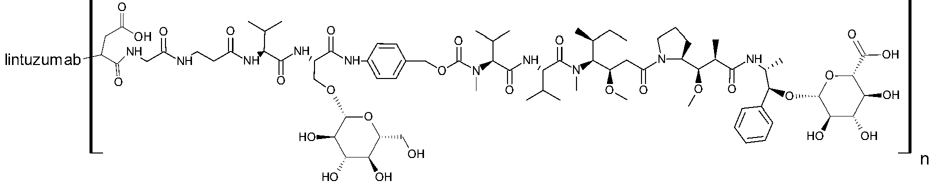 wherein n is 8.

In an embodiment, the targeting unit-linker-payload conjugate is gemtuzumab-maleimidoacetyl-β-Ala-Val-Ser(Glc)-PAB-MMAU 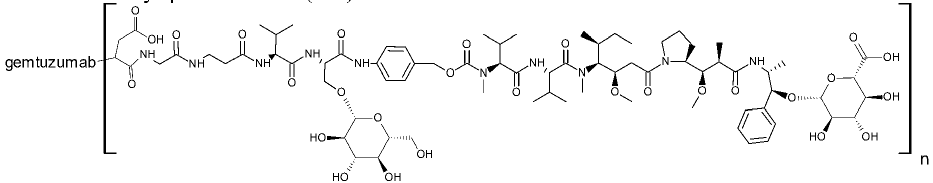 wherein n is 8.

In an embodiment, the targeting unit-linker-payload conjugate is anti-TYRP1 antibody-maleimidoacetyl-β-Ala-Val-Ser(Glc)-PAB-MMAU 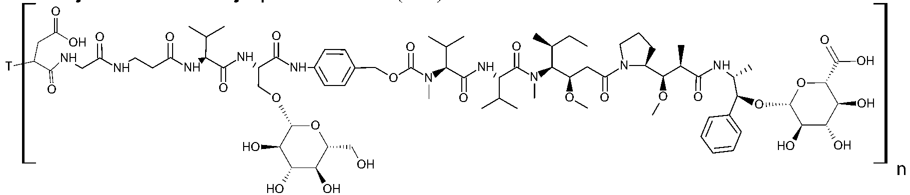 wherein T is an anti-TYRP1 antibody. In an embodiment, n is 6, 7 or 8. In an embodiment, n is 8.

In an embodiment, the targeting unit-linker-payload conjugate is flanvotumab-maleimidoacetyl-β-Ala-Val-Ser(Glc)-PAB-MMAU 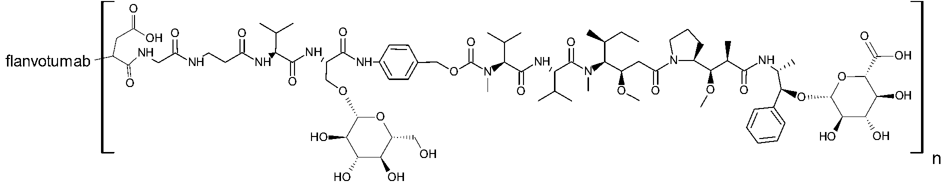 wherein n is 6, 7 or 8. In an embodiment, n is 8.

In an embodiment, the targeting unit-linker-payload conjugate is anti-CD22 antibody-maleimidoacetyl-β-Ala-Val-Ser(Glc)-PAB-MMAU 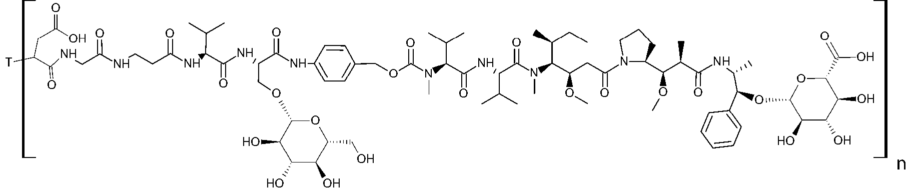 wherein T is an anti-CD22 antibody. In an embodiment, n is 6, 7 or 8. In an embodiment, n is 8.

In an embodiment, the targeting unit-linker-payload conjugate is epratuzumab-maleimidoacetyl-p-Ala-Val-Ser(Glc)-PAB-MMAU 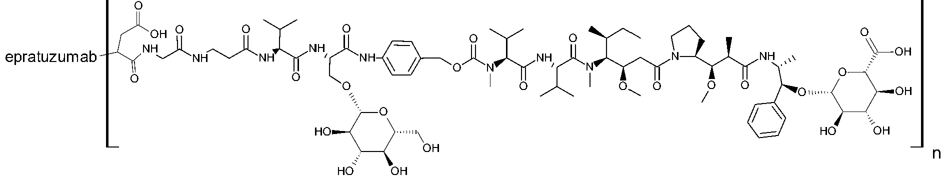 wherein n is 6, 7 or 8. In an embodiment, n is 8.

In an embodiment, the targeting unit-linker-payload conjugate is anti-CD19 antibody-maleimidoacetyl-β-Ala-Val-Ser(Glc)-PAB-MMAU 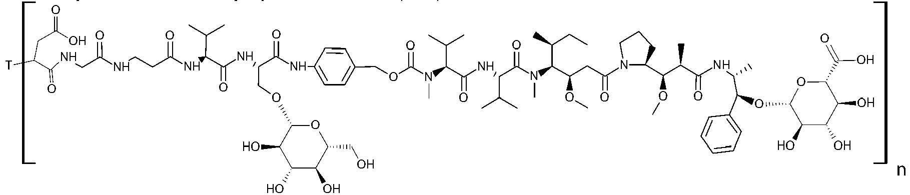 wherein T is an anti-CD19 antibody. In an embodiment, n is 6, 7 or 8. In an embodiment, n is 8.

In an embodiment, the targeting unit-linker-payload conjugate is coltuximab-maleimidoacetyl-p-Ala-Val-Ser(Glc)-PAB-MMAU 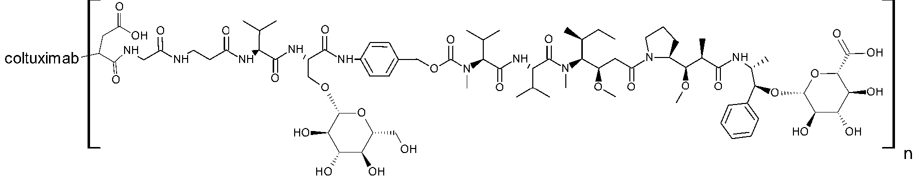 wherein n is 6, 7 or 8. In an embodiment, n is 8.

In an embodiment, the targeting unit-linker-payload conjugate is denintuzumab-maleimidoacetyl-6-Ala-Val-Ser(Glc)-PAB-MMAU 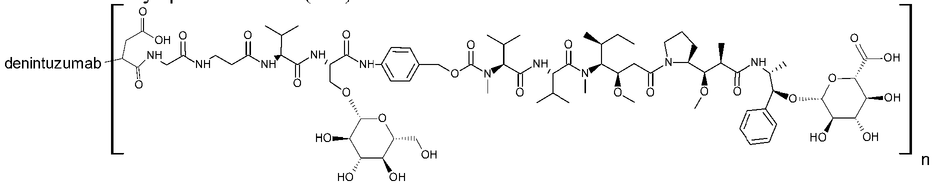 wherein n is 6, 7 or 8. In an embodiment, n is 8.

In an embodiment, the targeting unit-linker-payload conjugate is loncastuximab-maleimidoacetyl-β-Ala-Val-Ser(Glc)-PAB-MMAU 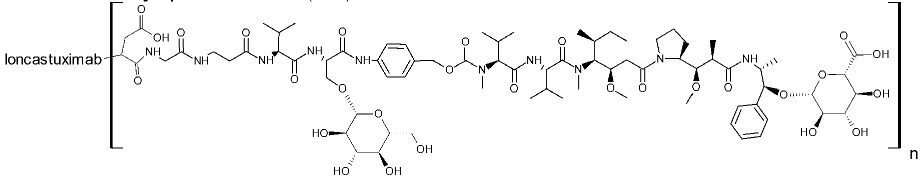 wherein n is 6, 7 or 8. In an embodiment, n is 8.

In an embodiment, the targeting unit-linker-payload conjugate is anti-CD52 antibody-maleimidoacetyl-β-Ala-Val-Ser(Glc)-PAB-MMAU 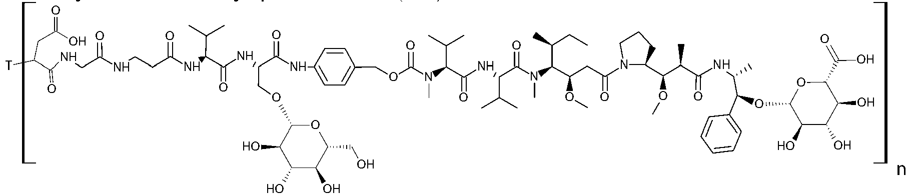 wherein T is an anti-CD52 antibody. In an embodiment, n is 6, 7 or 8. In an embodiment, n is 8.

In an embodiment, the targeting unit-linker-payload conjugate is alemtuzumab-maleimidoacetyl-β-Ala-Val-Ser(Glc)-PAB-MMAU 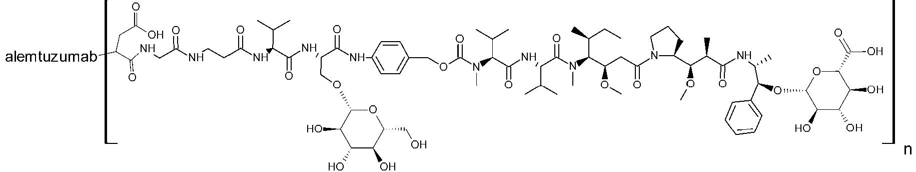 wherein n is 6, 7 or 8. In an embodiment, n is 8.

In an embodiment, the targeting unit-linker-payload conjugate is of Formula TMsz 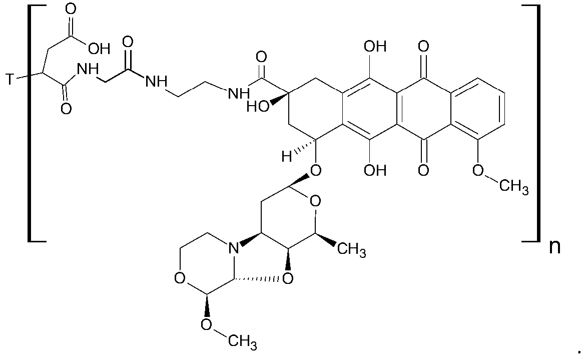 wherein T is an anti-TYRP1 antibody or a cysteine engineered anti-TYRP1 antibody. In an embodiment, n is 2, 3 or 4. In an embodiment, n is 2.

In an embodiment, the targeting unit-linker-payload conjugate is of Formula TMsz' 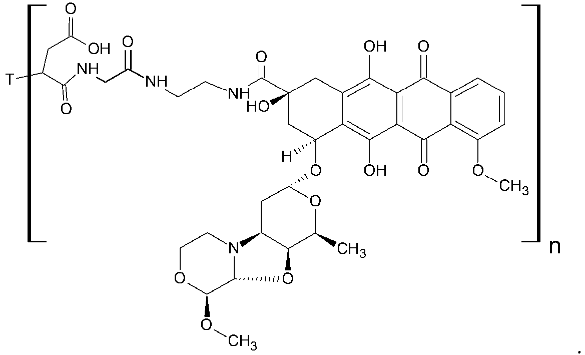 wherein T is an anti-TYRP1 antibody or a cysteine engineered anti-TYRP1 antibody. In an embodiment, n is 2, 3 or 4. In an embodiment, n is 2.

In an embodiment, the targeting unit-linker-payload conjugate is of Formula TMsz 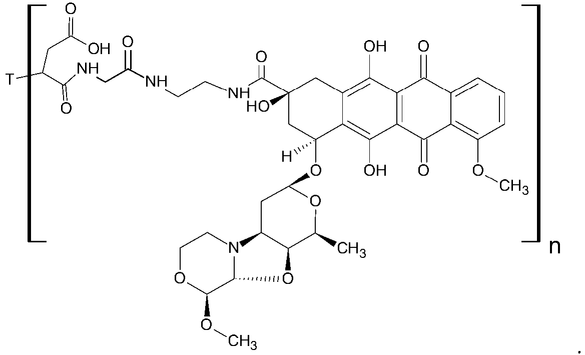 wherein T is a flanvotumab or a cysteine engineered flanvotumab. In an embodiment, n is 2, 3 or 4. In an embodiment, n is 2.

In an embodiment, the targeting unit-linker-payload conjugate is of Formula TMsz' 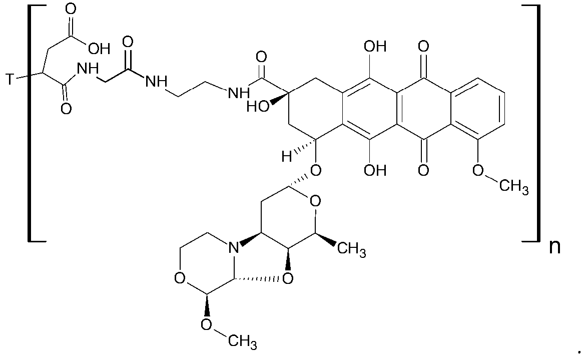 wherein T is a flanvotumab or a cysteine engineered flanvotumab. In an embodiment, n is 2, 3 or 4. In an embodiment, n is 2.

In an embodiment, the targeting unit-linker-payload conjugate is 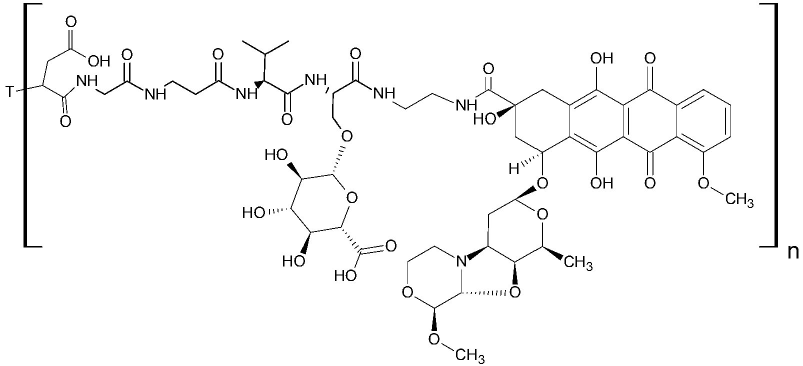 wherein T is the cysteine engineered antibody chimeric TA99 having HC N301C. n may be any value or range of values of n described in this specification.

In an embodiment, the targeting unit-linker-payload conjugate is 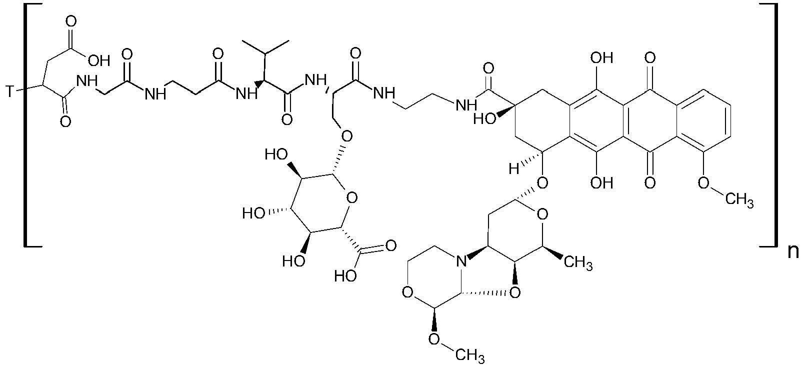 wherein T is the cysteine engineered antibody chimeric TA99 having HC N301C. n may be any value or range of values of n described in this specification.

In the context of this specification, the phrase "HC N301C" may be understood as an antibody having the substitution N301C in the heavy chain sequence.

In an embodiment the targeting unit-linker-payload conjugate is 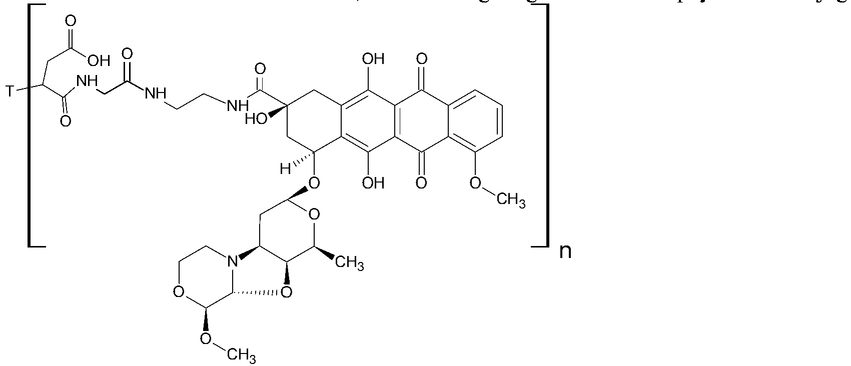 wherein T is lintuzumab HC N296C. n may be any value or range of values of n described in this specification.

In an embodiment, the targeting unit-linker-payload conjugate is 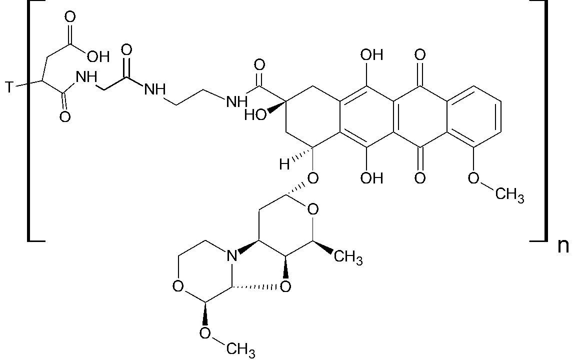 wherein T is lintuzumab HC N296C. n may be any value or range of values of n described in this specification.

In an embodiment, the targeting unit-linker-payload conjugate is 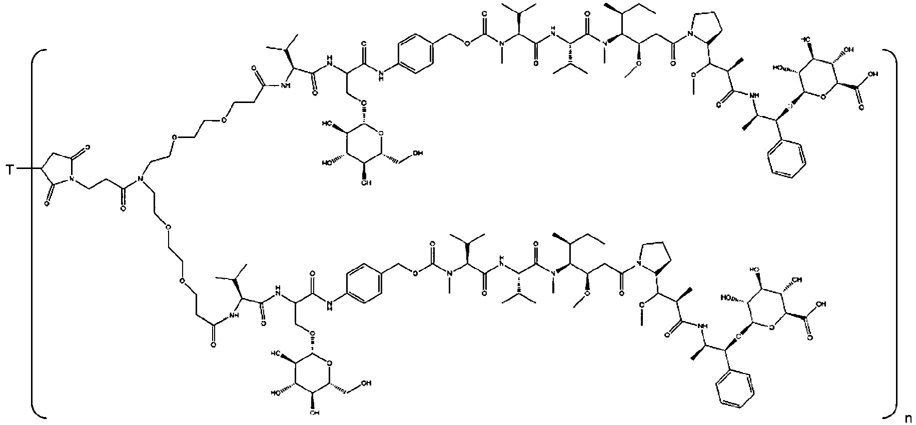 wherein T is lintuzumab HC N296C. n may be any value or range of values of n described in this specification.

In an embodiment, the targeting unit-linker-payload conjugate is cysteine engineered antibody-maleimidoacetyl-β-Ala-Val-Ser(Glc)-PAB-MMAU 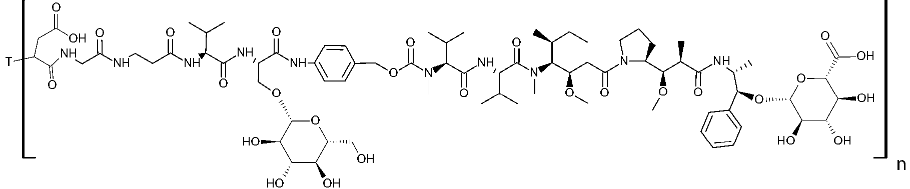 wherein T is a cysteine engineered antibody and n is 2, 3, 4, 5, 6, 7 or 8.

In an embodiment, the targeting unit-linker-payload conjugate is cysteine engineered antibody-maleimidoacetyl-β-Ala-Val-Ser(Glc)-PAB-MMAU 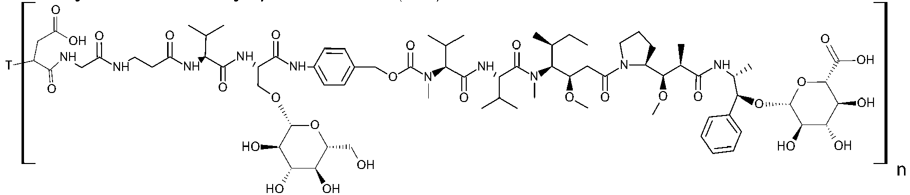 wherein T is a cysteine engineered antibody having heavy chain substitution in A40, P41, A84, V89, S112, S113, A114, S115, T116, G118, V152, S153, N155, A168, Q171, C220, 225, 226, 229, 247, V278, N297, 339, S371, 375, 376, S396, or E400, and/or light chain substitution in V110, S114, S121, S127, 143, 147, A153, 159, 163, 165, S168, V205, or 214 (according to Kabat). n may be any value or range of values of n described in this specification.

In an embodiment, the targeting unit-linker-payload conjugate is cysteine engineered antibody-maleimidoacetyl-β-Ala-Val-Ser(Glc)-PAB-MMAU 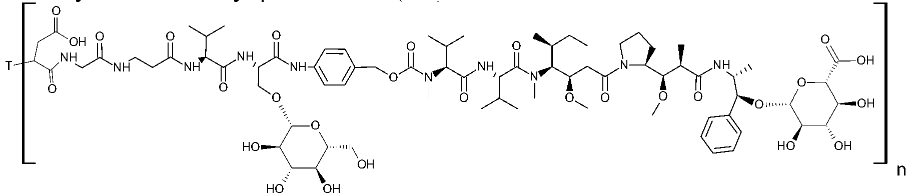 wherein T is a cysteine engineered antibody having heavy chain substitution in 220 or 297 (according to Kabat). n may be any value or range of values of n described in this specification.

In an embodiment, the targeting unit-linker-payload conjugate is cysteine engineered antibody-maleimidoacetyl-β-Ala-Val-Ser(Glc)-PAB-MMAU 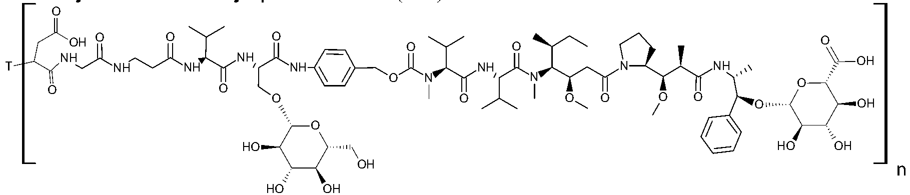 wherein T is a cysteine engineered anti-TYRP1 or anti-CD33 antibody. n may be any value or range of values of n described in this specification.

In an embodiment, the targeting unit-linker-payload conjugate is cysteine engineered antibody-maleimidoacetyl-β-Ala-Val-Ser(Glc)-PAB-MMAU 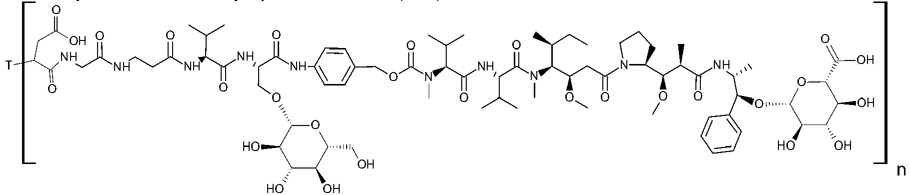 wherein T is a cysteine engineered anti-TYRP1 or anti-CD33 antibody having HC substitution in 220 or 297 (according to Kabat). n may be any value or range of values of n described in this specification.

In an embodiment, the targeting unit-linker-payload conjugate is cysteine engineered antibody-maleimidoacetyl-β-Ala-Val-Ser(Glc)-PAB-MMAU 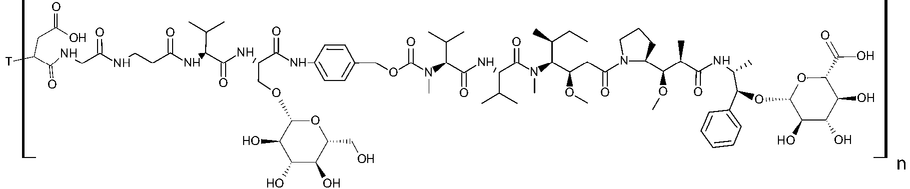 wherein T is a cysteine engineered lintuzumab, flanvotumab or gemtuzumab. n may be any value or range of values of n described in this specification.

In an embodiment, the targeting unit-linker-payload conjugate is cysteine engineered antibody-maleimidoacetyl-β-Ala-Val-Ser(Glc)-PAB-MMAU 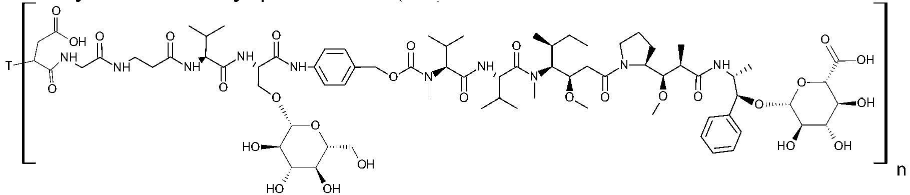 wherein T is a cysteine engineered lintuzumab, flanvotumab or gemtuzumab having HC substitution in 220 or 297 (according to Kabat). n may be any value or range of values of n described in this specification.

In an embodiment, the targeting unit-linker-payload conjugate is cysteine engineered lintuzumab-maleimidoacetyl-β-Ala-Val-Ser(Glc)-PAB-MMAU 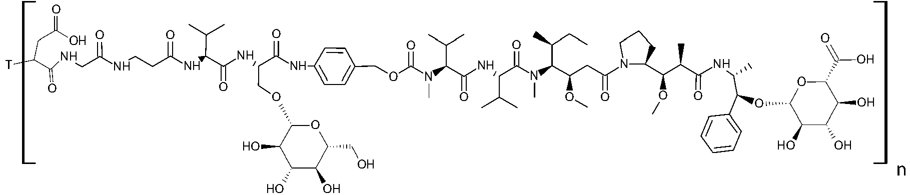 wherein T is a cysteine engineered lintuzumab having HC substitution N296C. n may be any value or range of values of n described in this specification.

In an embodiment, the targeting unit is an antibody and a bioorthogonal linking group connects the linker to an amino acid side chain of the antibody with a covalent bond.

In an embodiment, the bioorthogonal linking group is an alkyne selected from the group of aliphatic alkyne such as a propargyl group or a cycloalkyne such as DBCO, DIBO, cyclononyne, cyclooctyne, and the like.

In an embodiment, the linker is or comprises β-Ala-Val-Ser(Glc). In an embodiment, the linker is according to Formula IG, Formula IIG or Formula IIGs, wherein m is 1 and n is 0.

In an embodiment, the linker is or comprises β-Ala-Val-Ser(Glc)-PAB. In an embodiment, the linker is according to Formula IGX, Formula IIGX or Formula IIGXs.

In an embodiment, the linker-payload conjugate is according to any one of the following Formulas CBa-CBj.

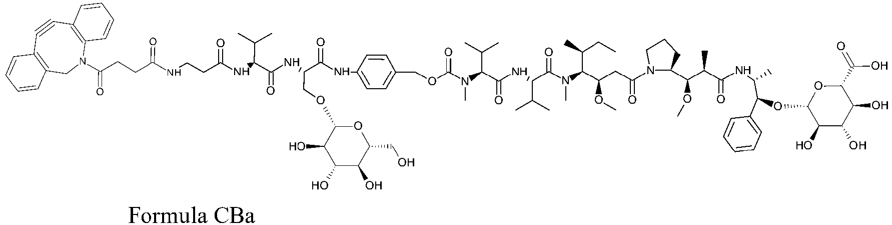

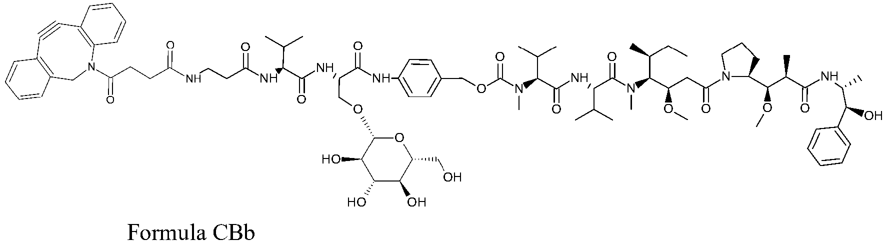

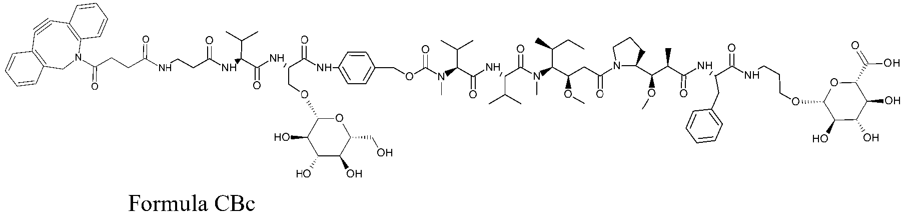

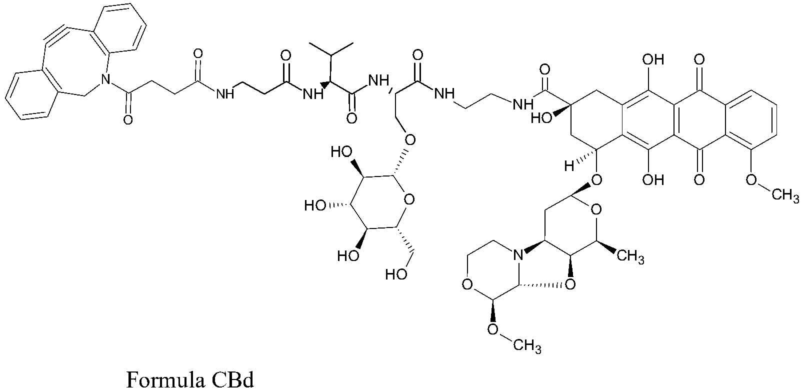

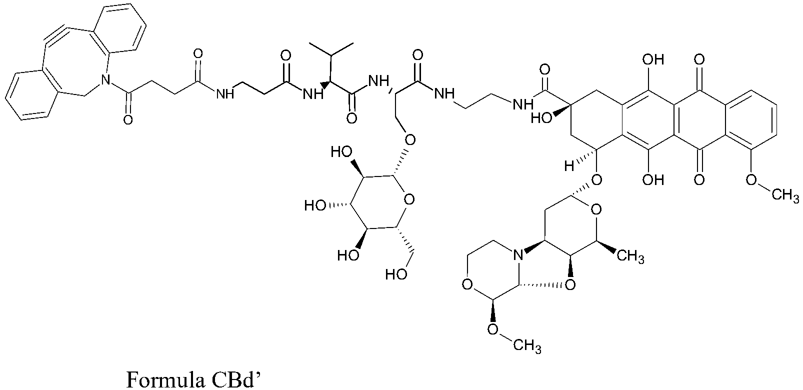

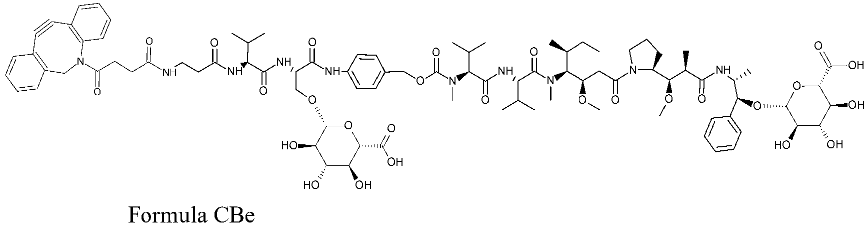

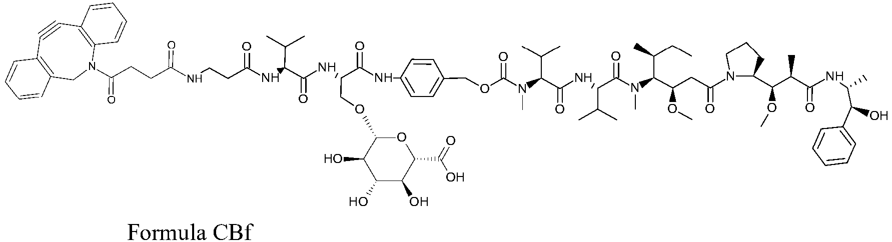

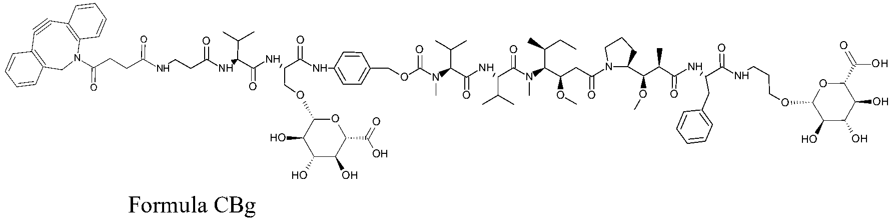

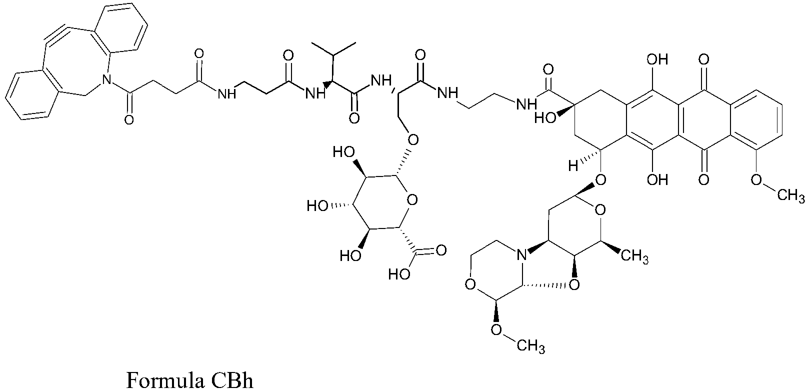

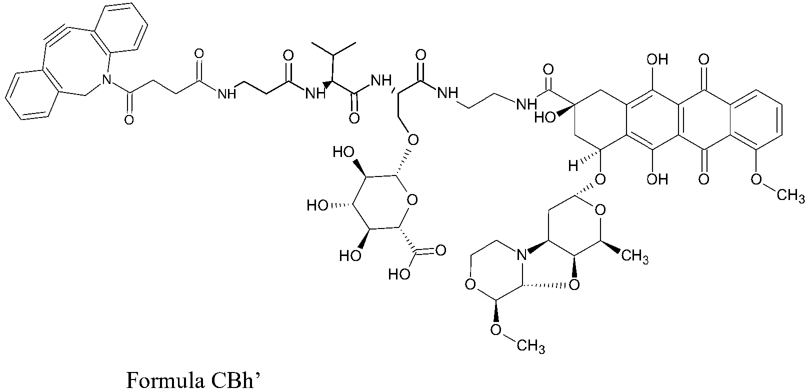

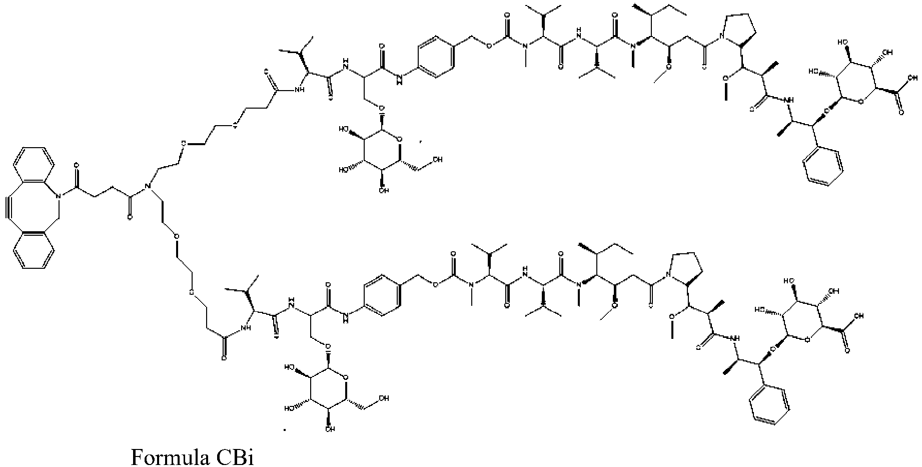

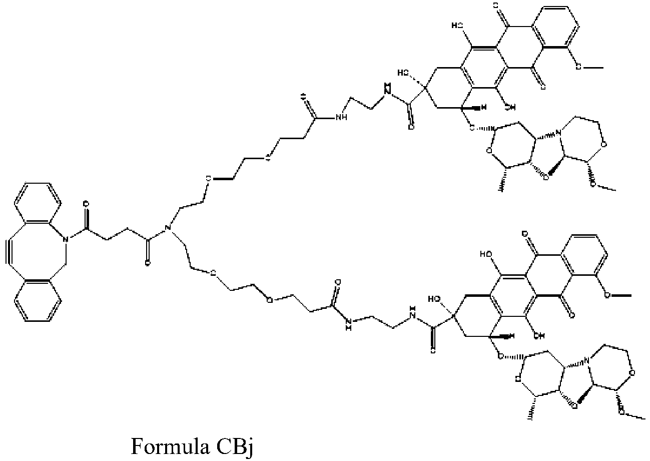

In an embodiment, the targeting unit-linker-payload conjugate is according to any one of the following Formulas TBa-TBj.

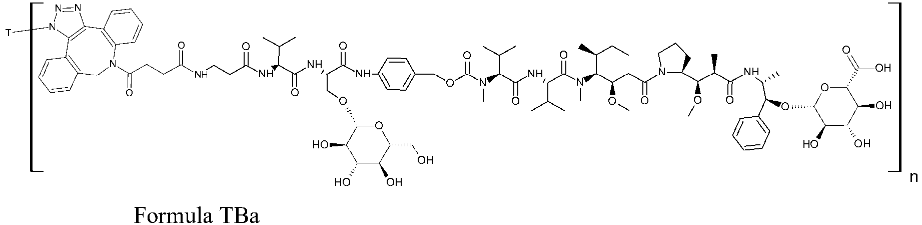

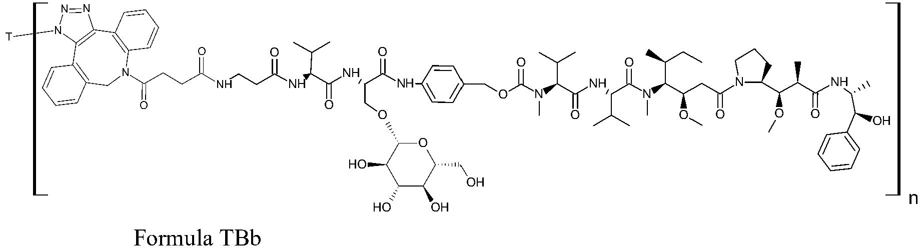

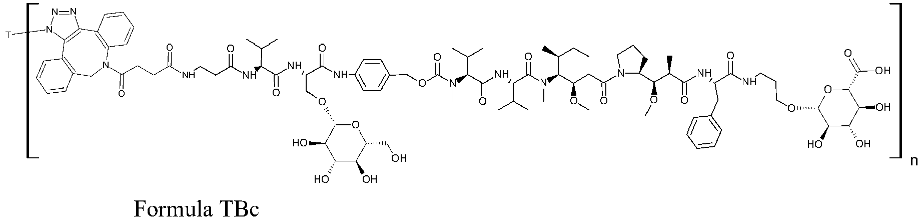

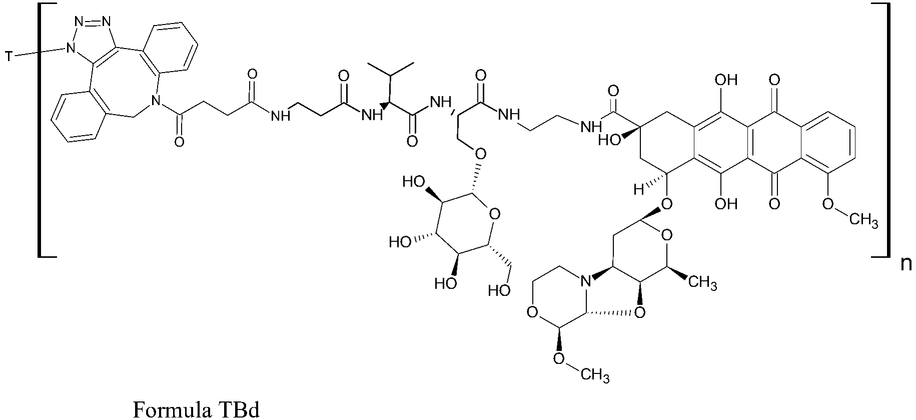

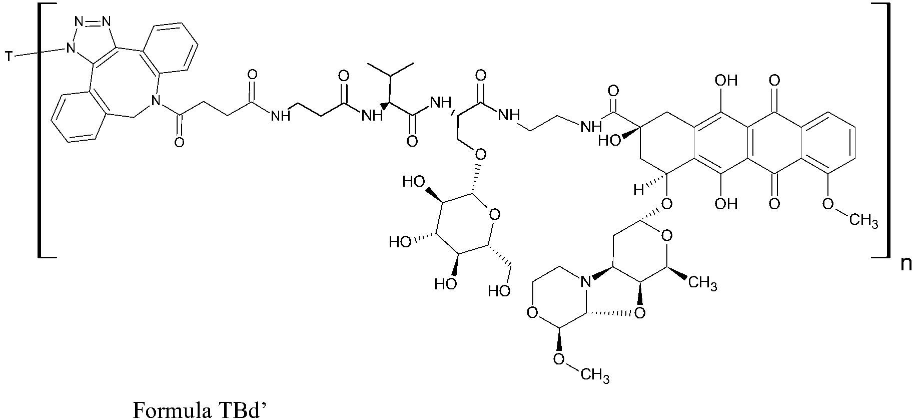

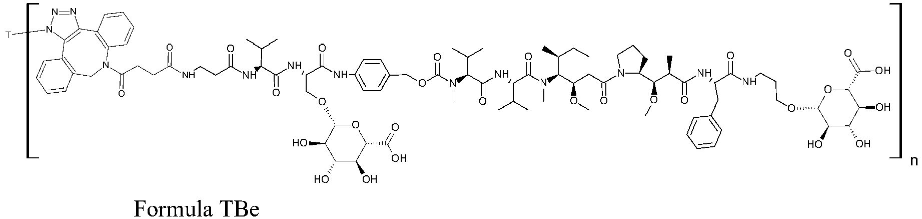

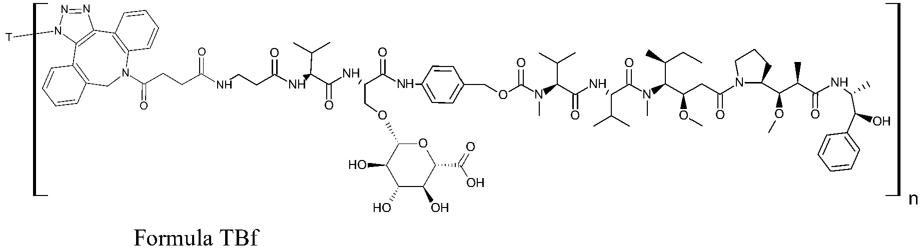

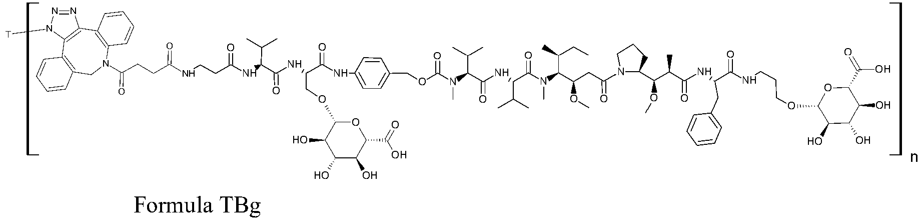

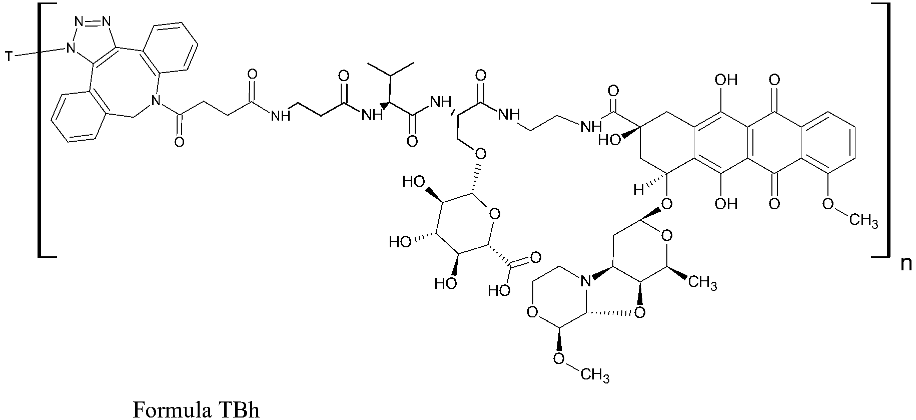

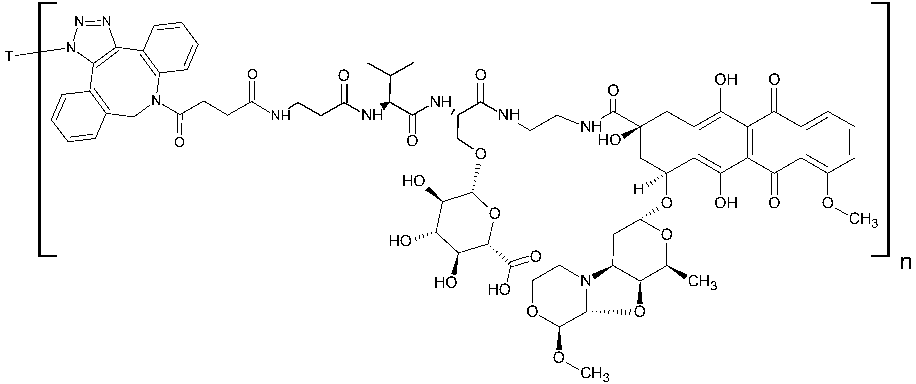

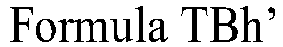

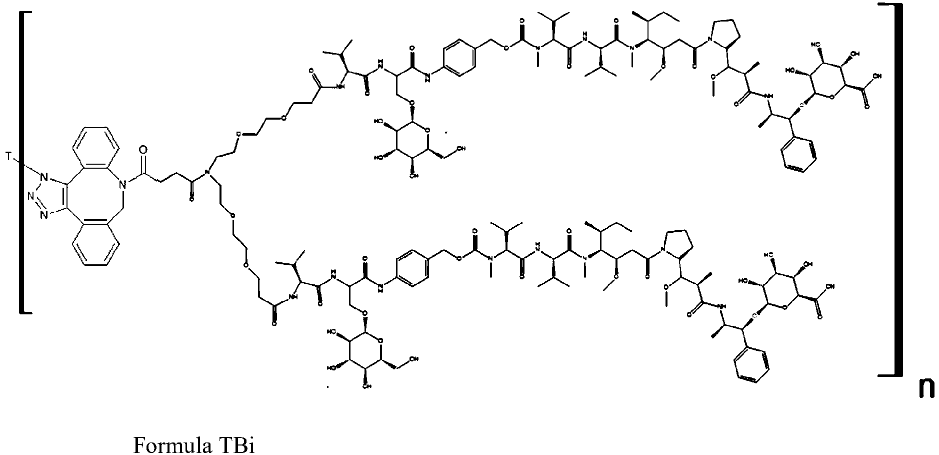

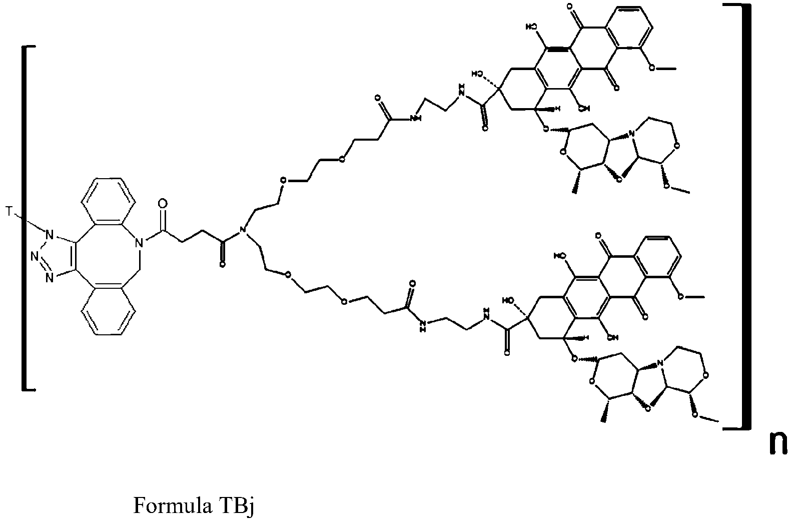

In an embodiment, n is in the range of 1 to about 20, or 1 to about 15, or 1 to about 10, or 2 to 10, or 2 to 6, or 2 to 5, or 2 to 4; or n is 1, 2, 3, 4, 5, 6, 7, 8, 9, 10, 11, 12, 13, 14, 15, 16, 17, 18, 19, or 20.

In an embodiment, n is in the range of 3 to about 20, or 3 to about 15, or 3 to about 10, or 3 to about 9, or 3 to about 8, or 3 to about 7, or 3 to about 6, or 3 to 5, or 3 to 4.

In an embodiment, n is in the range of 4 to about 20, or 4 to about 15, or 4 to about 10, or 4 to about 9, or 4 to about 8, or 4 to about 7, or 4 to about 6, or 4 to 5.

In an embodiment, n is 5.

In an embodiment, n is 6.

In an embodiment, n is 7.

In an embodiment, n is 8.

In an embodiment, n is 9.

In an embodiment, n, or drug-to-antibody (DAR) ratio, of a targeting unit-linker-payload conjugate may be determined using a MALDI-TOF MS.

In an embodiment, n, or drug-to-antibody ratio, of a targeting unit-linker-payload conjugate may be determined using an ESI-MS.

Exemplary methods to determine n, or drug-to-antibody ratio, is described in Chen J, Yin S, Wu Y, Ouyang J. Development of a native nanoelectrospray mass spectrometry method for determination of the drug-to-antibody ratio of antibody-drug conjugates. Anal Chem. 2013 Feb 5;85(3):1699-1704. doi:10.1021/ac302959p.

As a skilled person will understand, a composition such as a pharmaceutical composition may comprise a mixture of different targeting unit-linker-payload conjugate molecules in which n is different. For example, when DAR for a pharmaceutical composition is 7.8, the pharmaceutical composition may predominantly comprise targeting unit-linker-payload conjugate molecules in which n is 8, as well as minor amounts of targeting unit-linker-payload conjugate molecules in which n is smaller than 8, for example 7 and 6, and possibly trace amounts of molecules in which n is smaller than 6. n, or DAR, is therefore not necessarily an integer. If the (theoretical) maximum number of payload molecules to be conjugated to the targeting unit-linker-payload conjugate molecule is 8, then the DAR should in principle not exceed 8 or about 8, but the composition may comprise minor amounts of targeting unit-linker-payload conjugate molecules in which n is larger than 8, for example 9 or larger than 9. The DAR may depend on e.g. the number of possible conjugation sites in the targeting unit (such as antibody), the number of payload molecules that may be conjugated to a single conjugation site, and/or the extent to which the possible conjugation sites in the targeting unit are in fact conjugated to a payload molecule. Targeting unit-linker-payload conjugates can be prepared using cross-linking reagents. For example, a cysteine, thiol or an amine, e.g. N-terminus or an amino acid side chain, such as lysine of the antibody, can form a bond with a functional group of a cross-linking reagent.

Suitable linkers can be prepared by standard methods known to a person skilled in the art. For example, the central amino acid and peptide groups of the linker can be prepared by standard peptide chemistry and automated peptide chemistry, and ordered from a commercial manufacturer of synthetic peptides; and the saccharide, sulfate, phosphate, phosphodiester and phosphonate group Y can be added to the amino acid and peptide groups during or after their synthesis from commercially available protected building blocks. Further, the self-immolative group Z can be added to the amino acid and peptide groups by standard chemistry forming amide bonds to the amino acid and peptide groups.

General methods to prepare the targeting unit-payload conjugates, i.e. addition of the payload D, linkers and the targeting unit T, are known for the skilled artisan, and for example, described in U.S. Patent No. 5635483; U.S. Patent No. 5780588; Pettit et al. (1989) J. Am. Chem. Soc. 111:5463-5465; WO/2005/081711; Pettit et al. (1998) Anti-Cancer Drug Design 13:243-277; Pettit et al. (1996) J. Chem. Soc. Perkin Trans. 1 5:859-863; Doronina et al. (2003) Nat. Biotech.21:778-784 and Doronina et al. (2006) Bioconjugate Chem. 17:114-124, WO/2016/001485, WO/2014/096551, WO/2014/177771 and WO/2018/234636.

The targeting unit-linker-payload conjugates and linker-payload conjugates can be characterized and selected for their physical/chemical properties and/or biological activities by various assays known in the art.

For example, a conjugate can be tested for its antigen binding activity by known methods such as ELISA, FACS, Biacore or Western blot.

Transgenic animals and cell lines are particularly useful in screening conjugates that have potential as prophylactic or therapeutic treatments of cancer of tumor-associated antigens and cell surface receptors. Screening for a useful conjugate may involve administering a candidate conjugate over a range of doses to the transgenic animal and assaying at various time points for the effect(s) of the conjugate on the disease or disorder being evaluated. Alternatively, or additionally, the drug can be administered prior to or simultaneously with exposure to an inducer of the disease, if applicable. The candidate conjugate may be screened serially and individually, or in parallel under medium or high-throughput screening format.

A method for preparing the targeting unit-linker-payload conjugate according to one or more embodiments is disclosed, comprising conjugating the linker-payload according to one or more embodiments to a targeting unit. The linker-payload may be conjugated to the targeting unit via a linker, such as a linker according to one or more embodiments described in this specification.

Many ways of conjugating payload molecules to targeting units, for example, antibodies are known, and in principle any way that is suitable for conjugating a payload to a targeting unit may be used. The linker-payload according to one or more embodiments may be conjugated to the targeting unit such as an antibody directly or indirectly. In an embodiment, the linker-payload according to one or more embodiments and comprising a maleimide is conjugated to the antibody by reducing hinge region cystines with a reducing agent and contacting the reduced antibody with the linker-payload to form thioether bond.

In this context, the antibody may in principle be any antibody, and in particular any antibody described in this specification.

In this context, the payload molecule may in principle be any payload molecule, and in particular any payload molecule described in this specification.

In an embodiment, the antibody is selected from the group consisting of an anti-EGFR antibody, an epidermal growth factor receptor 2 (HER2/neu) antibody, an anti-CD22 antibody, an anti-CD30 antibody, an anti-CD33 antibody, an anti-CD20 antibody, an anti-TYRP-1 antibody, an anti-PD-L1 antibody, an anti-PD-L2 antibody, an anti-TIM-3 antibody, an anti-MUC1 antibody, and an anti-CA6 antibody.

A pharmaceutical composition comprising the linker-payload conjugate according to one or more embodiments or the targeting unit-linker-payload conjugate according to one or more embodiments is disclosed. The targeting unit-linker-payload conjugate may be obtainable by the method according to one or more embodiments described in this specification.

The pharmaceutical composition may further comprise a pharmaceutically acceptable carrier. Examples of suitable pharmaceutically acceptable carriers are well known in the art and may include e.g. phosphate buffered saline solutions, water, oil/water emulsions, wetting agents, and liposomes. Compositions comprising such carriers may be formulated by methods well known in the art. The pharmaceutical composition may further comprise other components such as vehicles, additives, preservatives, other pharmaceutical compositions administrated concurrently, and the like.

In an embodiment, the pharmaceutical composition comprises an effective amount of the linker-payload conjugate according to one or more embodiments.

In an embodiment, the pharmaceutical composition comprises an effective amount of the targeting unit-linker-payload conjugate according to one or more embodiments.

In an embodiment, the pharmaceutical composition comprises a therapeutically effective amount of the linker-payload conjugate according to one or more embodiments.

In an embodiment, the pharmaceutical composition comprises a therapeutically effective amount of the targeting unit-linker-payload conjugate according to one or more embodiments.

The term "therapeutically effective amount" or "effective amount" of the targeting unit-linker-payload conjugate should be understood as referring to the dosage regimen for modulating the growth of cancer cells and/or treating a patient's disease. The therapeutically effective amount can also be determined by reference to standard medical texts, such as the Physicians Desk Reference 2004. The patient may be male or female, and may be an infant, child or adult.

The term "treatment" or "treat" is used in the conventional sense and means attending to, caring for and nursing a patient with the aim of combating, reducing, attenuating or alleviating an illness or health abnormality and improving the living conditions impaired by this illness, such as, for example, with a cancer disease.

In an embodiment, the pharmaceutical composition comprises a composition for e.g. oral, parenteral, transdermal, intraluminal, intraarterial, intrathecal and/or intranasal administration or for direct injection into tissue. Administration of the pharmaceutical composition may be effected in different ways, e.g. by intravenous, intraperitoneal, subcutaneous, intramuscular, intratumoral, topical or intradermal administration.

The pharmaceutical composition may have a drug-to-antibody ratio of ≥ 1, or in the range of 1 to about 20, or 1 to about 15, or 1 to about 10, or 2 to 10, or 2 to 6, or 2 to 5, or 2 to 4; or about 1, about 2, about 3, about 4, about 5, about 6, about 7, about 8, about 9, about 10, about 11, about 12, about 13, about 14, about 15, about 16, about 17, about 18, about 19, or about 20; or about 1 to about 8, or about 6 to about 8.

In an embodiment, the pharmaceutical composition has a drug-to-antibody ratio in the range of about 1 to about 8, or 2 to 9, or 3 to 9, or 4 to 9, or 5 to 8.5, or 6 to 8.5, or 7 to 8.5, or 7.5 to 8.5, or 7 to 8, or 7.5 to 8, or about 8.

In an embodiment, the pharmaceutical composition has an average drug-to-antibody ratio between 6 to 9, or 7 to 8.5, or 7 to 8, or 7.5 to 8.5, or 7.5 to 8, or about 8.

In an embodiment, the pharmaceutical composition has a drug-to-antibody ratio in the range of 0 to about 8, or 1 to 7, or 2 to 6, or 3 to 5, or 3.5 to 4.5, or about 4.

In an embodiment, the pharmaceutical composition has an average drug-to-antibody ratio between 3 to 5, or 3.5 to 4.5, or about 4.

In an embodiment, the pharmaceutical composition has a drug-to-antibody ratio in the range of 0 to about 4, or 1 to 3, or 1.5 to 2.5, or about 2.

In an embodiment, the pharmaceutical composition has an average drug-to-antibody ratio between 1 to 3, or 1.5 to 2.5, or about 2.

In an embodiment, the targeting unit-linker-payload conjugate is the targeting unit-linker-payload conjugate represented by the following formula 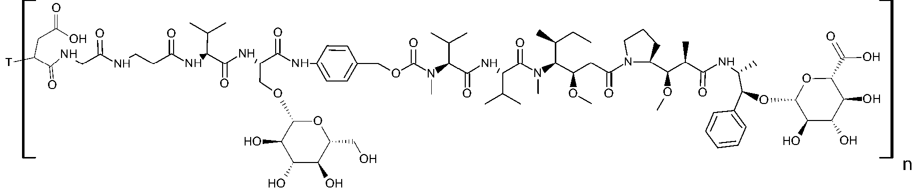 In the context of the above formula, n may be any value or range of values described in this specification. In such a pharmaceutical composition, the drug-to-antibody ratio may be in the range of about 3 to 5, about 3.5 to 4.5, about 4, about 7.5 to 8.3, or about 7.8-8.1. The targeting unit may be any targeting unit, such as an antibody, described in this specification.

A method of treating and/or modulating the growth of tumor cells in humans or animals is disclosed, wherein the linker-payload conjugate according to one or more embodiments, targeting unit-linker-payload conjugate according to one or more embodiments or the pharmaceutical composition according to one or more embodiments is administered to a human or animal in an effective amount.

A targeting unit-linker-payload conjugate according to one or more embodiments or the pharmaceutical composition according to one or more embodiments for use as a medicament is disclosed.

A linker-payload conjugate according to one or more embodiments for use as a medicament is disclosed.

A targeting unit-linker-payload conjugate according to one or more embodiments or the pharmaceutical composition according to one or more embodiments for use in the treatment of cancer is disclosed.

A linker-payload conjugate according to one or more embodiments for use in the treatment of cancer is disclosed.

The targeting unit-linker-payload conjugate according to one or more embodiments or the pharmaceutical composition according to one or more embodiments may be particularly useful in combination with anti-cancer agents. Thus, the present disclosure provides a combination of targeting unit-linker-payload conjugates, or their pharmaceutical composition, of Formula II, Formula IIs, Formula IIG, Formula IIGs, Formula IIGX, Formula IIGXs, Formula IV, Formula IVs, Formulas TMa-TMz, or Formulas TMsa-TMsz in a combination with anti-cancer agents for simultaneous, separate or sequential administration. The targeting unit-linker-payload conjugates, of Formula II, Formula IIs, Formula IIG, Formula IIGs, Formula IIGX, Formula IIGXs, Formula IV, Formula IVs, Formulas TMa-TMz, or Formulas TMsa-TMsz and an anticancer agent can act additively or synergistically. A synergistic combination of the targeting unit-linker-payload conjugates of Formula II, Formula IIs, Formula IIG, Formula IIGs, Formula IIGX, Formula IIGXs, Formula IV, Formula IVs, Formulas TMa-TMz, or Formulas TMsa-TMsz and an anticancer agent might allow the use of lower dosages of one or both of these agents and/or less frequent dosages of one or both of the targeting unit-linker-payload conjugates of Formula II, Formula IIs, Formula IIG, Formula IIGs, Formula IIGX, Formula IIGXs, Formula IV, Formula IVs, Formulas TMa-TMz, or Formulas TMsa-TMsz and the anticancer agents and/or to administer the anticancer agent less frequently can reduce any toxicity associated with the administration of the agents to a patient without reducing the efficacy of the agents in the treatment of cancer. In addition, a synergistic effect might result in the improved efficacy of these agents in the treatment of cancer and/or the reduction of any adverse or unwanted side effects associated with the use of the agent.

The anti-cancer agent can be administered according to therapeutic protocols well known in the art. It will be apparent to those skilled in the art that the administration of the anti-cancer agent can be varied depending on the disease being treated and the known effects of the anti-cancer agent on that disease. Also, in accordance with the knowledge of the skilled clinician, the therapeutic protocols (e.g., dosage amounts and times of administration) can be varied in view of the observed effects of the administered anticancer agents on the patient, and in view of the observed responses of the disease to the agents, and observed adverse effects.

In an embodiment, the targeting unit-linker-payload conjugates of Formula II, Formula IIs, Formula IIG, Formula IIGs, Formula IIGX, Formula IIGXs, Formula IV, Formula IVs, Formulas TMa-TMz, or Formulas TMsa-TMsz may be administered in combination with one or more anti-cancer agents.

In an embodiment, the targeting unit is an antibody.

In an embodiment, the targeting unit-linker-payload conjugate of Formula II, Formula IIs, Formula IIG, Formula IIGs, Formula IIGX, Formula IIGXs, Formula IV, Formula IVs, Formulas TMa-TMz, or Formulas TMsa-TMsz, wherein targeting unit is an antibody, is capable of binding an anti-hematologic target molecule selected from the group consisting of CD19, CD20, CD22, CD25, CD30, CD33, CD37, CD38, CD52, CD56, CD70, CD74, CD79, CD98, CD117, CD105, CD123, CD138, CD157, BCMA and CD319 (SLAMF7).

In an embodiment, the targeting unit-linker-payload conjugate of Formula II, Formula IIs, Formula IIG, Formula IIGs, Formula IIGX, Formula IIGXs, Formula IV, Formula IVs, Formulas TMa-TMz, or Formulas TMsa-TMsz, wherein targeting unit is an antibody, is capable of binding the target molecule selected from the group consisting of CD19, CD22, CD33, CD52 and CD123.

In an embodiment, the targeting unit-linker-payload conjugate of Formula II, Formula IIs, Formula IIG, Formula IIGs, Formula IIGX, Formula IIGXs, Formula IV, Formula IVs, Formulas TMa-TMz, or Formulas TMsa-TMsz, wherein targeting unit is an antibody, the antibody is an anti-hematologic target antibody selected from the group consisting of loncastuximab, blinatumomab, tafasitamab, coltuximab, denintuzumab, obexelimab, inebilizumab, MOR00208, MDX-1342, MEDI-551, SAR3419, rituximab, ofatumumab, veltuzumab, ocrelizumab, obinutuzumab, ocaratuzumab, ublituximab, nofetumomab, ibritumomab, epratuzumab, inotuzumab ozogamicin, bectumomab, moxetumomab, pinatuzumab, DCDT2980S, basiliximab, daclizumab, camidanlumab, inolimomab, ADCT-301, IMTOX-25, brentuximab, iratumumab, AVE9633, lintuzumab, gemtuzumab, vadastuximab, otlertuzumab, lilotomab, naratuximab, BI836826, AGS67E, IMGN529, daratumumab, isatuximab, mezagitamab, felzartamab, MOR202, MOR03087, alemtuzumab, lorvotuzumab mertansine, vorsetuzumab mafodotin, SGN-70A, polatuzumab, indatuximab, MDX-1203, milatuzumab-doxorubicin, IGN523, LOP-628, CSL360, talacotuzumab, XmAb14045, KHK2823, BT062, belantamab mafodotin, teclistamab and elotuzumab.

In an embodiment of the targeting unit-linker-payload conjugate of Formula II, Formula IIs, Formula IIG, Formula IIGs, Formula IIGX, Formula IIGXs, Formula IV, Formula IVs, Formulas TMa-TMz, or Formulas TMsa-TMsz, wherein targeting unit is an antibody, the antibody is selected from the group consisting of epratuzumab, lintuzumab, coltuximab, denintuzumab, loncastuximab, alemtuzumab and talacotuzumab.

The targeting unit may be an antibody capable of binding the target molecule selected from the group consisting of CD19, CD22, CD33, CD52 and CD123, and the targeting unit-linker-payload conjugate or the pharmaceutical composition is administered in combination with an FLT3 inhibitor, an IDH1 inhibitor, an IDH2 inhibitor, a BCL2 inhibitor, a KRAS inhibitor, a NRAS inhibitor or a MEK1/2 inhibitor.

The FLT3 inhibitor may be selected from the group consisting of midostaurin, gilteritinib fumarate, quizartinib, crenolanib, sunitinib, ponatinib and sorafenib. The MEK1/2 inhibitor may be trametinib, cobimetinib, selumetinib or binimetinib. The IDH1/IDH2 inhibitor may be enasidenib or ivosidenib. The BCL2 inhibitor may be venetoclax, navitoclax or obatoclax. The KRAS inhibitor may be sotorasib or adagrasib.

The treatment of cancer may further comprise administering an anti-cancer agent selected from the group consisting of acalabrutinib, arsenic trioxide, asciminib hydrochloride, axicabtagene ciloleucel, azacytidine, belinostat, bendamustine hydrochloride, bleomycin sulfate, bortezomib, bosutinib, brexucabtagene autoleucel, busulfan, carmustine, chlorambucil, cladribine, clofarabine, copanlisib hydrochloride, crizotinib, cyclophosphamide, cytarabine, dacarbazine, dasatinib, daunorubicin hydrochloride, denileukin diftitox, dexamethasone, doxorubicin hydrochloride, duvelisib, enasidenib mesylate, fludarabine phosphate, gilteritinib fumarate, glasdegib maleate, hydroxyurea, ibrutinib, idarubicin hydrochloride, idelalisib, imatinib mesylate, ivosidenib, lenalidomide, lisocabtagene maraleucel, lomustine, mercaptopurine, methotrexate sodium, midostaurin, mitoxantrone hydrochloride, nelarabine, nilotinib, nivolumab, omacetaxine mepesuccinate, plerixafor, ponatinib hydrochloride, pralatrexate, prednisone, procarbazine hydrochloride, recombinant interferon alfa-2b, rituximab, romidepsin, selinexor, tafasitamab-cxix, tagraxofusp-erzs, tazemetostat hydrobromide, thioguanine, tisagenlecleucel, umbralisib tosylate, venetoclax, navitoclax, obatoclax, vinblastine sulfate, vorinostat, zanubrutinib, gilteritinib, quizartinib, crenolanib and sorafenib.

The targeting unit-linker-payload conjugate or pharmaceutical composition may be administered in combination with arsenic trioxide, azacytidine, daunorubicin hydrochloride, cyclophosphamide, cytarabine, glasdegib maleate, dexamethasone, doxorubicin hydrochloride, midostaurin, gilteritinib fumarate, quizartinib, crenolanib, sunitinib, ponatinib, sorafenib, enasidenib, ivosidenib, sotorasib, adagrasib, etoposide hydrochloride, gemtuzumab ozogamicin, idarubicin hydrochloride, midostaurin, mitoxantrone hydrochloride, prednisone, thioguanine, venetoclax, navitoclax, obatoclax or vincristine sulfate.

In an embodiment, the cancer is selected from the group consisting of leukemia, lymphoma, breast cancer, prostate cancer, ovarian cancer, colorectal cancer, gastric cancer, squamous cancer, small-cell lung cancer, head-and-neck cancer, multidrug resistant cancer, glioma, melanoma and testicular cancer.

In an embodiment, the tumor cells are selected from the group consisting of leukemia cells, lymphoma cells, breast cancer cells, prostate cancer cells, ovarian cancer cells, colorectal cancer cells, gastric cancer cells, squamous cancer cells, small-cell lung cancer cells, head-and-neck cancer cells, multidrug resistant cancer cells, and testicular cancer cells.

In an embodiment, the anti-cancer agent is selected from the group consisting of acalabrutinib, arsenic trioxide, asciminib hydrochloride, axicabtagene ciloleucel, azacytidine, belinostat, bendamustine hydrochloride, bleomycin sulfate, bortezomib, bosutinib, brexucabtagene autoleucel, busulfan, carmustine, chlorambucil, cladribine, clofarabine, copanlisib hydrochloride, crizotinib, cyclophosphamide, cytarabine, dacarbazine, dasatinib, daunorubicin hydrochloride, denileukin diftitox, dexamethasone, doxorubicin hydrochloride, duvelisib, enasidenib mesylate, fludarabine phosphate, gilteritinib fumarate, glasdegib maleate, hydroxyurea, ibrutinib, idarubicin hydrochloride, idelalisib, imatinib mesylate, ivosidenib, lenalidomide, lisocabtagene maraleucel, lomustine, mercaptopurine, methotrexate sodium, midostaurin, mitoxantrone hydrochloride, nelarabine, nilotinib, nivolumab, omacetaxine mepesuccinate, plerixafor, ponatinib hydrochloride, pralatrexate, prednisone, procarbazine hydrochloride, recombinant interferon alfa-2b, rituximab, romidepsin, selinexor, tafasitamab-cxix, tagraxofusp-erzs, tazemetostat hydrobromide, thioguanine, tisagenlecleucel, umbralisib tosylate, venetoclax, navitoclax, obatoclax, vinblastine sulfate, vorinostat, zanubrutinib, gilteritinib, quizartinib, crenolanib and sorafenib.

In an embodiment, the targeting unit-linker-payload conjugate of Formula II, Formula IIs, Formula IIG, Formula IIGs, Formula IIGX, Formula IIGXs, Formula IV, Formula IVs, Formulas TMa-TMz, or Formulas TMsa-TMsz, wherein targeting unit is an antibody capable of binding an anti-hematologic target molecule selected from the group consisting of CD19, CD20, CD22, CD25, CD30, CD33, CD37, CD38, CD52, CD56, CD70, CD74, CD79, CD98, CD117, CD105, CD123, CD138, CD157, BCMA and CD319 (SLAMF7) is administered in combination with the anti-cancer agent selected from the group consisting of acalabrutinib, arsenic trioxide, asciminib hydrochloride, axicabtagene ciloleucel, azacytidine, belinostat, bendamustine hydrochloride, bleomycin sulfate, bortezomib, bosutinib, brexucabtagene autoleucel, busulfan, carmustine, chlorambucil, cladribine, clofarabine, copanlisib hydrochloride, crizotinib, cyclophosphamide, cytarabine, dacarbazine, dasatinib, daunorubicin hydrochloride, denileukin diftitox, dexamethasone, doxorubicin hydrochloride, duvelisib, enasidenib mesylate, fludarabine phosphate, gilteritinib fumarate, glasdegib maleate, hydroxyurea, ibrutinib, idarubicin hydrochloride, idelalisib, imatinib mesylate, ivosidenib, lenalidomide, lisocabtagene maraleucel, lomustine, mercaptopurine, methotrexate sodium, midostaurin, mitoxantrone hydrochloride, nelarabine, nilotinib, nivolumab, omacetaxine mepesuccinate, plerixafor, ponatinib hydrochloride, pralatrexate, prednisone, procarbazine hydrochloride, recombinant interferon alfa-2b, rituximab, romidepsin, selinexor, tafasitamab-cxix, tagraxofusp-erzs, tazemetostat hydrobromide, thioguanine, tisagenlecleucel, umbralisib tosylate, venetoclax, navitoclax, obatoclax, vinblastine sulfate, vorinostat, zanubrutinib, gilteritinib, quizartinib, crenolanib and sorafenib.

In an embodiment, the targeting unit-linker-payload conjugate of Formula II, Formula IIs, Formula IIG, Formula IIGs, Formula IIGX, Formula IIGXs, Formula IV, Formula IVs, Formulas TMa-TMz, or Formulas TMsa-TMsz, wherein targeting unit is an antibody capable of binding the target molecule selected from the group consisting of CD19, CD22, CD33, CD52 and CD123 is administered in combination with the anti-cancer agent selected from the group consisting of acalabrutinib, arsenic trioxide, asciminib hydrochloride, axicabtagene ciloleucel, azacytidine, belinostat, bendamustine hydrochloride, bleomycin sulfate, bortezomib, bosutinib, brexucabtagene autoleucel, busulfan, carmustine, chlorambucil, cladribine, clofarabine, copanlisib hydrochloride, crizotinib, cyclophosphamide, cytarabine, dacarbazine, dasatinib, daunorubicin hydrochloride, denileukin diftitox, dexamethasone, doxorubicin hydrochloride, duvelisib, enasidenib mesylate, fludarabine phosphate, gilteritinib fumarate, glasdegib maleate, hydroxyurea, ibrutinib, idarubicin hydrochloride, idelalisib, imatinib mesylate, ivosidenib, lenalidomide, lisocabtagene maraleucel, lomustine, mercaptopurine, methotrexate sodium, midostaurin, mitoxantrone hydrochloride, nelarabine, nilotinib, nivolumab, omacetaxine mepesuccinate, plerixafor, ponatinib hydrochloride, pralatrexate, prednisone, procarbazine hydrochloride, recombinant interferon alfa-2b, rituximab, romidepsin, selinexor, tafasitamab-cxix, tagraxofusp-erzs, tazemetostat hydrobromide, thioguanine, tisagenlecleucel, umbralisib tosylate, venetoclax, navitoclax, obatoclax, vinblastine sulfate, vorinostat, zanubrutinib, gilteritinib, quizartinib, crenolanib and sorafenib.

In an embodiment, the targeting unit-linker-payload conjugate of Formula II, Formula IIs, Formula IIG, Formula IIGs, Formula IIGX, Formula IIGXs, Formula IV, Formula IVs, Formulas TMa-TMz, or Formulas TMsa-TMsz, wherein targeting unit is an antibody capable of binding the target molecule selected from the group consisting of CD19, CD22, CD33, CD52 and CD123 is administered in combination with an FLT3 inhibitor, an IDH1 inhibitor, an IDH2 inhibitor, a KRAS inhibitor, a NRAS inhibitor or a MEK1/2 inhibitor.

In an embodiment, the targeting unit-linker-payload conjugate of Formula II, Formula IIs, Formula IIG, Formula IIGs, Formula IIGX, Formula IIGXs, Formula IV, Formula IVs, Formulas TMa-TMz, or Formulas TMsa-TMsz, wherein targeting unit is an antibody capable of binding the CD33 is administered in combination with an FLT3 inhibitor, IDH1 inhibitor, IDH2 inhibitor, BCL2 inhibitor, KRAS inhibitor, NRAS inhibitor or MEK1/2 inhibitor.

In an embodiment, the targeting unit-linker-payload conjugate of Formula II, Formula IIs, Formula IIG, Formula IIGs, Formula IIGX, Formula IIGXs, Formula IV, Formula IVs, Formulas TMa-TMz, or Formulas TMsa-TMsz, wherein targeting unit is lintuzumab is administered in combination with an FLT3 inhibitor, IDH1 inhibitor, IDH2 inhibitor, BCL2 inhibitor, KRAS inhibitor, NRAS inhibitor or MEK1/2 inhibitor.

In an embodiment, the targeting unit-linker-payload conjugate of Formula LNAuM 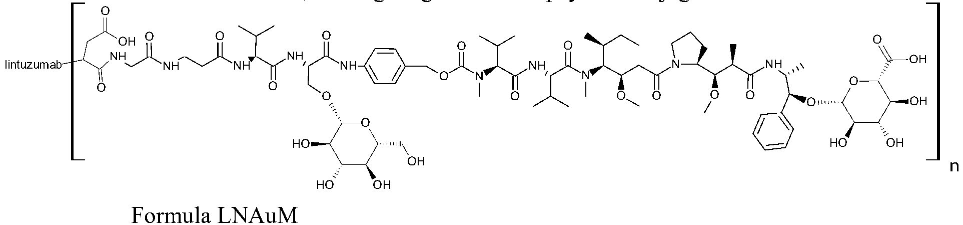 wherein n is 8, is administered in combination with an FLT3 inhibitor, an IDH1 inhibitor, an IDH2 inhibitor, a BCL2 inhibitor, a KRAS inhibitor, a NRAS inhibitor or a MEK1/2 inhibitor.

In an embodiment, the FLT3 inhibitor is selected from the group consisting of midostaurin, gilteritinib fumarate, quizartinib, crenolanib, sunitinib, ponatinib and sorafenib.

In an embodiment, the MEK1/2 inhibitor is trametinib, cobimetinib, selumetinib or binimetinib.

In an embodiment, the IDH1/IDH2 inhibitor is enasidenib or ivosidenib.

In an embodiment, the BCL2 inhibitor is venetoclax, navitoclax or obatoclax.

In an embodiment, the KRAS inhibitor is sotorasib or adagrasib.

In an embodiment, the targeting unit-linker-payload conjugate of Formula LNAuM 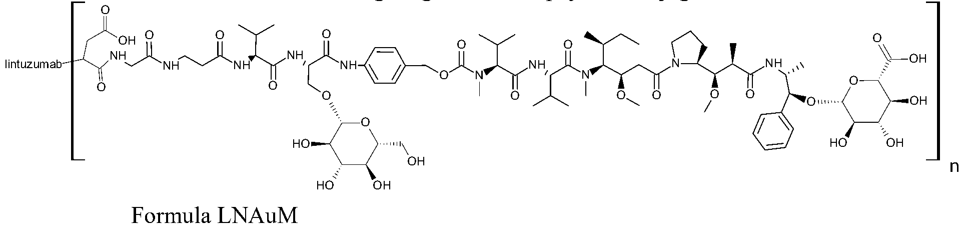 wherein n is 8, is administered in combination with arsenic trioxide, azacytidine, daunorubicin hydrochloride, cyclophosphamide, cytarabine, glasdegib maleate, dexamethasone, doxorubicin hydrochloride, midostaurin, gilteritinib fumarate, quizartinib, crenolanib, sunitinib, ponatinib, sorafenib, enasidenib, ivosidenib, sotorasib, adagrasib, etoposide hydrochloride, gemtuzumab ozogamicin, idarubicin hydrochloride, midostaurin, mitoxantrone hydrochloride, prednisone, thioguanine, venetoclax, navitoclax, obatoclax or vincristine sulfate.

A method of treating cancer in humans is disclosed, wherein the linker-payload conjugate according to one or more embodiments, the targeting unit-linker-payload conjugate according to one or more embodiments or the pharmaceutical composition according to one or more embodiments is administered to a human in an effective amount.

In an embodiment, the effective amount is a therapeutically effective amount.

In an embodiment, the linker-payload conjugate according to one or more embodiments, the targeting unit-linker-payload conjugate according to one or more embodiments or the pharmaceutical composition according to one or more embodiments is administered intravenously to a human in a therapeutically effective amount.

In an embodiment, the linker-payload conjugate according to one or more embodiments, the targeting unit-linker-payload conjugate according to one or more embodiments or the pharmaceutical composition according to one or more embodiments is administered intratumorally to a human in a therapeutically effective amount.

In an embodiment, the cancer is selected from the group consisting of head-and-neck cancer, leukemia, lymphoma, breast cancer, prostate cancer, ovarian cancer, colorectal cancer, gastric cancer, squamous cancer, small-cell lung cancer, multidrug resistant cancer and testicular cancer.

The embodiments of the invention described hereinbefore may be used in any combination with each other. Several of the embodiments may be combined together to form a further embodiment of the invention. A product or a method to which the invention is related may comprise at least one of the embodiments of the invention described hereinbefore.

The linker-payload conjugate according to one or more embodiments and the targeting unit-linker-payload conjugate according to one or more embodiments may have a number of beneficial properties.

The presence of the cleavable hydrophilic group renders the otherwise relatively poorly water-soluble linker more soluble in aqueous and physiological solutions. The improved solubility also improves the retention of the targeting unit-linker-payload conjugate in serum. It may also have high uptake in cells to which it is targeted and low uptake in cells and organs to which it is not targeted.

The targeting unit-linker-payload conjugate according to one or more embodiments is less toxic in the absence or low activity of lysosomal and intracellular enzymes. Since cancer cells typically display high activity of lysosomal and/or intracellular enzymes, the toxic payload moiety is preferentially released in cancer cells as compared to non-cancer cells.

The conjugate has low antigenicity.

The targeting unit-linker-payload conjugate according to one or more embodiments also exhibits good pharmacokinetics. It has suitable retention in blood, high uptake in cells to which it is targeted and low uptake in cells and organs to which it is not targeted.

The targeting unit-linker-payload conjugate according to one or more embodiments is sufficiently stable towards chemical or biochemical degradation during manufacturing or in physiological conditions, e.g. in blood, serum, plasma or tissues.

The following embodiments are also disclosed:
1. A linker-payload conjugate of Formula I, Formula IG, Formula IGX or Formula III 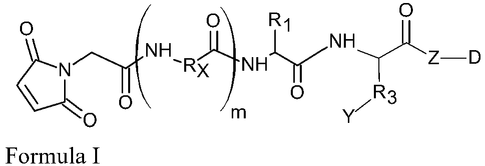 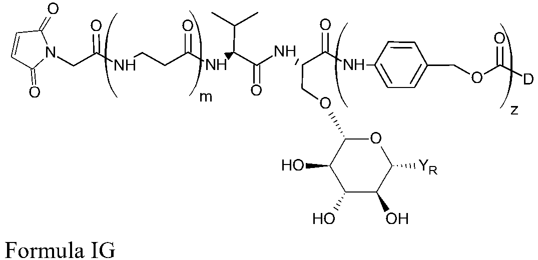 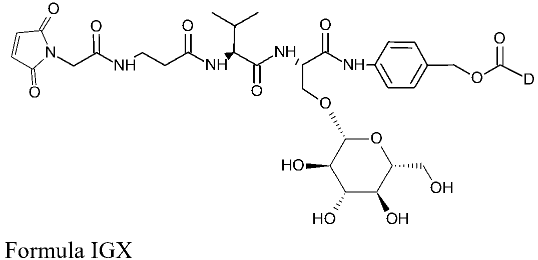 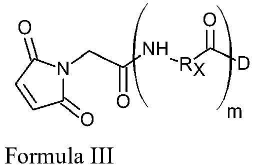 wherein R₁ is an amino acid side chain; R_{X} is a linear C₁-C₆ alkylene group, a branched C₁-C₆ alkylene group, -CH₂CH₂-, or -CH(R₂)-, wherein R₂ is an amino acid side chain; Y is absent or a hydrophilic group; R₃ is an amino acid side chain; Z is either absent or a self-immolative group; D is a payload molecule; and m is either 0 or 1.
2. A targeting unit-linker-payload conjugate of Formula II, Formula IIs, Formula IIG, Formula IIGs, Formula IIGX, Formula IIGXs, Formula IV or Formula IVs 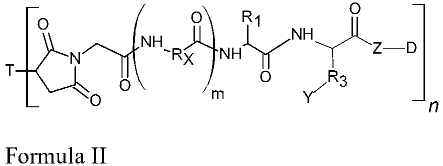 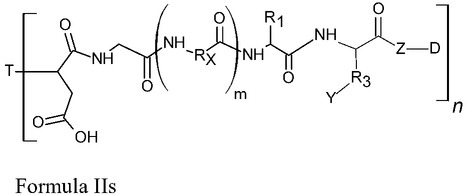 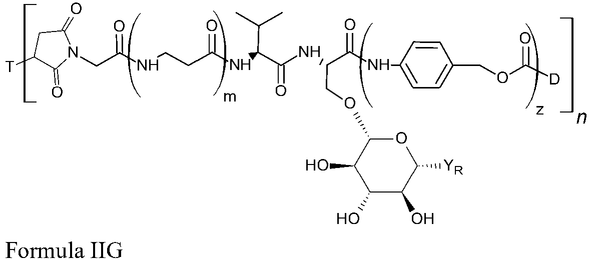 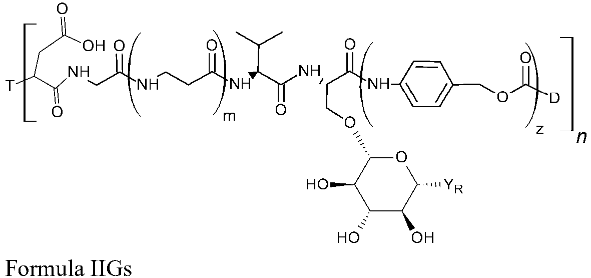 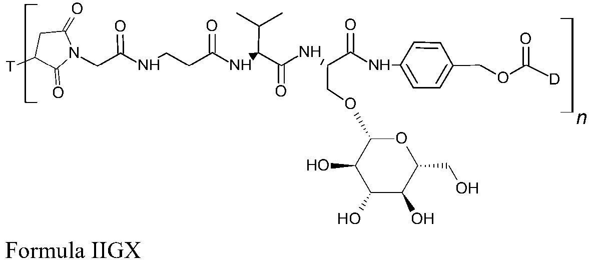 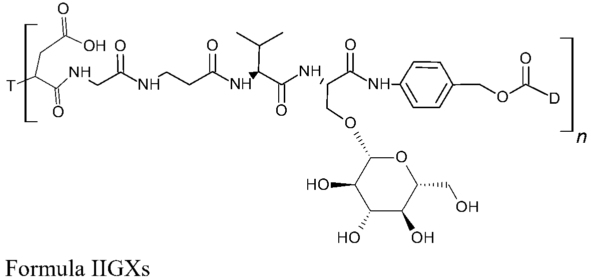 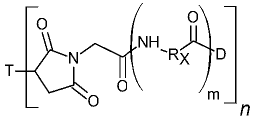 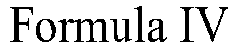 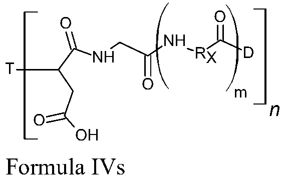 wherein T is a targeting unit; R₁ is an amino acid side chain; R_{X} is a linear C₁-C₆ alkylene group, a branched C₁-C₆ alkylene group, -CH₂CH₂-, or -CH(R₂)-, wherein R₂ is an amino acid side chain; Y is absent or a hydrophilic group; R₃ is an amino acid side chain; Z is either absent or a self-immolative group; D is a payload molecule; m is either 0 or 1; and n ≥ 1, or n is in the range of 1 to about 20, or 1 to about 15, or 1 to about 10, or 2 to 10, or 2 to 6, or 2 to 5, or 2 to 4; or n is 1, 2, 3, 4, 5, 6, 7, 8, 9, 10, 11, 12, 13, 14, 15, 16, 17, 18, 19, or 20.
3. The targeting unit-linker-payload conjugate of embodiment 2, wherein the targeting unit-linker-payload conjugate comprises
   i) at least 1 hydrolysed maleimide according to Formula IIs, Formula IIGs, Formula IIGXs or Formula IVs,
   ii) at least 2 hydrolysed maleimides according to Formula IIs, Formula IIGs, Formula IIGXs or Formula IVs,
   iii) least 3 hydrolysed maleimides according to Formula IIs, Formula IIGs, Formula IIGXs or Formula IVs,
   iv) at least 4 hydrolysed maleimides according to Formula IIs, Formula IIGs, Formula IIGXs or Formula IVs,
   v) at least 5 hydrolysed maleimides according to Formula IIs, Formula IIGs, Formula IIGXs or Formula IVs,
   vi) at least 6 hydrolysed maleimides according to Formula IIs, Formula IIGs, Formula IIGXs or Formula IVs,
   vii) at least 7 hydrolysed maleimides according to Formula IIs, Formula IIGs, Formula IIGXs or Formula IVs,
   viii) at least 8 hydrolysed maleimides according to Formula IIs, Formula IIGs, Formula IIGXs or Formula IVs,
   ix) at least 9 hydrolysed maleimides according to Formula IIs, Formula IIGs, Formula IIGXs or Formula IVs,
   x) at least 10 hydrolysed maleimides according to Formula IIs, Formula IIGs, Formula IIGXs or Formula IVs,
   xi) 100 % of hydrolysed maleimides according to Formula IIs, Formula IIGs, Formula IIGXs or Formula IVs,
   xii) at least 1/n hydrolysed maleimides according to Formula IIs, Formula IIGs, Formula IIGXs or Formula IVs, wherein n is at least 1,
   xiii) at least 2/n hydrolysed maleimides according to Formula IIs, Formula IIGs, Formula IIGXs or Formula IVs, wherein n is at least 2,
   xiv) at least 3/n hydrolysed maleimides according to Formula IIs, Formula IIGs, Formula IIGXs or Formula IVs, wherein n is at least 3,
   xv) at least 4/n hydrolysed maleimides according to Formula IIs, Formula IIGs, Formula IIGXs or Formula IVs, wherein n is at least 4,
   xvi) at least 5/n hydrolysed maleimides according to Formula IIs, Formula IIGs, Formula IIGXs or Formula IVs, wherein n is at least 5,
   xvii) at least 6/n hydrolysed maleimides according to Formula IIs, Formula IIGs, Formula IIGXs or Formula IVs, wherein n is at least 6,
   xviii) at least 7/n hydrolysed maleimides according to Formula IIs, Formula IIGs, Formula IIGXs or Formula IVs, wherein n is at least 7,
   xix) at least 8/n hydrolysed maleimides according to Formula IIs, Formula IIGs, Formula IIGXs or Formula IVs, wherein n is at least 8,
   xx) at least 9/n hydrolysed maleimides according to Formula IIs, Formula IIGs, Formula IIGXs or Formula IVs, wherein n is at least 9 or
   xxi) at least 10/n hydrolysed maleimides according to Formula IIs, Formula IIGs, Formula IIGXs or Formula IVs, wherein n is at least 10.
4. The linker-payload conjugate of embodiment 1 or the targeting unit-linker-payload conjugate of embodiment 2 or 3, wherein Y is selected from the group consisting of a saccharide, phosphate ester, sulfate ester, a phosphodiester and a phosphonate.
5. The linker-payload conjugate of any preceding embodiment or the targeting unit-linker-payload conjugate of any preceding embodiment, wherein the saccharide comprises or consists of β-D-galactose, N-acetyl-β-D-galactosamine, N-acetyl-α-D-galactosamine, N-acetyl-β-D-glucosamine, β-D-glucuronic acid, α-L-iduronic acid, α-D-galactose, α-D-glucose, β-D-glucose, α-D-mannose, β-D-mannose, α-L-fucose, β-D-xylose, a neuraminic acid or any analogue or modification thereof, or sulfate, phosphate, carboxyl, amino, or O-acetyl modification thereof.
6. The linker-payload conjugate of any preceding embodiment or the targeting unit-linker-payload conjugate of any preceding embodiment, wherein D is a cytotoxic drug selected from the group consisting of dolastatin; auristatin; epothilone; daunorubicin; doxorubicin; an alkylating agent, such as thiotepa or cyclophosphamide (CYTOXAN^{™}); alkyl sulfonate such as busulfan, improsulfan or piposulfan; aziridine, such as benzodopa, carboquone, meturedopa, or uredopa; ethylenimine and/or methylamelamine, such as altretamine, triethylenemelamine, trietylene-phosphoramide, triethylenethiophosphaoramide or trimethylolomelamine; acetogenin, such as bullatacin or bullatacinone; camptothecin, such as the synthetic analogue topotecan; bryostatin; callystatin; CC-1065 and/or its adozelesin, carzelesin or bizelesin synthetic analogue; cryptophycin, such as cryptophycin 1 or cryptophycin 8); duocarmycin (including the synthetic analogues, KW-2189 and CBI-TMI); eleutherobin; pancratistatin; sarcodictyins; spongistatin; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics, such as the enediyne antibiotics (e.g. calicheamicins, especially calicheamicin γ1; dynemicin, including dynemicin A; esperamicin; as well as neocarzinostatin chromophore and related chromoprotein enediyne antiobiotic chromomophores), aclacinomysins, actinomycin, authramycin, azaserine, bleomycins, cactinomycin, carabicin, caminomycin, carzinophilin; chromomycins, dactinomycin, detorubicin, 6-diazo-5-oxo-L-norleucine, other doxorubicin derivatives including morpholino-doxorubicin, cyanomorpholino-doxorubicin, 2-pyrrolino-doxorubicin and deoxydoxorubicin, epirubicin, esorubicin, idarubicin, marcellomycin, nitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites, such as methotrexate and 5-fluorouracil (5-FU); folic acid analogues, such as denopterin, methotrexate, pteropterin, trimetrexate; purine analogs, such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-fluorouracil; androgens, such as calusterone, dromostanolone propionate, epitiostanol, mepitiostane, testolactone; anti-adrenals, such as aminoglutethimide, mitotane, trilostane; folic acid replenisher, such as frolinic acid; aceglatone; aldophosphamide glycoside; aminolevulinic acid; amsacrine; bestrabucil; bisantrene; edatraxate; defofamine; demecolcine; diaziquone; elfomithine; elliptinium acetate; etoglucid; gallium nitrate; hydroxyurea; lentinan; lonidamine; maytansinoids, such as maytansine and N-glucosylmaytansinoids, ansamitocins, DM-1, DM-4; mitoguazone; mitoxantrone; mopidamol; nitracrine; pentostatin; phenamet; pirarubicin; podophyllinic acid; 2-ethylhydrazide; procarbazine; PSK^{®}; razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g. paclitaxel (TAXOL^{®}, Bristol-Myers Squibb Oncology, Princeton, N.J.) and doxetaxel (TAXOTERE^{®}, Rhone-Poulenc Rorer, Antony, France); chlorambucil; gemcitabine; 6-thioguanine; mercaptopurine; methotrexate; platinum coordination complex such as cisplatin, carboplatin and vinblastine; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylomithine (DMFO); retinoic acid; capecitabine; tamoxifen, raloxifene, aromatase inhibiting 4(5)-imidazoles, 4-hydroxytamoxifen, trioxifene, keoxifene, LY117018, onapristone, and toremifene (Fareston); and anti-androgens, such as flutamide, nilutamide, bicalutamide, leuprolide, and goserelin; tubulysins; amanitins, such as α-amanitin; and pharmaceutically acceptable salts, acids; dolastatin 10 or any derivative thereof; dolastatin 15 or any derivative thereof; auristatin F or any derivative thereof; monomethyl and desmethyl dolastatins 10, 15, C, D and H, monomethyl and desmethyl isodolastatin H, and analogues and derivatives thereof; monomethyl and desmethyl auristatins E, F, EB, EFP, PY, PYE, PE, PHE, TP, 2-AQ and 6-AQ; maytansinoids; N-glucosylmaytansinoid; maytansine, an ansamitocin, DM1 (also known as mertansine) or DM4 (also known as DM-4); daunorubicins, doxorubicins, detorubicin, other doxorubicin derivatives including morpholino-doxorubicin, cyanomorpholino-doxorubicin, 2-pyrrolino-doxorubicin, deoxydoxorubicin, epirubicin, esorubicin, idarubicin, rodorubicin, zorubicin, and pirarubicin; duocarmycin A, duocarmycin B1, duocarmycin B2, duocarmycin C1, duocarmycin C2, duocarmycin D, duocarmycin SA, duocarmycin MA, and CC-1065; synthetic analogs of duocarmycins, such as adozelesin, bizelesin, carzelesin, KW-2189 and CBI-TMI; duocarmycin-saccharide conjugate of Formula DS; tubulysin; α-amanitin; cryptophycin; monomethylauristatin E; an auristatin saccharide conjugate of Formula AS; MMAU; monomethylauristatin F, W or M; a pyrrolobenzodiazepine (PBD), abbeymycin, chicamycin, DC-81, mazethramycin, neothramycins A and B, porothramycin, prothracarcin, sibiromycin, tomamycin, and a PBD dimer; or an analogue of any of the above.
7. The linker-payload conjugate of any preceding embodiment or the targeting unit-linker-payload conjugate of any preceding embodiment, wherein (i) R₃ is selected from the group consisting of a side chain of α-amino acid, serine, threonine and tyrosine, (ii) Z is selected from the group consisting of para-aminobenzyloxycarbonyl (PABC); orto-aminobenzyloxycarbonyl; an amino acid; and a peptide; or Z is absent; (iii) R₁ is selected from the group consisting of the side chain of valine, sthe ide chain of phenylalanine, the side chain of tyrosine, the side chain of leusine, the side chain of isoleusine, the side chain of arginine, the side chain of alanine, the side chain of lysine and the side chain of glycine; and/or (iv) R_{X} is selected from the group consisting of a linear C₁-C₆ alkylene group; a branched C₁-C₆ alkylene group; CH(R₂), wherein R₂ is an amino acid side chain; and -CH₂CH₂-.
8. The linker-payload conjugate of any preceding embodiment, wherein the conjugate is 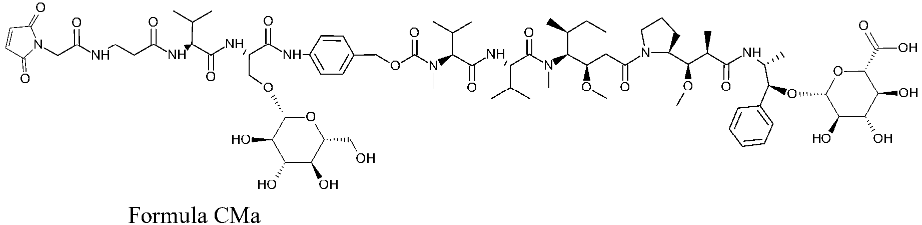 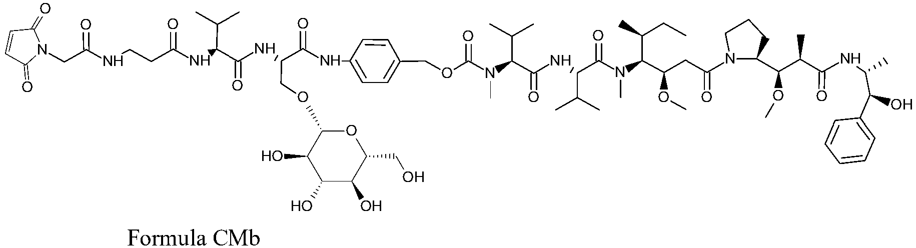 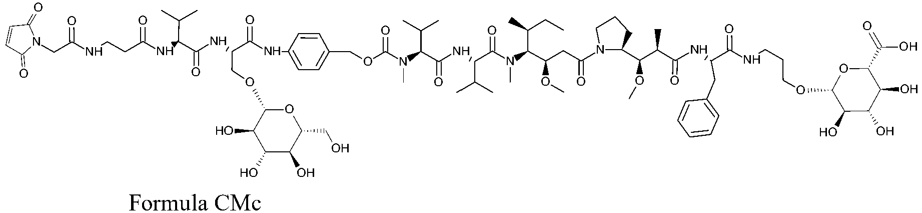 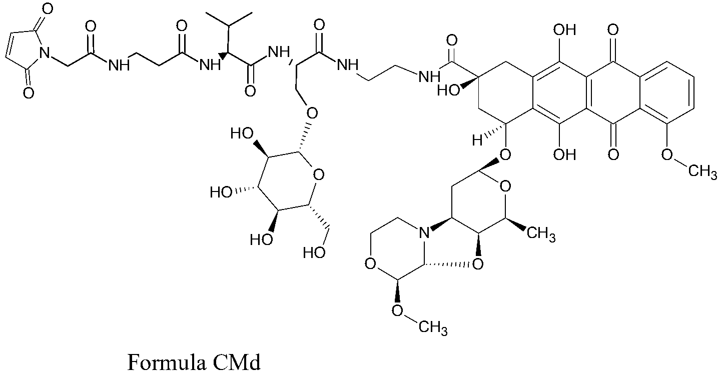 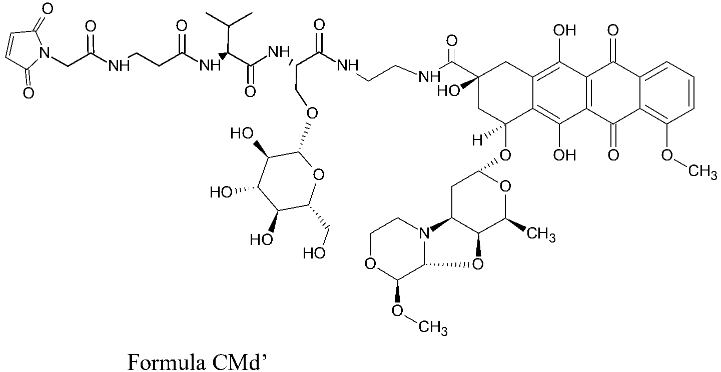 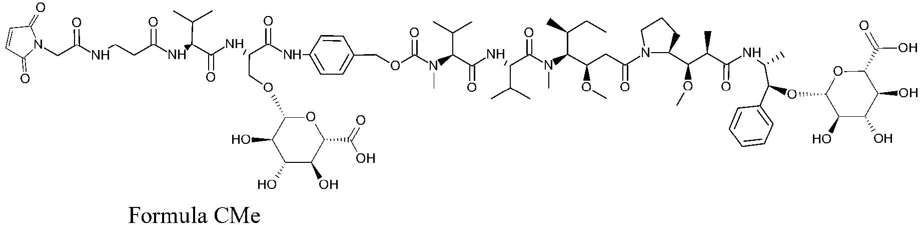 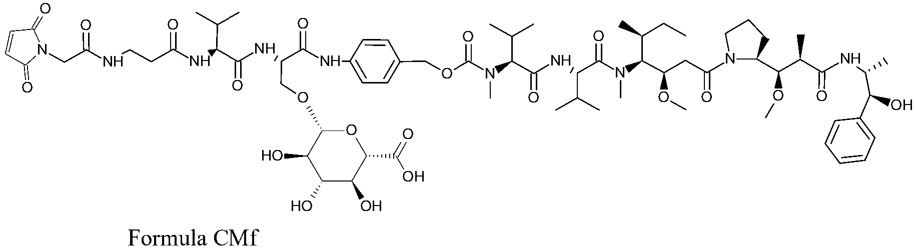 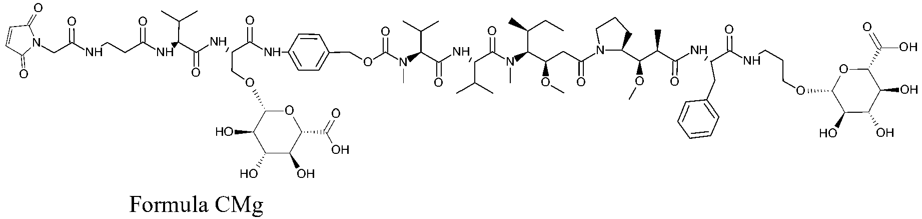 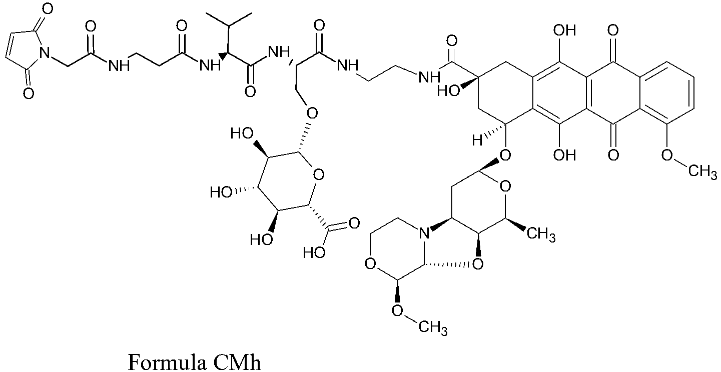 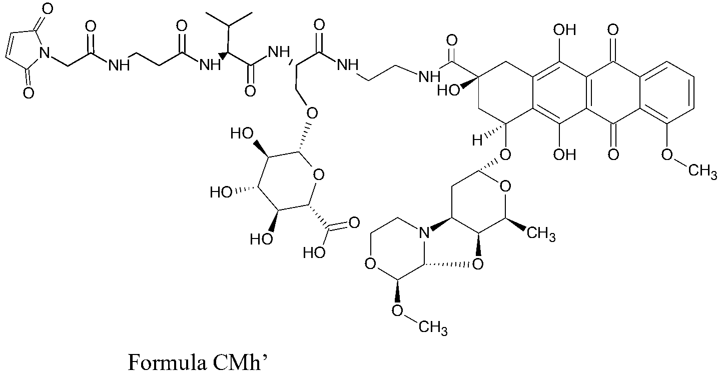 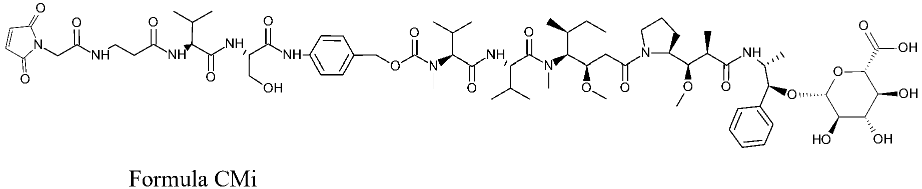 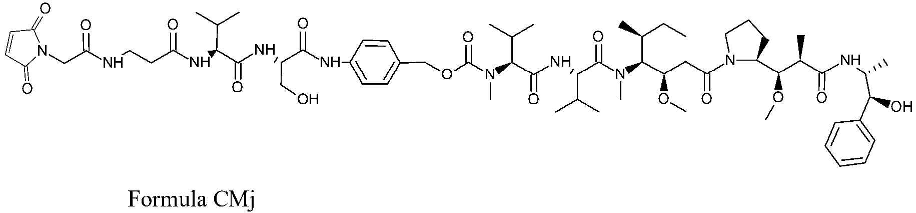 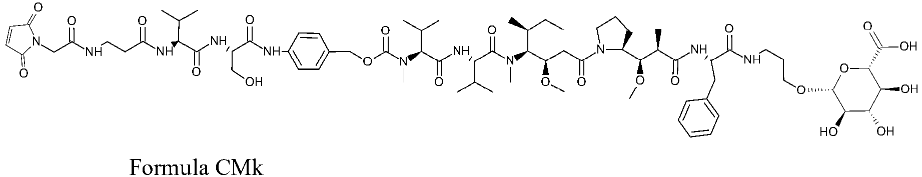 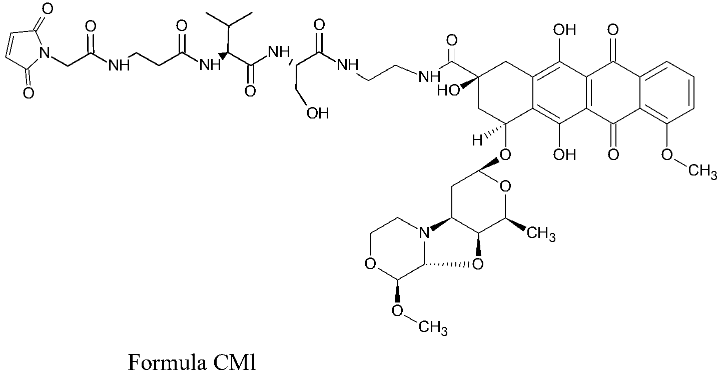 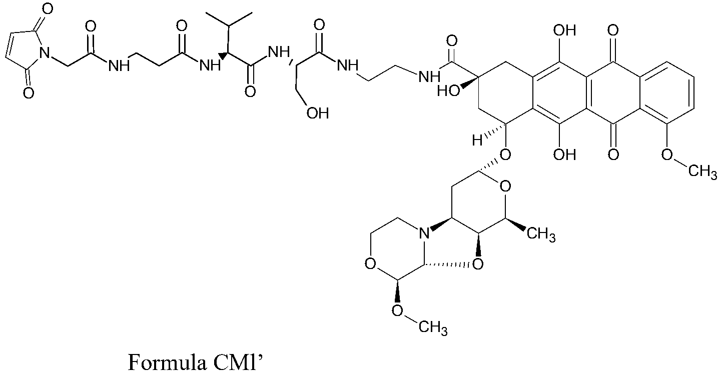 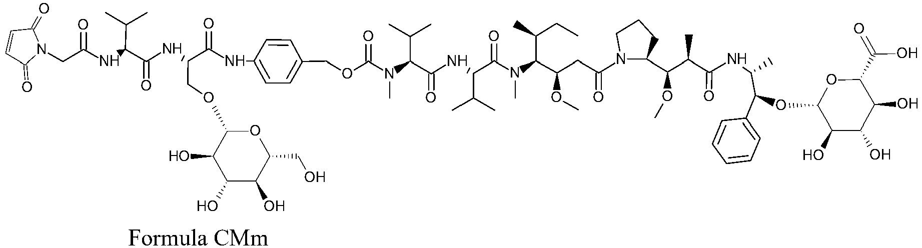 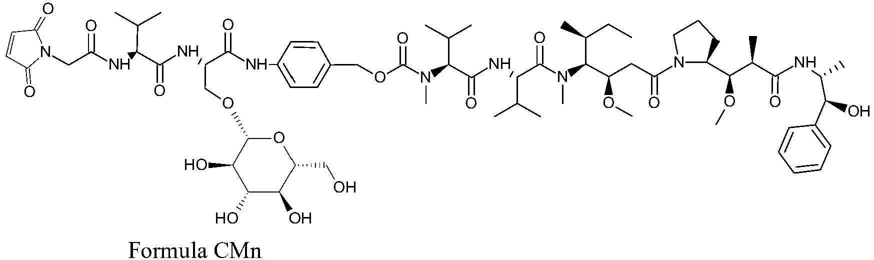 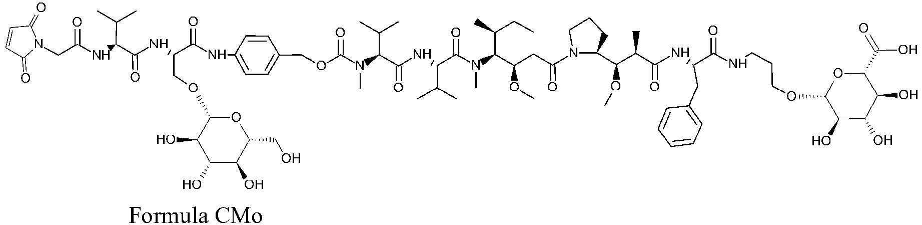 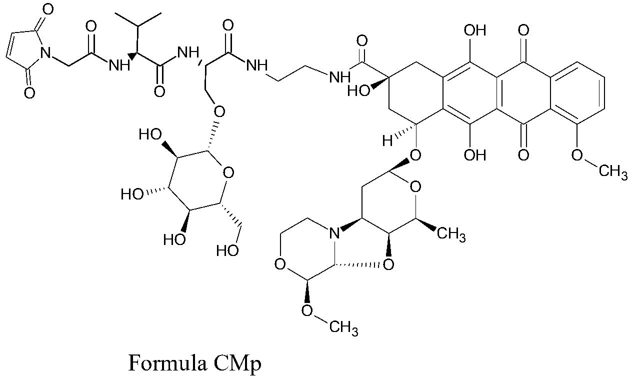 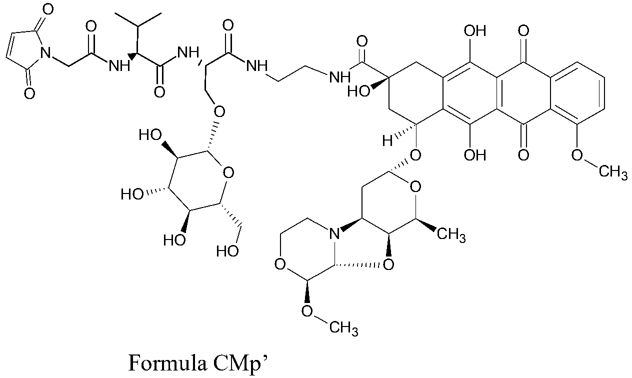 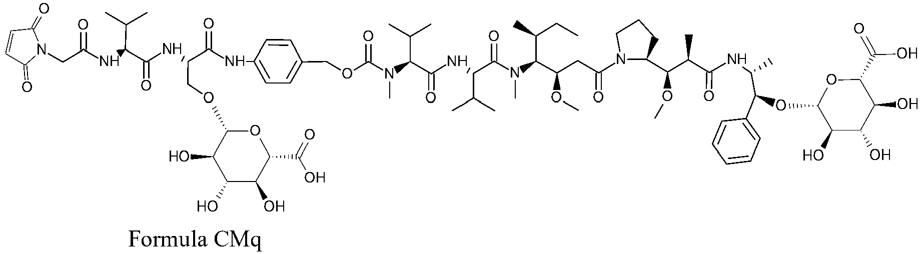 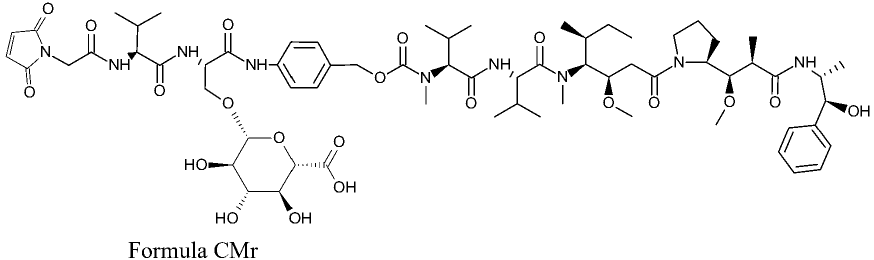 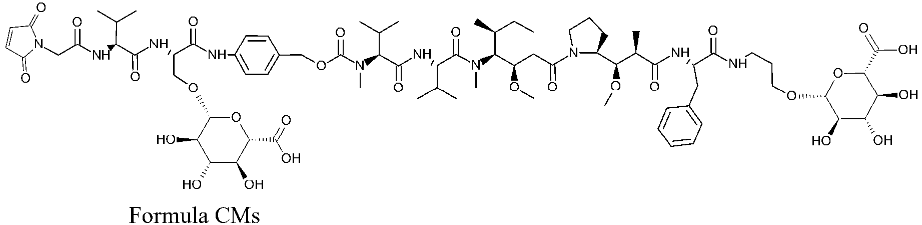 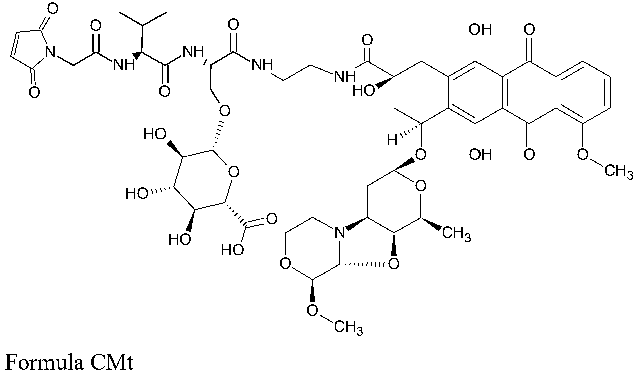 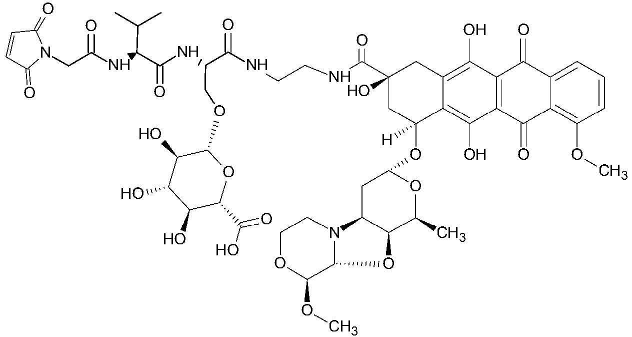 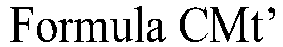 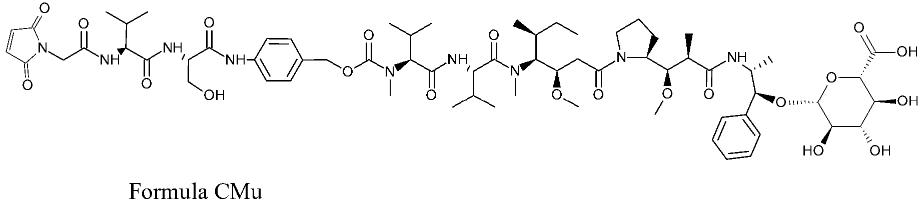 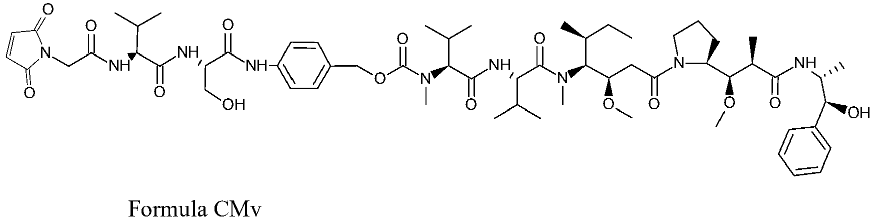 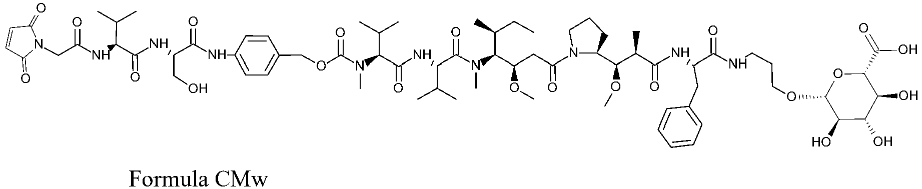 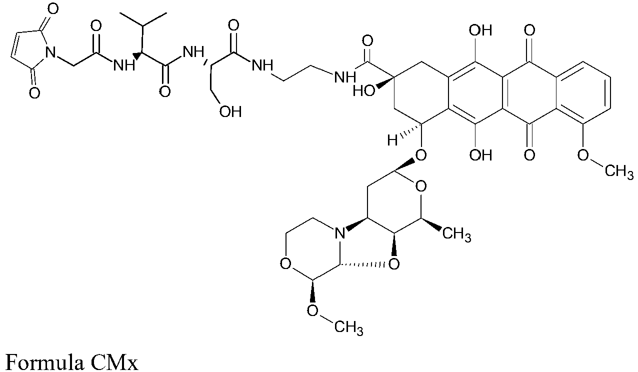 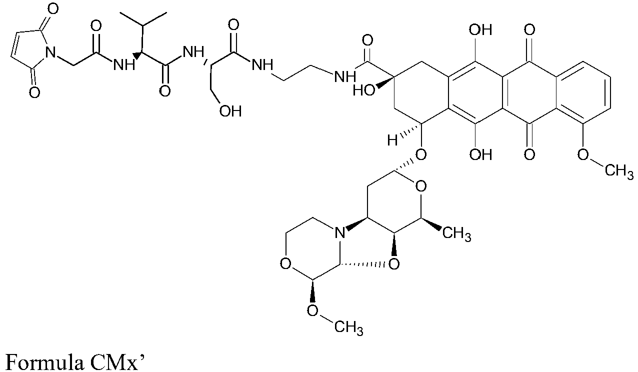 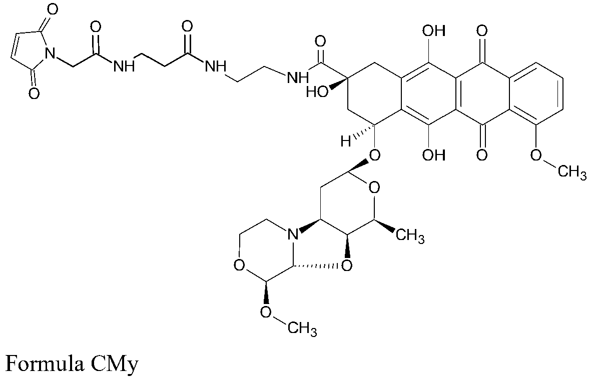 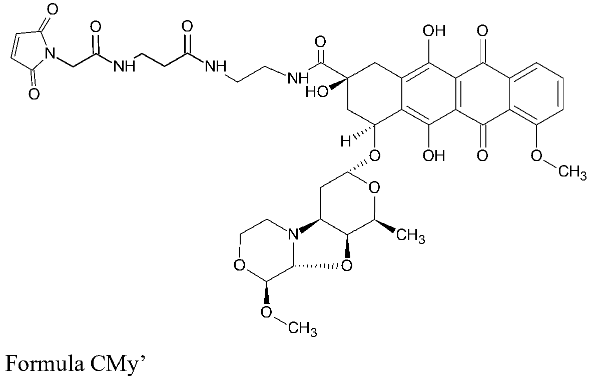 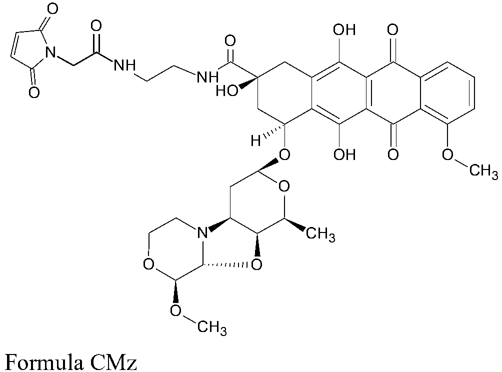 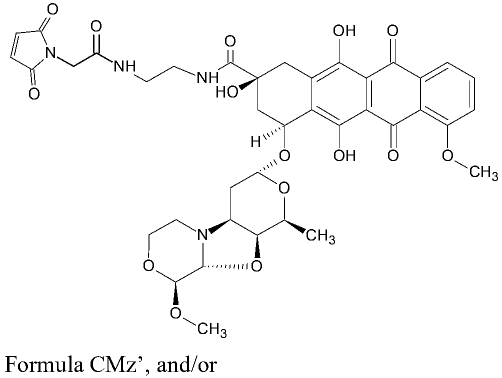 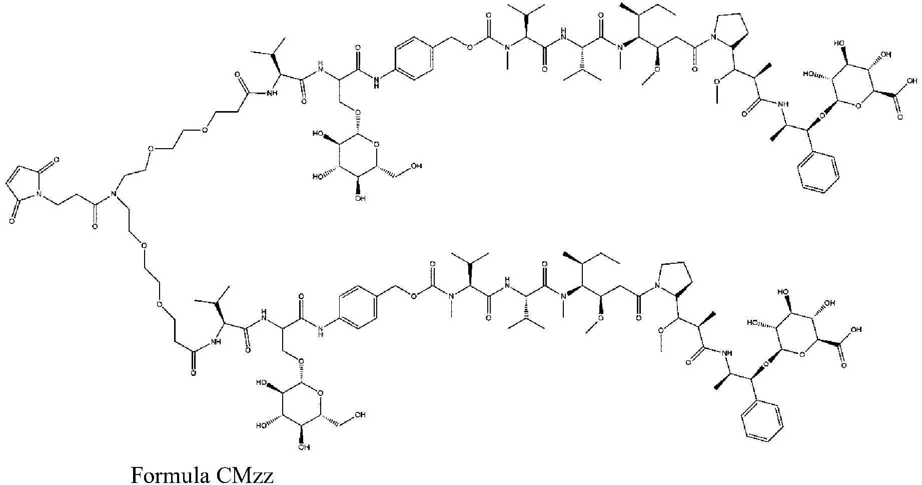
9. The targeting unit-linker-payload conjugate of any preceding embodiment, wherein the targeting unit is an antibody.
10. The targeting unit-linker-payload conjugate of embodiment 9, wherein the antibody is selected from the group consisting of bevacizumab, tositumomab, etanercept, trastuzumab, adalimumab, alemtuzumab, gemtuzumab ozogamicin, efalizumab, flanvotumab, rituximab, infliximab, abciximab, basiliximab, palivizumab, omalizumab, daclizumab, cetuximab, panitumumab, epratuzumab, 2G12, lintuzumab, nimotuzumab and ibritumomab tiuxetan, or the antibody is selected from the group consisting of an anti-EGFR antibody, an epidermal growth factor receptor 2 (HER2/neu) antibody, an anti-CD22 antibody, an anti-CD30 antibody, an anti-CD33 antibody, an anti-Lewis y antibody, anti-TYRP-1, an anti-CD20 antibody and an anti-hematologic target antibody.
11. The targeting unit-linker-payload conjugate of embodiment 9 or 10, wherein the antibody is capable of binding an anti-hematologic target molecule selected from the group consisting of CD19, CD20, CD22, CD25, CD30, CD33, CD37, CD38, CD52, CD56, CD70, CD74, CD79, CD98, CD117, CD105, CD123, CD138, CD157, BCMA and CD319 (SLAMF7).
12. The targeting unit-linker-payload conjugate of embodiment 11, wherein the target molecule is selected from the group consisting of CD19, CD22, CD33, CD52 and CD123.
13. The targeting unit-linker-payload conjugate of any one of embodiments 9-12, wherein the antibody is a hematologic target antibody selected from the group consisting of Loncastuximab, Blinatumomab, Tafasitamab, Coltuximab, denintuzumab, Obexelimab, Inebilizumab, MOR00208, MDX-1342, MEDI-551, SAR3419, Rituximab, Ofatumumab, Veltuzumab, Ocrelizumab, Obinutuzumab, Ocaratuzumab, Ublituximab, nofetumomab, ibritumomab, Epratuzumab, Inotuzumab ozogamicin, bectumomab, moxetumomab, pinatuzumab, DCDT2980S, Basiliximab, Daclizumab, camidanlumab, inolimomab, ADCT-301, IMTOX-25, Brentuximab, iratumumab, AVE9633, lintuzumab, gemtuzumab, vadastuximab, otlertuzumab, lilotomab, naratuximab, BI836826, AGS67E, IMGN529, Daratumumab, Isatuximab, mezagitamab, felzartamab, MOR202, MOR03087, Alemtuzumab, Lorvotuzumab mertansine, Vorsetuzumab mafodotin, SGN-70A, polatuzumab, indatuximab, MDX-1203, Milatuzumab-doxorubicin, IGN523, LOP-628, CSL360, Talacotuzumab, XmAb14045, KHK2823, BT062, Belantamab mafodotin, teclistamab and Elotuzumab.
14. The targeting unit-linker-payload conjugate of embodiment 13, wherein the antibody is selected from the group consisting of epratuzumab, lintuzumab, coltuximab, denintuzumab, loncastuximab, alemtuzumab and talacotuzumab.
15. The targeting unit-linker-payload conjugate of any one of embodiments 9 to **14,** wherein the antibody is a cysteine engineered antibody.
16. The targeting unit-linker-payload conjugate of embodiment 15, wherein the antibody has one or more amino acid deletions or substitutions of heavy chain selected from the group consisting of A40, **P41,** A84, V89, S112, S113, A114, S115, T116, G118, V152, S153, N155, A168, Q171, C220, 225, 226, 229, 247, V278, N297, 339, S371, 375, 376, S396, and E400 (according to Kabat)
17. The targeting unit-linker-payload conjugate of embodiment 15, wherein the antibody has one or more amino acid deletions or substitutions of light chain selected from the group consisting of V110, S114, S121, S127, 143, 147, A153, 159, 163, 165, S168, V205, and 214 (according to Kabat).
18. The targeting unit-linker-payload conjugate of embodiment 16 or 17, wherein the asparagine at position 297 (N297) on the IgG heavy chain is deleted or substituted, the cysteine at position 220 (C220), C226 or C229 on the IgG heavy chain is deleted or substituted, or the cysteine at position 214 (C214) of the IgG light chain (kappa or lambda) is deleted or substituted.
19. The targeting unit-linker-payload conjugate of any preceding embodiment, wherein the conjugate is 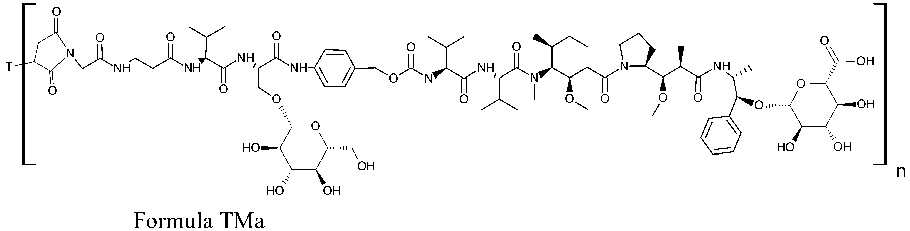 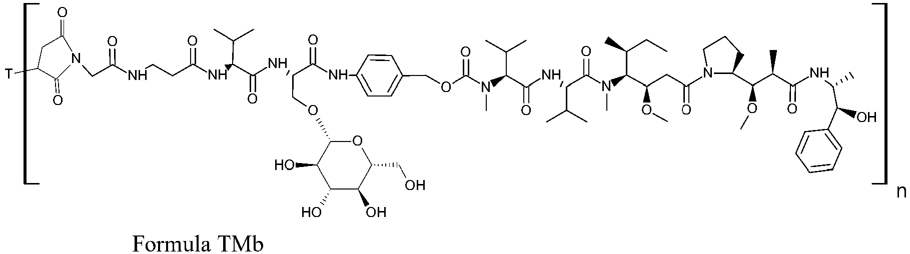 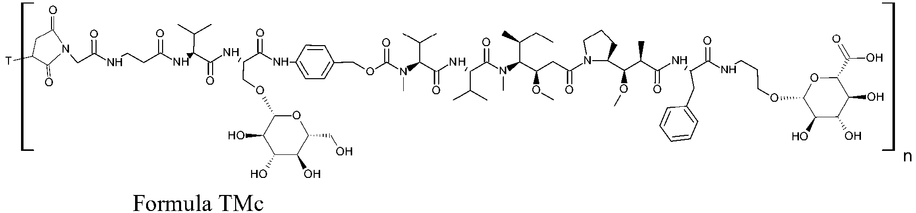 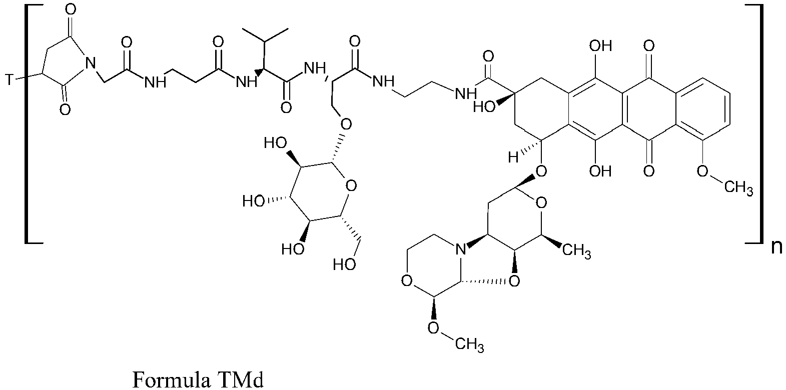 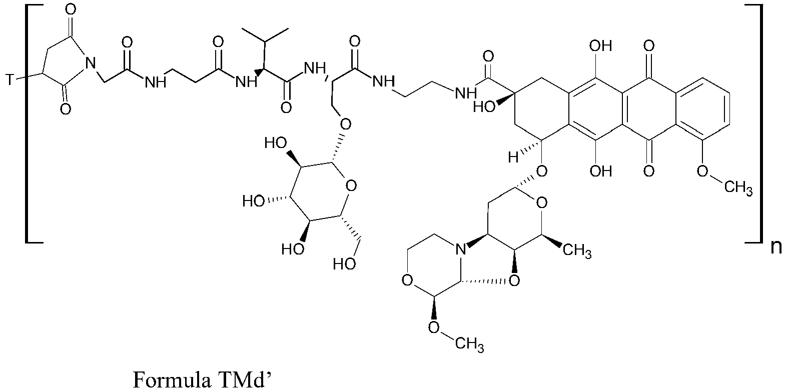 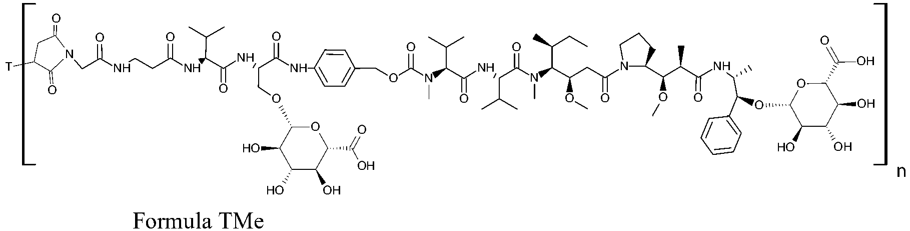 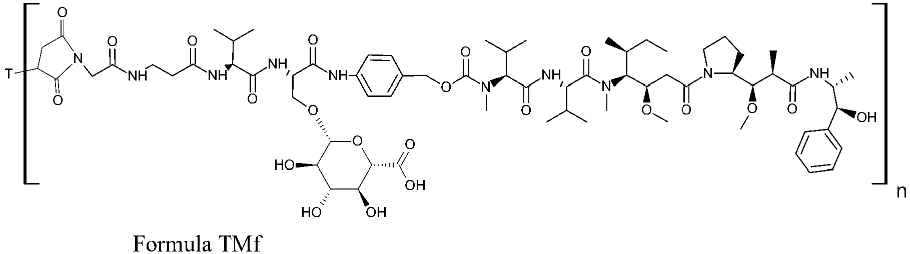 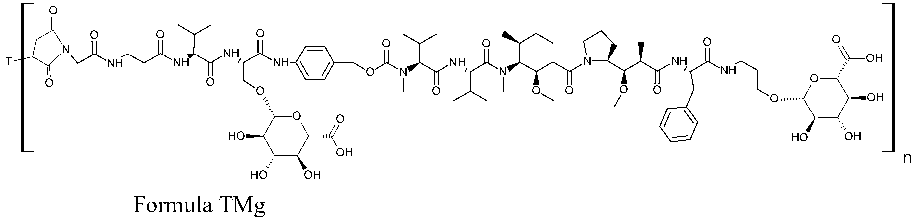 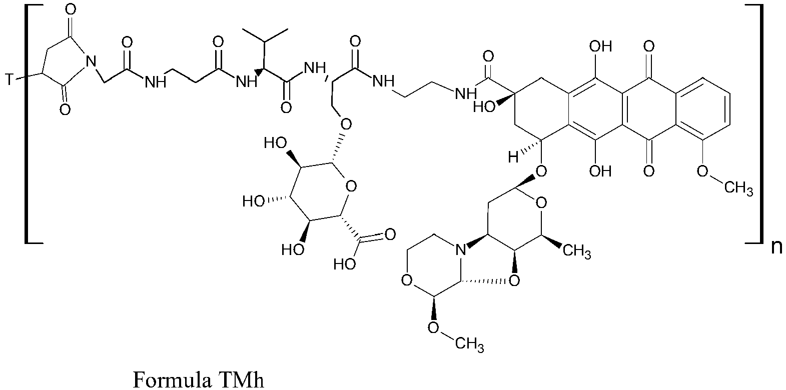 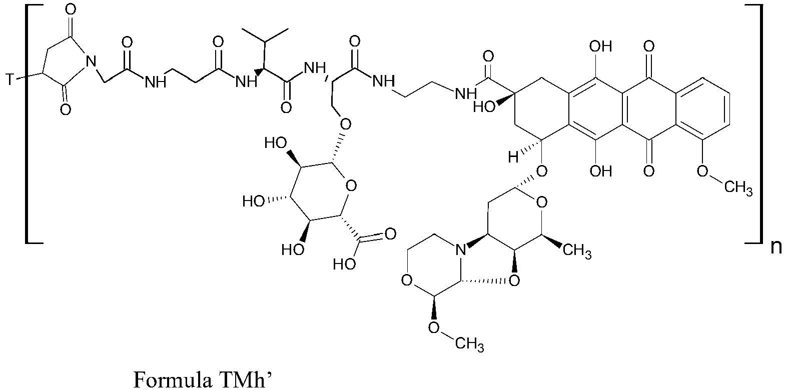 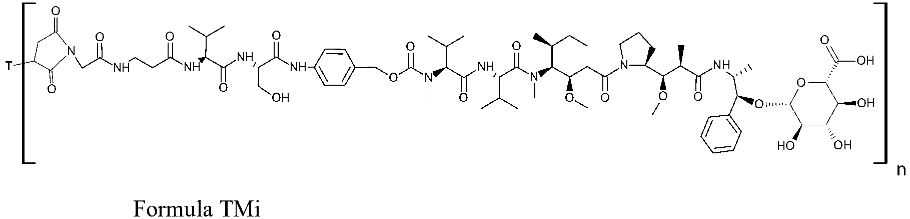 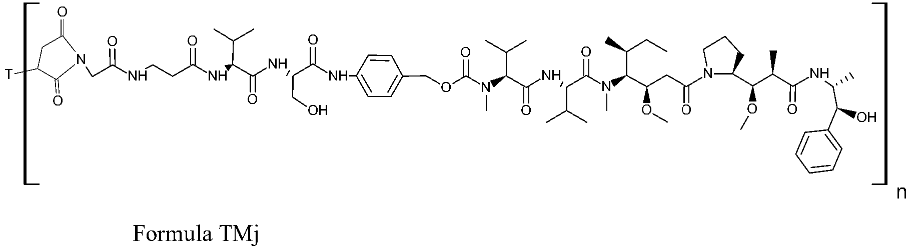 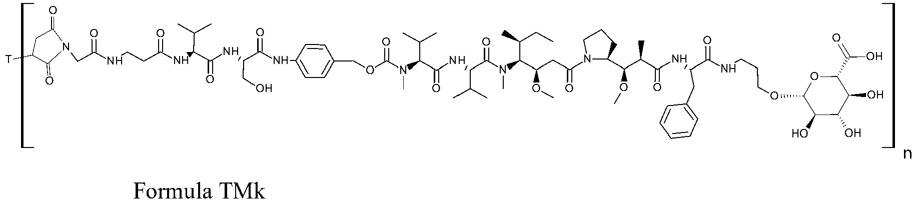 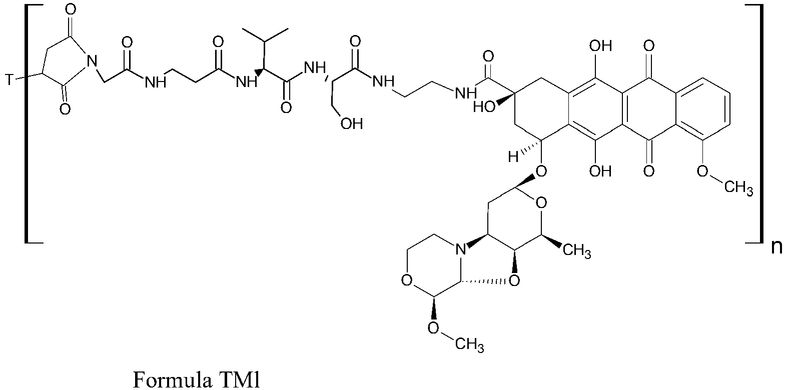 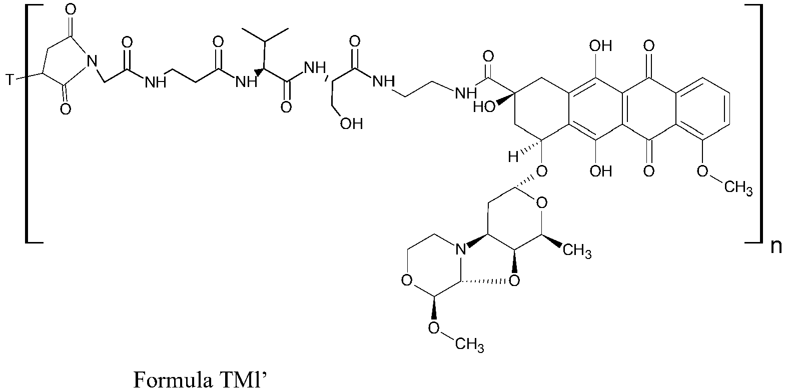 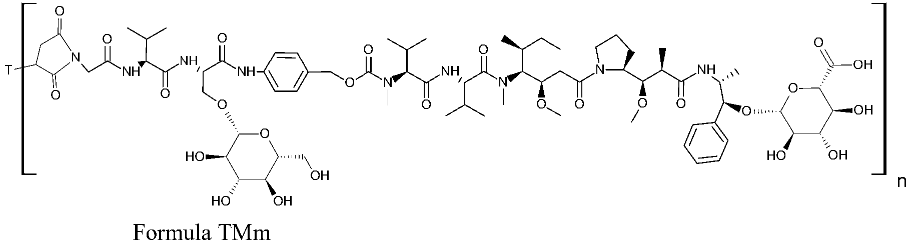 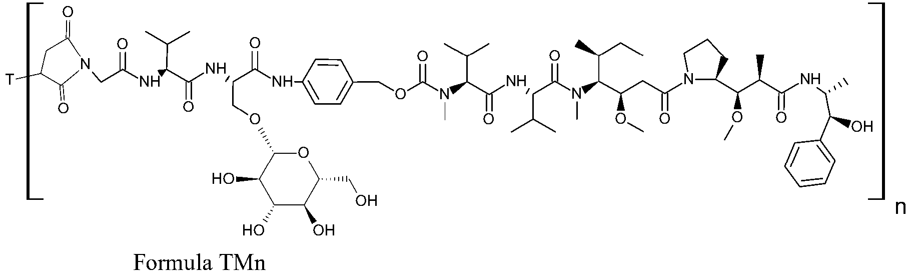 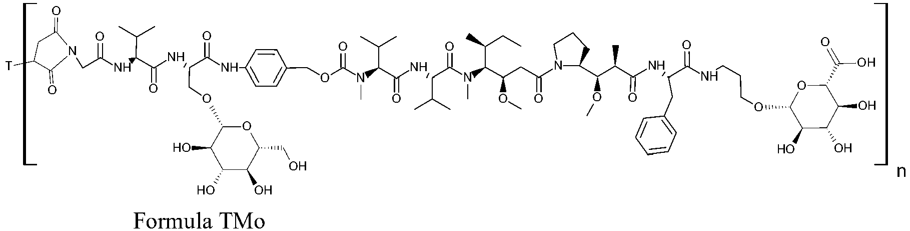 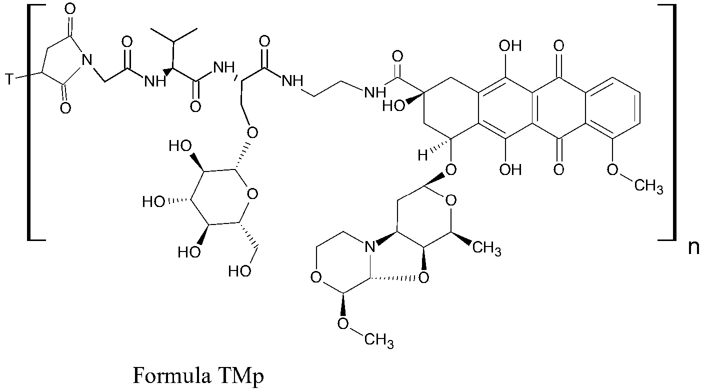 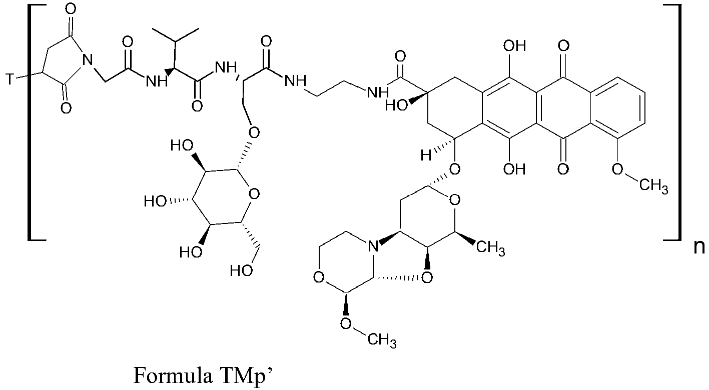 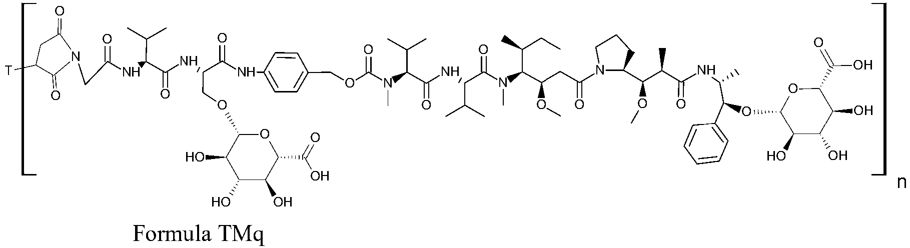 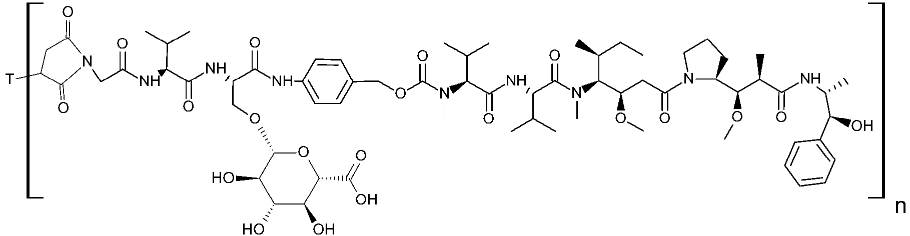 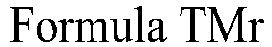 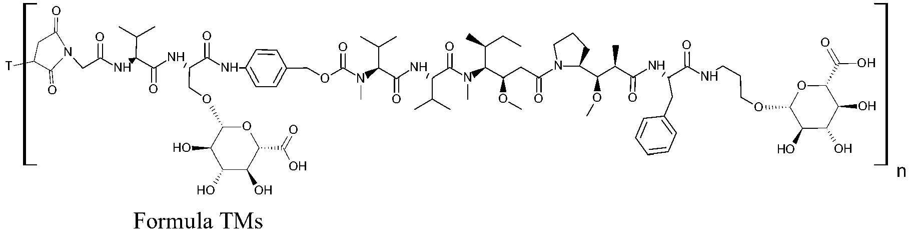 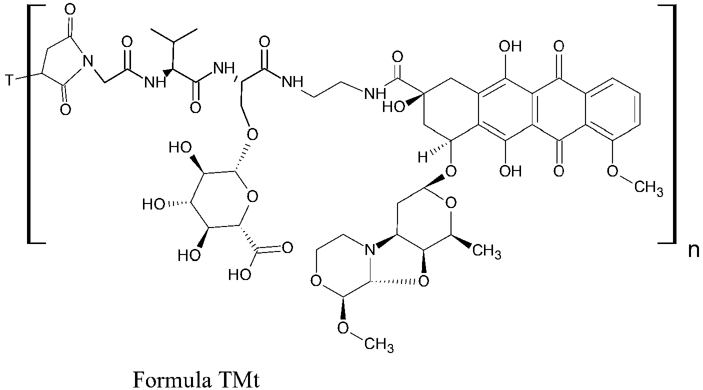 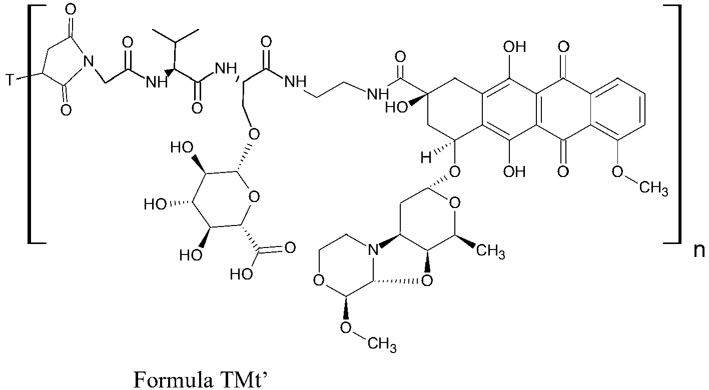 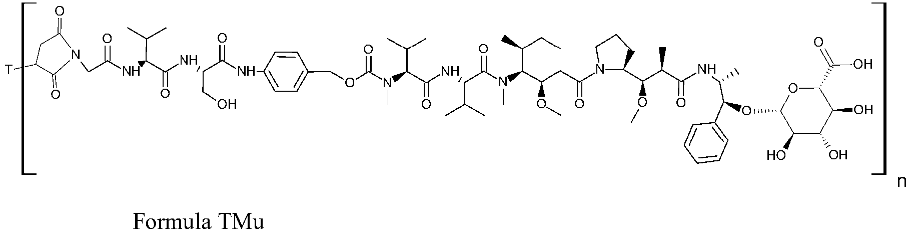 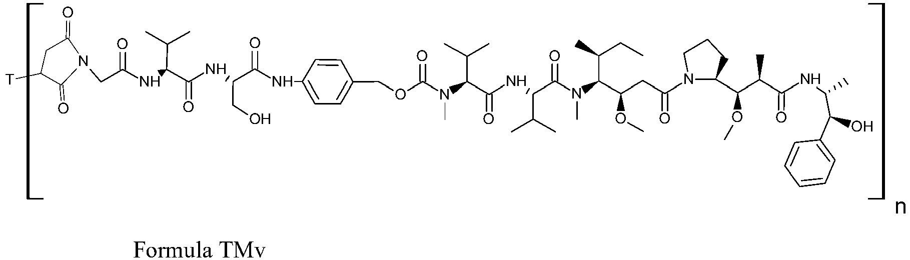 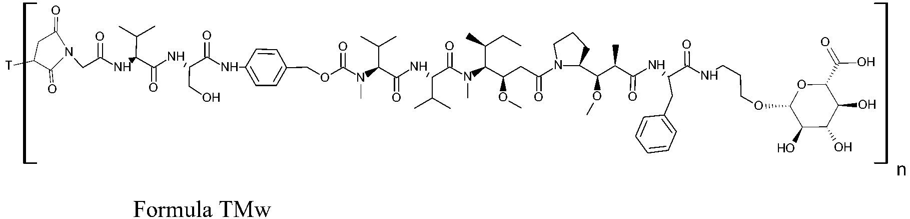 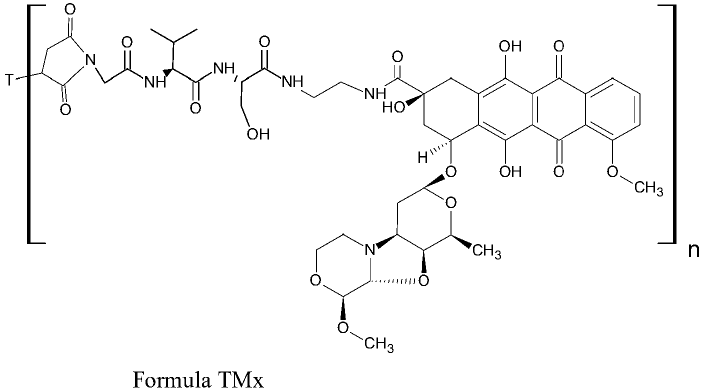 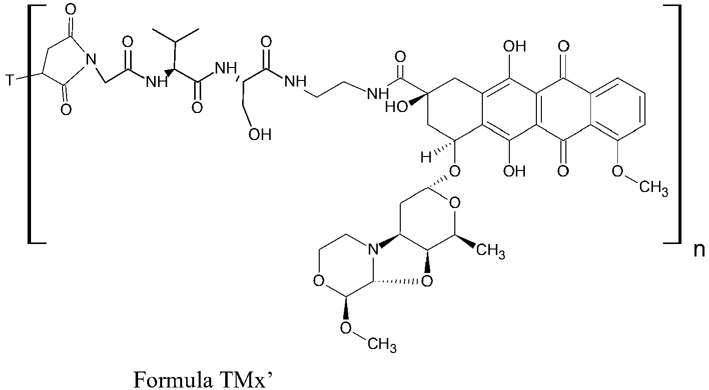 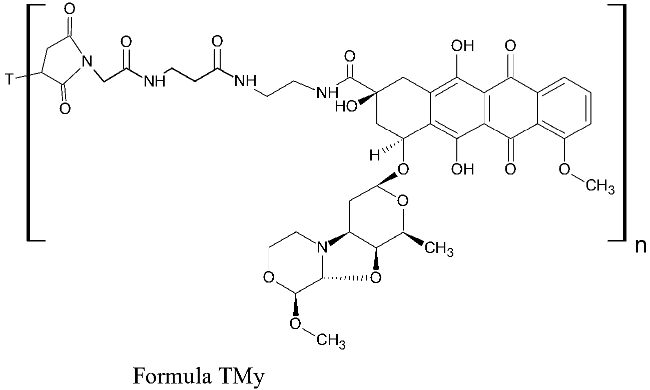 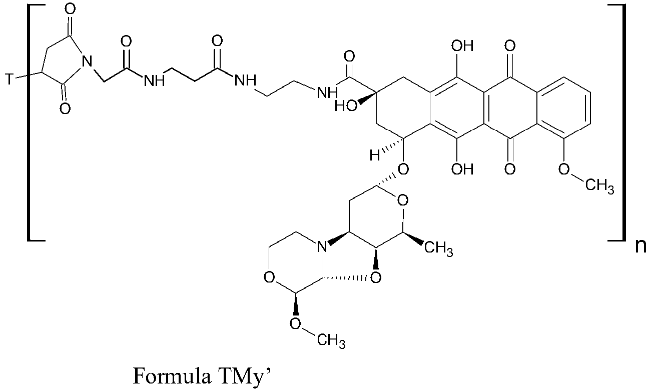 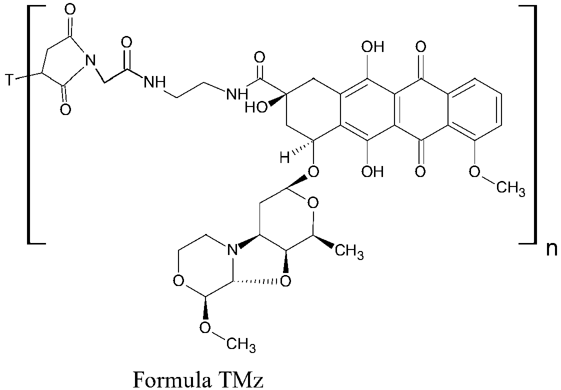 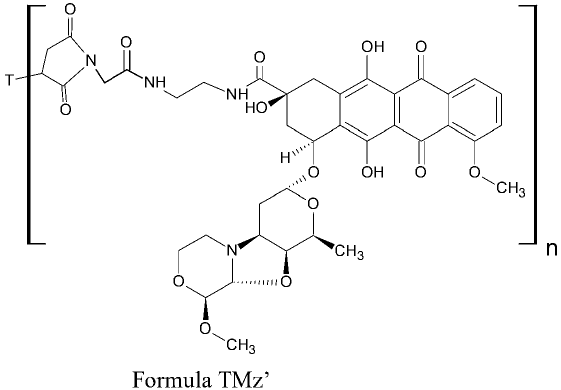 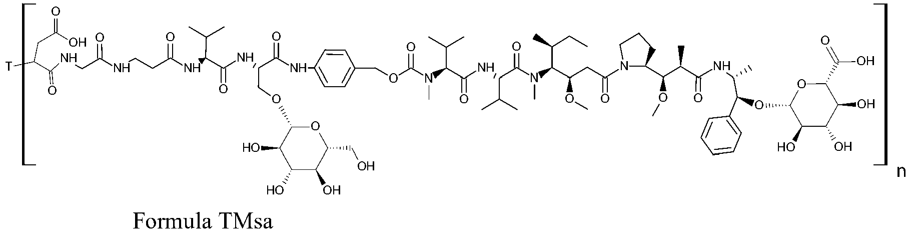 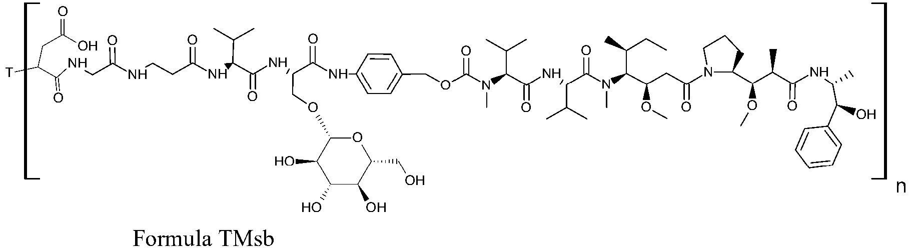 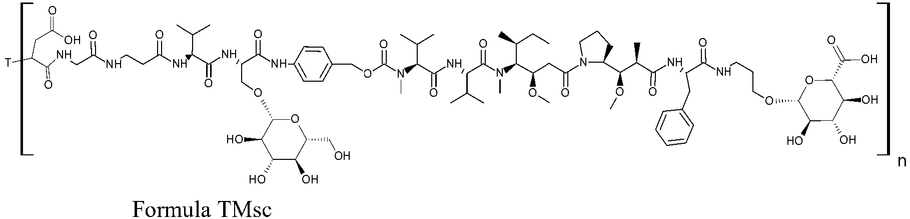 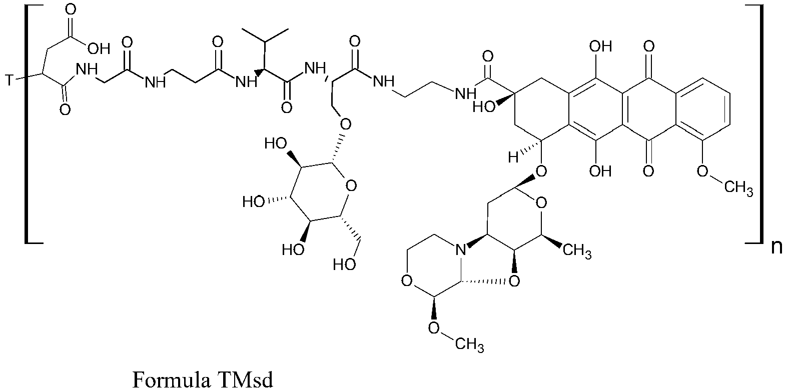 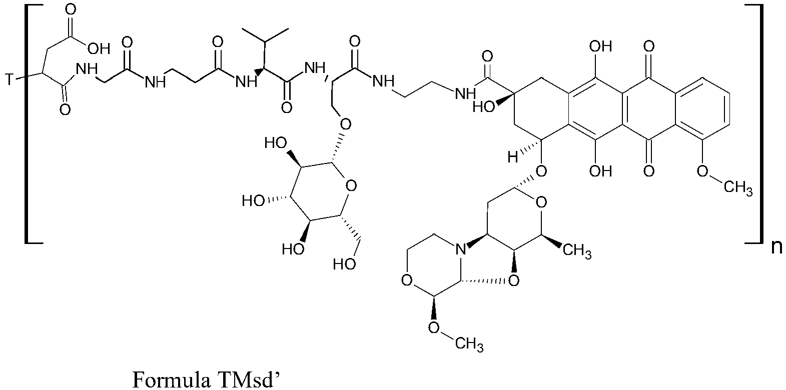 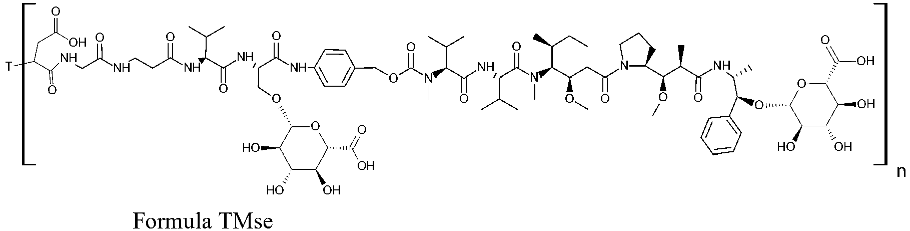 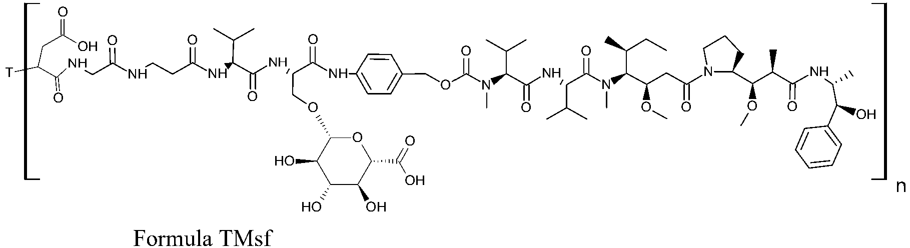 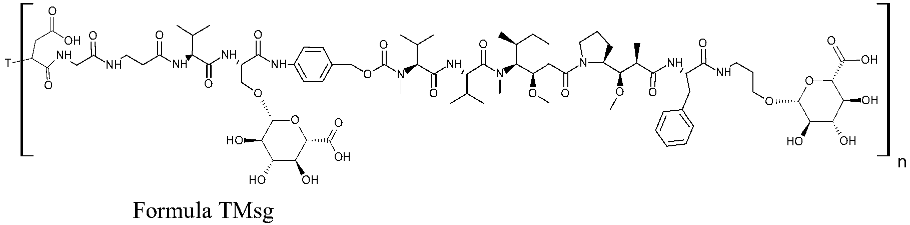 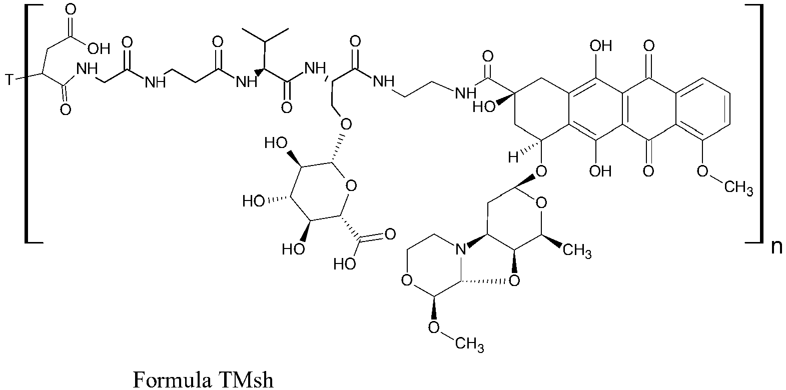 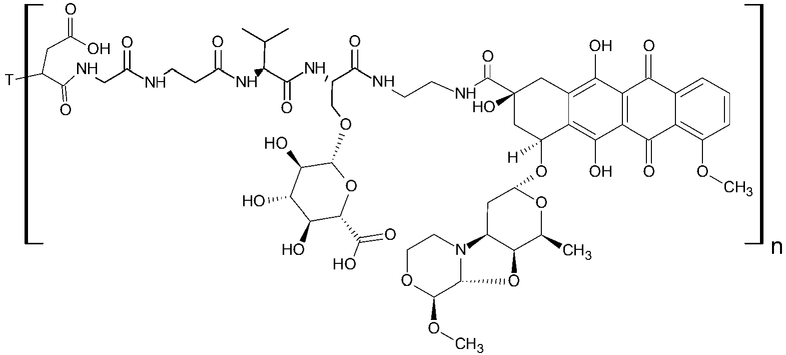 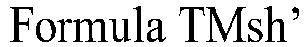 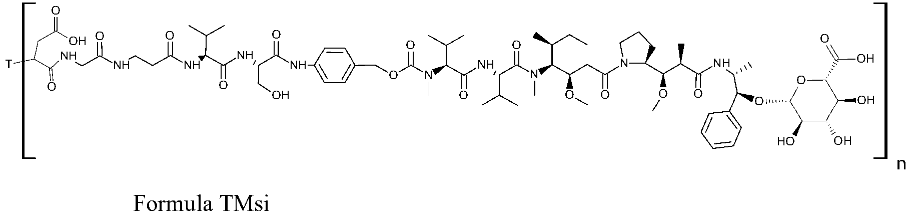 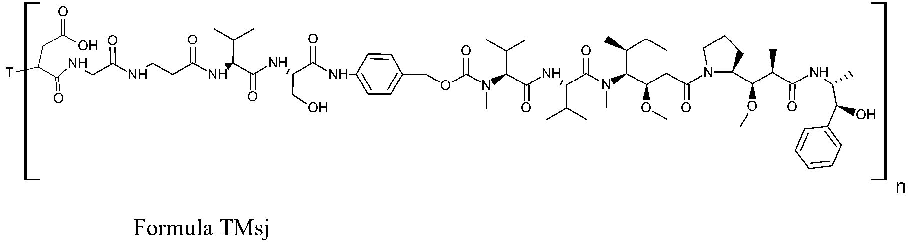 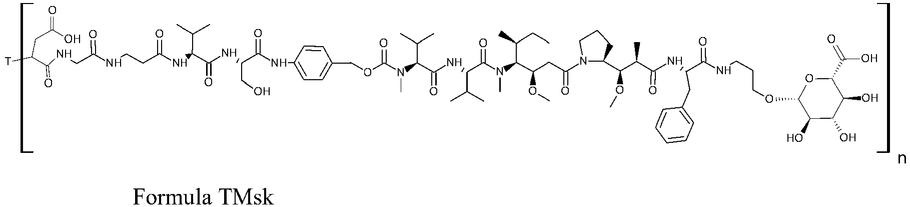 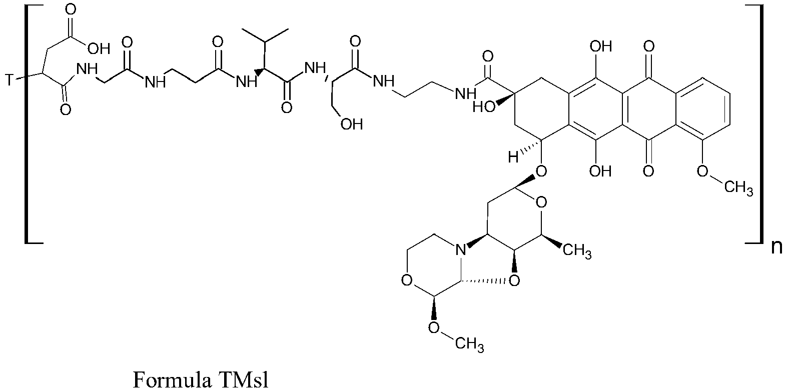 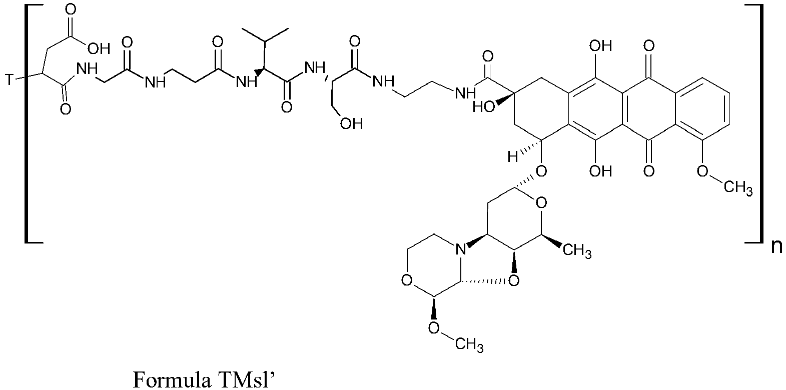 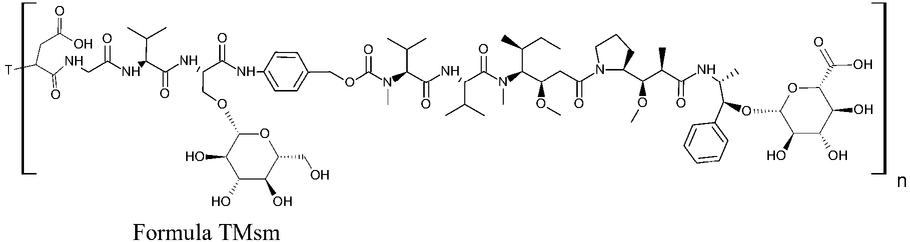 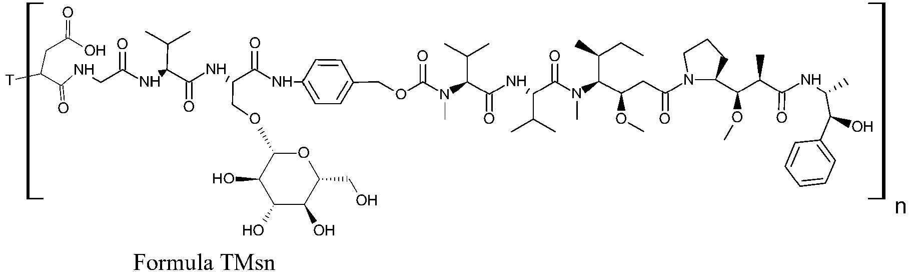 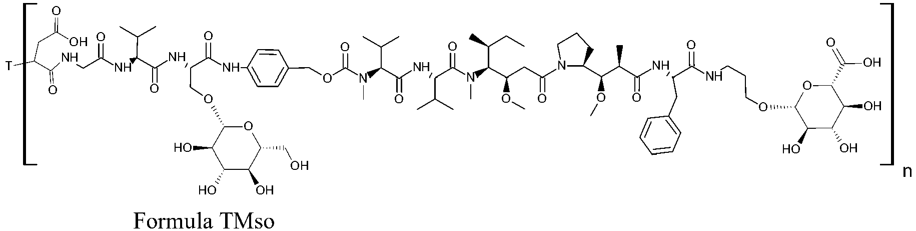 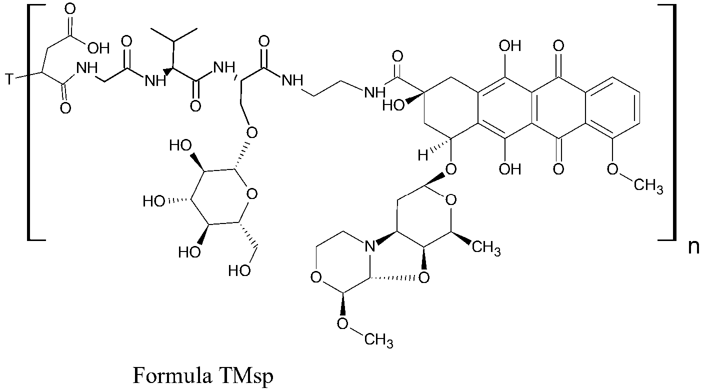 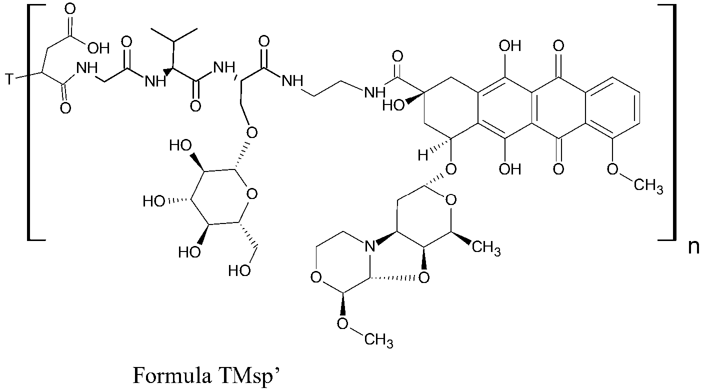 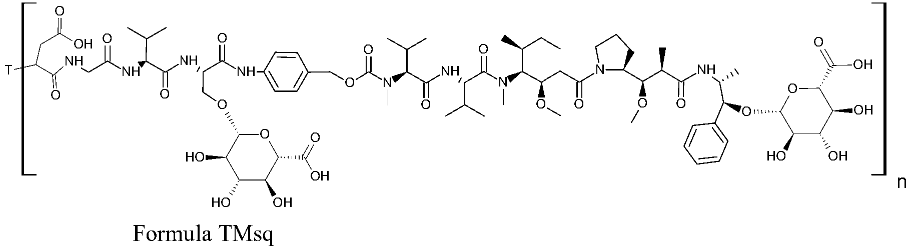 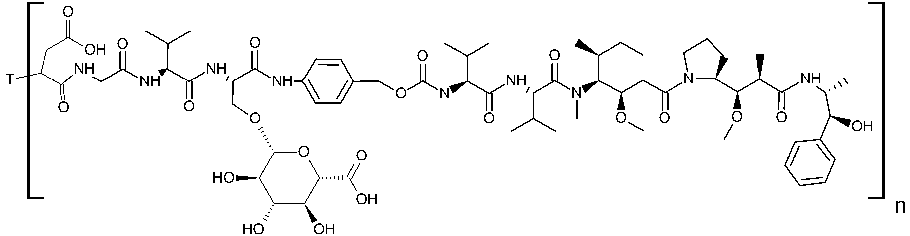 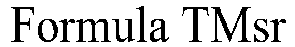 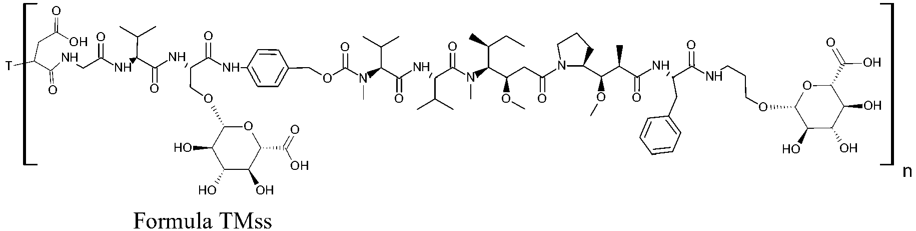 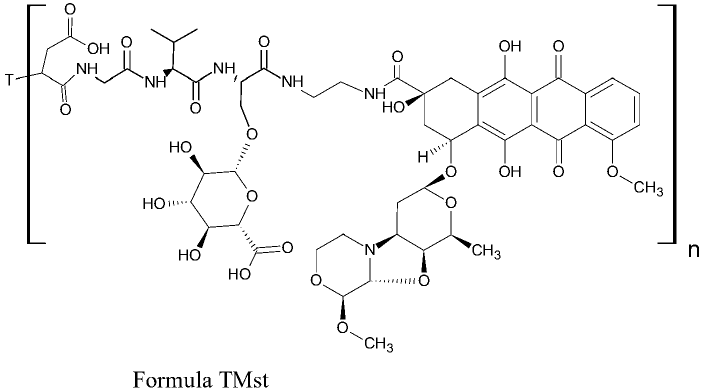 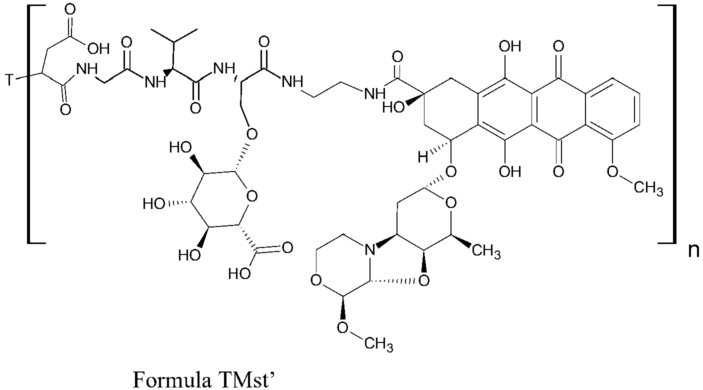 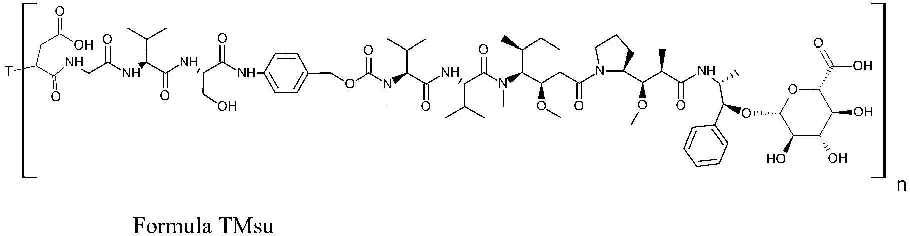 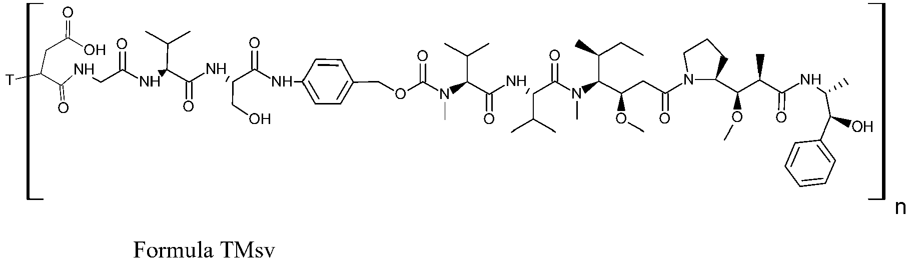 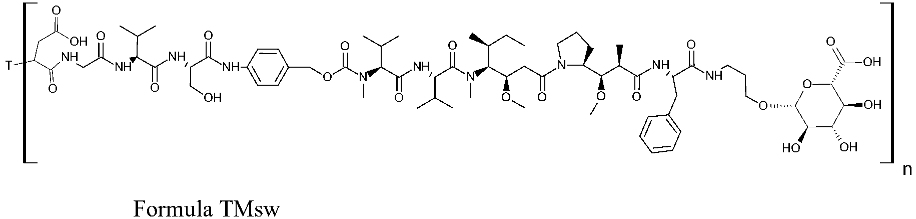 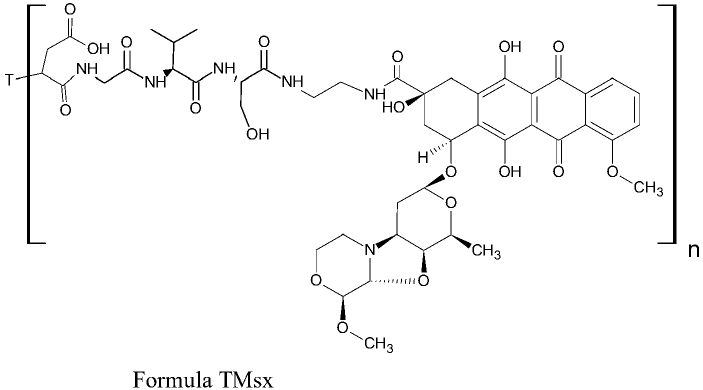 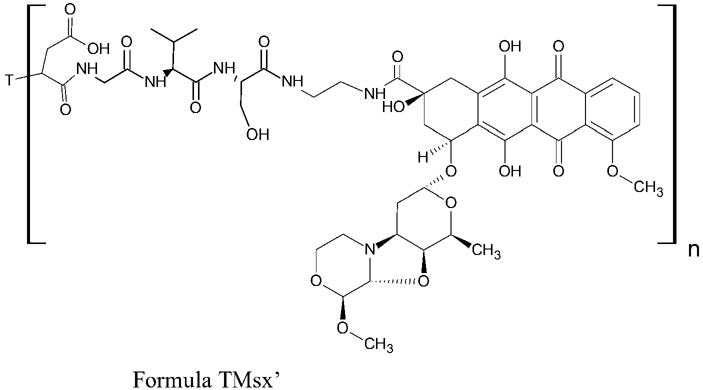 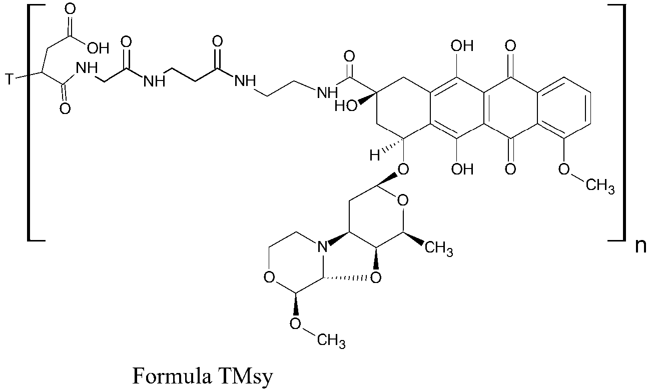 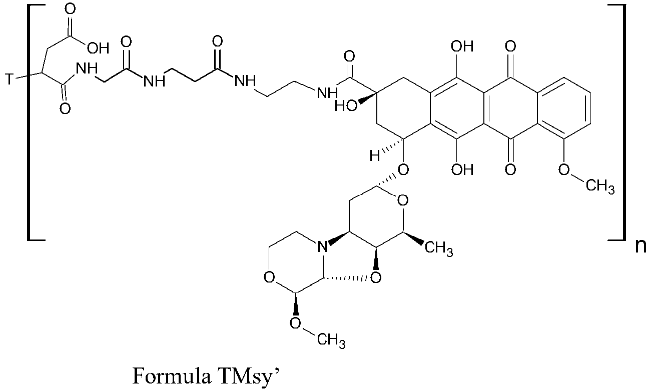 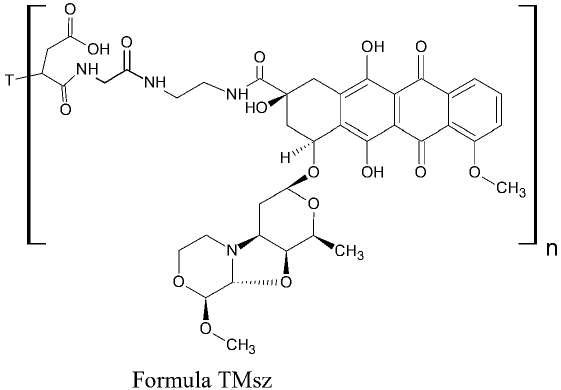 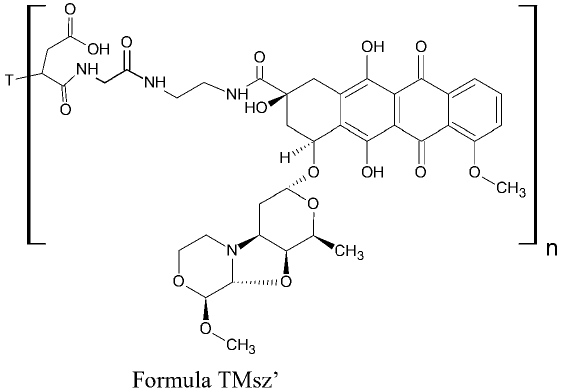 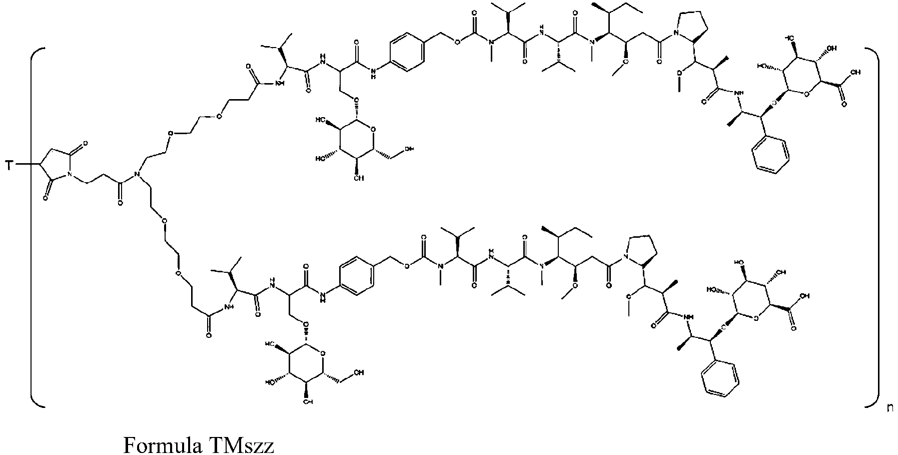
20. A targeting unit-linker-payload conjugate selected from the group consisting of 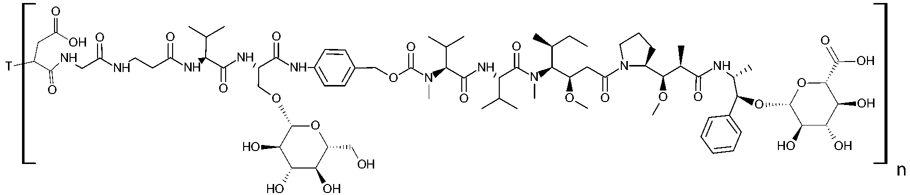
   wherein T is an antibody; 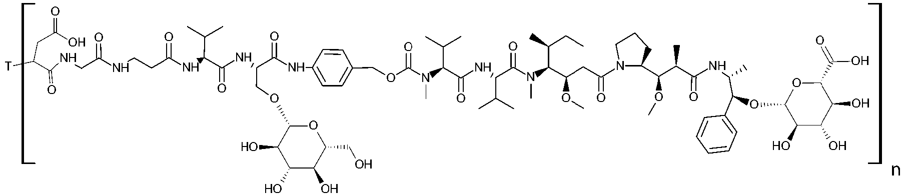
   wherein T is an antibody and n is 8; 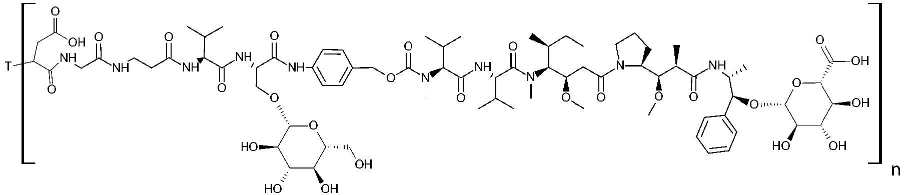
   wherein T is an anti-HER2 antibody; 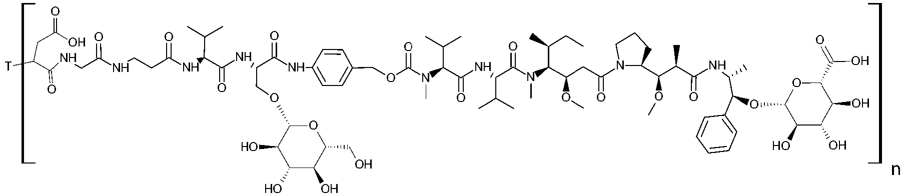
   wherein T is trastuzumab; 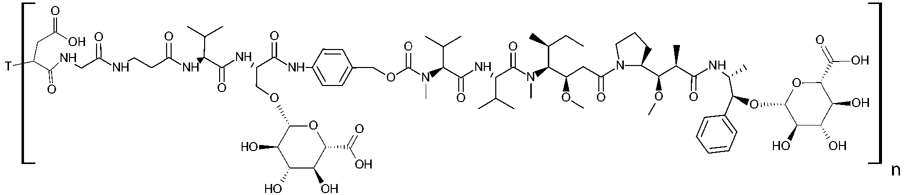
   wherein T is an anti-HER2 antibody; 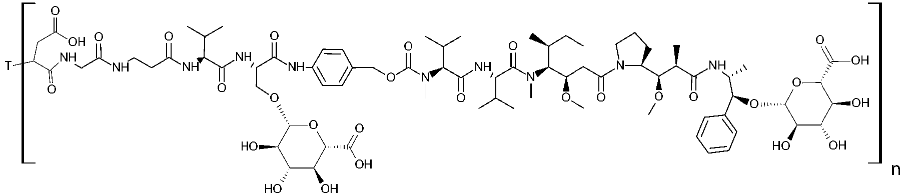
   wherein T is trastuzumab; 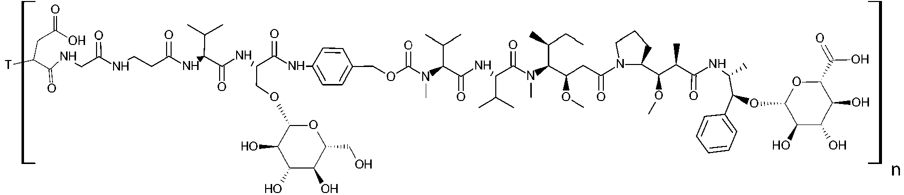
   wherein T is an anti-CD33; 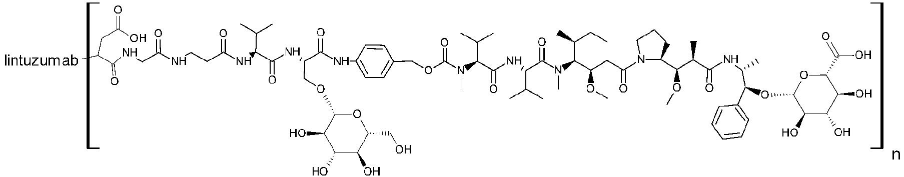 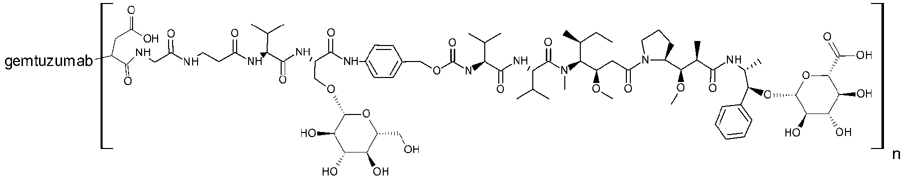 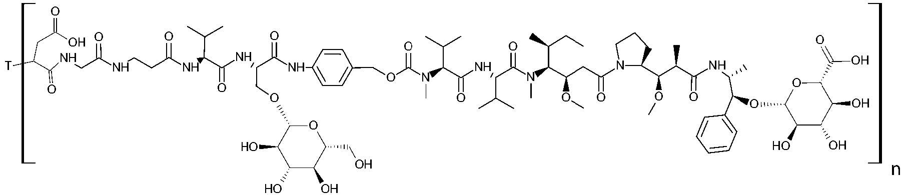
   wherein T is an anti-TYRP1 antibody; 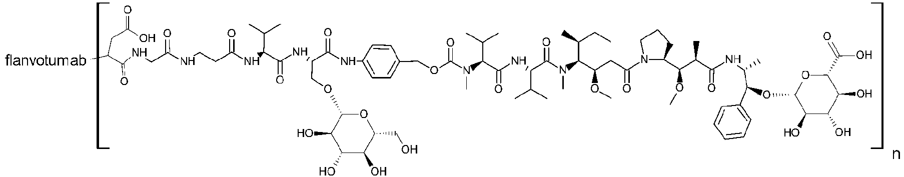 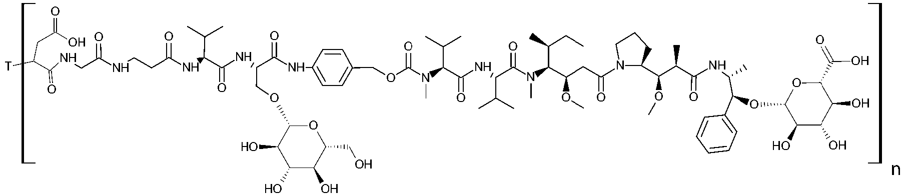
   wherein T is an anti-CD22 antibody; 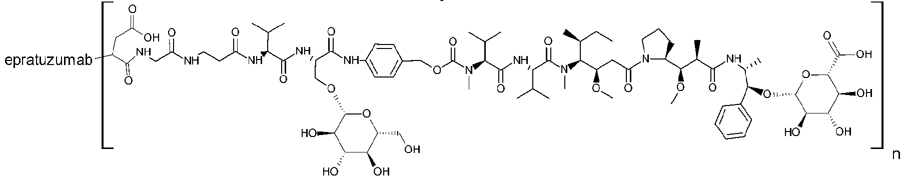 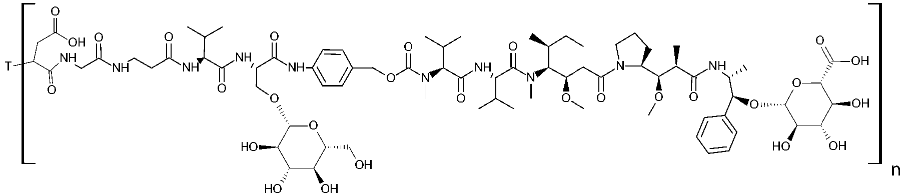
   wherein T is an anti-CD19 antibody; 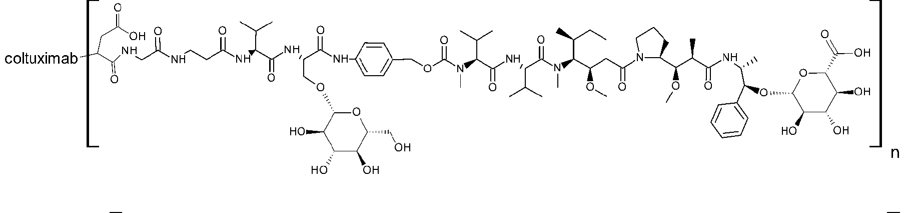 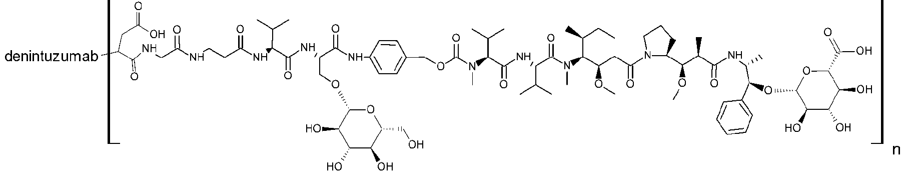 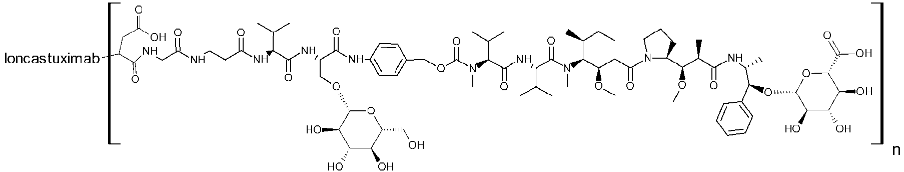 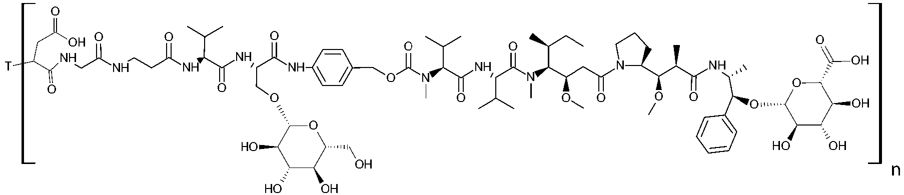
      wherein T is an anti-CD52 antibody; 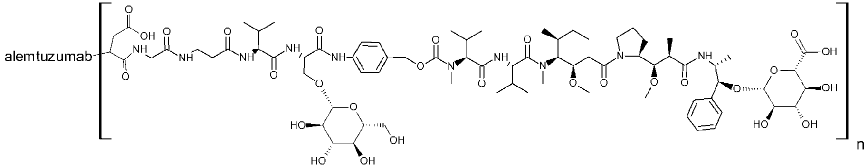 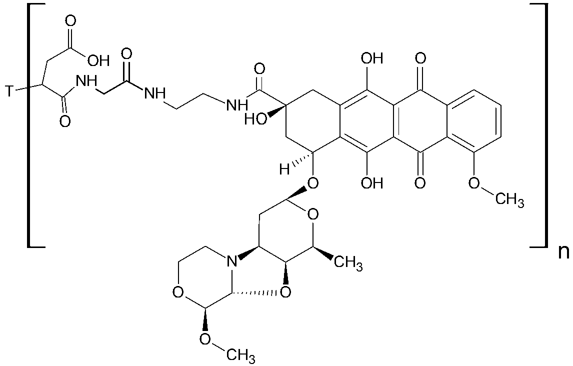
      wherein T is lintuzumab HC N296C; 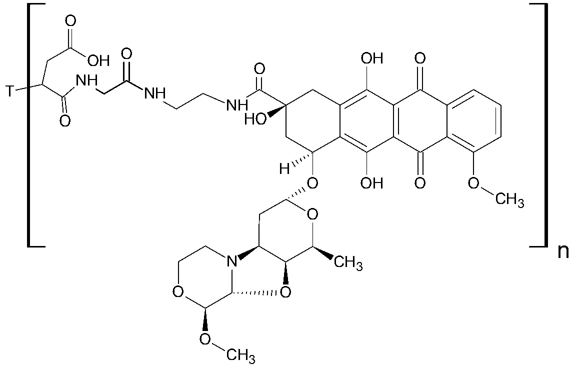
      wherein T is lintuzumab HC N296C; 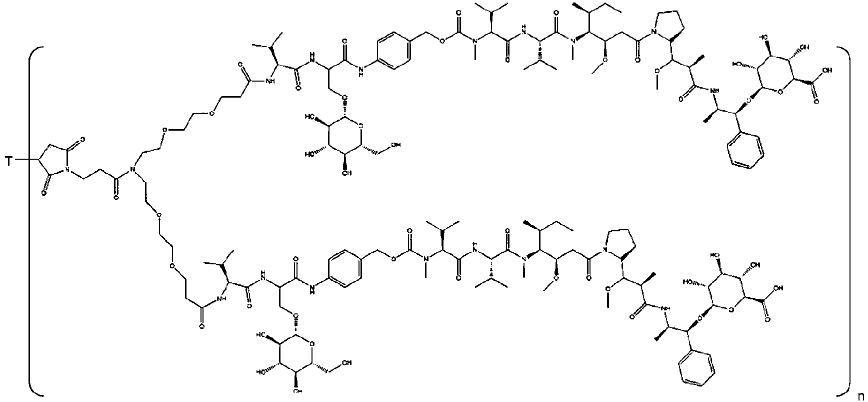
   wherein T is cysteine engineered lintuzumab having the HC substitution N296C; 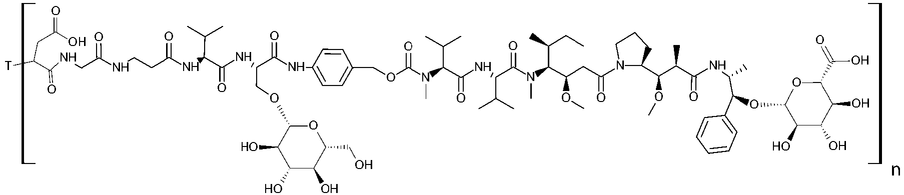
   wherein T is a cysteine engineered lintuzumab having the HC substitution N296C; 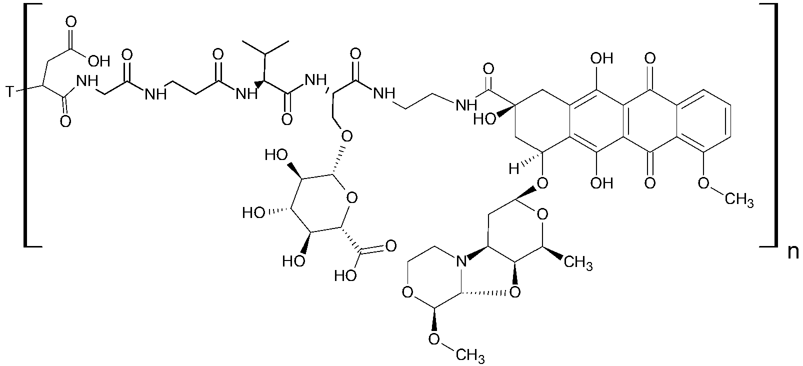
   wherein T is a cysteine engineered flanvotumab having the HC substitution N299C; 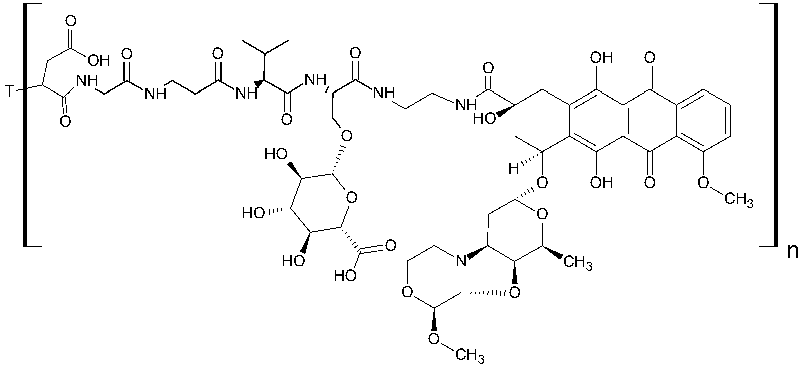
   wherein T is a cysteine engineered flanvotumab having the HC substitution N299C; 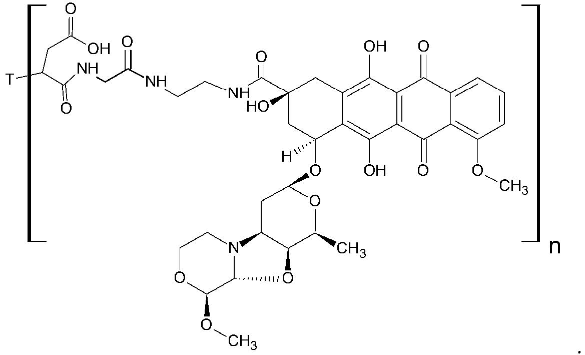
   wherein T is an anti-TYRP1 antibody; 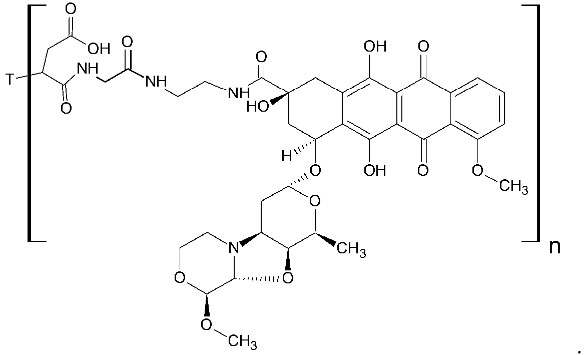
   wherein T is an anti-TYRP1 antibody; and 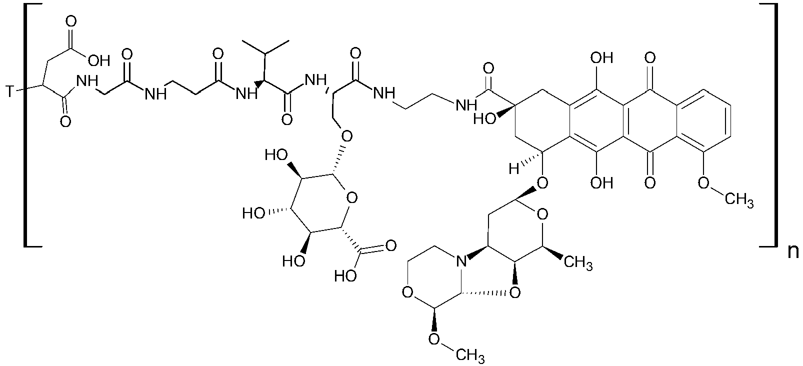
   wherein T is the cysteine engineered antibody chimeric TA99 having the HC substitution N301 C 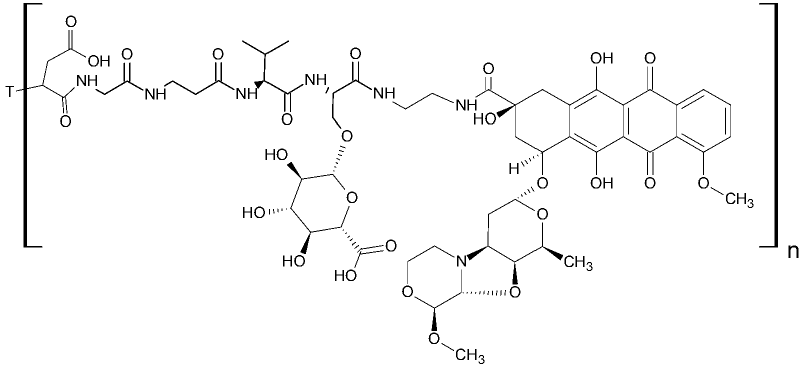
   wherein T is the cysteine engineered antibody chimeric TA99 having the HC substitution N301C; and
   wherein in any of the formulas above, n ≥ 1, or n is in the range of 1 to about 20, or 1 to about 15, or 1 to about 10, or 2 to 10, or 2 to 6, or 2 to 5, or 2 to 4; or n is 1, 2, 3, 4, 5, 6, 7, 8, 9, 10, 11, 12, 13, 14, 15, 16, 17, 18, 19, or 20; or n is 8.
21. A linker-payload conjugate or a targeting unit-linker-payload conjugate, wherein the linker-payload conjugate is any one of formulas CBa-CBj or the targeting unit-linker-payload conjugate is any one of formulas TBa-TBj 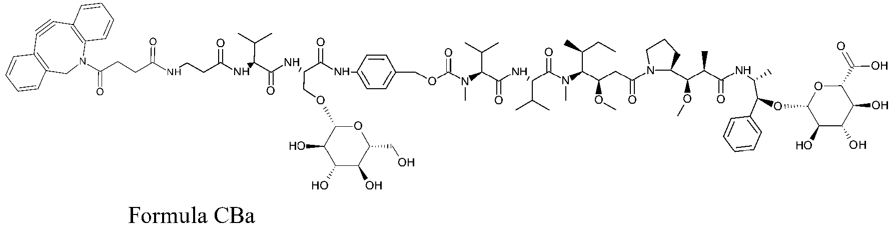 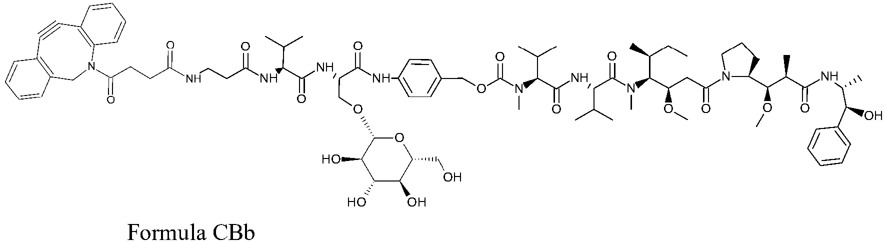 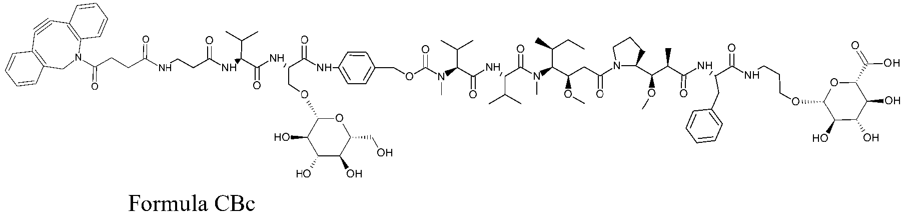 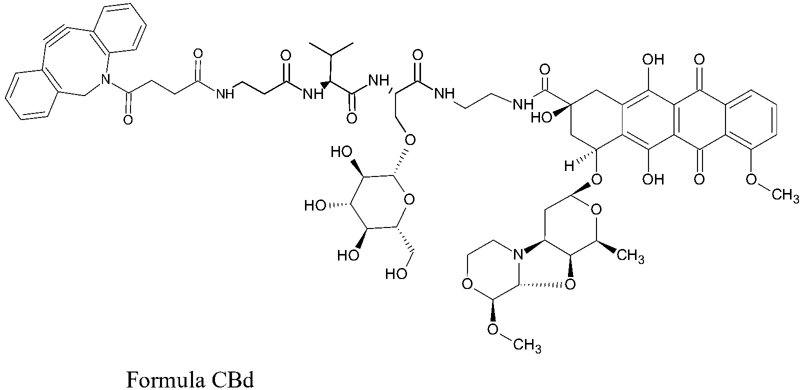 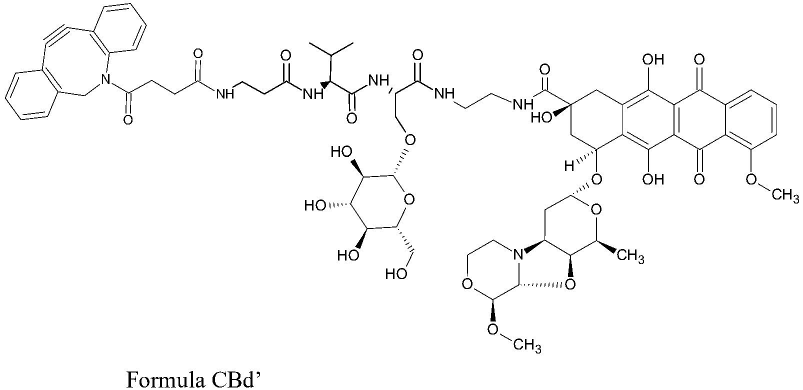 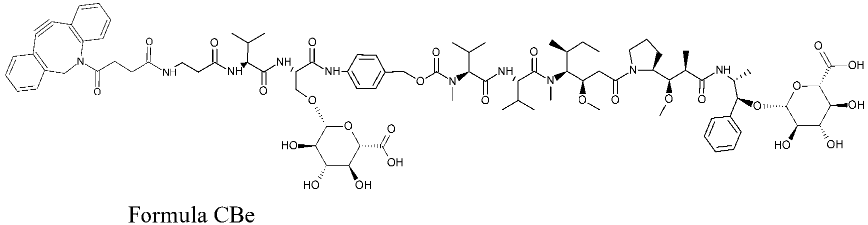 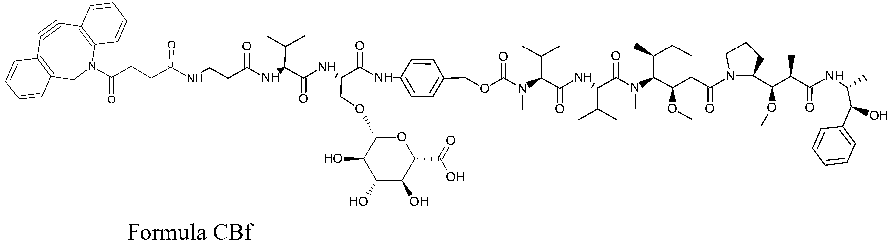 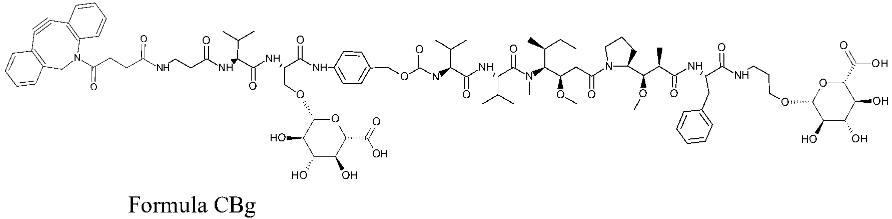 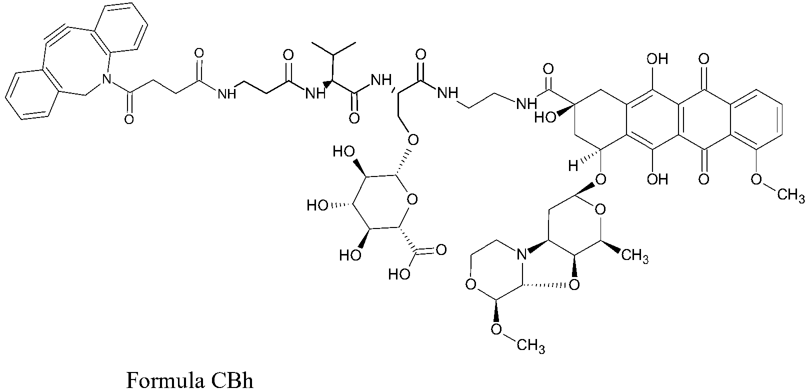 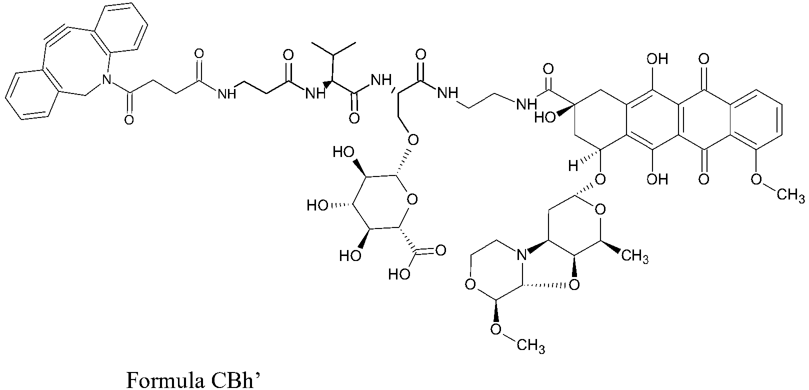 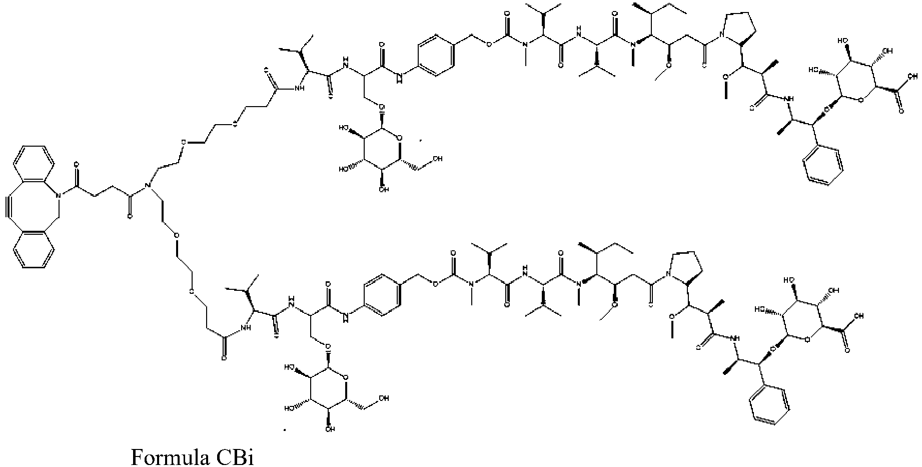 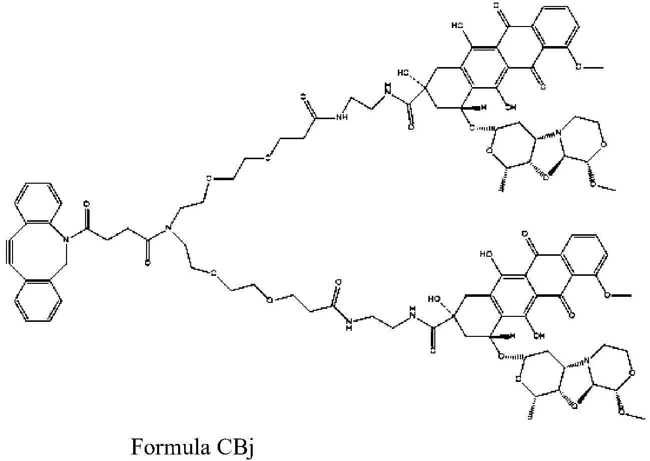 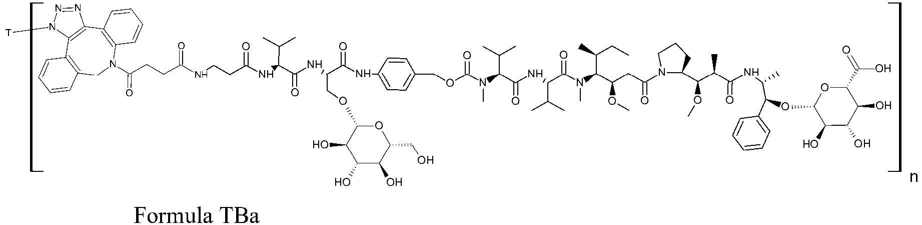 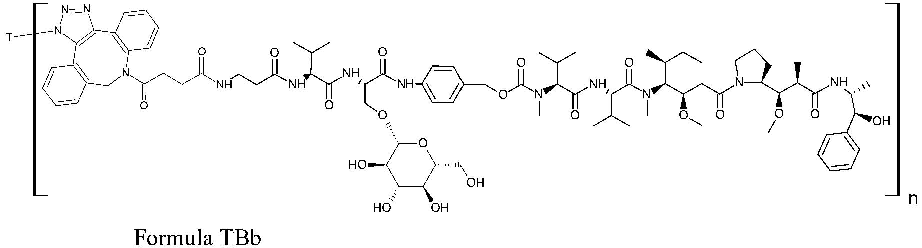 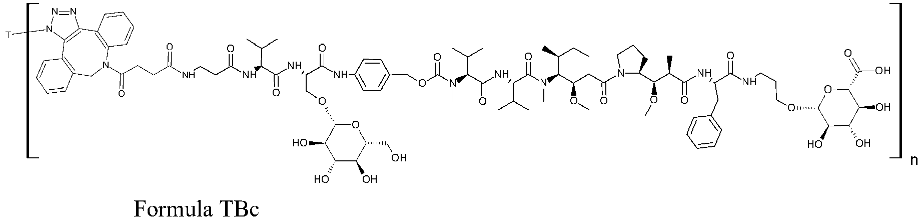 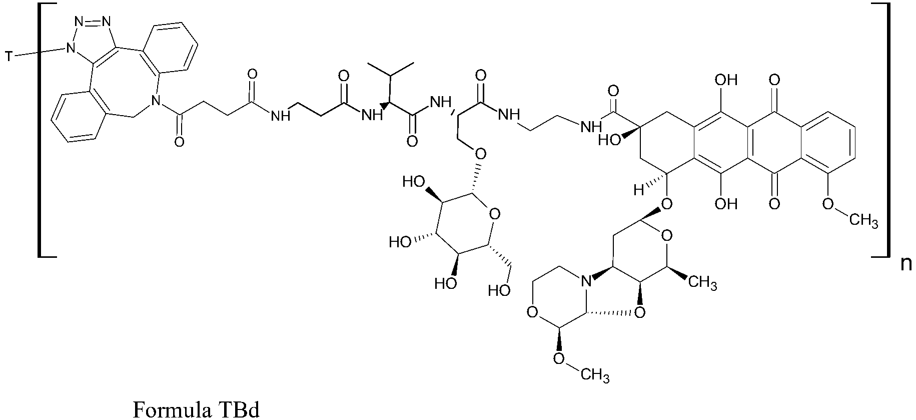 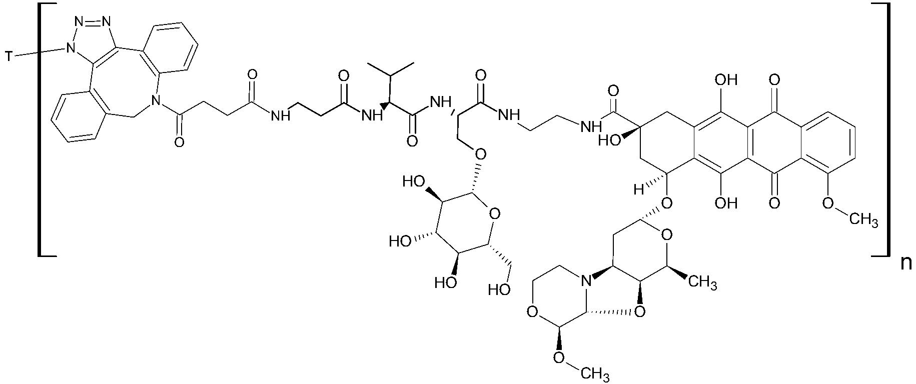 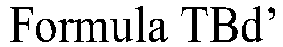 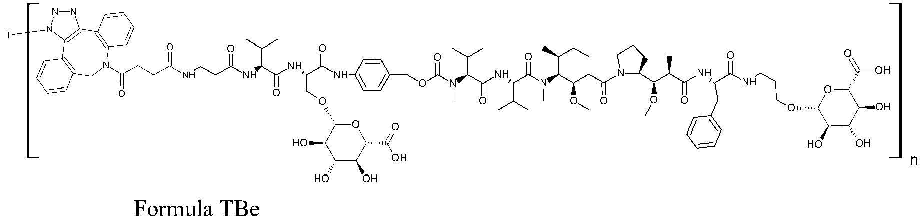 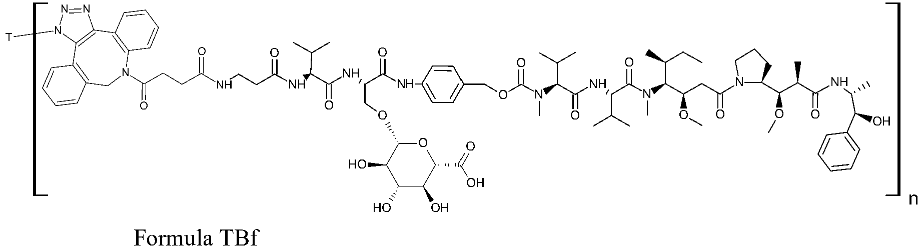 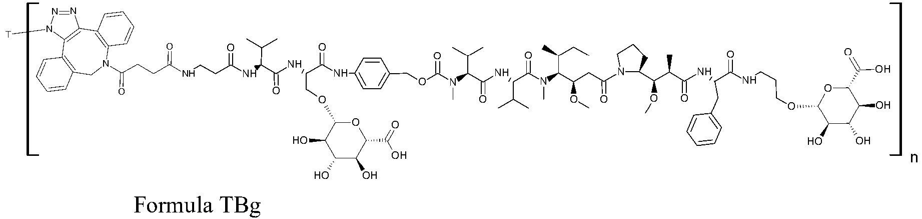 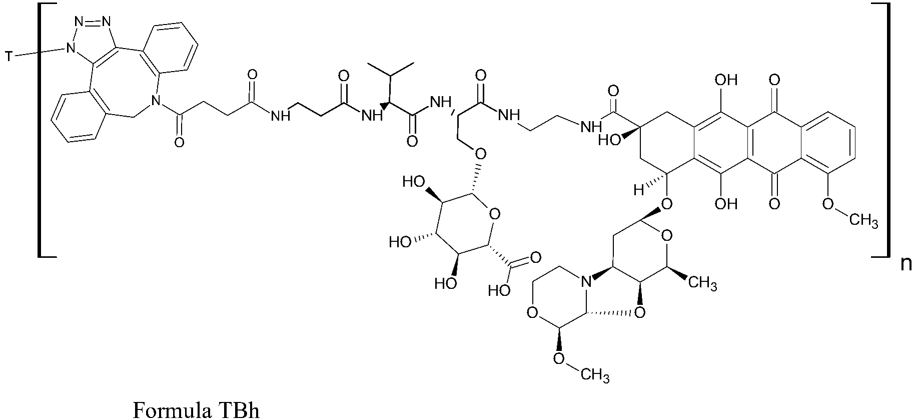 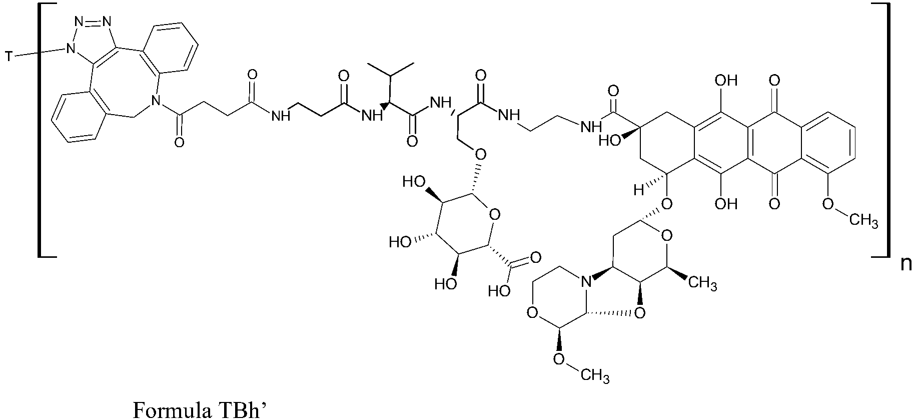 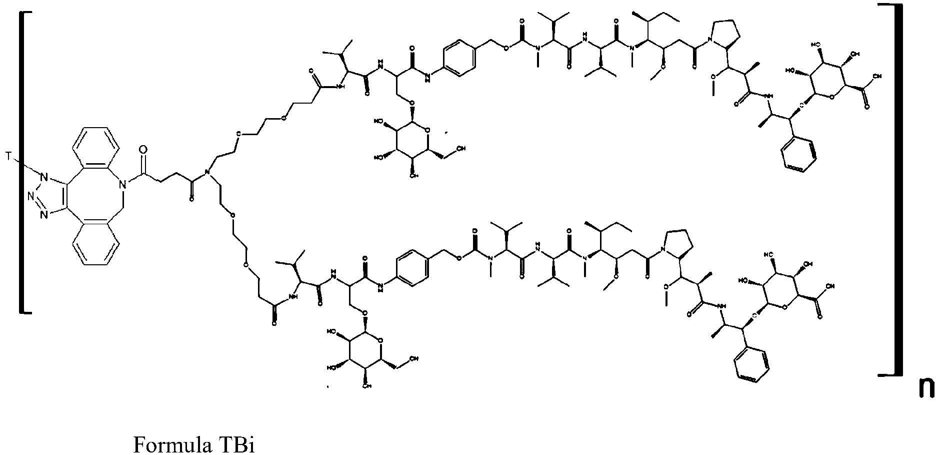 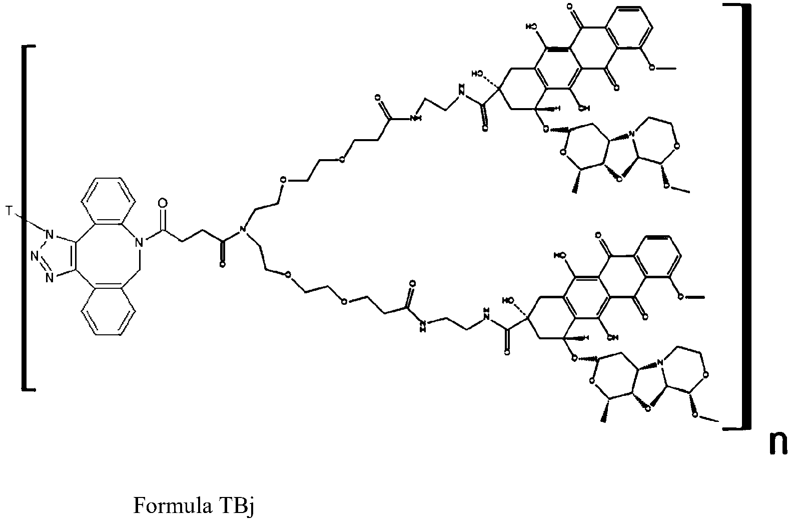 wherein in any one of the formulas Tba to TBj n ≥ 1, or n is in the range of 1 to about 20, or 1 to about 15, or 1 to about 10, or 2 to 10, or 2 to 6, or 2 to 5, or 2 to 4; or n is 1, 2, 3, 4, 5, 6, 7, 8, 9, 10, 11, 12, 13, 14, 15, 16, 17, 18, 19, or 20; or n is 8.
22. A method for preparing the targeting unit-linker-payload conjugate of any preceding embodiment comprising conjugating the linker-payload conjugate of any preceding embodiment to a targeting unit, for example, an antibody, and optionally via a linker.
23. A pharmaceutical composition comprising the linker-payload conjugate of any preceding embodiment, the targeting unit-linker-payload conjugate of any preceding embodiment, or the targeting unit-linker-payload conjugate obtainable by the method of embodiment 22.
24. The pharmaceutical composition according to embodiment 23, wherein the pharmaceutical composition has a drug-to-antibody ratio of ≥ 1, or in the range of 1 to about 20, or 1 to about 15, or 1 to about 10, or 2 to 10, or 2 to 6, or 2 to 5, or 2 to 4; or about 1, about 2, about 3, about 4, about 5, about 6, about 7, about 8, about 9, about 10, about 11, about 12, about 13, about 14, about 15, about 16, about 17, about 18, about 19, or about 20; or about 1 to about 8, or about 6 to about 8.
25. The pharmaceutical composition according to embodiment 23 or 24, wherein the targeting unit-linker-payload conjugate is the targeting unit-linker-payload conjugate represented by the following formula 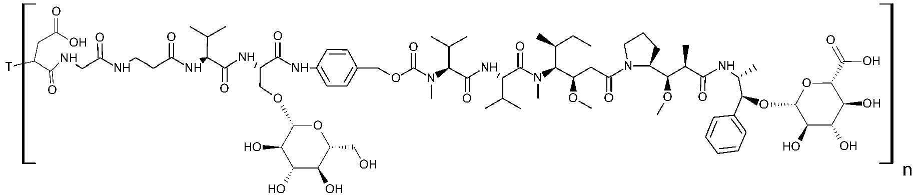 wherein n is in the range of 1 to about 20, or 1 to about 15, or 1 to about 10, or 2 to 10, or 2 to 6, or 2 to 5, or 2 to 4; or n is 1, 2, 3, 4, 5, 6, 7, 8, 9, 10, 11, 12, 13, 14, 15, 16, 17, 18, 19, or 20; or n is 8.
26. The pharmaceutical composition according to embodiment 25, wherein the composition has a drug-to-antibody ratio in the range of about 7.5 to 8.4, or about 7.8-8.1.
27. The targeting unit-linker-payload conjugate according to any one of embodiments 2-21 or pharmaceutical composition according to any one of embodiments 23-26 for use as a medicament or for use in the treatment of cancer.
28. The targeting unit-linker-payload conjugate or pharmaceutical composition for use according to embodiment 27, wherein the treatment of cancer further comprises administering an anti-cancer agent selected from the group consisting of acalabrutinib, arsenic trioxide, asciminib hydrochloride, axicabtagene ciloleucel, azacytidine, belinostat, bendamustine hydrochloride, bleomycin sulfate, bortezomib, bosutinib, brexucabtagene autoleucel, busulfan, carmustine, chlorambucil, cladribine, clofarabine, copanlisib hydrochloride, crizotinib, cyclophosphamide, cytarabine, dacarbazine, dasatinib, daunorubicin hydrochloride, denileukin diftitox, dexamethasone, doxorubicin hydrochloride, duvelisib, enasidenib mesylate, fludarabine phosphate, gilteritinib fumarate, glasdegib maleate, hydroxyurea, ibrutinib, idarubicin hydrochloride, idelalisib, imatinib mesylate, ivosidenib, lenalidomide, lisocabtagene maraleucel, lomustine, mercaptopurine, methotrexate sodium, midostaurin, mitoxantrone hydrochloride, nelarabine, nilotinib, nivolumab, omacetaxine mepesuccinate, plerixafor, ponatinib hydrochloride, pralatrexate, prednisone, procarbazine hydrochloride, recombinant interferon alfa-2b, rituximab, romidepsin, selinexor, tafasitamab-cxix, tagraxofusp-erzs, tazemetostat hydrobromide, thioguanine, tisagenlecleucel, umbralisib tosylate, venetoclax, navitoclax, obatoclax, vinblastine sulfate, vorinostat, zanubrutinib, gilteritinib, quizartinib, crenolanib and sorafenib.
29. The targeting unit-linker-payload conjugate or pharmaceutical composition for use according to embodiment 27 or 28, wherein the targeting unit is an antibody capable of binding the target molecule selected from the group consisting of CD19, CD22, CD33, CD52 and CD123, and the targeting unit-linker-payload conjugate or the pharmaceutical composition is administered in combination with an FLT3 inhibitor, an IDH1 inhibitor, an IDH2 inhibitor, a BCL2 inhibitor, a KRAS inhibitor, a NRAS inhibitor or a MEK1/2 inhibitor.
30. The targeting unit-linker-payload conjugate or pharmaceutical composition for use according to embodiment 29, wherein the FLT3 inhibitor is selected from the group consisting of midostaurin, gilteritinib fumarate, quizartinib, crenolanib, sunitinib, ponatinib and sorafenib, the MEK 1/2 inhibitor is trametinib, cobimetinib, selumetinib or binimetinib, the IDH1/IDH2 inhibitor is enasidenib or ivosidenib, the BCL2 inhibitor is venetoclax, navitoclax or obatoclax, and/or the KRAS inhibitor is sotorasib or adagrasib.
31. The targeting unit-linker-payload conjugate or pharmaceutical composition for use according to any one of embodiments 27-30, wherein the targeting unit-linker-payload conjugate or pharmaceutical composition is administered in combination with arsenic trioxide, azacytidine, daunorubicin hydrochloride, cyclophosphamide, cytarabine, glasdegib maleate, dexamethasone, doxorubicin hydrochloride, midostaurin, gilteritinib fumarate, quizartinib, crenolanib, sunitinib, ponatinib, sorafenib, enasidenib, ivosidenib, sotorasib, adagrasib, etoposide hydrochloride, gemtuzumab ozogamicin, idarubicin hydrochloride, midostaurin, mitoxantrone hydrochloride, prednisone, thioguanine, venetoclax, navitoclax, obatoclax or vincristine sulfate.
32. The targeting unit-linker-payload conjugate or pharmaceutical composition for use according to any one of embodiments 27-31, wherein the cancer is selected from the group consisting of leukemia, lymphoma, breast cancer, prostate cancer, ovarian cancer, colorectal cancer, gastric cancer, squamous cancer, small-cell lung cancer, head-and-neck cancer, multidrug resistant cancer, glioma, melanoma and testicular cancer.
33. A method of treating and/or modulating the growth of and/or prophylaxis of tumor cells in a human or an animal, wherein the linker-payload conjugate of any preceding embodiment, the targeting unit-linker-payload conjugate of any preceding embodiment, or the pharmaceutical composition according to any one of embodiments 23-26 is administered to the human or the animal in an effective amount.
34. The method of treating and/or modulating the growth of and/or prophylaxis of tumor cells in a human or an animal of embodiment 33, further comprising administering an anti-cancer agent selected from the group consisting of acalabrutinib, arsenic trioxide, asciminib hydrochloride, axicabtagene ciloleucel, azacytidine, belinostat, bendamustine hydrochloride, bleomycin sulfate, bortezomib, bosutinib, brexucabtagene autoleucel, busulfan, carmustine, chlorambucil, cladribine, clofarabine, copanlisib hydrochloride, crizotinib, cyclophosphamide, cytarabine, dacarbazine, dasatinib, daunorubicin hydrochloride, denileukin diftitox, dexamethasone, doxorubicin hydrochloride, duvelisib, enasidenib mesylate, fludarabine phosphate, gilteritinib fumarate, glasdegib maleate, hydroxyurea, ibrutinib, idarubicin hydrochloride, idelalisib, imatinib mesylate, ivosidenib, lenalidomide, lisocabtagene maraleucel, lomustine, mercaptopurine, methotrexate sodium, midostaurin, mitoxantrone hydrochloride, nelarabine, nilotinib, nivolumab, omacetaxine mepesuccinate, plerixafor, ponatinib hydrochloride, pralatrexate, prednisone, procarbazine hydrochloride, recombinant interferon alfa-2b, rituximab, romidepsin, selinexor, tafasitamab-cxix, tagraxofusp-erzs, tazemetostat hydrobromide, thioguanine, tisagenlecleucel, umbralisib tosylate, venetoclax, navitoclax, obatoclax, vinblastine sulfate, vorinostat, zanubrutinib, gilteritinib, quizartinib, crenolanib and sorafenib.
35. The method of treating and/or modulating the growth of and/or prophylaxis of tumor cells in a human or an animal of embodiment 33 or 34, wherein the targeting unit is an antibody capable of binding the target molecule selected from the group consisting of CD19, CD22, CD33, CD52 and CD123, and the targeting unit-linker-payload conjugate is administered in combination with an FLT3 inhibitor, an IDH1 inhibitor, an IDH2 inhibitor, a BCL2 inhibitor, a KRAS inhibitor, a NRAS inhibitor or a MEK1/2 inhibitor.
36. The method of treating and/or modulating the growth of and/or prophylaxis of tumor cells in a human or an animal of any one of embodiments 33-35, wherein the targeting unit-linker-payload conjugate of Formula LNAuM 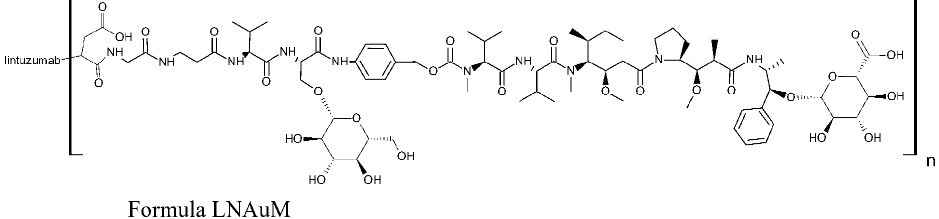 and wherein n is 8, is administered in combination with an FLT3 inhibitor, an IDH1 inhibitor, an IDH2 inhibitor, a BCL2 inhibitor, a KRAS inhibitor, a NRAS inhibitor or a MEK1/2 inhibitor.
37. The method of treating and/or modulating the growth of and/or prophylaxis of tumor cells in a human or an animal of any one of embodiments 35-36, wherein the FLT3 inhibitor is selected from the group consisting of midostaurin, gilteritinib fumarate, quizartinib, crenolanib, sunitinib, ponatinib and sorafenib, the MEK1/2 inhibitor is trametinib, cobimetinib, selumetinib or binimetinib, the IDH1/IDH2 inhibitor is enasidenib or ivosidenib, the BCL2 inhibitor is venetoclax, navitoclax or obatoclax, and/or the KRAS inhibitor is sotorasib or adagrasib.
38. The method of treating and/or modulating the growth of and/or prophylaxis of tumor cells in a human or an animal of embodiment 36 or 37, wherein the targeting unit-linker-payload conjugate of Formula LNAuM is administered in combination with arsenic trioxide, azacytidine, daunorubicin hydrochloride, cyclophosphamide, cytarabine, glasdegib maleate, dexamethasone, doxorubicin hydrochloride, midostaurin, gilteritinib fumarate, quizartinib, crenolanib, sunitinib, ponatinib, sorafenib, enasidenib, ivosidenib, sotorasib, adagrasib, etoposide hydrochloride, gemtuzumab ozogamicin, idarubicin hydrochloride, midostaurin, mitoxantrone hydrochloride, prednisone, thioguanine, venetoclax, navitoclax, obatoclax or vincristine sulfate.
39. The method of any one of embodiments 33-38, wherein the cancer is selected from the group consisting of leukemia, lymphoma, breast cancer, prostate cancer, ovarian cancer, colorectal cancer, gastric cancer, squamous cancer, small-cell lung cancer, head-and-neck cancer, multidrug resistant cancer, glioma, melanoma and testicular cancer; and/or the tumor cells are selected from the group consisting of leukemia cells, lymphoma cells, breast cancer cells, prostate cancer cells, ovarian cancer cells, colorectal cancer cells, gastric cancer cells, squamous cancer cells, small-cell lung cancer cells, head-and-neck cancer cells, multidrug resistant cancer cells, and testicular cancer cells.

### EXAMPLES

In the following, the present invention will be described in more detail. Reference will now be made in detail to the embodiments, examples of which are illustrated in the accompanying drawings. The description below discloses some embodiments in such detail that a person skilled in the art is able to utilize the invention based on the disclosure. Not all steps of the embodiments are discussed in detail, as many of the steps will be obvious for the person skilled in the art based on this specification.

### EXAMPLE 1. Synthesis of MMAU linker-payloads.

Protocols for MMAU and MMAU linker-payload synthesis are provided in WO2016001485, pages 57-60 and Examples 1-2. Protocols for Fmoc-Val-Ser(GlcOAc₄)-PAB-pNP, Val-Ser(Glc)-PAB-MMAU and MMAU linker-payload synthesis are provided in WO2018234636, Examples 1-2 and Example 36. Maleimidoacetyl-β-Ala-Val-Ser(Glc)-PAB-MMAU was prepared according to Scheme 1-1.

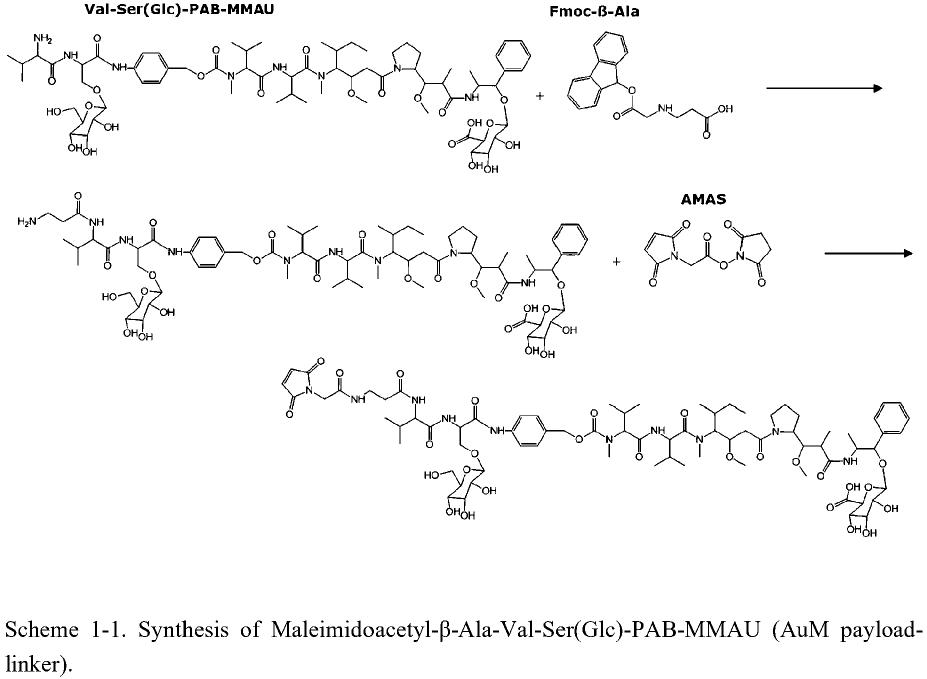

9.8 mg (6.5 µmol) Val-Ser(Glc)-PAB-MMAU and Fmoc-β-Ala-OH (3.4x molar excess) in dimethylformamide (DMF; 475 µl), 1.9x molar excess of Fmoc-Val-Ser(GlcOAc₄)-PABC-paranitrophenyl, 2.9x molar excess of HBTU and 60 µl diisopropylethylamine (DIPEA) were reacted for 1 hour at room temperature. After removing the Fmoc protecting group with DMF/DEA the product was purified by HPLC with C18 reversed phase column. AMAS (maleimidoacetyl N-hydroxysuccinimide ester, 4x molar excess) was added together with 200 µl of DMF and 8 µl of DIPEA, and the mixture was incubated for 4 hours at room temperature. The successfully prepared and HPLC-purified product was analyzed with matrix-assisted laser desorption-ionization time-of- flight (MALDI-TOF) mass spectrometry using Bruker UltraFlex III TOF/TOF instrument, showing the expected mass at m/z 1621.752 for the [M+Na]⁺ ion as well as at m/z 1643.770 for the [M-H+2Na]⁺ (Figure 1), which demonstrated successful preparation of the title compound.

### EXAMPLE 2. Preparation of maleimidoacetyl-linker-drug conjugates.

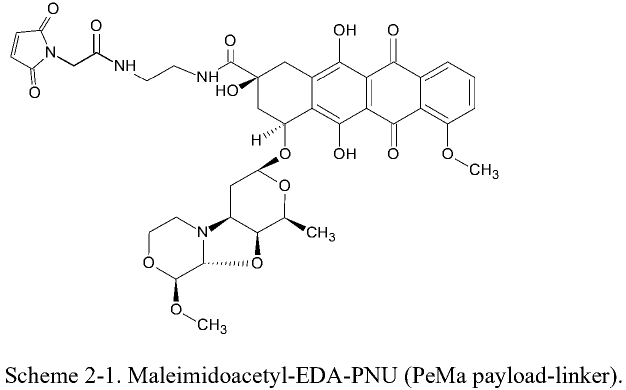

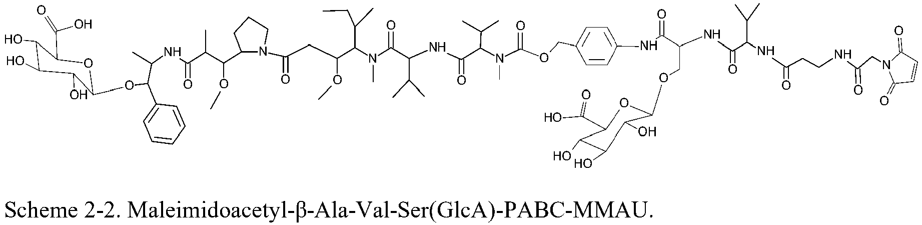

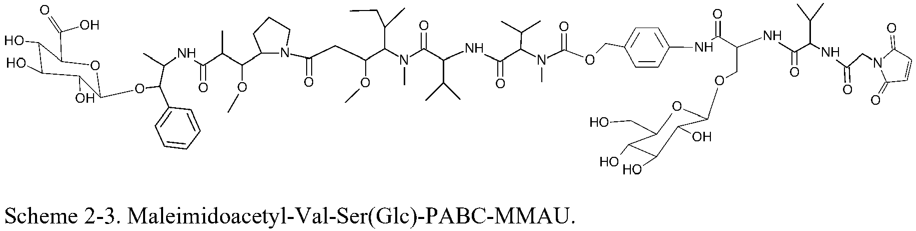

Maleimidoacetyl-EDA-PNU (Scheme 2-1) was prepared as follows: 1.5 µmol EDA-PNU in 80 µl DMSO was combined with equimolar amount of AMAS (N-α-maleimidoacet-oxysuccinimide ester; Thermo Fisher) in 20 µl DMSO together with 2 µl of DIPEA:DMSO (1:2, vol/vol) and reacted for 40 minutes at room temperature (RT). The product was purified by RP-HPLC with Gemini C18 column in 20 mM aqueous ammoniumacetate using acetonitrile (ACN) gradient. The yield of the product was 1.04 µmol according to integration of the absorbance of the peak in the chromatogram. The identity of the product was verified with MALDI-TOF MS, showing the expected mass at m/z 807.3 for the [M+H]⁺ ion and at m/z 829.3 for the [M+Na]⁺ ion.

Maleimidoacetyl-β-Ala-Val-Ser(GlcA)-PABC-MMAU (Scheme 2-2) is prepared similarly as the maleimidoacetyl-β-Ala-Val-Ser(Glc)-PABC-MMAU (Scheme 1-1), except that the Ser(Glc) residue is differently handled so as to obtain a Ser(GlcA) residue in the end product. It can be incorporated already in the synthesis as a Ser(GlcA) residue so that Val-Ser(GlcA)-PAB-MMAU is obtained and then first Fmoc-β-Ala-OH and then AMAS are added similarly as above. Alternatively Val-Ser(Glc)-PAB-MMAU is first obtained and the Glc is oxidized to GlcA by TEMPO oxidation according to standard procedures, after which first Fmoc-β-Ala-OH and then AMAS are added similarly as above. The product is purified by RP-HPLC and verified by MALDI-TOF MS at m/z 1613 for the [M+H]⁺ ion.

Maleimidoacetyl-Val-Ser(Glc)-PABC-MMAU (Scheme 2-3) is prepared similarly as the maleimidoacetyl-β-Ala-Val-Ser(Glc)-PABC-MMAU (Scheme 1-1), except that only AMAS is added similarly as above. The product is purified by RP-HPLC and verified by MALDI-TOF MS at m/z 1528 for the [M+H]⁺ ion.

### EXAMPLE 3. Preparation of trastuzumab-maleimidoacetyl-linker-MMAU conjugates.

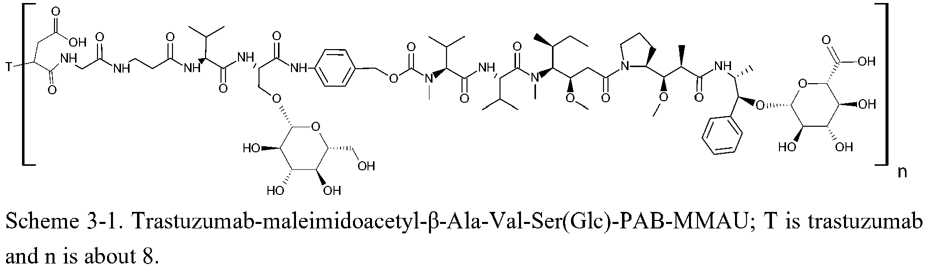

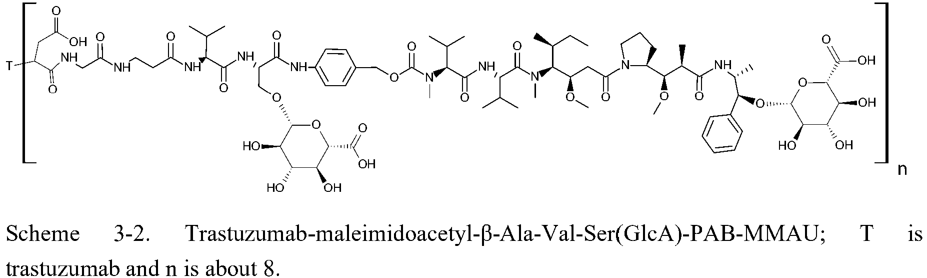

Two aliquots, 2 mg each, of trastuzumab (Herceptin; Roche) in 260 µl volume of phosphate-buffered saline (PBS) were reduced in the presence of 230 nmol of DTPA and 600 nmol of TCEP at +37°C for 55 minutes. After the reaction, the reduced antibodies were conjugated with maleimide-payload by adding 600 nmol of either maleimidoacetyl-β-Ala-Val-Ser(Glc)-PAB-MMAU or maleimidoacetyl-β-Ala-Val-Ser(GlcA)-PAB-MMAU, dissolved in 23 µl and 17 µl of DMSO, respectively, together with 700 µl of PBS, and incubating at +37°C for 3.5 hours. The reagents were removed by buffer exchange into PBS with Amicon 30K centrifugal filters according to manufacturer's instructions. The ADCs were analyzed by MALDI-TOF mass spectrometric analysis after digestion with FabRICATOR enzyme (Genovis, Sweden) and microscale purification of the resulting antibody fragments with Poros R1 material. The analysis showed that the prepared ADCs had a drug-to-antibody ratio (DAR) of about 8, since the light chain (LC) and Fd fragments were essentially changed into LC+1 payload and Fd+3 payload fragements, respectively. Original trastuzumab: LC at m/z 23442.2; trastuzumab-maleimidoacetyl-β-Ala-Val-Ser(Glc)-PAB-MMAU: LC + payload at m/z 25044.8 and Fd + 3 payloads; trastuzumab-maleimidoacetyl-β-Ala-Val-Ser(GlcA)-PAB-MMAU: LC + payload at m/z 25059.6 and Fd + 3 payloads at m/z 30246.8; all [M+H]⁺ ions. Larger aliquots of ADCs were prepared similarly, however with Protein A HPLC purification with in-line desalting instead of Amicon buffer-exchange.

Alternatively, 2 mg of trastuzumab in PBS was reduced in the presence of 20x molar excess of TCEP at +37°C for 1.5 hours. Then 28x molar excess of MA-Ac-β-Ala-Val-Ser(β-Glc)-PAB-MMAU was added and reaction allowed to proceed at +37°C for 1 hour.

### EXAMPLE 4. Stabilization of maleimides.

Maleimide stabilization after conjugation to cysteine was studied with glutathione conjugates of both maleimidoacetyl-β-Ala-Val-Ser(Glc)-PAB-MMAU(Scheme 3-1, wherein T is glutathione) and maleimidoacetyl-β-Ala-Val-Ser(GlcA)-PAB-MMAU (Scheme 3-2, wherein T is glutathione). Conjugation to glutathione was performed in aqueous solution (PBS) for a few hours in RT, after which conjugate formation was verified by MALDI-TOF MS. Glutathione-maleimidoacetyl-β-Ala-Val-Ser(Glc)-PAB-MMAU had m/z of 1929.5 [M+Na]⁺ and glutathione-maleimidoacetyl-β-Ala-Val-Ser(GlcA)-PAB-MMAU had m/z of 1943.4 [M+Na]⁺. In parallel experiments, the pH of the conjugate solution was changed with buffers into 6.0 (MES buffer), 7.2 (MOPS buffer) and 8.0 (Tris-HCl). After overnight incubation at +37°C, the maleimides of Tris-HCl buffer incubated conjugate had completely stabilized by hydrolysis (+18 mass units) into m/z 1947.6 and m/z 1961.4 for the two conjugates, respectively, demonstrating efficient stabilization in the mildly basic pH of 8.0. In contrast, in pH 6.0 (mildly acidic) and pH 7.2 (neutral) buffers, the conversion into the stabilized maleimide was only partial.

### EXAMPLE 5. Conjugate stability in serum.

Similar glutathione conjugate as above was also prepared from maleimidocaproyl-Val-Cit-PAB-MMAU and glutathione, purified and characterized by MALDI-TOF MS at m/z 1822.2 [M+Na]⁺. All three glutathione-linker-MMAU conjugates were incubated in parallel in mouse serum at +37°C and analyzed by MALDI-TOF MS after overnight incubation and after four days' incubation. With glutathione-maleimidocaproyl-Val-Cit-PAB-MMAU, overnight incubation showed appearance of free MMAU at m/z 916.8 [M+Na]⁺. After 4 days' incubation, only little glutathione-linker-MMAU was left at m/z 1821.9 [M+Na]⁺ while the major peak was free MMAU at m/z 916.5 [M+Na]⁺, demonstrating that the maleimidocaproyl-Val-Cit-PAB linker was relatively unstable in serum. With glutathione-maleimidoacetyl-β-Ala-Val-Ser(GlcA)-PAB-MMAU, overnight incubation showed appearance of small peak at free MMAU at m/z 916.8 [M+Na]⁺. However, after 4 days' incubation, only little free MMAU had appeared at m/z 916.5 [M+Na]⁺, while the vast majority of the glutathione-linker-MMAU was left at m/z 1960.8 [M+Na]⁺. The glutathione-maleimidoacetyl-β-Ala-Val-Ser(Glc)-PAB-MMAU behaved similarly, showing that the maleimidoacetyl-β-Ala-Val-Ser(GlcA)-PAB and maleimidoacetyl-β-Ala-Val-Ser(Glc)-PAB linkers had excellent stability in serum.

### EXAMPLE 6. Preparation of ADCs.

Trastuzumab-maleimidoacetyl-EDA-PNU ADCs were prepared by conjugation to reduced hinge cysteines as described above for MMAU ADCs. However, DAR was limited to 2-4 by partial reduction as follows: Two aliquots, 1.5 mg each, of trastuzumab (Herceptin; Roche) 5 mg/ml in PBS were reduced in the presence of 1 mM DTPA and either 6x molar excess or 9x molar excess of TCEP at +37°C for 1 hour. After the reaction, the reduced antibodies were conjugated with 10x molar excess of maleimidoacetyl-EDA-PNU payload by adding it, dissolved in 20 µl of DMSO, to the reaction mixture, and incubating at +37°C for 1 hour. The ADCs were purified by Protein A HPLC purification (MabSelect Sure, Thermo). The ADCs were analyzed by MALDI-TOF MS after digestion with FabRICATOR as above. The analysis showed that the ADCs had been successfully conjugated, since the light chain (LC) was effectively changed into LC + 1 payload. Original trastuzumab: LC at m/z 23426.1; trastuzumab-maleimidoacetyl-EDA-PNU: LC at m/z 23434.7 and LC + payload at m/z 24241.0; all [M+H]⁺ ions. The DAR was analyzed from the purified ADCs by comparison of PNU-specific absorbance at 480 nm to absorbance at 280 nm (both PNU and antibody) in Nanodrop One spectrophotometer (Thermo Fisher; by using in the calculations the absorption coefficients at 280/480 nm of doxorubicin and the original antibody, respectively). The two ADCs were determined to have DAR≈2.5 and DAR≈3.3, respectively.

Similarly as above, TA99-maleimidoacetyl-EDA-PNU DAR≈4 ADC (TA99-M-PNU DAR=4) was produced from anti-TYRP-1 mouse IgG2a antibody TA99 (anti-mGP75-mIgG2a, Invivogen, France) with maleimidoacetyl-EDA-PNU payload. The DAR was calculated as on average 4 by MALDI-TOF MS as described above.

TA99-maleimidoacetyl-β-Ala-Val-Ser(Glc)-PAB-MMAU DAR=10 ADC was prepared as follows. 2 mg of TA99 antibody in 1 ml of 150 mM NaCl, 20 mM Na-phosphate, 5% saccharose was reduced in the presence of 1 mM of DTPA and 12x molar excess of TCEP to antibody at +37°C for 1 hour. After the reaction, the reduced antibody was conjugated with the maleimide-payload by adding 20x molar excess to antibody of maleimidoacetyl-β-Ala-Val-Ser(Glc)-PAB-MMAU dissolved in DMSO and incubating at +37°C for 1 hour. The ADC was purified by protein A HPLC. The ADC was analyzed by MALDI-TOF mass spectrometric analysis. The analysis showed that the prepared ADC had a drug-to-antibody ratio (DAR) of about 10, since the light chain (LC) and heavy chain (HC) fragments were essentially changed into LC+1 payload and HC+4 payloads fragments, respectively. Original TA-99: LC at m/z 23594; TA-99-maleimidoacetyl-β-Ala-Val-Ser(Glc)-PAB-MMAU: LC + payload at m/z 25189; Original TA-99: [HC]²⁺ at m/z 25609; TA-99-maleimidoacetyl-β-Ala-Val-Ser(Glc)-PAB-MMAU: [HC +4 payloads] ²⁺ at m/z 28803.

### EXAMPLE 7. Cytotoxicity of ADCs.

Cytotoxicity of the DAR≈2.5 and DAR≈3.3 trastuzumab-maleimidoacetyl-EDA-PNU ADCs towards HER2+ SK-BR-3 ovarian cancer cells was evaluated by incubating a serial dilution series of the conjugates with the cells in parallel experiments and evaluating the viability with PrestoBlue reagent essentially as described in Satomaa et al. Antibodies 2018, 7(2), 15. The ADCs had high specific cytotoxicity with IC50 below 300 pM, since at that concentration nearly all of the SK-BR-3 cells had been killed in both experiments after 4 days, showing that both ADCs had high activity against HER2+ cancer cells.

### EXAMPLE 8. In vivo efficacy of trastuzumab and MMAU-ADC in tumor xenograft mice.

In vivo anti-tumor efficacy of ADC of trastuzumab and maleimidoacetyl-β-Ala-Val-Ser(Glc)-PAB-MMAU, DAR=8, prepared and maleimide-stabilized as described above (MMAU-ADC) was compared to trastuzumab. HCC-1954 cancer cells were obtained from the ATCC (USA) and cultured according to the manufacturer's inctructions to study efficay of antibody-drug conjugates in trastuzumab-resistant Her2-positive xenograft tumors in immunodeficient nude mice (Balb/cAnNRj-Foxn1nu-nu). The study was performed at the TCDM / Central Animal Laboratory, University of Turku, Finland, according to the appropriate ethical committee approval. Cells for inoculation to mice were prepared in vigorous exponential growth phase. 5 million cells in 50% Matrigel were inoculated s.c. to the flank of each mouse. Clinical signs and general behavior of the animals was observed regularly. No potential signs of toxicity were recorded. At the end of the study, the mice were examined for potential macroscopic changes in major organs, but none were detected. Tumor growth was followed by palpation. After caliper measurement, tumor volume was calculated according to 0.5 x length x width². The first dosings were administered when average tumor volume reached 100 cm³. Mice were evenly divided into study groups, six mice/group, so that each group received similar distribution of different-sized tumors and the average tumor volumes were similar in each group. Intravenous (i.v.) treatments of 10 mg/kg either antibody or ADC in PBS were given four times at seven day intervals (QWx4 i.e. once weekly for four weeks). Unconjugated antibody (trastuzumab: Herceptin, Roche) was used in this study as the control treatment. Figure 2 shows the results of the study. The tumors grew steadily in the control group (trastuzumab) to over 500 mm³ average size during the 61 day treatment and follow-up period, while in the MMAU-ADC group the ADC had effectively inhibited tumor growth in vivo and the tumors shrinked in all six mice (6/6) without regrowth during the 61 days.

### EXAMPLE 9. In vivo efficacy of maleimide-stabilized PNU-ADC in syngeneic tumor mice.

In vivo anti-tumor efficacy of ADC of monoclonal TA99 IgG2a antibody, glycoconjugated TA99 ADC with DBCO-Val-Ser(GlcA)-EDA-PNU payloads (TA99-PNU ADC, DAR=2) and TA99 ADC with maleimidoacetyl-EDA-PNU payloads (TA99-M-PNU ADC, DAR=4), prepared and maleimide-stabilized as described above, was compared to non-treated mice. B16-F10 mouse melanoma cells were obtained from the ATCC (USA) and cultured according to the manufacturer's inctructions to study efficacy of antibody-drug conjugates in highly treatment-resistant syngeneic tumors in female adult C57BL/6J mice at the age of about 8-10 weeks. The study was performed at the TCDM / Central Animal Laboratory, University of Turku, Finland, according to the appropriate ethical committee approval. Cells for inoculation to mice were prepared in vigorous exponential growth phase. 0.25 million cells in 50% Matrigel were inoculated s.c. to the flank of each mouse. Clinical signs and general behavior of the animals was observed regularly. Signs of toxicity were monitored by measuring the body weight, but no weight loss was observed in any of the study groups. Tumor growth was followed by palpation. After caliper measurement, tumor volume was calculated according to 0.5 x length x width². The first dosings were administered two days after the inoculation due to the high take rate and very rapid growth of the tumors. Mice were randomly divided into study groups. A single intravenous (i.v.) treatment of 5 mg/kg either antibody or ADC in PBS was given. Unconjugated antibody (TA99/anti-gp75, Invivogen) was used in this study as the control treatment. Figure 3 shows the results of the study. The tumors grew rapidly in the control groups (no treatment or antibody) and most of them had to be sacrificed before the end of the follow-up period (day 26). Both ADCs effectively shrinked tumors in all mice and they survived to the end of the experiment.

### EXAMPLE 10. Branched linker-payloads and ADCs.

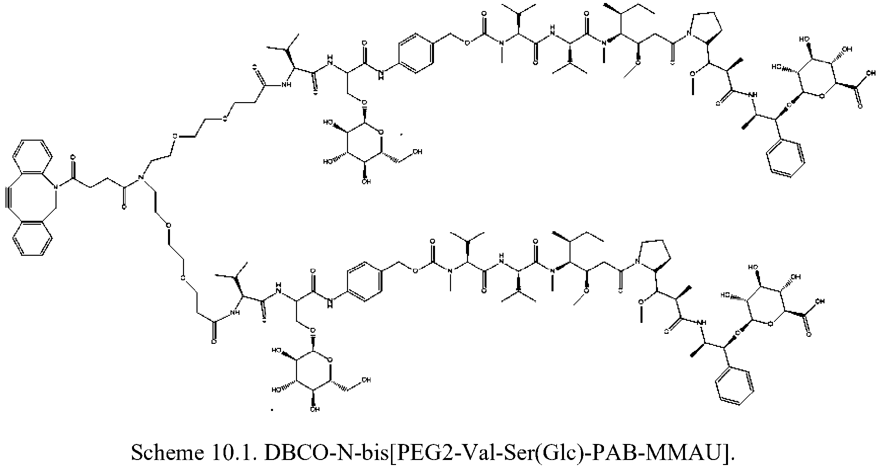

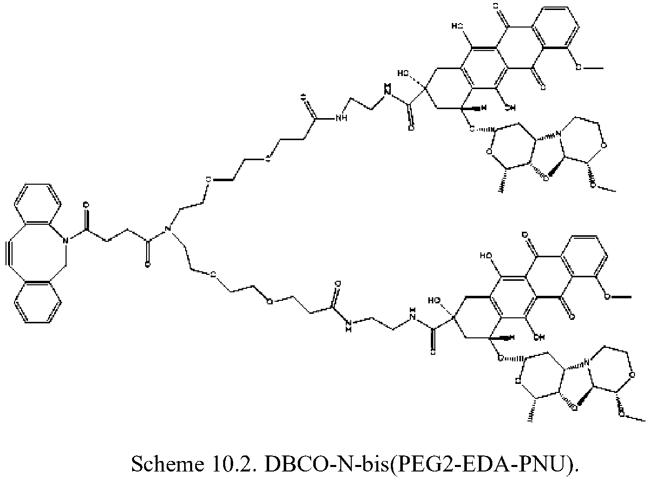

Branched DBCO-linker-payload according to Scheme 10.1. was prepared by reacting 1.20 µmol of Val-Ser(Glc)-PAB-MMAU with 256 nmol of N-DBCO-N-bis(PEG2-NHS ester) reagent (Broadpharm) in 110 µl DMSO containing 1/3 µl DIPEA at RT for 30 minutes. MALDI-TOF MS from the reaction mixture showed expected signal at 2139.03 for the [M+Na]+ ion, showing that the correct structure was generated. The branhed DBCO-linker payload is purified by RP-HPLC and conjugated to azide-labeled antibody by glycoconjugation as above to generate ADC with DAR=4.

Branched DBCO-linker-payload according to Scheme 10.2. was prepared by reacting 1.20 µmol of EDA-PNU with 256 nmol of N-DBCO-N-bis(PEG2-NHS ester) reagent (Broadpharm) in 103 µl DMSO containing 1/3 µl DIPEA at RT for 30 minutes. MALDI-TOF MS from the reaction mixture showed expected signal at 1949.82 for the [M+Na]+ ion, showing that the correct structure was generated. The branhed DBCO-linker payload is purified by RP-HPLC and conjugated to azide-labeled antibody by glycoconjugation as above to generate ADC with DAR=4.

### EXAMPLE 11. General methods for production of antibodies in CHO cells.

### Cloning of native antibodies

Amino terminus of LC was added a leader peptide MVSTPQFLVFLLFWIPASRS (SEQ ID NO: 53) and amino terminus of HC was added a leader peptide MAVLGLLFCLVTFPSCVLS (SEQ ID NO: 54). Then heavy chain and light chain coding sequences were codon optimized for CHO cells and subcloned into pcDNA3.4-TOPO expression vector (GeneArt). Plasmids were transformed into *E*. *coli* NEB10β competent cells (New England Biolabs) and plasmid maxipreps were extracted by PureLink HiPure Plasmid FP (Filter and Precipitator) Maxiprep Kit (Invitrogen).

### Cloning of cysteine engineered antibody constructs flanvotumab HC (N299C), flanvotumab HC (C222S), chimeric TA99 HC (N301C), chimeric TA99 HC (N301C), chimeric TA99 HC (C224S), lintuzumab HC (N296C), lintuzumab HC (C219S) and gemtuzumab HC (C130S)

To generate cysteine engineered antibody expression plasmids, DNA strings with desired substitutions were ordered from GeneArt, digested with appropriate restriction enzymes and combined with vector backbones by NEBuilder HiFi DNA Assembly method according to manufacturer's instructions (New England Biolabs). After transformation to *E. coli* mutated DNA sequences were confirmed by sequencing.

### Transfection and expression of antibodies

Culturing and transfection ExpiCHO-S cells (Thermo Fisher Scientific; Cat.No. A29133) was performed according to manufacturer's instructions. Post transfection ExpiCHO-S cells were cultured for 6-8 days and at harvesting, culture medium was transferred to 50 ml tubes, centrifuged, and filtered followed by purification.

### Antibody purification by HPLC

Antibody purifications from supernatant samples with HiTrap MabSelect SuRe column were done by Äkta HPLC purifier system. HiTrap MabSelect SuRe column (1 ml or 5 ml, GE Healthcare) was used for antibody purification. Sample was loaded to column and washed with 12-14 column volumes of PBS. Five column volumes of 0.1 M citrate pH 3.0 was used for elution. After elution, buffer of antibody sample was changed for PBS using 1-4 of HiTrap Desalting columns (5 ml, GE Healthcare). Concentration was determined by spectrophotometer (Nanodrop one, Thermo Fisher Scientific).

Aggregates were purified with Superdex 200 increase column (10 x 300 mm, GE Healthcare) if aggregation level was over 10 %. Antibody sample was concentrated by Amicon Ultra centrifugal filter (30K, Sigma Aldrich) before purification. 10-27 mg of antibody was purified at one cycle. Sample was injected to column and monomers purified by isocratic run with PBS, (0.75 ml/min, 34 min).

### ADC purification by HPLC

ADC purifications with Mab Select sure column were done with Äkta HPLC purifier system. Mab Select Sure column (1 ml, GE Healthcare) was used for ADC purification. Sample was loaded to column and washed with 12 column volumes of PBS. Five column volumes of 0.1 M citrate pH 3.0 was used for elution followed by PBS buffer change with Desalting column (5 ml, GE Healthcare). Concentration was determined by spectrophotometer (Nanodrop one, Thermo Fisher Scientific).

### HIC Protocol

Hydrophobic interaction chromatography (HIC) analysis with TSKgel ButylNPR column (4.6 mm x 3.5 cm, Tosoh Biosciences) was done with Äkta HPLC purifier system. 80 µg of ADC or antibody was injected into the column and separated by gradient elution: 100 % buffer A (1.5 M ammoniumsulfate, 25 mM K-phosphate) to 100% buffer B (25 % isopropanol, 25 mM K-phosphate) for 15 minutes (1 ml/min) continued with 100% B for 2 minutes.

### Aggregation analysis

Level of aggregation was analysed by Superdex 200 column (10 x 300 mm, GE Healthcare) using Äkta HPLC purifier system. 80 - 100 µg of antibody or ADC was injected to the column and separated by isocratic run with 0.2 M K-phosphate pH 7, 0.25 M KCl, (0.75 ml / min, 34 min).

### Determination of DAR with PLRP-S column

If sample volume was over 100 µl:
   Drug-to-antibody ratio (DAR) was calculated using PLRP-S chromatogram. 25 µg antibody or 50 µg ADC in 0.5 - 1.5 ml PBS was reduced with 30 µl 0.5 M DTT for 30 minutes at + 37 °C. 2 % TFA was added to getting 0.1 % TFA solution to injection. Analysis with PLRP-S column (1000 Å, 8µM, 150x2.1 mm, Agilent) was done by Äkta HPLC purifier system. Sample was loaded in 5 % ACN, 0.1 % TFA 0.4 ml / min and eluted by ACN gradient 30 - 50 % ACN, 0.25 ml / min for 40 minutes. Column oven at + 70 °C was used. DAR of the LC was calculated by relative portion of LC with payload (Area 280 nm) and DAR of the HC by relative portions of HC with 1, 2, 3 or 4 payloads.
If sample volume was under 100 µl:
   Drug-to-antibody ratio (DAR) was calculated using PLRP-S chromatogram. 25 µg antibody or 50 µg ADC was reduced with 1 µl 0.5 M DTT for 30 minutes at + 37 °C. Analysis with PLRP-S column (1000 Å, 8µM, 150x2.1 mm, Agilent) was done by Äkta HPLC purifier system. Sample was loaded to column and eluted by ACN gradient 30 - 50 % ACN, 0.25 ml / min for 40 minutes. Column oven at + 70 °C was used. DAR of the LC was calculated by relative portion of LC with payload (Area 280 nm) and DAR of the HC by relative portions of HC with 1, 2, 3 or 4 payloads.

### Determination of DAR with A280/A480 method

DAR was calculated by NanoDrop spectrophotometer (Nanodrop one, Thermo Fisher Scientific) with the equation: $DAR = \frac{\frac{A 480}{payload extinction coefficient}}{\frac{A 280 - payload \frac{A 280}{A 480} ∗ A 480}{antibody extinction coefficient}}$
Antibody extinction coefficient = 210000 M⁻¹ cm⁻¹
Payload extinction coefficient = doxorubicin extinction coefficient = 10410 M⁻¹ cm⁻¹
Payload A280/A480 = measured A280/A480 for Maleimidoacetyl-EDA-PNU (PeMa) = 0.8

### MALDI-TOF MS analysis

Purified antibodies were fragmented before MALDI analysis as above by treating with the antibodies with FabRICATOR^{®} (IdeS; Genovis) and GlycINATOR^{®} (EndoS2; Genovis). For analysis fragments were treated with TCEP.

### EXAMPLE 12. Preparation of glycoconjugated flanvotumab-DBCO-Val-Ser(GlcA)-EDA-PNU ADC (FLPeD)

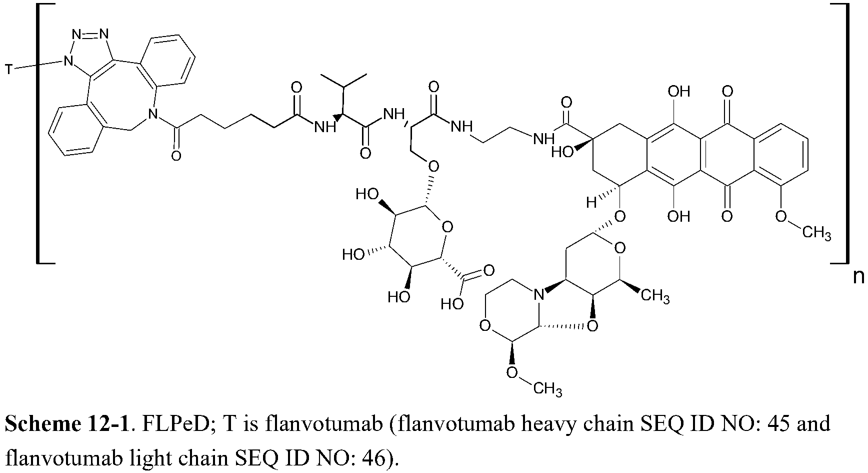

2 mg of flanvotumab was buffer exchanged into 50 mM MOPS pH 7.2, 150 mM NaCl by ultrafiltration with Amicon ultra-4 30K filter devices (Millipore). Flanvotumab-GalNAz with DAR=2 was synthesized in a one pot -reaction with immobilized GlyciNATOR^{®} gel (Genovis), UDP-GalNAz 2.1 mg/ml (Thermo Fisher Scientific), beta-1,4-Galactosyltransferase Y289L 133 µg/ml (Thermo Fischer Scientific), 5 mM MnCl₂, in a total volume of 600 µl. The reaction was incubated with gentle mixing at 37°C overnight. Two times molar excess of PeD, DBCO(C6)-ValSer(GlcA)-EDA-PNU-159682 (Levena Biopharma) vs. GalNAz was mixed with 0.89 mg of HPLC-purified flanvotumab-GalNAz DAR=2 in 1 ml PBS, and the reaction was incubated at room temperature overnight. ADC was purified with Mab Select sure column by Äkta HPLC purifier system. An aliquot of the reaction was digested with FabriCATOR^{®} (Genovis) and analyzed by MALDI-TOF MS (flanvotumab-GalNAz: Fc at m/z 24380; FLPeD: Fc+payload at m/z 25730; DAR=2).

### EXAMPLE 13. Preparation of glycoconjugated chimeric TA99-DBCO-Val-Ser(GlcA)-EDA-PNU ADC (CHPeD)

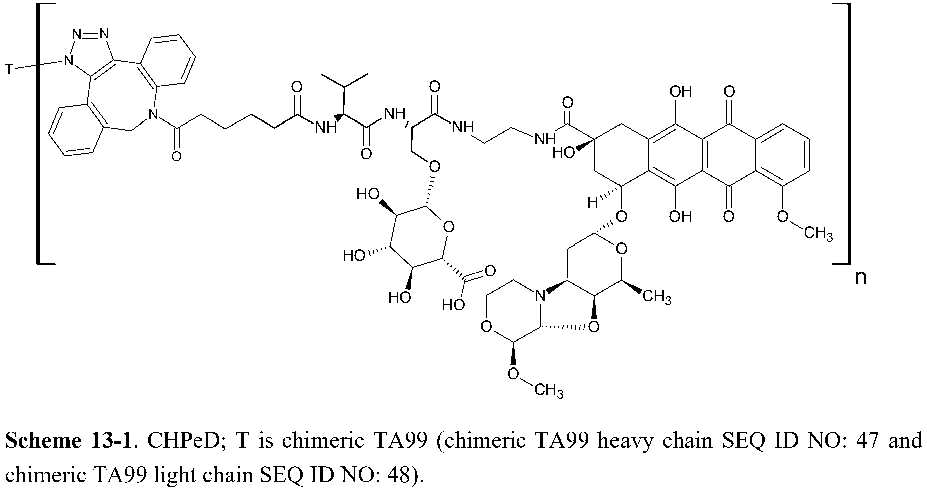

2 mg of chimeric TA99 antibody was buffer exchanged into 50 mM MOPS pH7.2, 150 mM NaCl by ultrafiltration and one pot synthesis of chimeric TA99-GalNAz was performed and the ADC was purified as described above for FLPeD and analysed by MALDI-TOF MS (chimeric TA99-GalNAz: Fc at m/z 24375; CHPeD: Fc+payload at m/z 25724; DAR=2).

### EXAMPLE 14. Preparation of anti-mGP75-maleimidoacetyl-β-Ala-Val-Ser(Glc)-PAB-MMAU (TAAuM)

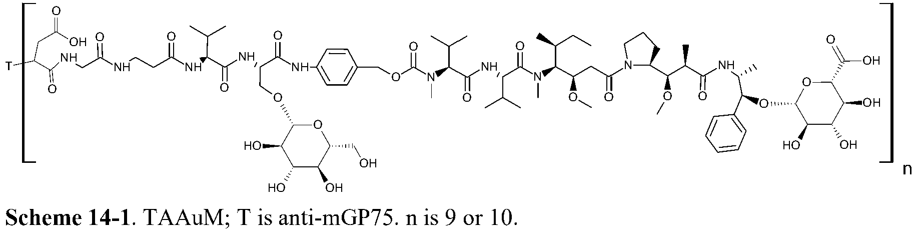

2 mg of anti-mGP75 (TA99 antibody; Invivogen) in PBS was reduced in the presence of 20x molar excess of TCEP at +37°C for 1.5 hours. 28x molar excess of MA-Ac-β-Ala-Val-Ser(β-Glc)-PAB-MMAU was added and reaction allowed to proceed at +37°C for 1 hour. TAAuM was purified as above, concentrated by Amicon ultra centrifugal filter, sterile filtered and stored until use.

DAR of TAAuM was determined with PLRP-S. DAR of TAAuM was 10.0.

### EXAMPLE 15. Preparation of glycoconjugated trastuzumab-DBCO-Val-Ser(GlcA)-EDA-PNU ADC (TRPeD)

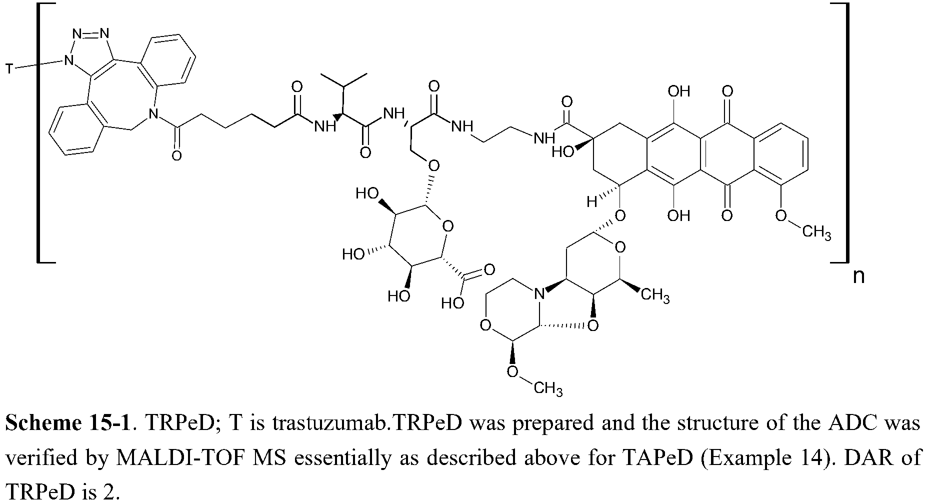

### EXAMPLE 16. Preparation of cysteine engineered flanvotumab ADC FLCPeMcv

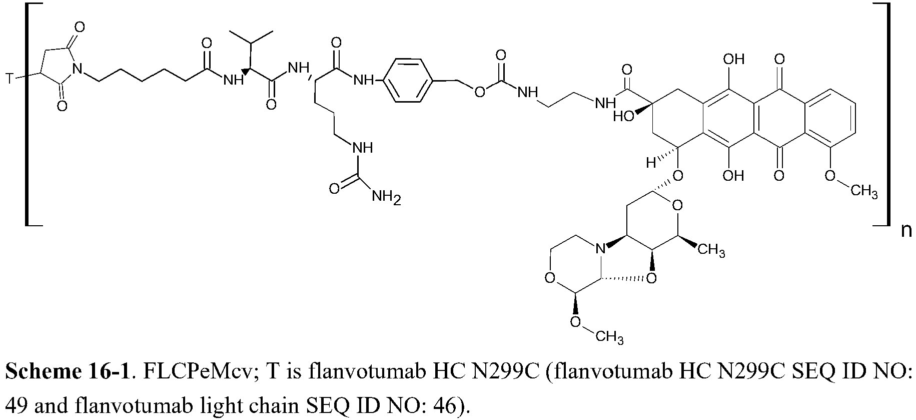

Preparation of PeMcv: MA-caproyl-Val-Cit-PAB-EDA-PNU: 0.9 µmol MA-caproyl-Val-Cit-PAB-PNP and 1.82 µmol PNU-EDA were mixed in 53 µl DMF. 1.4 µmol 0.5 M HOBt in DMF was added and reaction allowed to proceed for 1 hour at room temperature. MA-caproyl-Val-Cit-PAB-EDA-PNU was used in ADC reaction without purification.

Cysteine disulfide bonds of flanvotumab HC N299C were reduced using TCEP followed by reoxidation of hinge region disulfide bonds as described for FLCPeMg. For FLCpeMcv synthesis 7 mg (V=3 ml, c=2.3 mg/ml, 47 nmol) of reoxidized flanvotumab HC N299C was mixed with about 10 molar equivalents of PeMcv: MA-caproyl-Val-Cit-PAB-EDA-PNU in DMSO (55 µL, estimated n=450 nmol). Reaction mixture became opaque upon payload addition. Reaction time 1.5 hours at 37°C. After reaction time sample was centrifugated five minutes @ 3200 rcf to remove precipitate. Clear supernatant was purified. Final sample was purified using HPLC and DAR was determined using the A280/A480 method. FLCPeMcv01: A280 = 3.38, A480 = 0.22. FLCPeMcv DAR was 1.4.

### EXAMPLE 17. Preparation of cysteine engineered flanvotumab ADC FLCPeMg

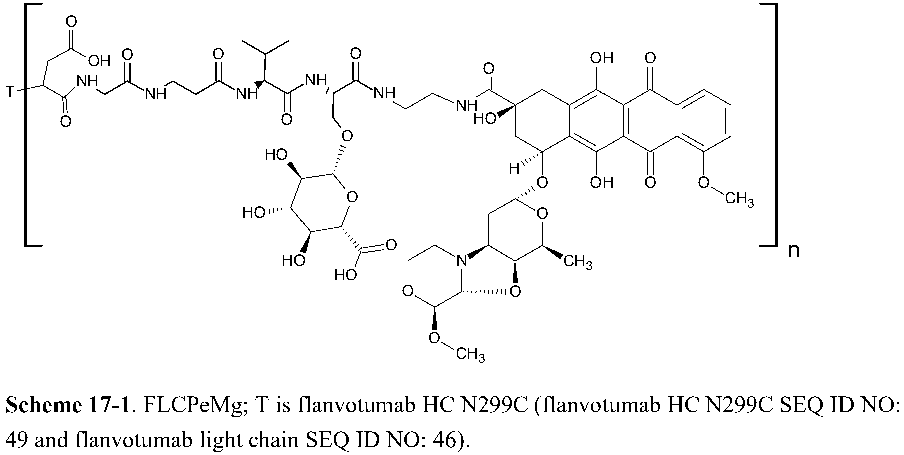

Flanvotumab HC N299C (45.0 mg, c=2.8 mg/ml, V=16 ml, n=300 nmol) was incubated with TCEP (30 molar equivalents, 9 µmol) in PBS for 1.5 hours at 37°C. Excess TCEP was removed from reduced sample using HPLC as described. Half of the purified sample was used for FLCpeMg synthesis. 30 molar equivalents of L-dehydroascorbic acid(L-DHAA) in PBS (350 µL, c=12.1 nmol/µL n=4.2 µmol) was added to reduced Flanvotumab HC N299C (20.9 mg, c=2.09 mg/ml, V=10.0 ml, n=139 nmol) and the sample was incubated for 1 hour at 37 °C. Reoxidized sample was purified from excess L-DHAA using using HPLC. For FLCpeMg synthesis 9 mg (V=4 ml, c=2.25 mg/ml, 60 nmol) of reoxidized Flanvotumab HC N299C was mixed with 10 molar equivalents of PeMg: MA-Ac-β-Ala-VS(GlcA)-EDA-PNU (SyntaBio, San Diego) in DMSO (21.8 µL, c=27.5 nmol/µL n=600 nmol). Reaction time 1.5 hours at 37°C. Final sample was purified using HPLC. DAR was determined using the A280/A480 method. DAR of FLCPeMg was 2.2 (A280 = 4.68, A480 = 0.47).

### EXAMPLE 18. Preparation of cysteine engineered flanvotumab ADC FLCPeMa

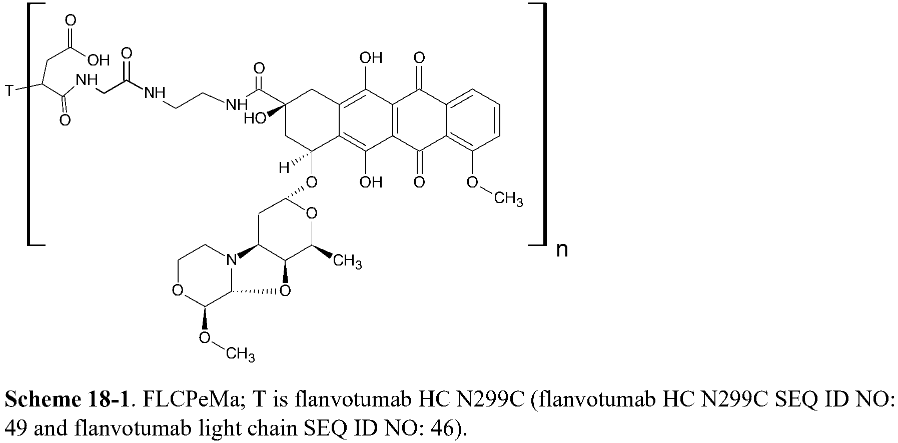

Cysteine disulfide bonds of flanvotumab HC N299C were reduced using TCEP followed by reoxidation of hinge region disulfide bonds as described for FLCPeMg. For FLCpeMa synthesis 7.7 mg (V=4 ml, c=1.93 mg/ml, 51 nmol) of reoxidized flanvotumab HC N299C was mixed with about 2 molar equivalents of PeMa: MA-Ac-EDA-PNU in DMSO (5 µL, estimated n=100 nmol. Reaction time 1.5 hours at 37°C. Final sample was purified using HPLC. DAR was determined using the A280/A480 method. DAR of FLCPeMa was 1.7 (A280 = 3.80, A480 = 0.30).

### EXAMPLE 19. Preparation of cysteine engineered flanvotumab ADC FLCPeMala

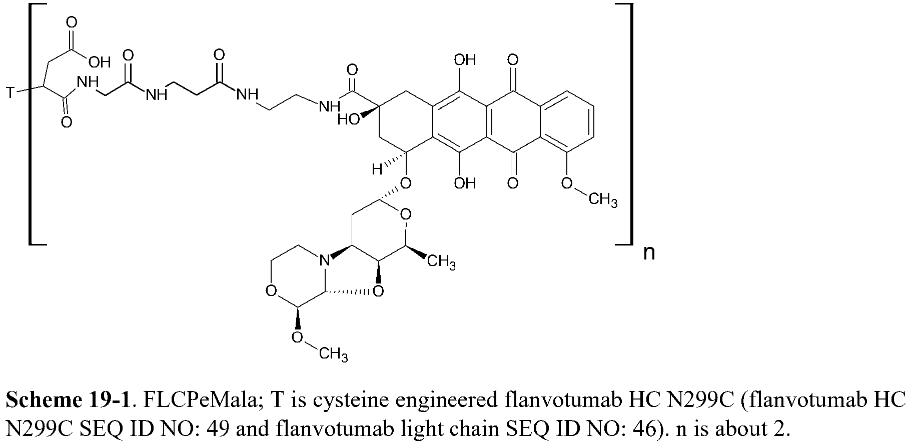

Preparation of PeMala, MA-Ac-β-Ala-EDA-PNU : 400 nmol PNU-EDA and 600 nmol MA-Ac-β-Ala-Pfp were mixed in 170 ml DMSO and allowed to react for 15 minutes at room temperature. The MA-Ac-β-Ala-EDA-PNU purification with Gemini-NX reversed phase column (4.6 x 250 mm, Phenomenex) was done with Äkta HPLC purifier system. Buffer A was 20 mM ammonium acetate pH 5.6 and buffer B was ACN. Column was stabilized with 20 % buffer B and MA-Ac-β-Ala-EDA-PNU eluted with linear gradient: 20 % buffer B to 80 % buffer B for 30 minutes (1 ml/min). Purified PeMala was dried by vacuum concentrator.

Cysteine disulfide bonds of flanvotumab HC N299C were reduced using TCEP followed by reoxidation of hinge region disulfide bonds as described for FLCPeMg. For FLCPeMala synthesis 7.7 mg (V=4 ml, c=1.93 mg/ml, 51 nmol) of reoxidized flanvotumab HC N299C was mixed with about 2 molar equivalents of PeMala: MA-Ac-β-Ala-EDA-PNU in DMSO (5 mL, estimated n=100 nmol. Reaction time 1.5 hours at 37°C. Final sample was purified using HPLC. DAR was determined using the A280/A480 method. DAR of FLCPeMala was 2.0 (A280 = 3.84, A480 = 0.35).

### EXAMPLE 20. Preparation of lintuzumab-AuM ADC LNAuM

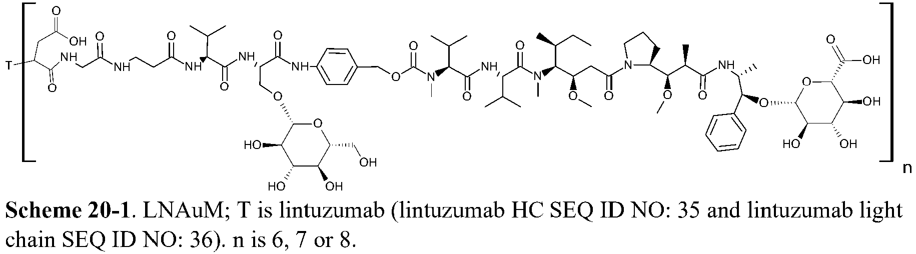

Lintuzumab (2.0-4.9 mg; V=1.17-2.9 mL) was incubated with TCEP (20-30 molar equivalents) in PBS for 1-1.5 hour at 37°C. 28-35 molar equivalents of AuM: MA-Ac-β-Ala-Val-Ser(Glc)-PAB-MMAU in DMSO (42 mL, c=27nmol/mL) was added to the reaction mixture and the mixture was incubatedfor 1 hour at 37°C. LNAuM was purified as described above.

DAR was determined with PLRP-S method. Three batches of produced LNAuM DAR were 7.8, 8.0 and 8.1.

### EXAMPLE 21. Preparation of gemtuzumab-AuM ADC GMAuM

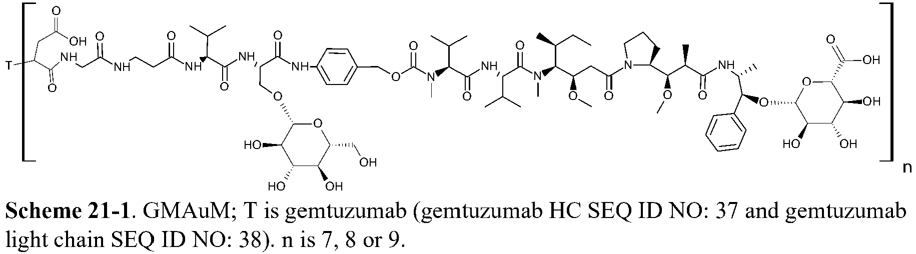

Gemtuzumab (3.8 mg) was incubated with TCEP (50 molar equivalents) in PBS at 37°C overnight for 23. Excess TCEP was removed using Amicon ultra 0.5 ml 30K concentrator tubes with PBS. Volume was adjusted to 1.6 ml with PBS and 30 molar equivalents of AuM: MA-Ac-β-Ala-Val-Ser(Glc)-PAB-MMAU in DMSO (28 mL) was added to the reaction mixture and the mixture was incubated for 2.5 hours at 37°C. GMAuM was purified, concentrated and sterile filtered as described above.

DAR was determined with PLRP-S method. GMAuM DAR was 8.0.

### EXAMPLE 22. Preparation of cysteine engineered lintuzumab-AuM ADC LNCAuM

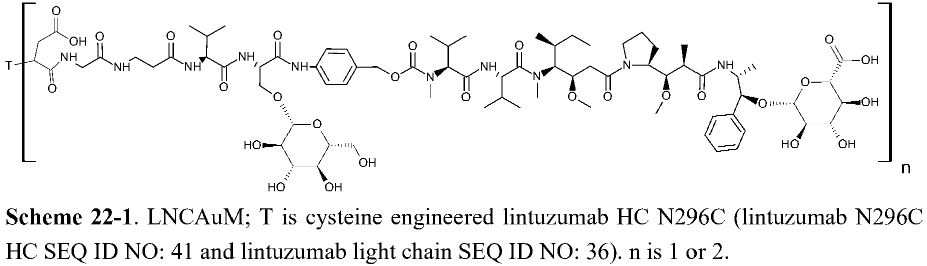

Lintuzumab HC N296C (4.2 mg; V=2.3 ml) was incubated with TCEP (25 molar equivalents) in PBS for 1 hour at 37°C. Excess TCEP was removed from reduced sample using Amicon ultra 0.5 ml 30K concentrator tubes with PBS and volume was adjusted to 1.8 ml. 30 molar equivalents of L-dehydroascorbic acid(L-DHAA) in PBS (84 mL, c=10 nmol/mL) was added to reduced Lintuzumab HC N296C and the sample was incubated for 1.5 hours at 37°C. Reoxidized sample was purified from excess L-DHAA using Amicon ultra 0.5 ml 30K concentrator tubes with PBS, volume was adjusted to 2 mL, and the sample was used to synthesize LNCAuM and LNCauMb.

For LNCAuM synthesis 2.1 mg (1 mL) of reoxidized lintuzumab HC N296C was mixed with 10 molar equivalents of AuM: MA-Ac-β-Ala-Val-Ser(Glc)-PAB-MMAU in DMSO (5.6 mL, c=25 nmol/mL) and the mixture was incubated for 1 h at 37°C. LNCAuM was purified, concentrated and sterile filtered as described above. DAR was determined with PLRP-S method. DAR of LNCAuM was 1.7.

### EXAMPLE 23. Preparation of cysteine engineered lintuzumab MMAU ADC LNCAuMb

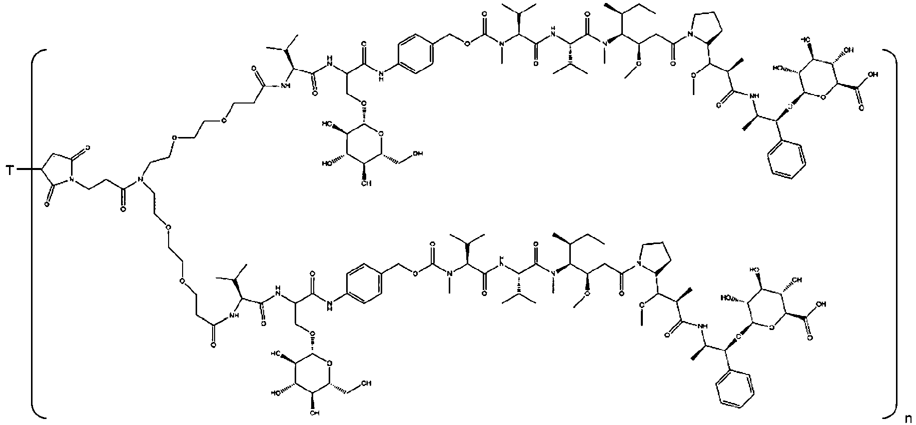

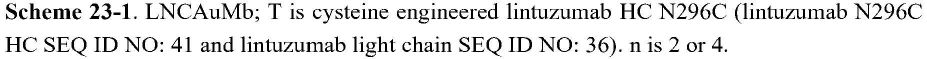

Preparation of AuMb: N-MAL-N-bis(Peg2)-Val-Ser(Glc)-PAB-MMAU : 1.4 mmol Val-Ser(Glc)-PAB-MMAU (SyntaBio) and 280 nmol N-Mal-N-bis(PEG2-NHS ester) (Broadpharm, San Diego) were mixed in 200 ml DMSO. 2 ml ¼ Dipea/DMSO was added for three hours' reaction at room temperature. The N-MAL-N-bis(Peg2)-Val-Ser(Glc)-PAB-purification with Gemini-NX reversed phase column (4.6 x 250 mm, Phenomenex) was done with Äkta HPLC purifier system. Buffer A was 20 mM ammonium acetate pH 5.6 and buffer B was ACN. Column was stabilized with 20 % buffer B and AuMb eluted with linear gradient: 20 % buffer B to 80 % buffer B for 40 minutes (1 ml/min). Purified AuMb was dried by vacuum concentrator.

For LNCAuMb synthesis 2.1 mg (1 mL, 14 nmol) of reoxidized intuzumab HC N296C was mixed with about10-20 molar equivalent of branched payload AuMb: N-MAL-N-bis(Peg2)-Val-Ser(Glc)-PAB-MMAU in 20 mL of DMSO and the mixture was incubated for 1 h at 37°C. LNCAuMb was purified, concentrated and sterile filtered as described above. DAR was determined with PLRP-S method. DAR of LNCAuMb was 3.3.

### EXAMPLE 24. Preparation of cysteine engineered lintuzumab MMAU ADC LNCPeMa

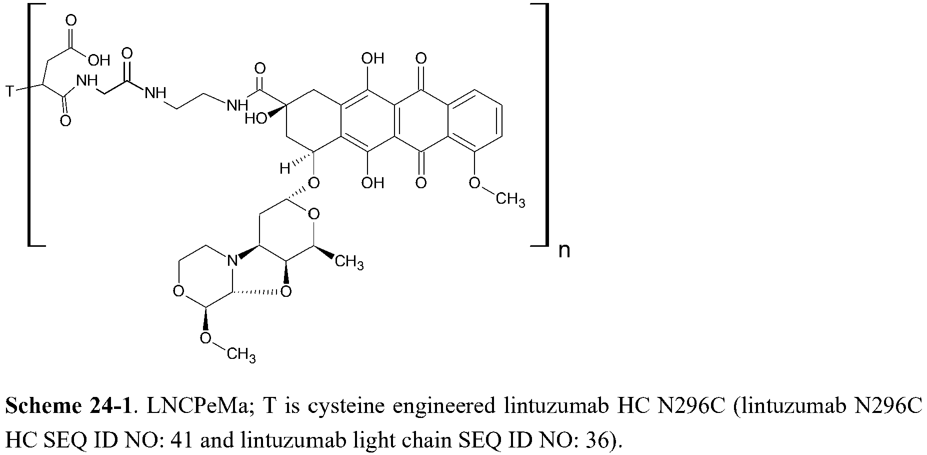

Lintuzumab HC N296C (4.0 mg, V=2.82 mL) was incubated with TCEP (40 molar equivalents) in PBS for 1 hour at 37°C. Excess TCEP was removed from reduced sample using Mab Select sure column/Äkta HPLC purifier system. 35 molar equivalents of L-dehydroascorbic acid(L-DHAA) in PBS (29 mL, c=17 nmol/mL) was added to reduced lintuzumab HC N296C (2.1 mg, V=2.0 mL) and the sample was incubated for 1.5 h at 37°C. Reoxidized sample was purified from excess L-DHAA using using Mab Select sure column/Äkta HPLC purifier system.

For LNCPeMa synthesis 1.6 mg (V=2 ml) of reoxidized lintuzumab HC N296C was mixed with 10 molar equivalents of PeMa: MA-Ac-EDA-PNU in DMSO (10 mL, c=10 nmol/mL) and mixture was incubated for 1 h at 37°C. Based on MALDI analysis, DAR of LNCPeMa is about 2.

### EXAMPLE 25. Preparation of cysteine engineered chimeric TA99 ADC CHCPeMg

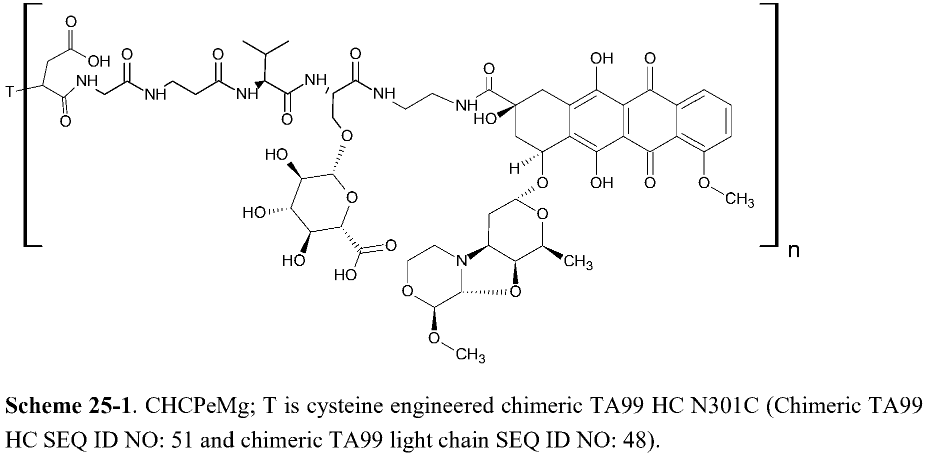

Chimeric TA99 HC N301C (6.0 mg, V=4.55 mL) was incubated with TCEP (40 molar equivalents) in PBS for 1.5 hours at 37°C. Excess TCEP was removed from reduced sample using HPLC. 35 molar equivalents of L-dehydroascorbic acid(L-DHAA) in PBS (42.8 mL, c=24 nmol/mL) was added to reduced Chimeric TA99 HC N301C (4.4 mg, V=2.5 ml) and the sample was incubated for 1 hour at 37°C. Reoxidized sample was purified from excess L-DHAA HPLC.

For CHCpeMg synthesis 3.7 mg (V=2.5 ml) of reoxidized Chimeric TA99 HC N301C was mixed with 10 molar equivalents of PeMg: MA-Ac-b-Ala-VS(GlcA)-EDA-PNU in DMSO (9 mL, c=27.5 nmol/mL) and incubated overnight at 37°C. Final sample was purified using HPLC as described above. MALDI analysis showed that DAR is about 2.

### EXAMPLE 26. Preparation of flanvotumab-AuM ADC FLAuM

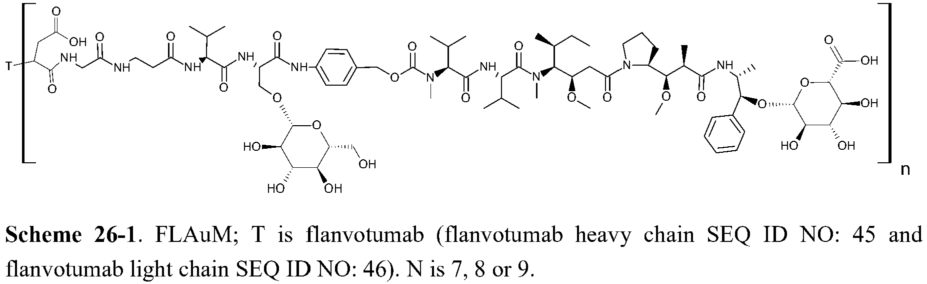

5 mg of Flanvotumab (c=3.06 mg/mL) was diluted to 2.0 mg/mL with PBS and was reduced in the presence of 25X molar excess of TCEP at +37°C for 1.5 hour. For FLAuM synthesis 30X molar excess of AuM: MA-Ac-β-Ala-Val-Ser(β-Glc)-PAB-MMAU was added and the mixture was incubated at +37°C for 1.5 h. Final FLAuM ADC was purified and sterile filtered as described above. DAR was determined with PLRP-S. DAR of FLAuM was 7.9.

### EXAMPLE 27. Preparation of chimeric TA99 ADC CHAuM

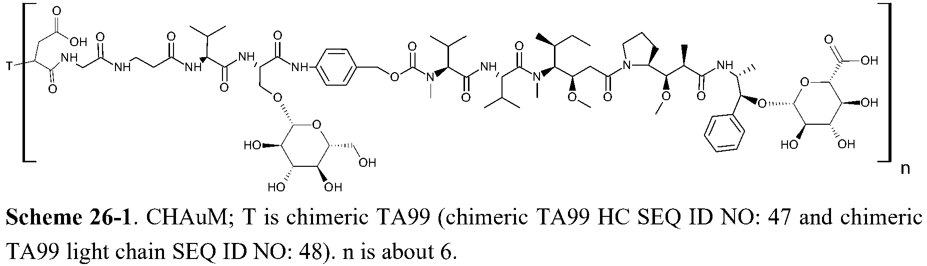

TA99 MMAU ADC was prepared as FLAuM and purified as described above. According to MALDI analysis, DAR of the prepared batch of CHAuM was 6.

### EXAMPLE 28. Maleimide ring stability caproyl vs β-Ala

### Preparation of TRAuM

10 mg of trastuzumab (Herceptin^{®}, Roche; c=2.0 mg/ml in PBS) was reduced in the presence of 25x molar excess of TCEP at +37°C for 1.5 h. 30x molar excess of MA-Ac-β-Ala-Val-Ser(β-Glc)-PAB-MMAU was added and reaction allowed to proceed at +37°C for 1.5 hours. Final TRAuM was purified as described above, sterile filtered and stored at 4°C. DAR was determined with PLRP-S. DAR of TRAuM was 7.9.

### Preparation of TRAuMc (trastuzumab-caproyl-MMAU)

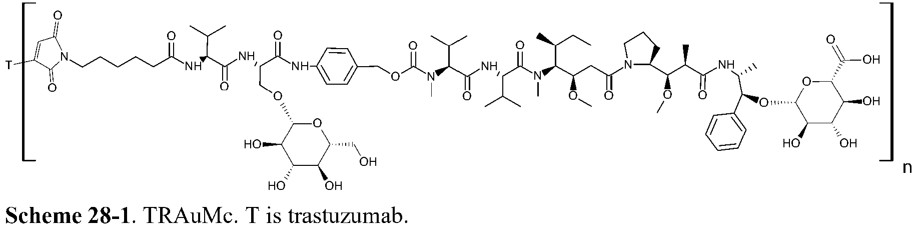

MMAU-PAB-(Glc)SerVal (3 µmol) in 300 µl DMF was reacted with 5 molar excess of EMCS in 500 µl DMF. 3 µl Dipea was added, and reaction incubated at room temperature. After one hour reaction 2 µl Dipea was added and incubation continued for 1.5 hours. MALDI: AuMc M+Na = 1606, M+2Na = 1628.

AuMc (MA-caproyl-ValSer(Glc)PAB-MMAU) purification with Gemini-NX reversed phase column (4.6 x 250 mm, Phenomenex) was done with Äkta HPLC purifier system. Buffer A was 0.1 % TFA in MilliQ-water and buffer B was ACN. Column was stabilized with 20 % buffer B and AuMc eluted with linear gradient: 20 % buffer B to 60 % buffer B for 40 minutes (1 ml/min). Purified AuMc was dried by vacuum concentrator.

2 mg of trastuzumab (c=2.5 mg/ml in PBS was reduced in the presence of 25X molar excess of TCEP at +37 °C for one hour. An estimated 30X molar excess of MA-caproyl-Val-Ser(β-Glc)-PAB-MMAU was added, and reaction allowed to proceed at +37°C for 1 hour.

ADC was purified as described above, sterile filtered and stored at 4°C. The purified trastuzumab-caproyl-MMAU ADC was sterile filtered, and the final ADC sample was assigned a code TRAuMc, yield was 1.4 mg. DAR was determined with PLRP-S. DAR of TRAuMc was 7.9.

Stabilization of maleimide ring by hydrolysis was compared between TRAuMc and TRAuM. Both ADCs were incubated in PBS at 37°C for 24 h. The stabilization reaction was followed at time points of 0 h, 5 h and 24 h by MALDI-TOF MS of the ADC. Figure 8 shows the light chain area of the mass spectrum for both TRAuMc (Figure 8A) and TRAuM (Figure 8B). The maleimides of TRAuMc did not stabilize by hydrolysis during the 24 h incubation, since the m/z of the [M+H]+ ion of the light chain + payload (LC+PL) component of the ADC did not change and was detected at m/z between 25015-25018 at every time point. In contrast, the maleimides of TRAuM effectively stabilized by hydrolysis during the 24 h incubation, since the m/z of the LC+PL ion changed from m/z 25032.785 at 0 h to 25049.032 at 24 h, corresponding to the hydrolysis reaction (observed change +16.2 Da, calculated addition of water +18.0 Da). At the 5 h time point of the TRAuM incubation, both the original non-hydrolyzed LC+PL ion and the hydrolyzed/stabilized product were visible. Thus, an ADC with the AuM linker-payload comprising maleimidoacetyl group was readily stabilized in a few hours in mild conditions, at pH 7.4 and 37°C. In contrast, an ADC with the AuMc linker-payload comprising maleimidocaproyl group was not stabilized in the same conditions.

### EXAMPLE 29. Maleimide conjugate stability in the presence of glutathione and albumin.

**TRAuMc and TRAuM ADCs were incubated in the presence of 5 mM oxidized** glutathione (Sigma) in 50 mM HEPES pH 7.4 containing 1 mM EDTA at 37°C. Transfer of the linker-payload from the ADC to glutathione was monitored by MALDI-TOF MS at 0, 1, 2, 3, 7 and 10 days. Cysteamine-linker-payload was made for use as an internal standard by incubating MA-Ac-β-Ala-Val-Ser(β-Gle)-PAB-MMAU with an excess of cysteamine for two hours at room temperature, after which the formation of the correct product was verified by MALDI-TOF MS. The timepoint samples (á 5 µL) were purified with miniaturized Poros R2 columns together with the internal standard, same amount of standard molecule in each analysis. The samples were eluted onto target plate into two spots. DHB was used as a matrix. The averages of the corresponding peaks of internal standard and sample in the two spots were calculated at the flowing m/z values: cysteamine-AuM (internal standard, maleimide not hydrolyzed) [M+H]⁺ 1676.9, [M+Na]⁺ 1698.9, [M+2Na-H]⁺ 1720.9; glutathione-AuMc [M+H]⁺ 1892.1, [M+Na]⁺ 1914.1, [M+2Na-H]⁺ 1936.1; glutathione-AuM (hydrolyzed maleimide) [M+H]⁺ 1925.1, [M+Na]⁺ 1947.1, [M+2Na-H]⁺ 1969.1. The corresponding peak intensities were summed, and the ratios of linker-associated signals and the internal standard were calculated. Figure 9A shows the results, demonstrating 10-fold higher de-conjugation rate for the maleimidocaproyl group comprising linker of TRAuMc compared to the maleimidoacetyl group comprising linker of TRAuM during the 10-day experiment.

Stability of TRAuMc and TRAuM ADCs were also compared in the presence of human serum albumin (HSA) in PBS buffer pH 7.4 at 37°C. Payload loss due to payload transfer to HSA was monitored by DAR analysis with RP-HPLC as described above. The ADCs (100 µg/ml) were incubated with HSA (40 mg/ml) at 37°C and samples were taken at 0, 1, 5, and 10 days. The samples were stored at -20°C before the analysis and purified by Protein A HPLC as described above to remove the HSA in order to avoid any potential interference of HSA in the DAR analysis. Figure 9B shows the results. DAR of TRAuM had a slight reduction from 7.8 to 7.2 during the 10 days experiment, whereas DAR of TRAuMc was reduced from 7.9 down to 5.4. Thus, the ADC with the AuM linker-payload comprising maleimidoacetyl group displayed greatly enhanced stability towards de-conjugation in the presence of HSA. In contrast, the ADC with the AuMc linker-payload comprising maleimidocaproyl group displayed continuos time-dependent payload loss and lesser stability in the same conditions. Figure 10 shows the RP-HPLC chromatograms of the experiment.

### EXAMPLE 30. In vitro efficacy of ADCs.

Toxicity of anti-TYRP ADCs was tested with SK-MEL-28 (ATCC: HTB^{®}-72^{™}), SK-MEL-30 (DSMZ: ACC 151) and/or IGR-1 (DSMZ: ACC 236) human melanoma cells.

SK-MEL-28 cells were seeded in 96-well plate, 2000 cells/well and cultivated in 10% FBS/EMEM -medium under standard cell culture conditions. After overnight culture, the diluted ADCs or unconjugated antibodies were added to cells (concentration range 0.02 nM - 200 nM or 0.1 nM-300 nM) and incubated for 3-5 days. For cell control, the cells were treated with medium without ADC. The viability of the cells was evaluated with PrestoBlue cell viability reagent (Life Technologies) according to the manufacturer's instructions.

SK-MEL-30 were seeded in 96-well plate, 2000 cells/well or 3000 cells/well and cultivated in 10% FBS/RPMI 1640 -medium under standard cell culture conditions. After overnight culture, diluted ADCs or antibodies were added to the cells, and incubated for 3-5 days. For cell control, the cells were treated with medium without ADC. The viability of the cells was evaluated with PrestoBlue cell viability reagent.

IGR-1 cells were seeded in 96-well plate, 2000 cells/well, and cultivated in 10% FBS/DMEM -medium under standard cell culture conditions. After overnight culture, the diluted ADCs or unconjugated antibodies (concentration range 0.1 nM - 300 nM) were added to cells, and incubated for 3-5 days. The viability of the cells was evaluated with PrestoBlue cell viability reagent.

The AVG % values and standard deviations were transferred to GraphPad Prism 9.1.2. Dose-response curves were generated and the IC50% values of the samples were obtained by non-linear regression analysis (Inhibitor vs. response, variable slope (four parameters)) using the software (IC50%, the concentration of compound needed to yield a 50% reduction in viability compared with vehicle-treated cells (control = 100%).

Results with the ADCs are shown in Tables 30-1 to 30-14 below. In IGR-1 and SK-MEL-30 cells unconjugated anti-mGP75 and flanvotumab antibodies had no effect up to 300 nM (the highest concentration tested). In HL-60 and K-562 cells unconjugated anti-CD33 antibodies lintuzumab and gemtuzumab had no effect up to 300 nM (the highest concentration tested). In KG-1, Ramos and Daudi cells unconjugated lintuzumab had no effect up to 300 nM (the highest concentration tested). Therefore, the observed high cytotoxicities of the ADCs are direct evidence for specific payload cytotoxicity and anti-cancer activity of the antibody-drug conjugates.

**Table 30-1. Range of IC50 values of FLCPeMcv ADC (n=2).**

| **Cell line** / **ADC** | **FLCPeMcv DAR 1.4** |
|---|---|
| **IGR-1** | 414 pM - 716 pM |
| **SK-MEL-30** | 2.8 nM - 4.0 nM |

**Table 30-2. Range of IC50 values of FLCPeMg ADC (n=2).**

| **Cell line** / **ADC** | **FLCPeMg DAR 2.2** |
|---|---|
| **IGR-1** | 417 pM - 484 pM |
| **SK-MEL-30** | 4.3 nM - 10 nM |

**Table 30-3. IC50 values of FLCPeMa ADC.**

| **Cell line** / **ADC** | **FLCPeMa DAR 1.7** |
|---|---|
| **IGR-1** | 1.2 nM (95% CI: 0.94-1.5 nM) |
| **SK-MEL-30** | 22 nM (95% CI: 19-25 nM) |

**Table 30-4. IC50 values of FLCPeMala ADC.**

| **Cell line** / **ADC** | **FLCPeMala DAR 2.0** |
|---|---|
| **IGR-1** | 649 pM (95% CI: 394-903 pM) |

**Table 30-5. IC50 values of FLPeD ADC.**

| **Cell line** / **ADC** | **FLPeD DAR 2** |
|---|---|
| **SK-MEL-28** | 100 nM (95% CI: 93-107 nM) |
| **IGR-1** | 428 pM - 601 pM (n=3) |
| **SK-MEL-30** | 1.3-8.2 nM (n=3) |

**Table 30-6. IC50 values of CHPeD ADC.**

| **Cell line** / **ADC** | **CHPeD DAR 2** |
|---|---|
| **SK-MEL-28** | 55 nM |
| **IGR-1** | 1.4 nM (95% CI: 1.0-1.8 nM) |
| **SK-MEL-30** | 5.7 nM (95% CI: 5.1-6.5 nM) |

**Table 30-7. IC50 values of TAPeD ADC.**

| **Cell line** / **ADC** | **TAPeD DAR 2** |
|---|---|
| **IGR-1** | 1.2 nM (95% CI: 0.97-1.4 nM) |
| **SK-MEL-30** | 8.6 nM (95% CI: 7.9-9.3 nM) |

**Table 30-8. IC50 values of TAAuM ADC.**

| **Cell line** / **ADC** | **TAAuM DAR 10** |
|---|---|
| **IGR-1** | 1.3 nM (95% CI: 0.89-1.7 nM) |
| **SK-MEL-30** | 7.9-26 nM (n=2) |

**Table 30-9. IC50 values of FLAuM ADC.**

| **Cell line** / **ADC** | **FLAuM DAR 6** |
|---|---|
| **SK-MEL-28** | > 300 nM |
| **IGR-1** | 4.1 nM (95% CI: 3.4-4.8 nM) |
| **SK-MEL-30** | 123 nM (95% CI: 96-158 nM) |

**Table 30-10. IC50 values of CHAuM ADC.**

| **Cell line** / **ADC** | **CHAuM DAR 6** |
|---|---|
| **SK-MEL-28** | > 300 nM |
| **IGR-1** | 2.2 nM (95% CI: 1.9-2.5 nM) |
| **SK-MEL-30** | 48 nM (95% CI: 36-62 nM) |

**Table 30-11. Range of IC50 values of LNAuM ADC (AVG ± standard deviation). HL-60 cells were treated for 5 days. KG-1 cells were treated for 3 or 4 days. MOLM-13, K562 and Daudi cells were treated for 3 days.**

| **Cell line** / **ADC** | **LNAuM DAR 8** |
|---|---|
| **HL-60** | **24-459** pM (n=4) |
| | 272 ± 181 pM |
| **MOLM-13** | 23-55 pM (n=3) |
| | 39 ± 16 pM |
| **KG-1** | 144-313 pM (n=3) |
| | 242 ± 88 pM |
| **K-562 (low CD33)** | 24-66 nM (n=2) |
| **Ramos.2G6.4C10 (no CD33 expression)** | No effect up to 300 nM |
| **Daudi (no CD33 expression)** | No effect up to 300 nM |

**Table 30-12. IC50 values of GMAuM ADC. HL-60 cells were treated for 5 days. MOLM-13 and K562 cells were treated for 3 days.**

| **Cell line** / **ADC** | **GMAuM DAR 8** |
|---|---|
| **HL-60** | 583 pM (95% CI: 487- 700 pM) |
| **MOLM-13** | 206 pM (95% CI: 189-224 pM) |
| **K-562 (low CD33)** | 241 nM (95% CI: 217-266 nM) |

**Table 30-13. IC50 values of LNCAuM ADC after 5 days' treatment.**

| **Cell line** / **ADC** | **LNCAuM DAR 1.7** |
|---|---|
| **HL-60** | 16 nM (95% CI: 11-24 nM) |

**Table 30-14. IC50 values of LNCAuMb ADC after 5 days' treatment.**

| **Cell line** / **ADC** | **LNCAuMb DAR3.3** |
|---|---|
| **HL-60** | 2.2 nM (95% CI: 1.5-3.1 nM) |

Taken together, Tables 30-1 to 30-14 show that both anti-TYRP1 ADCs and anti-CD33 ADCs demonstrated high and specific cytotoxic efficacy against target antigen-expressing cancer cells.

Amount of TYRP1 antigen in the cell lines were in the order of IGR-1 > SK-MEL-30 > SK-MEL-28. Thus, anti-TYRP1 ADCs were the most efficient against IGR-1 cells, while they showed intermediate activity against SK-MEL-30 cells and were the least effective against SK-MEL-28 cells. All the anti-TYRP1 ADCs prepared from flanvotumab, TA99 and chimeric TA99 antibodies had effective anti-cancer and anti-melanoma activity. However, flanvotumab-based ADCs FLCPeMcv (Table 30-1), FLCPeMg (Table 30-2), FLCPeMala (Table 30-4) and FLCPeD (Table 30-5) had picomolar IC50 values against IGR-1 cells, demonstrating highest anti-cancer and anti-melanoma efficacy for flanvotumab-based ADCs. The efficacy of DAR=2 PNU ADCs was comparable regardless of whether the conjugation was to glycans (for example, FLPeD in Table 30-5, IC50 down to 428 pM for IGR-1 cells) or to engineered cysteines (for example, FLCPeMg in Table 30-2, IC50 down to 417 pM for IGR-1 cells), showing effective payload delivery to cancer cells with both ADCs. In addition to the PNU payloads, also MMAU payloads had effective anti-cancer and anti-melanoma activity, for example TAAuM (Table 30-8), FLAuM (Table 30-9) and CHAuM (Table 30-10), all with IC50 values of about 1-4 nM against IGR-1 cells.

LNAuM (Table 30-11) was more effective against all tested CD33+ cancer cell lines than GMAuM (Table 30-12), although both ADCs were highly active with picomolar IC50 values. LNAuM had very high efficacy against CD33+ cells with high CD33 expression showing IC50 values down to low picomolar (23-24 pM) against HL-60 and MOLM-13 cells (Table 30-11). Further, LNAuM IC50 value against cells with low CD33 expression such as K-562 was low nanomolar (down to 24 nM) corresponding to effective anti-cancer cell activity. However, IC50 was not reached even at 300 nM concentration of LNAuM with CD33- cell lines Ramos and Daudi (Table 30-11), indicating that the in vitro therapeutic window of LNAuM was greater than 10000. Experiments with ADCs of different DAR showed that ADC efficacy increased with higher DAR, for example with HL-60 cells and LN ADCs with the same MMAU payload but different DAR: LNCAuM (DAR=1.7), LNCAuMb (DAR=3.3) and LNAuM (DAR=8) had anti-HL-60 cell IC50 values of 16 nM, 2.2 nM and down to 24 pM, respectively (Tables 30-13, 30-14 and 30-11). Although IC50 values of 2.2 nM and 16 nM are still effective anti-cancer activities, LNAuM with 8 payloads/antibody (DAR=8; IC50 down to 23 pM) was shown to have superior anti-cancer activity.

### EXAMPLE 31. In vivo efficacy of PNU-ADCs.

In vivo anti-tumor efficacy of anti-TYRP1 ADCs (TAPeD, FLPeD, CHPeD and FLCPeMg) was evaluated in the B16-F10 mouse melanoma model as described above. 0.25 million cells were inoculated s.c. to the flank of each mouse. Tumor growth was followed by palpation, and ADC dosings were administered when tumor sizes reached 60-110 mm³, between 2-7 days after the inoculation. On each administration day, mice with suitably sized tumors were randomly divided into the study groups so that the groups were comparable with regard to both injection days and tumor sizes (6 mice/group, average tumor size/group 81-83 mm³). A single i.v. injection of 5 mg/kg ADC in PBS was given on the injection day and the tumor sizes were followed for at least 28 days. In all ADC treatment groups, 2 mice showed effective tumor growth inhibition (<200 mm³ tumors). Another xenograft experiment was performed similarly as above. In this experiment, 10 xenografted mice received no treatment, while 10 xenografted mice were given three i.v. doses of 10 mg/kg TA99 antibody on days 2, 7 and 12 after the inoculation (3 x 10 mg/kg at 5 day intervals). In every one of these 20 mice the tumors grew to >200 mm³ during 28 days' follow-up period. Taken together, with all four tested ADCs the treatment with a single dose of 5 mg/kg was more effective at reducing tumor growth than three doses of 10 mg/kg of the naked antibody, showing anti-tumor efficacy with each payload-linker. The results of these experiments are summarized in Table 31-1.

**Table 31-1. B16-F10 xenograft results.**

| **Treatment (i.v.)** | **Number of mice/group** | **Number of mice with <200 mm³ tumor at end of study** |
|---|---|---|
| Single 5 mg/kg dose of TAPeD on days 2-7 when tumors reach 60-110 mm³ | 6 | 2 |
| Single 5 mg/kg dose of FLPeD on days 2-7 when tumors reach 60-110 mm³ | 6 | 2 |
| Single 5 mg/kg dose of CHPeD on days 2-7 when tumors reach 60-110 mm³ | 6 | 2 |
| Single 5 mg/kg dose of FLCPeMg on days 2-7 when tumors reach 60-110 mm³ | 6 | 2 |
| 3 x 10 mg/kg TA99 antibody on days 2, 7 and 12 after inoculation | 10 | 0 |
| No treatment | 10 | 0 |

### EXAMPLE 32. In vivo tolerability of PNU-ADCs.

Tolerability and safety of ADCs conjugated with PNU-EDA linker-payloads were tested in normal C57BL/6J mice. Female young adult mice at the age of 8-10 weeks were used, 3 mice/group (n=3). The study was performed at the TCDM / Central Animal Laboratory, University of Turku, Finland, according to the appropriate ethical committee approval. Clinical signs, body weight and general behavior of the animals were observed regularly. Dose-limiting toxicity was observed as either over 10 % reduction of average body weight in the group or acute toxicity in any animal in the group. Maximum tolerated dose (MTD) was determined as the next lower dose below the dose-limiting toxicity dose level. ADCs were prepared as described above and a single i.v. dose of the ADC was given on the first day of the study. The mice were followed for up to 30 days if no dose-limiting toxicity was observed. No toxicity was observed with either 20 mg/kg or 30 mg/kg TA99 antibody, and the average body weight of control mice grew over 10% during the study. The results of the study are shown in Table 32-1. Taken together, the ADCs showed safety at or above the efficacious dose level in vivo, therefore demonstrating a useful therapeutic window.

**Table 32-1. Results of mouse tolerability study.**

| **ADC** | **Dose-limiting toxicity dose level** | **MTD (maximum tolerated dose)** |
|---|---|---|
| **TAPeD** | 15 mg/kg | 10 mg/kg |
| **FLPeD** | 15 mg/kg | 10 mg/kg |
| **FLCPeMg** | 12.5 mg/kg | 10 mg/kg |
| **FLCPeMa** | 20 mg/kg | 15 mg/kg |
| **FLCPeMala** | 10 mg/kg | 5 mg/kg |
| **FLCPeMcv** | 15 mg/kg | 10 mg/kg |
| **TRPeD** | 20 mg/kg | 10 mg/kg |

### EXAMPLE 33. In vivo efficacy of anti-CD33 ADCs.

In vivo anti-leukemia xenograft efficacy was evaluated for anti-CD33 ADCs LNAuM (DAR=8.0) and GMAuM (DAR=8.0), as well as anti-CD33 antibodies LN and GM. HL-60 acute myeloid leukemia (AML) cells were obtained from the ATCC and cultured according to the manufacturer's inctructions. The study was performed at the TCDM / Central Animal Laboratory, University of Turku, Finland, according to the appropriate ethical committee approval. Cells for inoculation to mice were prepared in vigorous exponential growth phase. 2 million cells in 50% Matrigel were inoculated s.c. to the flank of each mouse (female athymic nude mice between 8-10 weeks of age). Clinical signs and general behavior of the animals was observed regularly. No signs of toxicity were recorded. At the end of the study, the mice were examined for potential macroscopic changes in major organs, but none were detected. Tumor growth was followed by palpation. After caliper measurement, tumor volume was calculated according to 0.5 x length x width². The first dosings were administered when average tumor volume reached 100 mm³. Mice were evenly divided into study groups, 5 mice in each treatment group and 8 mice in the control group, so that each group received similar distribution of different-sized tumors and the average tumor volumes were similar in each group (100-102 mm³). Intravenous (i.v.) treatment of 10 mg/kg either antibody or ADC in PBS was given once (single dose regimen), while the control group received no treatment.

Figures 6-7 show the results of the study. The tumors grew fast both in the control group and the GM treatment group during the follow-up period of 30 days, and in both of these groups part of the animals had to be sacrificed due to tumor growth before the end of the experiment. In the LN treatment group tumor growth was inhibited for about 20 days, after which all the tumors continued growing slowly. In both ADC treatment group the tumors disappeared in all five mice (5/5) without regrowth during the experiment (Figure 6). In all groups, the average body weight of the mice grew during the experiment and no treatment-related decrease in body weight was detected (Figure 7). Taken together, both LNAuM and GMAuM anti-CD33 MMAU ADCs showed effective anti-cancer and anti-AML activity, and no toxicity was detected.

Serum samples (from 40 µl blood) were taken from each mouse in the treatment groups as well as two mice in the control group to follow systemic exposure of the compounds on day 1, day 5 and day 9 after dosing. ADC concentrations in the serum samples were analyzed by ELISA using FastELISA Human immunoglobulin quantification kit (RD-Biotech) according to the manufacturer's instructions. The results are shown in Table 15.1. Between 1 d - 9 d, LNAuM had better systemic exposure than GMAuM and GMAuM was eliminated faster from circulation by Day 9 than LNAuM.

**Table 15.1. Average concentrations of ADCs in serum samples 1, 5 and 9 days after dosing, expressed in µg/ml as average ADC concentration ± standard deviation (n = 5). AUC(1-9d) shows partial area under curve between 1 d - 9 d in mg x d / L, calculated by the trapezoidal method.**

| | **GMAuM** | **LNAuM** |
|---|---|---|
| **Day 1** | 56.2±15.6 | 37.1±17.9 |
| **Day 5** | 10.1±4.8 | 16.6±8.8 |
| **Dav 9** | 3.3±1.6 | 22.0±7.8 |
| **AUC(1-9d)** | 160 | 200 |

## Claims

1. A linker-payload conjugate of Formula IGX 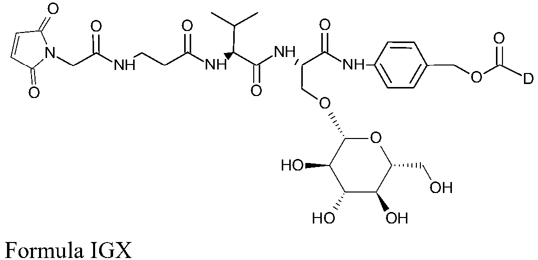
wherein D is a payload molecule, wherein the payload molecule is monomethylauristatin E, monomethylauristatin F, monomethylauristatin E β-D-glucuronide (MMAU), or a monomethylauristatin E saccharide conjugate of Formula AS 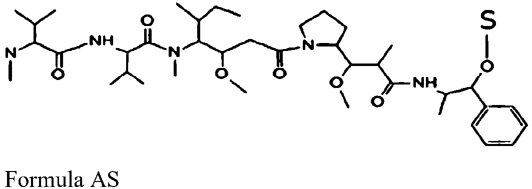
wherein S is a saccharide, wherein the saccharide comprises or is a monosaccharide selected from the group consisting of β-D-galactose, N-acetyl-β-D-galactosamine, N-acetyl-α-D-galactosamine, N-acetyl-β-D-glucosamine, β-D-glucuronic acid, α-L-iduronic acid, α-D-galactose, α-D-glucose, β-D-glucose, α-D-mannose, β-D-mannose, α-L-fucose, β-D-xylose, and neuraminic acid.

2. The linker-payload conjugate of claim 1, wherein the conjugate is 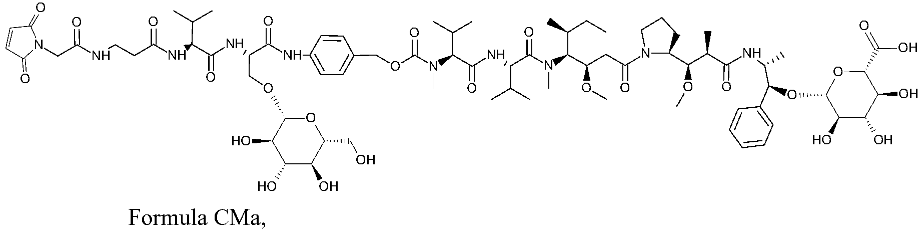 or

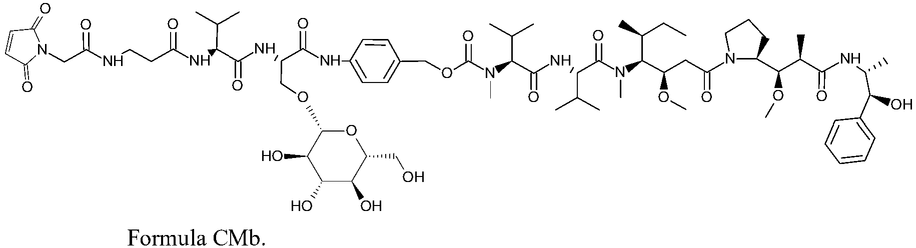

3. A targeting unit-linker-payload conjugate of Formula IIGX or Formula IIGXs 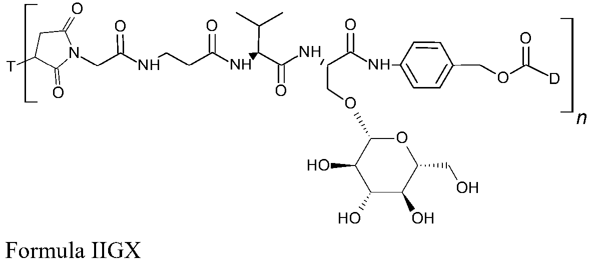 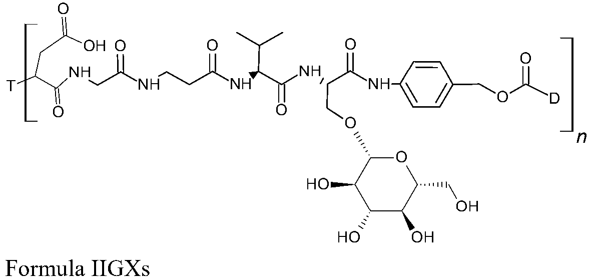
wherein T is an antibody; D is a payload molecule; and n ≥ 1, or n is in the range of 1 to about 20, or 1 to about 15, or 1 to about 10, or 2 to 10, or 2 to 6, or 2 to 5, or 2 to 4; or n is 1, 2, 3, 4, 5, 6, 7, 8, 9, 10, 11, 12, 13, 14, 15, 16, 17, 18, 19, or 20;
wherein the payload molecule is monomethylauristatin E, monomethylauristatin F, monomethylauristatin E β-D-glucuronide (MMAU), or a monomethylauristatin E saccharide conjugate of Formula AS 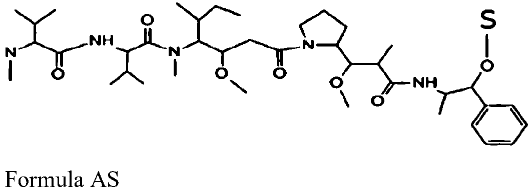
wherein S is a saccharide, wherein the saccharide comprises or is a monosaccharide selected from the group consisting of β-D-galactose, N-acetyl-β-D-galactosamine, N-acetyl-α-D-galactosamine, N-acetyl-β-D-glucosamine, β-D-glucuronic acid, α-L-iduronic acid, α-D-galactose, α-D-glucose, β-D-glucose, α-D-mannose, β-D-mannose, α-L-fucose, β-D-xylose, and neuraminic acid.

4. The targeting unit-linker-payload conjugate of claim 3, wherein the antibody is selected from the group consisting of bevacizumab, tositumomab, etanercept, trastuzumab, adalimumab, alemtuzumab, gemtuzumab ozogamicin, efalizumab, flanvotumab, rituximab, infliximab, abciximab, basiliximab, palivizumab, omalizumab, daclizumab, cetuximab, panitumumab, epratuzumab, 2G12, lintuzumab, nimotuzumab and ibritumomab tiuxetan, or the antibody is selected from the group consisting of an anti-EGFR antibody, an epidermal growth factor receptor 2 (HER2/neu) antibody, an anti-CD22 antibody, an anti-CD30 antibody, an anti-CD33 antibody, an anti-Lewis y antibody, anti-TYRP-1, an anti-CD20 antibody and an anti-hematologic target antibody.

5. The targeting unit-linker-payload conjugate of claim 3 or 4, wherein the antibody is capable of binding an anti-hematologic target molecule selected from the group consisting of CD19, CD20, CD22, CD25, CD30, CD33, CD37, CD38, CD52, CD56, CD70, CD74, CD79, CD98, CD117, CD105, CD123, CD138, CD157, BCMA and CD319 (SLAMF7).

6. The targeting unit-linker-payload conjugate of any one of claims 3-5, wherein the antibody is a hematologic target antibody selected from the group consisting of Loncastuximab, Blinatumomab, Tafasitamab, Coltuximab, denintuzumab, Obexelimab, Inebilizumab, MOR00208, MDX-1342, MEDI-551, SAR3419, Rituximab, Ofatumumab, Veltuzumab, Ocrelizumab, Obinutuzumab, Ocaratuzumab, Ublituximab, nofetumomab, ibritumomab, Epratuzumab, Inotuzumab ozogamicin, bectumomab, moxetumomab, pinatuzumab, DCDT2980S, Basiliximab, Daclizumab, camidanlumab, inolimomab, ADCT-301, IMTOX-25, Brentuximab, iratumumab, AVE9633, lintuzumab, gemtuzumab, vadastuximab, otlertuzumab, lilotomab, naratuximab, BI836826, AGS67E, IMGN529, Daratumumab, Isatuximab, mezagitamab, felzartamab, MOR202, MOR03087, Alemtuzumab, Lorvotuzumab mertansine, Vorsetuzumab mafodotin, SGN-70A, polatuzumab, indatuximab, MDX-1203, Milatuzumab-doxorubicin, IGN523, LOP-628, CSL360, Talacotuzumab, XmAb14045, KHK2823, BT062, Belantamab mafodotin, teclistamab and Elotuzumab.

7. The targeting unit-linker-payload conjugate of any one of claims 3 to 6, wherein the antibody is a cysteine engineered antibody.

8. The targeting unit-linker-payload conjugate of claim 7, wherein the antibody has one or more amino acid deletions or substitutions of heavy chain selected from the group consisting of A40, P41, A84, V89, S112, S113, A114, S115, T116, G118, V152, S153, N155, A168, Q171, C220, 225, 226, 229, 247, V278, N297, 339, S371, 375, 376, S396, and E400 (according to Kabat).

9. The targeting unit-linker-payload conjugate of claim 7, wherein the antibody has one or more amino acid deletions or substitutions of light chain selected from the group consisting of V110, S114, S121, S127, 143, 147, A153, 159, 163, 165, S168, V205, and 214 (according to Kabat).

10. The targeting unit-linker-payload conjugate of any one of claims 7 to 9, wherein the asparagine at position 297 (N297) on the IgG heavy chain is deleted or substituted, the cysteine at position 220 (C220), C226 or C229 on the IgG heavy chain is deleted or substituted, or the cysteine at position 214 (C214) of the IgG light chain (kappa or lambda) is deleted or substituted.

11. The targeting unit-linker-payload conjugate of any preceding claim, wherein the conjugate is

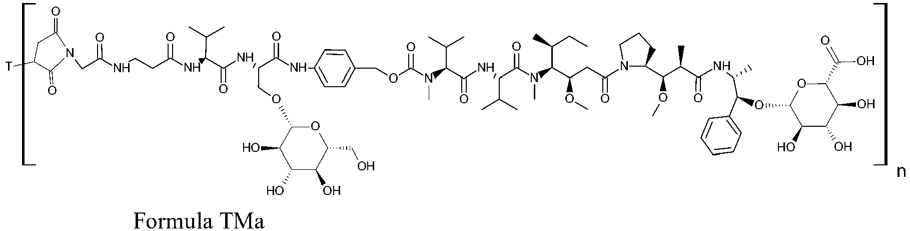

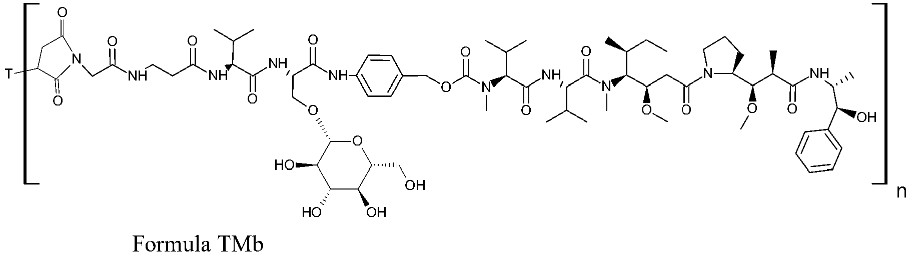

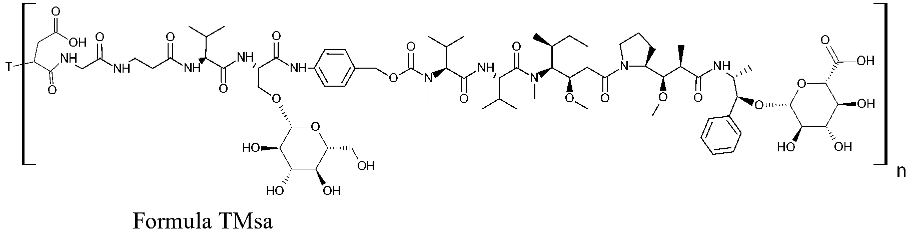

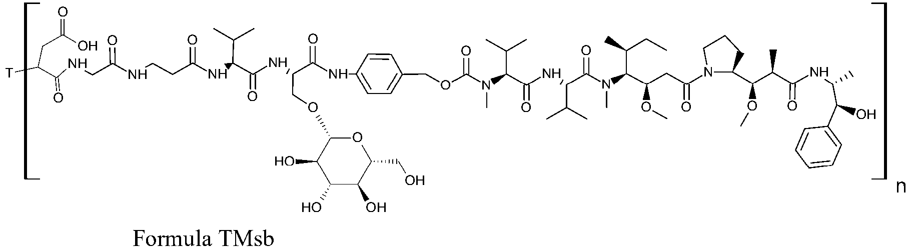

12. A pharmaceutical composition comprising the targeting unit-linker-payload conjugate of any preceding claim.

13. The pharmaceutical composition according to claim 12, wherein the pharmaceutical composition has a drug-to-antibody ratio of ≥ 1, or in the range of 1 to about 20, or 1 to about 15, or 1 to about 10, or 2 to 10, or 2 to 6, or 2 to 5, or 2 to 4; or about 1, about 2, about 3, about 4, about 5, about 6, about 7, about 8, about 9, about 10, about 11, about 12, about 13, about 14, about 15, about 16, about 17, about 18, about 19, or about 20; or about 1 to about 8, or about 6 to about 8.

14. The targeting unit-linker-payload conjugate according to any one of claims 1-11 or the pharmaceutical composition according to any one of claims 12-13 for use as a medicament or for use in the treatment of cancer.

15. The targeting unit-linker-payload conjugate or pharmaceutical composition for use according to claim 14, wherein the cancer is selected from the group consisting of leukemia, lymphoma, breast cancer, prostate cancer, ovarian cancer, colorectal cancer, gastric cancer, squamous cancer, small-cell lung cancer, head-and-neck cancer, multidrug resistant cancer, glioma, melanoma and testicular cancer.
